(19) Europäisches Patentamt
European Patent Office
Office européen des brevets

(11) EP 4 308 551 B1

(12) **EUROPEAN PATENT SPECIFICATION**

(45) Date of publication and mention of the grant of the patent:
**09.04.2025 Bulletin 2025/15**

(21) Application number: **22714807.9**

(22) Date of filing: **14.03.2022**

(51) International Patent Classification (IPC):
***C07D 257/02*** (2006.01) ***C07F 5/00*** (2006.01)
***A61K 49/10*** (2006.01)

(52) Cooperative Patent Classification (CPC):
**C07D 257/02; A61K 49/085; A61K 49/106; A61K 49/12; C07F 5/003**

(86) International application number:
**PCT/EP2022/056541**

(87) International publication number:
**WO 2022/194777 (22.09.2022 Gazette 2022/38)**

(54) **NEW CONTRAST AGENT FOR USE IN MAGNETIC RESONANCE IMAGING**

NEUES KONTRASTMITTEL ZUR VERWENDUNG IN DER MAGNETRESONANZBILDGEBUNG

NOUVEL AGENT DE CONTRASTE POUR UNE UTILISATION DANS L’IMAGERIE PAR RÉSONANCE MAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA TN**

(30) Priority: **15.03.2021 EP 21162650**

(43) Date of publication of application:
**24.01.2024 Bulletin 2024/04**

(73) Proprietor: **Bayer Aktiengesellschaft**
**51373 Leverkusen (DE)**

(72) Inventors:
- **BRUMBY, Thomas**
  **12163 Berlin (DE)**
- **LOHRKE, Jessica**
  **12351 Berlin (DE)**
- **HERBERT, Simon Anthony**
  **13467 Berlin (DE)**
- **PANKNIN, Olaf**
  **438 53 Hindås (SE)**
- **FRENZEL, Thomas**
  **85614 Kirchseeon (DE)**
- **GREEN, Claudia**
  **50996 Köln (DE)**
- **JOST, Gregor**
  **10318 Berlin (DE)**
- **PIETSCH, Hubertus**
  **14532 Kleinmachnow (DE)**
- **BERGER, Markus**
  **12167 Berlin (DE)**
- **WITTROCK, Sven**
  **10117 Berlin (DE)**

(74) Representative: **BIP Patents**
**c/o Bayer Intellectual Property GmbH**
**Alfred-Nobel-Straße 50**
**40789 Monheim am Rhein (DE)**

(56) References cited:
**WO-A1-03/009874 WO-A1-2020/220023**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 308 551 B1

## Description

## FIELD OF THE INVENTION

The present invention relates to the items characterized in the patent claims, *i.e*. to new gadolinium chelate compounds, to methods of preparing said compounds, to the use of said compounds as MRI contrast agents and to their use in a mammalian body.

## BACKGROUND

### 1. Introduction

The Magnetic Resonance Imaging (MRI) technique is non-invasive and can provide information on the anatomy, function and metabolism of tissues in vivo. Unenhanced MRI scans of tissue anatomy and function make use of the hydrogen atoms in water to generate the image. Apart from differences in the local water content, the basic contrast in the MR image mainly results from regional differences in the intrinsic relaxation times T(1) and T(2), each of which can be chosen to dominate image contrast. However, the intrinsic contrast provided by the water T(1) and T(2) and changes in their values brought about by tissue pathology are often too limited to enable a sensitive and specific diagnosis. To overcome these limits the proton relaxation times can be influenced by the presence of paramagnetic ions. Commercial Gadolinium-based Contrast Agents (GBCAs) contain at least one paramagnetic ion of the rare earth metal Gadolinium ($Gd^{3+}$), which possesses the highest number of unpaired electrons of any stable ion (seven), creating a high magnetic moment that is effective at enhancing proton relaxation.

Paramagnetic contrast media shorten the T1 (longitudinal) and T2 (transversal) relaxation times of surrounding water protons to indirectly produce a signal-enhancing effect. The efficacy of an agent to shorten relaxation times is called relaxivity (r1 and r2), which is dependent on the ligand surrounding the $Gd^{3+}$ ion and influenced by extrinsic factors including temperature, magnetic field strength and the matrix (water, solid tissue, or blood) (Lauffer RB et al., Paramagnetic metal complexes as water proton relaxation agents for NMR imaging: theory and design. Chem Rev. 1987;87(5):901-27; Caravan P et al., Gadolinium(III) chelates as MRI contrast agents: structure, dynamics, and applications. Chem Rev. 1999;99(9):2293-352).

Besides non-targeted agents, two linear contrast agents (i.e., contrast agents where ethe Gadolinium ion is bound to a linear ligand structure) are available for Magnetic Resonance Imaging (MRI) of the liver: Gd-BOPTA (gadobenic acid, marketed as Multihance) and Gd-EOB-DTPA (gadoxetic acid, marketed as Primovist in Europe and as Eovist in the USA). Gd-EOB-DTPA (Bayer AG) and Gd-BOPTA (Bracco) have been approved for detection and differentiation of focal liver lesions at clinical doses of 0.025 mmol/kg body weight and 0.05 mmol/kg body weight, respectively. For both agents the T1 effect dominates and yields bright contrast in the image. Both targeted agents have been used for MRI of the liver, for the detection of focal liver lesions in patients with known or suspected primary liver cancer (e.g. hepatocellular carcinoma HCC) or metastatic disease. They provide information regarding lesion vascularity in the arterial and venous phases, hepatocyte presence and function in the delayed hepatobiliary phase. The liver specific contrast agents are taken up by healthy liver cells (hepatocytes) while there is no uptake into malignant tumor tissue due to the lack of intact organic anion-transporting polypeptide (OATP) transporters. Therewith they improve the detection of focal liver lesions by increasing the lesion-to-liver contrast. The contrastenhanced MRI (CE-MRI) provides important information for differential diagnosis. HCCs do not express the respective uptake transporters and thus do not accumulate the liver specific GBCA to an extent which is observed in healthy liver tissue.

Gd-BOPTA is secreted 3-to-5% into the bile and enables the capture of images in the liver-specific phase 1 to 2 hours after its administration (Seale MK et al. Radiographics 2009; 29: 1725-1748).

In the early 2000's, research for new contrast agents with improved tropism for liver cells led to the development of Gd-EOB-DTPA. Gd-EOB-DTPA is excreted into the bile up to about 50% of the administered dose. Gd-EOB-DTPA can be administered as a bolus, guarantees a satisfactory assessment of the vascular interstitial phase and subsequently (after 10-20 minutes), an evaluation of the hepatobiliary phase. Literature studies have confirmed that liver MRI with Gd-EOB-DTPA is able to detect and identify focal liver lesions with high specificity and sensitivity, either in patients with a healthy liver or oncologic/cirrhotic liver patients (Fidler J et al. Hepatology 2011; 53 (2): 678-82; Park Y et al. Korean J Radiol 2010; 11(4): 433-40; Bluemke DA et al. Radiology 2005; 237: 89-98).

Gd-EOB-DTPA is mainly taken up by hepatocytes via organic anion-transporting polypeptides (OATP1B1, OATP1B3) and is subsequently primarily excreted into the bile canaliculi via the multidrug resistance-associated protein 2 (MRP2, synonym cMOAT: canalicular multispecific organic anion transporter) (Ringe KI et al. American Journal of Roentgenology. 2010;195: 13-28; Leonhardt M et al. Drug Metab Dispos. 2010 Jul;38(7):1024-8).

The molecular structure of both marketed products (Gd-EOB-DTPA and Gd-BOPTA) includes a hydrophilic and lipophilic group. The linear DTPA-like ligand for the Gadolinium complexation comprises the hydrophilic group and the

benzene (e.g. ethyl-oxy-benzyl, EOB) side chain is the lipophilic group. The lipophilic group is not only responsible for the marked biliary excretion but also results in some weak protein binding of approximately 10% (Weinmann HJ et al. Magn Reson Med 1991; 22:233-237). Compared to commercially available extracellular GBCAs, Gd-EOB-DTPA and Gd-BOPTA show higher r1 and r2 relaxivities (Rohrer M et al. Invest Radiol. 2005 Nov;40(11):715-24). The high relaxivity values depend on the affinity of the molecules with plasma proteins through the lipophilic group, which is also responsible for specific hepatocyte uptake.

Some recent reports have shown an increased signal intensity (SI) in the dentate nucleus (DN) and globus pallidus (GP) brain areas on unenhanced T1-weighted (T1w) MR images in patients with normal renal function who received multiple linear gadolinium-based contrast agent (GBCA) administrations. A visible SI increase is highly associated with linear GBCAs only. So far, this does not correlate with any clinical symptoms. However, in Europe, the European Medicines Agency (EMA) proposed to withdraw GBCAs with a linear structure from the market, except for both liver-specific market products Gd-BOPTA - if exclusively used in the liver - and Gd-EOB-DTPA. In March 2016, the Pharmacovigilance Risk Assessment Committee (PRAC) of EMA started a procedure to review linear GBCAs and recommended, in March 2017, a suspension of the marketing authorization for all multipurpose linear GBCAs in the European Union (EU) while supporting the continued use of macrocyclic GBCAs (i.e., contrast agents where ethe Gadolinium ion is bound to a macrocyclic ligand structure). Following this decision, the GBCA market has broadly moved away from linear Gadolinium-based agents. As of early 2022, liver specific GBCAs (Gd-EOB-DTPA and Gd-BOPTA) are still accepted, because no alternative liver specific macrocyclic GBCA is commercially available. The observed increased signal intensity in the dentate nucleus was also seen after repeated administration of the linear Gd-EOB-DTPA (Kahn J et al. Radiology. 2017;282(3):708 - 716). It is known that macrocyclic GBCAs are more stable against Gd release (Frenzel et al. Invest Radiol. 2008 Dec;43(12):817-28.) Thus, there is an increased medical need for new macrocyclic liver-specific contrast agents.

## 2. Description of the Prior Art, Problem to be solved and its Solution

WO199532741 describes bile acid conjugates claimed to be useful for imaging of liver and bile duct.

WO2001082795 describes an MRI agent with a covalently bound therapeutic blocking moiety attached which elicits a change in signal intensity of said agent when therapeutic agent interacts with its intended target.

WO2007009638 discloses metal complexes containing perfluoroalkyl groups which can be used as MRI and X-ray contrast agents in particular for lymphography.

WO2004006965 discloses perfluoroalkyl containing MRI contrast agents, which exhibit micelle formation leading to high $r_1$ relaxivity, for representing intravascular thrombi. WO1997032862 describes a class of polychelates linked to alkene bridged amino groups for diagnostic imaging.

WO1999005145 details a process for the preparation of tetraazamacrocycles. WO1996016677 describes metal complexes for use as X-ray contrast media for imaging of liver and bile ducts.

WO1995028392 reveals the use of amphiphilic chelates and their use for hepatobiliary imaging.

WO2013083535 describes the preparation of hyperpolarized imaging agents for MR diagnostic analysis.

The medical need for CE-MRI of the liver is high and since no macrocyclic GBCA is available linear GBCAs are still being used e.g. for differential diagnosis of focal liver lesions or the detection of small liver lesions. However, currently no marketed product containing macrocyclic GBCA with liver uptake is available which displays the favorable attributes of macrocyclic GBCAs with the essential attributes of a liver imaging agent. Among the different properties desirable for compounds suitable as MRI contrast agents are e.g. high-water solubility, high relaxivity, complete and intact excretion, good tolerability and a good safety profile.

EP405704 relates to $Gd^{3+}$ complexes with derivatives of diethylentriaminopentaacetic acid (DTPA) such as Gd-EOB-DTPA and their use as contrast agents in magnetic resonance imaging of the liver, among others. However, as described above, these linear compounds and complexes have come under increased scrutiny from the health authorities in recent years and are only still being used in a clinical environment due to the lack of suitable alternatives.

Thus, there is an unmet medical need to provide macrocyclic GBCAs for magnetic resonance imaging, in particular for magnetic resonance imaging of the liver, which combine the beneficial properties of linear liver specific agents and macrocyclic GBCAs. Specifically, there is an unmet medical need to provide liver specific GBCAs which show as many of the below-listed criteria as possible:

- exhibit high water solubility,
- are chemically stable,
- are stable against metal release from the chelate,

- exhibit high relaxivity,
- exhibit high in vitro uptake into human transfected OATP1 B1 HEK cells,
- exhibit high in vitro uptake into human transfected OATP1 B3 HEK cells,
- have low protein binding,
- show a favorable pharmacokinetic profile and dual elimination pathway,
- are fast and completely excreted,
- exhibit no long-term retention of $Gd^{3+}$ both in tissues and in organs,
- are stable against metabolic degradation,
- are well tolerated,
- are suitable for liver imaging,
- are suitable for biliary imaging,
- are suitable for the imaging of liver diseases
- and may exhibit high in vitro uptake (e.g. into rat hepatocytes).

The state of the art described above does not disclose the compounds of general formula (I) of the present invention as defined herein, or a stereoisomer, a tautomer, an N-oxide, a hydrate, a solvate, or a salt thereof, or a mixture of the same, as described and defined herein. Nor does the art disclose the compounds of general formula (I) of the present invention in the form of a complex with $Gd^{3+}$, and stereoisomers, tautomers, N-oxides, hydrates, solvates, or salts thereof, or mixtures of same. Together or separately, the compounds of general formula (I) of the present invention and the compounds of general formula (I) of the present invention in the form of a complex with $Gd^{3+}$, and stereoisomers, tautomers, N-oxides, hydrates, solvates, or salts thereof, or mixtures of same are hereinafter referred to as "compounds of the present invention". The compounds of general formula (I) of the present invention in the form of a complex with $Gd^{3+}$, and stereoisomers, tautomers, N-oxides, hydrates, solvates, or salts thereof, or mixtures of same may also be referred to as "$Gd^{3+}$-containing compounds of the present invention".

It has been found, and this constitutes the basis of the present invention, that the compounds of the present invention have surprising and advantageous properties.

In particular, the compounds of general formula (I) of the present invention allow for the preparation of complexes with $Gd^{3+}$, *i.e.* compounds of general formula (I) of the present invention in the form of a complex with $Gd^{3+}$ and stereoisomers, tautomers, N-oxides, hydrates, solvates, or salts thereof, or mixtures of same. The $Gd^{3+}$-containing compounds of the present invention display the favorable stability of macrocyclic GBCAs and a high uptake in the liver. Further, the $Gd^{3+}$-containing compounds of the present invention show high tolerability, improved relaxivity, excellent water solubility and a fast and complete excretion, making them well suited for magnetic resonance imaging, in particular for liver magnetic resonance imaging.

## SUMMARY

The present invention describes a new class of liver-specific (non-linear) gadolinium chelate complexes, methods for their preparation and their use as MRI contrast agents.

## DESCRIPTION OF THE INVENTION

In accordance with a first aspect, the present invention covers compounds of general formula (I),

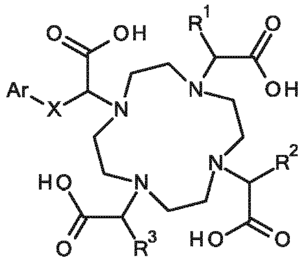

(I) ,

in which:

Ar represents a group selected from

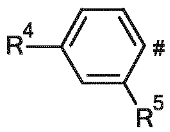 , 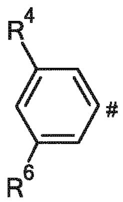 , 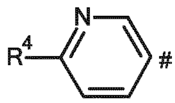 and 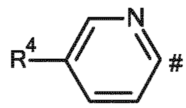 ,

wherein # indicates the point of attachment to X,

X represents a group selected from $CH_2$, $(CH_2)_2$, $(CH_2)_3$, $(CH_2)_4$ and $*-(CH_2)_2-O-CH_2-\#$, wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, and

$R^6$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

and stereoisomers, tautomers, N-oxides, hydrates, solvates, or salts thereof, or mixtures of same.

In accordance with a second aspect, the present invention covers compounds of general formula (I), *supra,* in the form of a complex with $Gd^{3+}$, and stereoisomers, tautomers, N-oxides, hydrates, solvates, or salts thereof, or mixtures of same.

Thus, in accordance said second aspect, the present invention covers compounds of general formula (I), *supra,* in the form of a complex with $Gd^{3+}$,
wherein:

Ar represents a group selected from

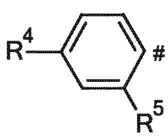 , 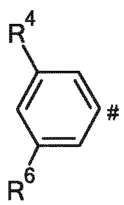 , 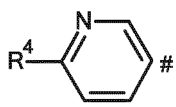 and 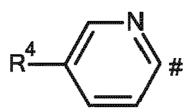 ,

wherein # indicates the point of attachment to X,

| | |
|---|---|
| X | represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety, |
| $R^1$, $R^2$ and $R^3$ | represent, independently for each occurrence, a hydrogen atom or a group selected from $C_1$-$C_3$-alkyl, -$CH_2OH$, -$(CH_2)_2OH$ and -$CH_2OCH_3$, |
| $R^4$ | represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom, |
| $R^5$ | represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, and |
| $R^6$ | represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, |

and stereoisomers, tautomers, N-oxides, hydrates, solvates, or salts thereof, or mixtures of same.

**DEFINITIONS**

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

Should a composite substituent be composed of more than one parts, e.g. ($C_1$-$C_3$-alkoxy)-($C_2$-$C_6$-alkyl)-, it is possible for the position of a given part to be at any suitable position of said composite substituent, *i.e.* the $C_1$-$C_3$-alkoxy part can be attached to any carbon atom of the $C_2$-$C_6$-alkyl part of said ($C_1$-$C_3$-alkoxy)-($C_2$-$C_6$-alkyl)- group. A hyphen at the beginning or at the end of such a composite substituent indicates the point of attachment of said composite substituent to the rest of the molecule.

The term "comprising" when used in the specification includes "consisting of" and "consisting essentially of".

If within the present text any item is referred to as "as mentioned herein", it means that it may be mentioned anywhere in the present text.

The terms as mentioned in the present text have the following meanings:
The term "halogen atom" means a fluorine, chlorine, bromine or iodine atom, particularly a fluorine, chlorine or bromine atom.

The term "$C_1$-$C_6$-alkyl" means a linear or branched, saturated, monovalent hydrocarbon group having 1, 2, 3, 4, 5 or 6 carbon atoms, *e.g*. a methyl, ethyl, propyl, isopropyl, butyl, *sec*-butyl, isobutyl, *tert*-butyl, pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer or stereoisomer thereof. Particularly, said group has 1, 2, 3 or 4 carbon atoms ("$C_1$-$C_4$-alkyl"), *e.g.* a methyl, ethyl, propyl, isopropyl, butyl, sec-butyl isobutyl, or *tert*-butyl group, more particularly 1, 2 or 3 carbon atoms ("$C_1$-$C_3$-alkyl"), *e.g.* a methyl, ethyl, *n*-propyl or isopropyl group.

The term "$C_1$-$C_3$-haloalkyl" means a linear or branched, saturated, monovalent hydrocarbon group in which the term "$C_1$-$C_3$-alkyl" is as defined *supra,* and in which one or more of the hydrogen atoms are replaced, identically or differently, with a halogen atom. Particularly, said halogen atom is a fluorine atom. Said $C_1$-$C_3$-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl or other polyfluorosubstituted alkyl group.

The term "$C_2$-$C_6$-hydroxyalkyl" means a linear or branched, saturated, monovalent hydrocarbon group in which the term "$C_2$-$C_6$-alkyl" is defined *supra,* and in which one or more, preferably 1, 2 or 3 of the hydrogen atoms are replaced with a hydroxy group, *e.g*. a 2-hydroxyethyl, 3-hydroxypropyl, 2-hydroxypropyl, 1-hydroxypropan-2-yl, 2,3-dihydroxypropyl, 1,3-dihydroxypropan-2-yl, 1,3-dihydroxy-2-(hydroxymethyl)propan-2-yl, 3-hydroxy-2-methyl-propyl, 2-hydroxy-2-methyl-propyl group.

The term "$C_1$-$C_3$-alkoxy" means a linear or branched, saturated, monovalent group of formula ($C_1$-$C_3$-alkyl)-O-,

in which the term "$C_1$-$C_3$-alkyl" is as defined *supra, e.g.* a methoxy, ethoxy, *n*-propoxy or isopropoxy group.

The term "$C_3$-$C_6$-cycloalkyl" means a saturated, monovalent, mono- or bicyclic hydrocarbon ring which contains 3, 4, 5 or 6 carbon atoms ("$C_3$-$C_6$-cycloalkyl"). Said $C_3$-$C_6$-cycloalkyl group is for example, a monocyclic hydrocarbon ring, *e.g*. a cyclopropyl, cyclobutyl, cyclopentyl or cyclohexyl group.

The term "$C_1$-$C_6$", as used in the present text, *e.g*. in the context of the definition of "$C_1$-$C_6$-alkyl" means an alkyl group having a finite number of carbon atoms of 1 to 6, *i.e.* 1, 2, 3, 4, 5 or 6 carbon atoms.

Further, as used herein, the term "$C_3$-$C_6$", as used in the present text, *e.g*. in the context of the definition of "$C_3$-$C_6$-cycloalkyl", means a cycloalkyl group having a finite number of carbon atoms of 3 to 6, *i.e.* 3, 4, 5 or 6 carbon atoms.

When a range of values is given, said range encompasses each value and sub-range within said range.

For example:

"$C_1$-$C_6$" encompasses $C_1$, $C_2$, $C_3$, $C_4$, $C_5$, $C_6$, $C_1$-$C_6$, $C_1$-$C_5$, $C_1$-$C_4$, $C_1$-$C_3$, $C_1$-$C_2$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$;

"$C_1$-$C_4$" encompasses $C_1$, $C_2$, $C_3$, $C_4$, $C_1$-$C_4$, $C_1$-$C_3$, $C_1$-$C_2$, $C_2$-$C_4$, $C_2$-$C_3$ and $C_3$-$C_4$;

"$C_1$-$C_3$" encompasses $C_1$, $C_2$, $C_3$, $C_1$-$C_3$, $C_1$-$C_2$ and $C_2$-$C_3$;

"$C_2$-$C_6$" encompasses $C_2$, $C_3$, $C_4$, $C_5$, $C_6$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_5$, and $C_5$-$C_6$; and

"$C_3$-$C_6$" encompasses $C_3$, $C_4$, $C_5$, $C_6$, $C_3$-$C_6$, $C_3$-$C_5$, $C_3$-$C_4$, $C_4$-$C_6$, $C_4$-$C_5$, and $C_5$-$C_6$.

The compounds of the present invention may contain one or more asymmetric centers, depending upon the location and nature of the various substituents desired. Asymmetric carbon atoms may be present in the (R) or (S) configuration, which can result in racemic mixtures, mixtures in which one enantiomer is present in a greater amount than the other enantiomer, or single enantiomers in the case of a single asymmetric center. In the case of multiple stereogenic centers, diastereomeric mixtures, single diastereomers or single enantiomers can be synthesized. In certain instances, asymmetry may also be present due to restricted rotation about a given bond, axial chirality or coordination of the metal center.

In the context of the present invention, the compounds of formula (I), the compounds of formula (I) in the form of a $Gd^{3+}$ complex, the compounds of formula (II), the compounds of formula (III) as well as any other compounds and/or intermediates herein described may include a group X. Said group X may, *inter alia,* represent a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety. One skilled in the art would recognize that the terms $CH_2$, $(CH_2)_2$, $(CH_2)_3$ and $(CH_2)_4$ refer to linear alkyl groups, *i.e.* $*CH_2-^{\#}$, $*-(CH_2)_2-^{\#}$, $*-(CH_2)_3-^{\#}$ and $*-(CH_2)_4-^{\#}$ groups, wherein * indicates the point of attachment to Ar and # indicates the point of attachment to the acetic acid moiety.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g*., chiral HPLC columns), with or without conventional derivatisation, optimally chosen to maximise the separation of the enantiomers. Suitable chiral HPLC columns are manufactured by Daicel, *e.g*., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials and/or reagents and catalysts.

In order to describe different types of isomers reference is made to IUPAC Rules Section E (Pure Appl Chem 45, 11-30, 1976).

The present invention includes all possible stereoisomers of the compounds of the present invention as single stereoisomers, or as any mixture of said stereoisomers, *e.g*. R- or S-isomers, or diastereoisomers, in any ratio. Isolation of a single stereoisomer, *e.g*. a single enantiomer or a single diastereomer, of a compound of the present invention may be achieved by any suitable state of the art method as described herein, such as chromatography, especially chiral

chromatography, for example.

Further, the compounds of the present invention can exist as N-oxides, which are defined in that at least one nitrogen of the compounds of the present invention is oxidized. The present invention includes all such possible N-oxides.

The present invention also relates to useful forms of the compounds as disclosed herein, such as hydrates, solvates, salts, in particular pharmaceutically acceptable salts, and coprecipitates.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or nonstoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in the form of a salt. Said salt may be either an inorganic or organic addition salt, particularly any pharmaceutically acceptable inorganic or organic addition salt, customarily used in pharmaceutical formulations.

Further, in the context of the present invention, the compounds of general formula (I) in the form of a complex with $Gd^{3+}$, *i.e.* $Gd^{3+}$ complexes of the compounds of general formula (I), as described throughout this document, may exist as salts in the form according to formula (Ia)

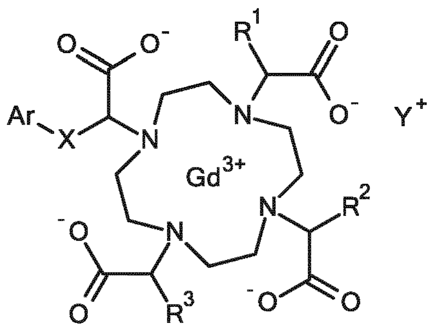

(**Ia**),

wherein:

Ar, X and $R^1$ to $R^6$ represent the groups indicated in the different aspects, embodiments, examples and any other descriptions and/or depictions of the compounds of general formula (I) in the form of a complex with $Gd^{3+}$, *i.e.* $Gd^{3+}$ complexes of the compounds of general formula (I), as described throughout this document, and $Y^+$ represents a hydrogen atom or a positively charged organic or inorganic counterion. In particularly preferred embodiments, the compounds of general formula (I) in the form of a complex with $Gd^{3+}$, *i.e.* $Gd^{3+}$ complexes of the compounds of general formula (I), as described throughout this document, may exist as salt of an alkali metal (group 1 element), such as sodium salts, *i.e.* as salts in the form according to formula (Ia), supra, wherein $Y^+$ represents a cation of an alkali metal (group 1 element), *e.g.* a positively charged sodium cation of an alkali metal (group 1 element). In more particularly preferred embodiments, the compounds of general formula (I) in the form of a complex with $Gd^{3+}$, *i.e.* $Gd^{3+}$ complexes of the compounds of general formula (I), as described throughout this document, may exist as sodium salts, *i.e.* as salts in the form according to formula (Ia), supra, wherein $Y^+$ represents a sodium cation, *i.e.* a positively charged sodium ion.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. The production of especially neutral salts is described in US Patent No. 5,560,903.

Pharmaceutically acceptable salts of the compounds according to the invention include salts with inorganic and/or organic bases or amino acids, in particular physiologically tolerable cations of inorganic and/or organic bases or amino acids, such as, inter alia, those of primary, secondary or tertiary amines. Examples may be, without being limited thereto, salts of sodium, lithium, potassium, calcium, magnesium, arginine, lysine, ammonia, creatinine, diethanolamine, ethanol amine, morpholine, glucamine, N,N-dimethylglucamine, N-methylglucamine, ornithine, histidine, imidazole, tromethamine, meglumine and the like.

Particularly preferred pharmaceutically acceptable salts of the compounds according to the invention are their corresponding sodium salts.

Those skilled in the art will further recognize that salts of the claimed compounds may be prepared by reaction of the compounds with the appropriate inorganic or organic base via any of a number of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

This applies analogously to cases in which synthesis intermediates or example compounds or salts thereof have been obtained, by the preparation and/or purification processes described, as solvates, such as hydrates with (if defined) unknown stoichiometric composition.

In accordance with a further embodiment of the first aspect, the present invention covers compounds of general formula (I), *supra,* wherein:

Ar represents a group selected from

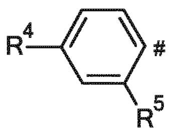 , 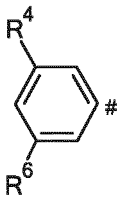 , 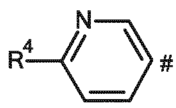 and 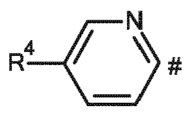 ,

wherein # indicates the point of attachment to X,

X represents a group selected from $CH_2$, $(CH_2)_2$, $(CH_2)_3$, $(CH_2)_4$ and $*-(CH_2)_2-O-CH_2-^{\#}$, wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom,

and

$R^6$ represents a hydrogen atom,

and stereoisomers, tautomers, N-oxides, hydrates, solvates, or salts thereof, or mixtures of same.

In accordance with a further embodiment of the first aspect, the present invention covers compounds of general formula (I), *supra,* wherein:

Ar represents a group selected from

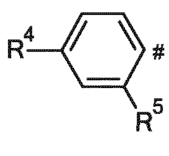 and 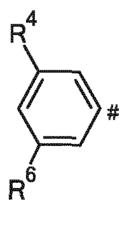 ,

wherein # indicates the point of attachment to X,

X represents a group selected from $CH_2$, $(CH_2)_2$, $(CH_2)_3$, $(CH_2)_4$ and $*-(CH_2)_2-O-CH_2\ ^{\#}$, wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom,
and
$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

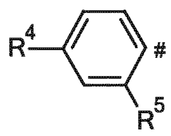 and 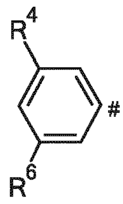 ,

wherein # indicates the point of attachment to X,
X represents a group selected from $CH_2$ and $(CH_2)_3$,
$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,
$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,
$R^5$ represents a hydrogen atom,
and
$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

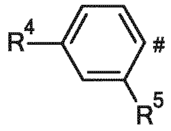 and 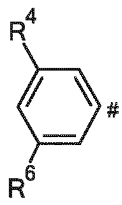 ,

wherein # indicates the point of attachment to X,
X represents a group selected from $CH_2$ and $(CH_2)_3$,
$R^1$ and $R^3$ represent, independently for each occurrence, a hydrogen atom or a $-CH_2OH$ group,
$R^2$ represents a hydrogen atom or a group selected from $C_1$-$C_3$-alkyl, $-CH_2OH$, $-(CH_2)_2OH$ and $-CH_2OCH_3$,
$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,
$R^5$ represents a hydrogen atom,
and
$R^6$ represents a hydrogen atom,

and stereoisomers, tautomers, hydrates, solvates, or salts thereof, or mixtures of same.

In accordance with a further embodiment of the first aspect, the present invention covers compounds of general

formula (I), *supra,* wherein:

Ar represents a group selected from

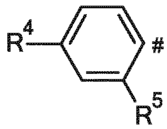 and 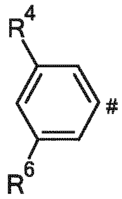 ,

wherein # indicates the point of attachment to X,

X represents a group selected from $CH_2$ and $(CH_2)_3$,

$R^1$, $R^2$ and $R^3$ represent, independently for each occurrence, a hydrogen atom or a $-CH_2OH$ group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom, and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

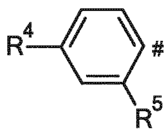 and 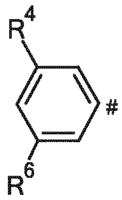 ,

wherein # indicates the point of attachment to X,

X represents a group selected from $CH_2$ and $(CH_2)_3$,

$R^1$, $R^2$ and $R^3$ represent a hydrogen atom or a $-CH_2OH$ group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom, and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

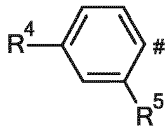 and 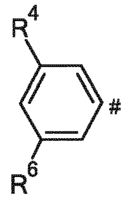 ,

wherein # indicates the point of attachment to X,

X represents a group selected from $CH_2$ and $(CH_2)_3$,

$R^1$ and $R^3$ represent a hydrogen atom,

$R^2$ represents a $-(CH_2)_2OH$ group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

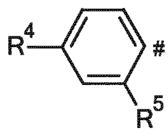 and 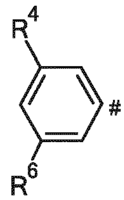 ,

wherein # indicates the point of attachment to X,

X represents a group selected from $CH_2$ and $(CH_2)_3$,

$R^1$ and $R^3$ represent a hydrogen atom,

$R^2$ represents a $-CH_2OCH_3$ group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

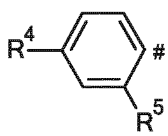 and 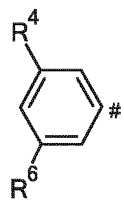 ,

wherein # indicates the point of attachment to X,

X represents a group selected from $CH_2$ and $(CH_2)_3$,

$R^1$ and $R^3$ represent a hydrogen atom,

$R^2$ represents a group selected from $C_1$-$C_3$-alkyl,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

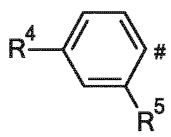 and 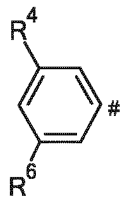 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#,
wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, -$CH_2OH$, -$(CH_2)_2OH$ and -$CH_2OCH_3$,

$R^4$ represents a group selected from $C_2$-$C_4$-alkoxy, ($H_3C$-$CH_2$)-O-$(CH_2)_2$-O-, ($H_3C$-$CH_2$)-O-$(CH_2)_2$-O-$(CH_2)_2$-O- and ($H_3C$-$CH_2$)-O-$(CH_2)_2$-O-$(CH_2)_2$-O-$(CH_2)_2$-O-,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

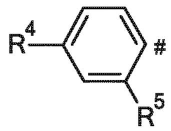 and 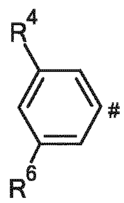 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#,
wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, -$CH_2OH$, -$(CH_2)_2OH$ and -$CH_2OCH_3$,

R4 represents a group selected from $(H_3C\text{-}CH_2)\text{-}O\text{-}(CH_2)_2\text{-}O\text{-}$, $(H_3C\text{-}CH_2)\text{-}O\text{-}(CH_2)_2\text{-}O\text{-}(CH_2)_2\text{-}O\text{-}$ and $(H_3C\text{-}CH_2)\text{-}O\text{-}(CH_2)_2\text{-}O\text{-}(CH_2)_2\text{-}O\text{-}(CH_2)_2\text{-}O\text{-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

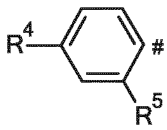

and

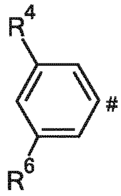

,

wherein # indicates the point of attachment to X,

X represents a group selected from $CH_2$ and $(CH_2)_3$,

$R^1$, $R^2$ and $R^3$ represent, independently for each occurrence, a hydrogen atom or a group selected from $C_1\text{-}C_3$-alkyl, $-CH_2OH$, $-(CH_2)_2OH$ and $-CH_2OCH_3$,

$R^4$ represents a group selected from $(H_3C\text{-}CH_2)\text{-}O\text{-}(CH_2)_2\text{-}O\text{-}$, $(H_3C\text{-}CH_2)\text{-}O\text{-}(CH_2)_2\text{-}O\text{-}(CH_2)_2\text{-}O\text{-}$ and $(H_3C\text{-}CH_2)\text{-}O\text{-}(CH_2)_2\text{-}O\text{-}(CH_2)_2\text{-}O\text{-}(CH_2)_2\text{-}O\text{-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

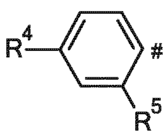

and

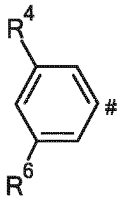

,

wherein # indicates the point of attachment to X,

X represents a group selected from $CH_2$ and $(CH_2)_3$,

$R^1$ and $R^3$ represent, independently for each occurrence, a hydrogen atom or a $-CH_2OH$ group,

$R^2$ represents a hydrogen atom or a group selected from $C_1\text{-}C_3$-alkyl, $-CH_2OH$, - $(CH_2)_2OH$ and $-CH_2OCH_3$,

$R^4$ represents a group selected from $(H_3C\text{-}CH_2)\text{-}O\text{-}(CH_2)_2\text{-}O\text{-}$, $(H_3C\text{-}CH_2)\text{-}O\text{-}(CH_2)_2\text{-}O\text{-}(CH_2)_2\text{-}O\text{-}$ and $(H_3C\text{-}CH_2)\text{-}O\text{-}(CH_2)_2\text{-}O\text{-}(CH_2)_2\text{-}O\text{-}(CH_2)_2\text{-}O\text{-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

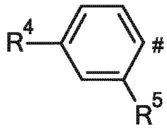 and 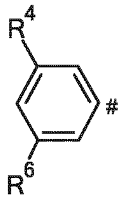 ,

wherein # indicates the point of attachment to X,

X represents a group selected from $CH_2$ and $(CH_2)_3$,

$R^1$, $R^2$ and $R^3$ represent, independently for each occurrence, a hydrogen atom or a $-CH_2OH$ group,

$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

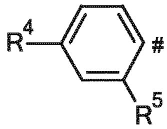 and 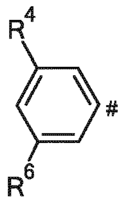 ,

wherein # indicates the point of attachment to X,

X represents a group selected from $CH_2$ and $(CH_2)_3$,

$R^1$, $R^2$ and $R^3$ represent a hydrogen atom or a $-CH_2OH$ group,

$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents

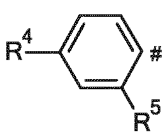 ,

wherein # indicates the point of attachment to X,

X represents a group selected from $CH_2$ and $(CH_2)_3$,

$R^1$, $R^2$ and $R^3$ represent a $-CH_2OH$ group,

$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

$R^5$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

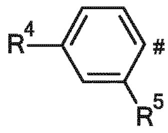 and 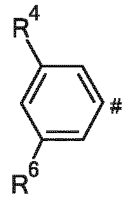 ,

wherein # indicates the point of attachment to X,
X represents a group selected from $CH_2$ and $(CH_2)_3$,
$R^1$ and $R^3$ represent a hydrogen atom,
$R^2$ represents a $-(CH_2)_2OH$ group,
$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom,
and
$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

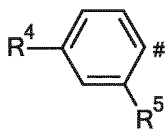 and 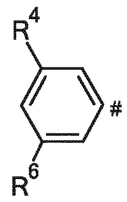 ,

wherein # indicates the point of attachment to X,
X represents a group selected from $CH_2$ and $(CH_2)_3$,
$R^1$ and $R^3$ represent a hydrogen atom,
$R^2$ represents a $-CH_2OCH_3$ group,
$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom,
and
$R^6$ represents a hydrogen atom,

and stereoisomers, tautomers, hydrates, solvates, or salts thereof, or mixtures of same.

In accordance with a further embodiment of the first aspect, the present invention covers compounds of general formula (I), *supra,* wherein:

Ar represents a group selected from

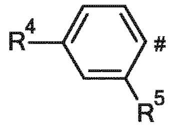 and 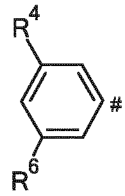 ,

wherein # indicates the point of attachment to X,

X represents a group selected from $CH_2$ and $(CH_2)_3$,

$R^1$ and $R^3$ represent a hydrogen atom,

$R^2$ represents a group selected from $C_1$-$C_3$-alkyl,

$R^4$ represents a group selected from $(H_3C\text{-}CH_2)\text{-}O\text{-}(CH_2)_2\text{-}O\text{-}$, $(H_3C\text{-}CH_2)\text{-}O\text{-}(CH_2)_2\text{-}O\text{-}(CH_2)_2\text{-}O\text{-}$ and $(H_3C\text{-}CH_2)\text{-}O\text{-}(CH_2)_2\text{-}O\text{-}(CH_2)_2\text{-}O\text{-}(CH_2)_2\text{-}O\text{-}$,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

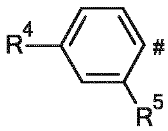 , 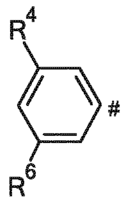 , 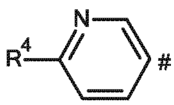 and 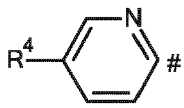 ,

wherein # indicates the point of attachment to X,

X represents a group selected from $CH_2$, $(CH_2)_2$, $(CH_2)_3$, $(CH_2)_4$ and $*\text{-}(CH_2)_2\text{-}O\text{-}CH_2\text{-}^{\#}$, wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

$R^4$ represents a group selected from $C_2$-$C_5$-alkoxy, $(C_1\text{-}C_3\text{-alkoxy})\text{-}(CH_2)_2\text{-}O\text{-}$, $(C_1\text{-}C_3\text{-alkoxy})\text{-}(CH_2)_2\text{-}O\text{-}(CH_2)_2\text{-}O\text{-}$ and $(C_1\text{-}C_3\text{-alkoxy})\text{-}(CH_2)_2\text{-}O\text{-}(CH_2)_2\text{-}O\text{-}(CH_2)_2\text{-}O\text{-}$, wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, N-oxides, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

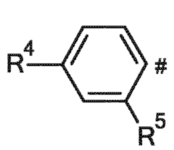 and 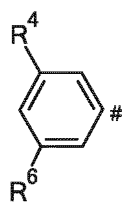 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#, wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, -$CH_2OH$, -$(CH_2)_2OH$ and -$CH_2OCH_3$,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom, and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

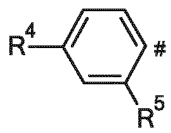 and 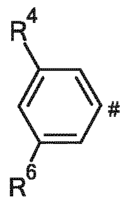 ,

wherein # indicates the point of attachment to X,

X represents a group selected from $CH_2$ and $(CH_2)_3$,

$R^1$, $R^2$ and $R^3$ represent, independently for each occurrence, a hydrogen atom or a group selected from $C_1$-$C_3$-alkyl, -$CH_2OH$, -$(CH_2)_2OH$ and -$CH_2OCH_3$,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom, and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

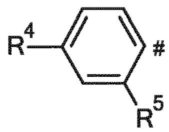 and 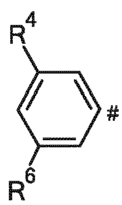 ,

wherein # indicates the point of attachment to X,

X represents a group selected from $CH_2$ and $(CH_2)_3$,

$R^1$ and $R^3$ represent, independently for each occurrence, a hydrogen atom or a -$CH_2OH$ group,

$R^2$ represents a hydrogen atom or a group selected from $C_1$-$C_3$-alkyl, -$CH_2OH$, -$(CH_2)_2OH$ and -$CH_2OCH_3$,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

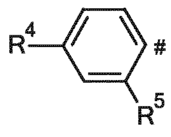 and 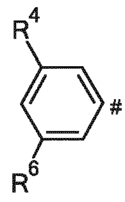 ,

wherein # indicates the point of attachment to X,

X represents a group selected from $CH_2$ and $(CH_2)_3$,

$R^1$, $R^2$ and $R^3$ represent, independently for each occurrence, a hydrogen atom or a -$CH_2$OH group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

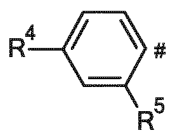 and 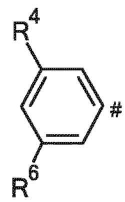 ,

wherein # indicates the point of attachment to X,

X represents a group selected from $CH_2$ and $(CH_2)_3$,

$R^1$, $R^2$ and $R^3$ represent a hydrogen atom or a -$CH_2$OH group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

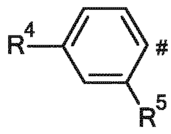
and
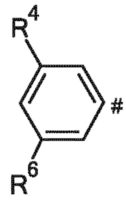
,

wherein # indicates the point of attachment to X,

X represents a group selected from $CH_2$ and $(CH_2)_3$,

$R^1$ and $R^3$ represent a hydrogen atom,

$R^2$ represents a $-(CH_2)_2OH$ group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

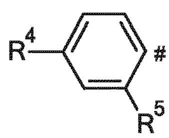
and
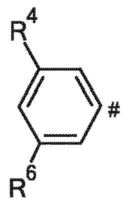
,

wherein # indicates the point of attachment to X,

X represents a group selected from $CH_2$ and $(CH_2)_3$,

$R^1$ and $R^3$ represent a hydrogen atom,

$R^2$ represents a $-CH_2OCH_3$ group,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

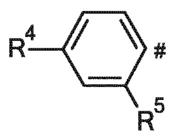 and 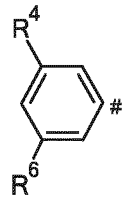 ,

wherein # indicates the point of attachment to X,

X represents a group selected from $CH_2$ and $(CH_2)_3$,

$R^1$ and $R^3$ represent a hydrogen atom,

$R^2$ represents a group selected from $C_1$-$C_3$-alkyl,

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

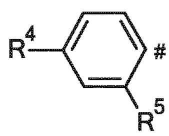 and 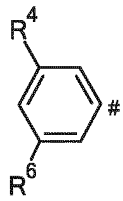 ,

wherein # indicates the point of attachment to X,

X represents a group selected from $CH_2$, $(CH_2)_2$, $(CH_2)_3$, $(CH_2)_4$ and *-$(CH_2)_2$-O-$CH_2$-#,
wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, -$CH_2OH$, -$(CH_2)_2OH$ and -$CH_2OCH_3$,

$R^4$ represents a group selected from $C_2$-$C_4$-alkoxy, ($H_3C$-$CH_2$)-O-$(CH_2)_2$-O-, ($H_3C$-$CH_2$)-O-$(CH_2)_2$-O-$(CH_2)_2$-O- and ($H_3C$-$CH_2$)-O-$(CH_2)_2$-O-$(CH_2)_2$-O-$(CH_2)_2$-O-,

$R^5$ represents a hydrogen atom,
and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

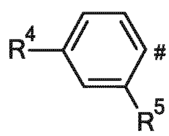 and 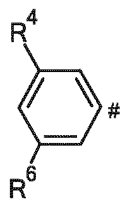 ,

wherein # indicates the point of attachment to X,

X represents a group selected from $CH_2$, $(CH_2)_2$, $(CH_2)_3$, $(CH_2)_4$ and $*\text{-}(CH_2)_2\text{-O-}CH_2\text{-}^{\#}$, wherein * indicates the point of attachment to Ar and # indicates the point of attachment to the acetic acid moiety,

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

$R^4$ represents a group selected from $(H_3C\text{-}CH_2)\text{-O-}(CH_2)_2\text{-O-}$, $(H_3C\text{-}CH_2)\text{-O-}(CH_2)_2\text{-O-}(CH_2)_2\text{-O-}$ and $(H_3C\text{-}CH_2)\text{-O-}(CH_2)_2\text{-O-}(CH_2)_2\text{-O-}(CH_2)_2\text{-O-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

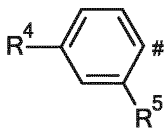

and

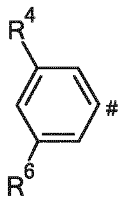

,

wherein # indicates the point of attachment to X,

X represents a group selected from $CH_2$ and $(CH_2)_3$,

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

$R^4$ represents a group selected from $(H_3C\text{-}CH_2)\text{-O-}(CH_2)_2\text{-O-}$, $(H_3C\text{-}CH_2)\text{-O-}(CH_2)_2\text{-O-}(CH_2)_2\text{-O-}$ and $(H_3C\text{-}CH_2)\text{-O-}(CH_2)_2\text{-O-}(CH_2)_2\text{-O-}(CH_2)_2\text{-O-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

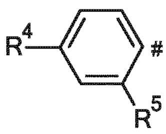

and

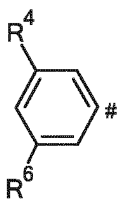

,

wherein # indicates the point of attachment to X,

X represents a group selected from $CH_2$ and $(CH_2)_3$,

$R^1$ and $R^3$ represent, independently for each occurrence, a hydrogen atom or a $-CH_2OH$ group,

$R^2$ represents a hydrogen atom or a group selected from $C_1$-$C_3$-alkyl, $-CH_2OH$, $-(CH_2)_2OH$ and $-CH_2OCH_3$,

$R^4$ represents a group selected from $(H_3C\text{-}CH_2)\text{-O-}(CH_2)_2\text{-O-}$, $(H_3C\text{-}CH_2)\text{-O-}(CH_2)_2\text{-O-}(CH_2)_2\text{-O-}$ and $(H_3C\text{-}CH_2)\text{-O-}(CH_2)_2\text{-O-}(CH_2)_2\text{-O-}(CH_2)_2\text{-O-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

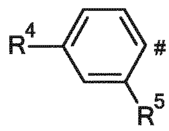 and 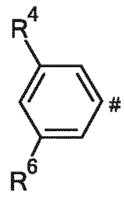 ,

wherein # indicates the point of attachment to X,

X represents a group selected from $CH_2$ and $(CH_2)_3$,

$R^1$, $R^2$ and $R^3$ represent, independently for each occurrence, a hydrogen atom or a $-CH_2OH$ group,

$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

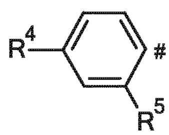 and 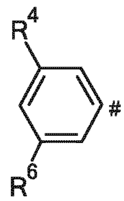 ,

wherein # indicates the point of attachment to X,

X represents a group selected from $CH_2$ and $(CH_2)_3$,

$R^1$, $R^2$ and $R^3$ represent a hydrogen atom or a $-CH_2OH$ group,

$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents

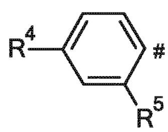 ,

wherein # indicates the point of attachment to X,
X represents a group selected from $CH_2$ and $(CH_2)_3$,
$R^1$, $R^2$ and $R^3$ represent a $-CH_2OH$ group,
$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

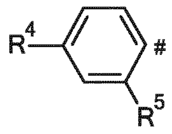
and
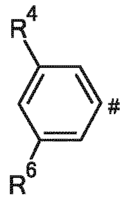
,

wherein # indicates the point of attachment to X,
X represents a group selected from $CH_2$ and $(CH_2)_3$,
$R^1$ and $R^3$ represent a hydrogen atom,
$R^2$ represents a $-(CH_2)_2OH$ group,
$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom,
and
$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

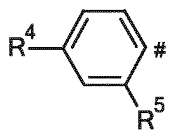
and
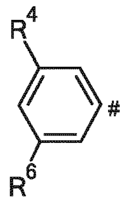
,

wherein # indicates the point of attachment to X,
X represents a group selected from $CH_2$ and $(CH_2)_3$,
$R^1$ and $R^3$ represent a hydrogen atom,
$R^2$ represents a $-CH_2OCH_3$ group,
$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom,
and
$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a complex with $Gd^{3+}$, wherein:

Ar represents a group selected from

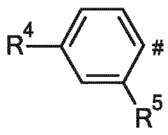 and 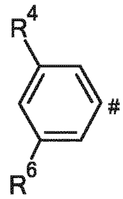 ,

wherein # indicates the point of attachment to X,

X represents a group selected from $CH_2$ and $(CH_2)_3$,

$R^1$ and $R^3$ represent a hydrogen atom,

$R^2$ represents a group selected from $C_1$-$C_3$-alkyl,

$R^4$ represents a group selected from $(H_3C\text{-}CH_2)\text{-}O\text{-}(CH_2)_2\text{-}O\text{-}$, $(H_3C\text{-}CH_2)\text{-}O\text{-}(CH_2)_2\text{-}O\text{-}(CH_2)_2\text{-}O\text{-}$ and $(H_3C\text{-}CH_2)\text{-}O\text{-}(CH_2)_2\text{-}O\text{-}(CH_2)_2\text{-}O\text{-}(CH_2)_2\text{-}O\text{-}$,

$R^5$ represents a hydrogen atom,

and

$R^6$ represents a hydrogen atom,

and stereoisomers, tautomers, hydrates, solvates, or salts thereof, or mixtures of same.

In accordance with a further embodiment of the second aspect, the present invention covers compounds of general formula (I), *supra,* in the form of a sodium ($Na^+$) salt of a complex with $Gd^{3+}$, wherein Ar, X, $R^1$, $R^2$, $R^3$, $R^4$, $R^5$ and $R^6$ are defined as described in any of the embodiments above, and stereoisomers, tautomers, hydrates, or solvates thereof, or mixtures of same

In a further embodiment of the first aspect, the invention relates to compounds of formula (I), wherein:

Ar represents a group selected from

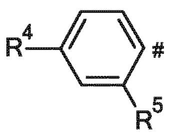 , 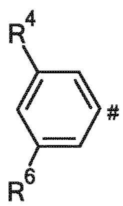 , 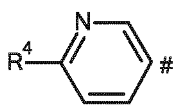 and 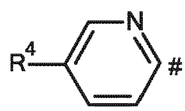 ,

wherein # indicates the point of attachment to X,

and stereoisomers, tautomers, N-oxides, hydrates, solvates, or salts thereof, or mixtures of same.

In a further embodiment of the first aspect, the invention relates to compounds of formula (I), wherein:

Ar represents a group selected from

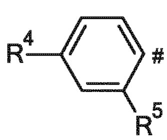 and 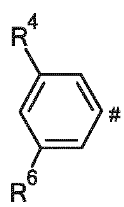 ,

wherein # indicates the point of attachment to X,

and stereoisomers, tautomers, hydrates, solvates, or salts thereof, or mixtures of same.

In a further embodiment of the first aspect, the invention relates to compounds of formula (I), wherein:

Ar represents a group selected from

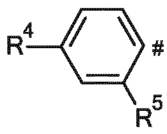 and 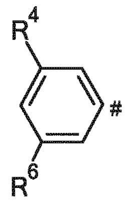 ,

wherein # indicates the point of attachment to X,

$R^5$ and $R^6$ represent a hydrogen atom,
and stereoisomers, tautomers, hydrates, solvates, or salts thereof, or mixtures of same.

In a further embodiment of the first aspect, the invention relates to compounds of formula (I), wherein:

Ar represents a group

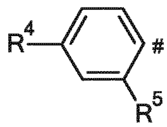 ,

wherein # indicates the point of attachment to X,

$R^5$ represents a hydrogen atom,
and stereoisomers, tautomers, hydrates, solvates, or salts thereof, or mixtures of same.

In a further embodiment of the first aspect, the invention relates to compounds of formula (I), wherein:

X represents a group selected from $CH_2$, $(CH_2)_2$, $(CH_2)_3$, $(CH_2)_4$ and $*-(CH_2)_2-O-CH_2-^{\#}$,

wherein * indicates the point of attachment to Ar and # indicates the point of attachment to the acetic acid moiety,
and stereoisomers, tautomers, N-oxides, hydrates, solvates, or salts thereof, or mixtures of same.

In a further embodiment of the first aspect, the invention relates to compounds of formula (I), wherein:

X represents a group selected from $CH_2$ and $(CH_2)_3$,

and stereoisomers, tautomers, N-oxides, hydrates, solvates, or salts thereof, or mixtures of same.
In a further embodiment of the first aspect, the invention relates to compounds of formula (I), wherein:

X represents $(CH_2)_3$,

and stereoisomers, tautomers, N-oxides, hydrates, solvates, or salts thereof, or mixtures of same.
In a further embodiment of the first aspect, the invention relates to compounds of formula (I), wherein:

$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,

and stereoisomers, tautomers, N-oxides, hydrates, solvates, or salts thereof, or mixtures of same.
In a further embodiment of the first aspect, the invention relates to compounds of formula (I),

wherein:

$R^1$ and $R^3$ represent, independently for each occurrence, a hydrogen atom or a $-CH_2OCH_3$ group,
$R^2$ represents a hydrogen atom or a group selected from $C_1$-$C_3$-alkyl, $-CH_2OH$, $-(CH_2)_2OH$ and $-CH_2OCH_3$,

and stereoisomers, tautomers, N-oxides, hydrates, solvates, or salts thereof, or mixtures of same.

In a further embodiment of the first aspect, the invention relates to compounds of formula (I), wherein:

$R^1$ and $R^3$ represent, independently for each occurrence, a hydrogen atom or a $-CH_2OH$ group,
$R^2$ represents a hydrogen atom or a group selected from $C_1$-$C_3$-alkyl, $-CH_2OH$, $-(CH_2)_2OH$ and $-CH_2OCH_3$,

and stereoisomers, tautomers, N-oxides, hydrates, solvates, or salts thereof, or mixtures of same.

In a further embodiment of the first aspect, the invention relates to compounds of formula (I), wherein:

$R^1$, $R^2$ and $R^3$ represent, independently for each occurrence, a hydrogen atom or a $-CH_2OH$ group,

and stereoisomers, tautomers, N-oxides, hydrates, solvates, or salts thereof, or mixtures of same.

In a further embodiment of the first aspect, the invention relates to compounds of formula (I), wherein:

$R^1$, $R^2$ and $R^3$ represent a hydrogen atom or a $-CH_2OH$ group,
and stereoisomers, tautomers, N-oxides, hydrates, solvates, or salts thereof, or mixtures of same.

In a further embodiment of the first aspect, the invention relates to compounds of formula (I), wherein:

$R^1$, $R^2$ and $R^3$ represent a $-CH_2OH$ group,
and stereoisomers, tautomers, N-oxides, hydrates, solvates, or salts thereof, or mixtures of same.

In a further embodiment of the first aspect, the invention relates to compounds of formula (I), wherein:

$R^1$ and $R^3$ represent a hydrogen atom,
$R^2$ represents a $-CH_2OH$ group,

and stereoisomers, tautomers, N-oxides, hydrates, solvates, or salts thereof, or mixtures of same.

In a further embodiment of the first aspect, the invention relates to compounds of formula (I), wherein:

$R^1$ and $R^3$ represent a hydrogen atom,
$R^2$ represents a $-CH_2OCH_3$ group,

and stereoisomers, tautomers, N-oxides, hydrates, solvates, or salts thereof, or mixtures of same.

In a further embodiment of the first aspect, the invention relates to compounds of formula (I), wherein:

$R^1$ and $R^3$ represent, a hydrogen atom,
$R^2$ represents a $C_1$-$C_3$-alkyl group,

and stereoisomers, tautomers, N-oxides, hydrates, solvates, or salts thereof, or mixtures of same.

In a further embodiment of the first aspect, the invention relates to compounds of formula (I), wherein:

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

and stereoisomers, tautomers, N-oxides, hydrates, solvates, or salts thereof, or mixtures of same.

In a further embodiment of the first aspect, the invention relates to compounds of formula (I), wherein:

$R^4$ represents a group selected from $C_2$-$C_4$-alkoxy, $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$, wherein said $C_2$-$C_4$-alkoxy group is optionally substituted, one, two, three or four times, with a fluorine atom,

and stereoisomers, tautomers, N-oxides, hydrates, solvates, or salts thereof, or mixtures of same.

In a further embodiment of the first aspect, the invention relates to compounds of formula (I), wherein:

$R^4$ represents a group selected from $C_2$-$C_4$-alkoxy, $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

and stereoisomers, tautomers, N-oxides, hydrates, solvates, or salts thereof, or mixtures of same.

In a further embodiment of the first aspect, the invention relates to compounds of formula (I), wherein:

$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,

and stereoisomers, tautomers, N-oxides, hydrates, solvates, or salts thereof, or mixtures of same.

In a further embodiment of the first aspect, the invention relates to compounds of formula (I), wherein:

$R^4$ represents $(H_3C-CH_2)-O-(CH_2)_2-O-(CH_2)_2-O-$,

and stereoisomers, tautomers, N-oxides, hydrates, solvates, or salts thereof, or mixtures of same.

In a further embodiment of the first aspect, the invention relates to compounds of formula (I), wherein:

$R^5$ represents a hydrogen atom or a group selected from $C_2$-$C_5$-alkoxy, $(C_1$-$C_3$-alkoxy$)-(CH_2)_2-O-$, $(C_1$-$C_3$-alkoxy$)-(CH_2)_2-O-(CH_2)_2-O-$ and $(C_1$-$C_3$-alkoxy$)-(CH_2)_2-O-(CH_2)_2-O-(CH_2)_2-O-$,

and stereoisomers, tautomers, hydrates, solvates, or salts thereof, or mixtures of same.

In a further embodiment of the first aspect, the invention relates to compounds of formula (I), wherein:

$R^5$ represents a hydrogen atom,

and stereoisomers, tautomers, hydrates, solvates, or salts thereof, or mixtures of same.

In a further embodiment of the first aspect, the invention relates to compounds of formula (I), wherein:

$R^6$ represents a hydrogen atom or a group selected from $C_2$-$C_5$-alkoxy, $(C_1$-$C_3$-alkoxy$)-(CH_2)_2-O-$, $(C_1$-$C_3$-alkoxy$)-(CH_2)_2-O-(CH_2)_2-O-$ and $(C_1$-$C_3$-alkoxy$)-(CH_2)_2-O-(CH_2)_2-O-(CH_2)_2-O-$,

and stereoisomers, tautomers, hydrates, solvates, or salts thereof, or mixtures of same.

In a further embodiment of the first aspect, the invention relates to compounds of formula (I), wherein:

$R^6$ represents a hydrogen atom,

and stereoisomers, tautomers, hydrates, solvates, or salts thereof, or mixtures of same.

In a further embodiment of the first aspect, the invention relates to compounds of formula (I), wherein:

$R^5$ and $R^6$ represent a hydrogen atom,

and stereoisomers, tautomers, hydrates, solvates, or salts thereof, or mixtures of same.

It is to be understood that the present invention relates also to any combination of the embodiments described above.

In the context of the present invention, the compounds of formula (I) may contain one or more chiral centers. When $R^1 = R^2 = R^3 = H$, the carbon atom attached to X can be in the (R) or (S) configuration. Thus, the present invention includes the (R) and (S) enantiomers of the compounds of formula (I) when $R^1 = R^2 = R^3 = H$, or mixtures thereof.

Further, when one or more of $R^1$, $R^2$ and/or $R^3$ are different from a hydrogen atom, the carbon atoms attached to $R^1$, $R^2$ and/or $R^3$ can be in the (R) or (S) configuration. Thus, the present invention includes all possible stereoisomers of the compounds of formula (I) when one or more of $R^1$, $R^2$ and/or $R^3$ are different from a hydrogen atom, or mixtures thereof. When one of $R^1$, $R^2$ or $R^3$ is different from a hydrogen atom, this includes the RR, SS, RS, SR stereoisomers of the compounds of formula (I), or mixtures thereof. When two of $R^1$, $R^2$ or $R^3$ are different from a hydrogen atom, this includes the RRR, SSS, RRS, SSR, SRR, RSS, RSR and SRS stereoisomers of the compounds of formula (I), or mixtures thereof.

When $R^1$, $R^2$ and $R^3$ are different from a hydrogen atom, this includes the RRRR, SRRR, RSRR, RRSR, RRRS, SSRR, SRSR, SRRS, RSSR, RSRS, RRSS, RSSS, SRSS, SSRS, SSSR, SSSS stereoisomers of the compounds of formula (I), or mixtures thereof.

Another embodiment of the first aspect are compounds of formula (I) selected from the group consisting of:

3-[2-(4-ethoxyphenyl)ethoxy]-2-[4,7,10-tris(carboxymethyl)-1,4,7,10-tetraazacyclododecan-1-yl]propanoic acid,
3-{4-[2-(2-ethoxyethoxy)ethoxy]phenyl}-2-[4,7,10-tris(carboxymethyl)-1,4,7,10-tetraazacyclododecan-1-yl]propanoic acid,
(2S)-3-{4-[2-(2-ethoxyethoxy)ethoxy]phenyl}-2-[4,7,10-tris(carboxymethyl)-1,4,7,10-tetraazacyclododecan-1-yl] propanoic acid,
(2R)-3-{4-[2-(2-ethoxyethoxy)ethoxy]phenyl}-2-[4,7,10-tris(carboxymethyl)-1,4,7,10-tetraaza-cyclododecan-1-yl] propanoic acid,
2-[7-(1-carboxyethyl)-4,10-bis(carboxymethyl)-1,4,7,10-tetraazacyclododecan-1-yl]-3-{4-[2-(2-ethoxyethoxy) ethoxy]phenyl}propanoic acid,
2-[7-{1-carboxy-2-[4-(2-ethoxyethoxy)phenyl]ethyl}-4,10-bis(carboxymethyl)-1,4,7,10-tetraazacyclododecan-1-yl] butanoic acid,
(2S)-6-(4-butoxyphenyl)-2-{4,7,10-tris[(1S)-1-carboxy-2-hydroxyethyl]-1,4,7,10-tetraazacyclododecan-1-yl}hexanoic acid,
3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid,
(2S)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid,
(2R)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid,
2-{7-[1-carboxy-2-(4-{2-[2-(2-ethoxyethoxy)ethoxy]ethoxy}phenyl)ethyl]-4,10-bis(carboxymethyl)-1,4,7,10-tetraazacyclododecan-1-yl}-3-methoxypropanoic acid,
3-(4-butoxyphenyl)-2-[4,7,10-tris(carboxymethyl)-1,4,7,10-tetraazacyclododecan-1-yl]propanoic acid,
(2S)-5-{4-[2-(2-ethoxyethoxy)ethoxy]phenyl}-2-{4,7,10-tris[(1S)-1-carboxy-2-hydroxyethyl]-1,4,7,10-tetraazacyclododecan-1-yl}pentanoic acid,
3-{6-[2-(2-ethoxyethoxy)ethoxy]pyridin-3-yl}-2-[4,7,10-tris(carboxymethyl)-1,4,7,10-tetraazacyclododecan-1-yl] propanoic acid,
5-{4-[2-(2-ethoxyethoxy)ethoxy]phenyl}-2-[4,7,10-tris(1-carboxy-2-hydroxyethyl)-1,4,7,10-tetraazacyclododecan-1-yl]pentanoic acid,
(2R)-5-{4-[2-(2-ethoxyethoxy)ethoxy]lphenyl}-2-{4,7,10-tris[(1S)-1-carboxy-2-hydroxyethyl]-1,4,7,10-tetraazacyclododecan-1-yl}pentanoic acid,
(2R)-5-{4-[2-(2-ethoxyethoxy)ethoxy]phenyl}-2-{4,7,10-tris[(1R)-1-carboxy-2-hydroxyethyl]-1,4,7,10-tetraazacyclododecan-1-yl}pentanoic acid,
(2S)-5-{4-[2-(2-ethoxyethoxy)ethoxy]phenyl}-2-{4,7,10-tris[(1R)-1-carboxy-2-hydroxyethyl]-1,4,7,10-tetraazacyclododecan-1-yl}pentanoic acid,
(2S)-5-{4-[2-(2-ethoxyethoxy)ethoxy]phenyl}-2-[4,7,10-tris(1-carboxy-2-hydroxyethyl)-1,4,7,10-tetraazacyclododecan-1-yl]pentanoic acid,
2,2',2''-{10-[1-carboxy-2-{4-[2-(2-ethoxyethoxy)ethoxy]phenyl}ethyl]-1,4,7,10-tetraazacyclododecane-1,4,7-triyl} tris(3-hydroxypropanoic acid),
3-{5-[2-(2-ethoxyethoxy)ethoxy]pyridin-2-yl}-2-[4,7,10-tris(carboxymethyl)-1,4,7,10-tetraazacyclododecan-1-yl]propanoic acid,
4-(4-propoxyphenyl)-2-[4,7,10-tris(carboxymethyl)-1,4,7,10-tetraazacyclododecan-1-yl]butanoic acid,
5-{4-[2-(2-ethoxyethoxy)ethoxy]phenyl}-2-[4,7,10-tris(carboxymethyl)-1,4,7,10-tetraazacyclododecan-1-yl]pentanoic acid,
(2S)-5-{4-[2-(2-ethoxyethoxy)ethoxy]phenyl}-2-[4,7,10-tris(carboxymethyl)-1,4,7,10-tetraazacyclododecan-1-yl] pentanoic acid,
(2S)-6-(4-ethoxyphenyl)-2-[4,7,10-tris(carboxymethyl)-1,4,7,10-tetraazacyclododecan-1-yl]hexanoic acid,
5-(4-ethoxyphenyl)-2-[4,7,10-tris(carboxymethyl)-1,4,7,10-tetraazacyclododecan-1-yl] pentanoic acid,
2-{7-[1-carboxy-2-(4-ethoxyphenyl)ethyl]-4,10-bis(carboxymethyl)-1,4,7,10-tetraazacyclododecan-1-yl}pentanoic acid,
(2S)-5-(4-butoxyphenyl)-2-[4,7,10-tris(carboxymethyl)-1,4,7,10-tetraazacyclododecan-1-yl]pentanoic acid,
3-[4-(2-ethoxyethoxy)phenyl]-2-[4,7,10-tris(carboxymethyl)-1,4,7,10-tetraazacyclododecan-1-yl]propanoic acid,
(2S)-2-[4,10-bis(carboxymethyl)-7-{1-carboxy-2-[4-(2,2,3,3-tetrafluoropropoxy)phenyl]ethyl}-1,4,7,10-tetraazacyclododecan-1-yl]-3-hydroxypropanoic acid,
(2S)-5-(3-butoxyphenyl)-2-{4,7,10-tris[(1S)-1-carboxy-2-hydroxyethyl]-1,4,7,10-tetraazacyclododecan-1-yl}pentanoic acid,
(2S)-2-{7-[(1 R)-1-carboxy-2-hydroxyethyl]-4, 1 0-bis[(1S)-1-carboxy-2-hydroxyethyl]-1,4, 7,10-tetraazacyclododecan-1-yl}-5-{4-[2-(2-ethoxyethoxy)ethoxy]phenyl}pentanoic acid,
(2S)-2-{7-[1-carboxy-3-hydroxypropyl]-4,10-bis[1-carboxy-3-hydroxypropyl]-1,4,7,10-tetraazacyclododecan-1-yl}-5-{4-[2-(2-ethoxyethoxy)ethoxy]phenyl}pentanoic acid,
2,2" 2"-[10-(1-carboxy-2-{4-[2-(2-ethoxyethoxy)ethoxy]phenyl}ethyl)-1,4,7,10-tetraazacyclododecane-1,4,7-triyl] tris(4-hydroxybutanoic acid),
2-[7-{1-carboxy-2-[4-(2-ethoxyethoxy)phenyl]ethyl}-4,10-bis(carboxymethyl)-1,4,7,10-tetraazacyclododecan-1-yl]-3-methoxypropanoic acid,
3-{3,5-bis[2-(2-ethoxyethoxy)ethoxy]phenyl}-2-[4,7,10-tris(carboxymethyl)-1,4,7,10-tetraazacyclododecan-1-yl] propanoic acid,
3-(2,4-bis{2-[2-(2-ethoxyethoxy)ethoxy]ethoxy}phenyl)-2-[4,7,10-tris(carboxymethyl)-1,4,7,10-tetraazacyclododecan-1-yl]propanoic acid,
2-[7-(1-carboxy-2-{4-[2-(2-ethoxyethoxy)ethoxy]phenyl}ethyl)-4,10-bis(carboxymethyl)-1,4,7,10-tetraazacyclododecan-1-yl]-3-hydroxypropanoic acid,
2-{7-[1-carboxy-2-(4-{2-[2-(2-ethoxyethoxy)ethoxy]ethoxy}phenyl)ethyl]-4,10-bis(carboxymethyl)-1,4,7,10-tetraazacyclododecan-1-yl}-3-hydroxypropanoic acid,
2-[7-(1-carboxy-2-{4-[2-(2-ethoxyethoxy)ethoxy]phenyl}ethyl)-4,10-bis(carboxymethyl)-1,4,7,10-tetraazacyclododecan-1-yl]butanoic acid,
3-(4-butoxyphenyl)-2-[4,7,10-tris(carboxymethyl)-1,4,7,10-tetraazacyclododecan-1-yl]propanoic acid,
2-[4,10-bis(carboxymethyl)-7-{1-carboxy-2-[4-(2,2,3,3-tetrafluoropropoxy) phenyl]ethyl}-1,4,7,10-tetraazacyclododecan-1-yl]-3-methoxypropanoic acid,
3-[4-(2,2,3,3-tetrafluoropropoxy)phenyl]-2-[4,7,10-tris(carboxymethyl)-1,4,7,10-tetraazacyclododecan-1-yl]propanoic acid,
3-[4-(2,2,2-trifluoroethoxy)phenyl]-2-[4,7,10-tris(carboxymethyl)-1,4,7,10-tetraazacyclododecan-1-yl]propanoic acid,
3-(4-propoxyphenyl)-2-[4,7,10-tris(carboxymethyl)-1,4,7,10-tetraazacyclododecan-1-yl]propanoic acid,
(2S)-2-{4,7-bis[(1R)-1-carboxy-2-hydroxyethyl]-10-[(1S)-1-carboxy-2-hydroxyethyl]-1,4,7,10-tetraazacyclododecan-1-yl}-5-{4-[2-(2-ethoxyethoxy)ethoxy]phenyl}pentanoic acid and
(2S)-2-{4-[(1R)-1-carboxy-2-hydroxyethyl]-7,10-bis[(1S)-1-carboxy-2-hydroxyethyl]-1,4,7,10-tetraazacyclododecan-1-yl}-5-{4-[2-(2-ethoxyethoxy)ethoxy]phenyl}pentanoic acid and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

Ar represents a group selected from

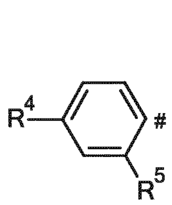, 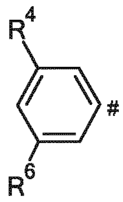, 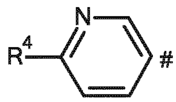 and 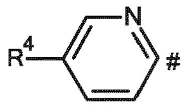,

wherein # indicates the point of attachment to X,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

Ar represents a group selected from

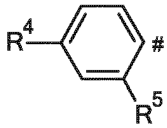 and 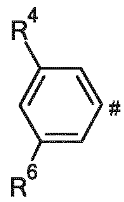,

wherein # indicates the point of attachment to X,

and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

Ar represents a group selected from

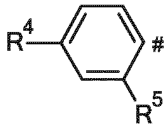 and 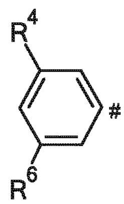,

wherein # indicates the point of attachment to X,

$R^5$ and $R^6$ represent a hydrogen atom,
and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

Ar represents

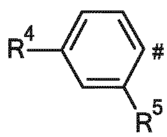,

wherein # indicates the point of attachment to X,

$R^5$ represents a hydrogen atom,
and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

X represents a group selected from $CH_2$, $(CH_2)_2$, $(CH_2)_3$, $(CH_2)_4$ and $*-(CH_2)_2-O-CH_2-^{\#}$,

wherein * indicates the point of attachment to Ar and # indicates the point of attachment to the acetic acid moiety, and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

X represents a group selected from $CH_2$, and $(CH_2)_3$,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

X represents $(CH_2)_3$,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$, $R^2$and $R^3$ represent, independently for each occurrence, a hydrogen atom or a group selected from $C_1$-$C_3$-alkyl, $-CH_2OH$, $-(CH_2)_2OH$ and $-CH_2OCH_3$,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

R' and $R^3$ represent, independently for each occurrence, a hydrogen atom or a $-CH_2OCH_3$ group,
$R^2$ represents a hydrogen atom or a group selected from $C_1$-$C_3$-alkyl, $-CH_2OH$, $-(CH_2)_2OH$ and $-CH_2OCH_3$,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

R' and $R^3$ represent, independently for each occurrence, a hydrogen atom or a $-CH_2OH$ group,
$R^2$ represents a hydrogen atom or a group selected from $C_1$-$C_3$-alkyl, $-CH_2OH$, $-(CH_2)_2OH$ and $-CH_2OCH_3$,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$, $R^2$ and $R^3$ represent, independently for each occurrence, a hydrogen atom or a $-CH_2OH$ group,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$, $R^2$ and $R^3$ represent a hydrogen atom or a $-CH_2OH$ group,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$, $R^2$ and $R^3$ represent a $-CH_2OH$ group,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

**R' and** $R^3$ **represent** a hydrogen atom,
$R^2$ represents a $-CH_2OH$ group,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

R' and $R^3$ represent a hydrogen atom,
$R^2$ represents a $-CH_2OCH_3$ group,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^1$ and $R^3$ represent, a hydrogen atom,
$R^2$ represents a $C_1$-$C_3$-alkyl group,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, wherein said $C_1$-$C_3$-alkoxy groups and $C_2$-$C_5$-alkoxy groups are optionally substituted, one, two, three or four times, with a fluorine atom,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^4$ represents a group selected from $C_2$-$C_4$-alkoxy, $(H_3C$-$CH_2)$-O-$(CH_2)_2$-O-, $(H_3C$-$CH_2)$-O-$(CH_2)_2$-O-$(CH_2)_2$-O- and $(H_3C$-$CH_2)$-O-$(CH_2)_2$-O-$(CH_2)_2$-O-$(CH_2)_2$-O-, wherein said $C_2$-$C_4$-alkoxy group is optionally substituted, one, two, three or four times, with a fluorine atom,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^4$ represents a group selected from $C_2$-$C_4$-alkoxy, $(H_3C$-$CH_2)$-O-$(CH_2)_2$-O-, $(H_3C$-$CH_2)$-O-$(CH_2)_2$-O-$(CH_2)_2$-O- and $(H_3C$-$CH_2)$-O-$(CH_2)_2$-O-$(CH_2)_2$-O-$(CH_2)_2$-O-,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^4$ represents a group selected from $(H_3C$-$CH_2)$-O-$(CH_2)_2$-O-, $(H_3C$-$CH_2)$-O-$(CH_2)_2$-O-$(CH_2)_2$-O- and $(H_3C$-$CH_2)$-O-$(CH_2)_2$-O-$(CH_2)_2$-O-$(CH_2)_2$-O-,

and stereoisomers, tautomers, N-oxides, hydrates, solvates, or salts thereof, or mixtures of same.

In a further embodiment of the second aspect, the invention relates to compounds of formula (I) in the form of a complex with $Gd^{3+}$, wherein:

$R^4$ represents $(H_{3C}$-$CH_2)$-O-$(CH_2)_2$-O-$(CH_2)_2$-O-,

and stereoisomers, tautomers, N-oxides, hydrates, solvates, or salts thereof, or mixtures of same.

In a further embodiment of the second aspect, the invention relates to compounds of formula (I) in the form of a

complex with $Gd^{3+}$, wherein:

$R^5$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

$R^5$ represents a hydrogen atom,

and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

$R^6$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

$R^6$ represents a hydrogen atom,

and stereoisomers, tautomers, hydrates, solvates, or salts thereof, or mixtures of same.

In a further embodiment of the second aspect, the invention relates to compounds of formula (I) in the form of a complex with $Gd^{3+}$, wherein:

$R^5$ and $R^6$ represent a hydrogen atom,

and stereoisomers, tautomers, hydrates, solvates, or salts thereof, or mixtures of same.

It is to be understood that the present invention relates also to any combination of the embodiments described above.

In the context of the present invention, the compounds of formula (I) in the form of a complex with $Gd^{3+}$ may contain one or more chiral centers. When $R^1 = R^2 = R^3 = H$, the carbon atom attached to X can be in the (R) or (S) configuration. Thus, the present invention includes the (R) and (S) enantiomers of the compounds of formula (I) in the form of a complex with $Gd^{3+}$ when $R^1 = R^2 = R^3 = H$, or mixtures thereof.

Further, when one or more of $R^1$, $R^2$ and/or $R^3$ are different from a hydrogen atom, the carbon atoms attached to $R^1$, $R^2$ and/or $R^3$ can be in the (R) or (S) configuration. Thus, the present invention includes all possible stereoisomers of the compounds of formula (I) in the form of a complex with $Gd^{3+}$ when one or more of $R^1$, $R^2$ and/or $R^3$ are different from a hydrogen atom, or mixtures thereof. When one of $R^1$, $R^2$ or $R^3$ is different from a hydrogen atom, this includes the RR, SS, RS, SR stereoisomers of the compounds of formula (I) in the form of a complex with $Gd^{3+}$, or mixtures thereof. When two of $R^1$, $R^2$ or $R^3$ are different from a hydrogen atom, this includes the RRR, SSS, RRS, SSR, SRR, RSS, RSR and SRS stereoisomers of the compounds of formula (I) in the form of a complex with $Gd^{3+}$, or mixtures thereof. When $R^1$, $R^2$ and $R^3$ are different from a hydrogen atom, this includes the RRRR, RRRS, RSRR, RSSR, RSRS, RRSS, RRSR, RSSS, SSSS, SSSR, SRSS, SRRS, SRSR, SSRR, SSRS, SRRR stereoisomers of the compounds of formula (I) in the form of a complex with $Gd^{3+}$, or mixtures thereof.

In accordance with a further embodiment of the second aspect, the present invention covers compounds of general formula (I), *supra,* in the form of a sodium ($Na^+$) salt of a complex with $Gd^{3+}$, wherein Ar, X, $R^1$, $R^2$, $R^3$, $R^4$, $R^5$ and $R^6$ are defined as described in any of the embodiments above, and stereoisomers, tautomers, hydrates, or solvates thereof, or mixtures of same.

Another embodiment of the second aspect are compounds of formula (I) in the form of a complex with $Gd^{3+}$, selected from the group consisting of:

gadolinium 2,2',2"-(10-{1-carboxy-2-[2-(4-ethoxyphenyl)ethoxy]ethyl}-1,4,7,10-tetraazacyclododecane-1,4,7-triyl) triacetate,
gadolinium 2,2',2"-{10-[1-carboxy-2-{4-[2-(2-ethoxyethoxy)ethoxy] phenyl}ethyl]-1,4,7,10-tetraazacyclododecane-1,4,7-triyl}triacetate,

gadolinium 2,2',2"-{10-[(1S)-1-carboxy-2-{4-[2-(2-ethoxyethoxy)ethoxy] phenyl}ethyl]-1,4,7,10-tetraazacyclododecane-1,4,7-triyl}triacetate,
gadolinium 2,2',2"-{10-[(1R)-1-carboxy-2-{4-[2-(2-ethoxyethoxy)ethoxy] phenyl}ethyl]-1,4,7,10-tetraazacyclododecane-1,4,7-triyl}triacetate,
gadolinium 2-[7-(1-carboxy-2-{4-[2-(2-ethoxyethoxy)ethoxy]phenyl} ethyl)-4,10-bis(carboxylatomethyl)-1,4,7,10-tetraazacyclododecan-1-yl]propanoate,
gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]butanoate,
gadolinium (2S,2'S,2"S)-2,2',2"-{10-[(1S)-5-(4-butoxyphenyl)-1-carboxypentyl]-1,4,7,10-tetraazacyclododecane-1,4,7-triyl}tris(3-hydroxypropanoate),
gadolinium 2,2',2"-{10-[1-carboxy-2-(4-{2-[2-(2-ethoxyethoxy)ethoxy] ethoxy}phenyl)ethyl]-1,4,7,10-tetraazacyclododecane-1,4,7-triyl} triacetate,
gadolinium 2,2',2"-{10-[(1S)-1-carboxy-2-(4-{2-[2-(2-ethoxyethoxy)ethoxy]ethoxy}phenyl)ethyl]-1,4,7,10-tetraazacyclododecane-1,4,7-triyl}triacetate,
gadolinium 2,2',2"-{10-[(1R)-1-carboxy-2-(4-{2-[2-(2-ethoxyethoxy)ethoxy]ethoxy}phenyl)ethyl]-1,4,7,10-tetraazacyclododecane-1,4,7-triyl}triacetate,
gadolinium 2-{7-[1-carboxy-2-(4-{2-[2-(2-ethoxyethoxy)ethoxy]ethoxy} phenyl)ethyl]-4,10-bis(carboxylatomethyl)-1,4,7,10-tetraazacyclodo decan-1-yl}-3-methoxypropanoate,
gadolinium 2,2',2"-{10-[2-(4-butoxyphenyl)-1-carboxyethyl]-1,4,7,10-tetraazacyclododecane-1,4,7-triyl}triacetate,
gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium 2,2',2"-{10-[1-carboxy-2-{6-[2-(2-ethoxyethoxy)ethoxy] pyridin-3-yl}ethyl]-1,4,7,10-tetraazacyclododecane-1,4,7-triyl}triacetate,
gadolinium-2,2',2"-{10-[1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium (25,2'S,2"S)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium (2R,2'R,2"R)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium (2R,2'R,2"R)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium 2,2',2"-{10-[1-carboxy-2-{4-[2-(2-ethoxyethoxy)ethoxy] phenyl}ethyl]-1,4,7,10-tetraazacyclododecane-1,4,7-triyl}tris(3-hydroxypropanoate),
gadolinium 2,2',2"-{10-[1-carboxy-2-{5-[2-(2-ethoxyethoxy)ethoxy] pyridin-2-yl}ethyl]-1,4,7,10-tetraazacyclododecane-1,4,7-triyl}triacetate,
gadolinium 2,2',2"-{10-[1-carboxy-3-(4-propoxyphenyl)propyl]-1,4,7,10-tetraazacyclododecane-1,4,7-triyl}triacetate,
gadolinium 2,2',2"-{10-[1-carboxy-4-{4-[2-(2-ethoxyethoxy)ethoxy] phenyl}butyl]-1,4,7,10-tetraazacyclododecane-1,4,7-triyl}triacetate,
gadolinium 2,2',2"-{10-[(1S)-1-carboxy-4-{4-[2-(2-ethoxyethoxy)ethoxy] phenyl}butyl]-1,4,7,10-tetraazacyclododecane-1,4,7-triyl}triacetate,
gadolinium 2,2',2"-{10-[(1S)-1-carboxy-5-(4-ethoxyphenyl)pentyl]-1,4,7,10-tetraazacyclododecane-1,4,7-triyl}triacetate,
gadolinium 2,2',2"-{10-[1-carboxy-4-(4-ethoxyphenyl)butyl]-1,4,7,10-tetraazacyclododecane-1,4,7-triyl}triacetate,
gadolinium 2-{7-[1-carboxy-2-(4-ethoxyphenyl)ethyl]-4,10-bis(carboxylatomethyl)-1,4,7,10-tetraazacyclododecan-1-yl}pentanoate,
gadolinium 2,2',2"-{10-[(1S)-4-(4-butoxyphenyl)-1-carboxybutyl]-1,4,7,10-tetraazacyclododecane-1,4,7-triyl}triacetate,
gadolinium 2,2',2"-(10-{1-carboxy-2-[4-(2-ethoxyethoxy)phenyl]ethyl}-1,4,7,10-tetraazacyclododecane-1,4,7-triyl) triacetate,
gadolinium (2S)-2-[4,10-bis(carboxylatomethyl)-7-{1-carboxy-2-[4-(2,2,3,3-tetrafluoropropoxy)phenyl]ethyl}-1,4,7,1 0-tetraazacyclododecan-1-yl]-3-hydroxypropanoate,
gadolinium (25,2'S,2"S)-2,2',2"-{10-[(1S)-4-(3-butoxyphenyl)-1-carboxybutyl]-1,4,7,10-tetraazacyclododecane-1,4,7-triyl}tris(3-hydroxypropanoate),
gadolinium (2S,2'S)-2,2'-{4-[(1S)-1-carboxy-4-{4-[2-(2-ethoxyethoxy) ethoxy]phenyl}butyl]-10-[(1R)-1-carboxylato-2-hydroxyethyl]-1,4,7,10-tetraazacyclododecane-1,7-diyl}bis(3-hydroxypropanoate),

gadolinium-2,2'-{4-[(1S)-1-carboxy-4-{4-[2-(2-ethoxyethoxy)ethoxy] phenyl}butyl]-10-[1-carboxylato-3-hydroxypropyl]-1,4,7,10-tetraazacyclododecane-1,7-diyl}bis(4-hydroxybutanoate),
gadolinium-2,2'-{4-[1-carboxy-2-{4-[2-(2-ethoxyethoxy)ethoxy] phenyl}ethyl]-10-[1-carboxylato-3-hydroxypropyl]-1,4,7,10-tetraazacyclododecane-1,7-diyl}bis(4-hydroxybutanoate),
gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]-3-methoxypropanoate,
gadolinium 2,2',2"-{10-[2-{3,5-bis[2-(2-ethoxyethoxy)ethoxy]phenyl}-1-carboxyethyl]-1,4,7,10-tetraazacyclododecane-1,4,7-triyl}triacetate,
gadolinium 2,2',2"-{10-[(1S)-2-(2,4-bis{2-[2-(2-ethoxyethoxy)ethoxy]ethoxy}phenyl)-1-carboxyethyl]-1,4,7,10-tetraazacyclododecane-1,4,7-triyl}triacetate,
gadolinium-2-{7-[1-carboxy-2-{4-[2-(2-ethoxyethoxy)ethoxy]phenyl} ethyl]-4,10-bis(carboxylatomethyl)-1,4,7,10-tetraazacyclododecan-1-yl}-3-hydroxypropanoate,
gadolinium-2-{7-[1-carboxy-2-(4-{2-[2-(2-ethoxyethoxy)ethoxy]ethoxy} phenyl)ethyl]-4,10-bis(carboxylatomethyl)-1,4,7,10-tetraazacyclododecan-1-yl}-3-hydroxypropanoate,
gadolinium-2-{4,10-bis(carboxylatomethyl)-7-[1-carboxypropyl]-1,4,7,10-tetraazacyclododecan-1-yl}-3-{4-[2-(2-ethoxyethoxy)ethoxy]phenyl}propanoate,
gadolinium 2,2',2"-{10-[2-(5-butoxypyridin-2-yl)-1-carboxyethyl]-1,4,7,10-tetraazacyclododecane-1,4,7-triyl}triacetate,
gadolinium-2-{4,10-bis(carboxylatomethyl)-7-[1-carboxy-2-methoxyethyl]-1,4,7,10-tetraazacyclododecan-1-yl}-3-[4-(2,2,3,3-tetrafluoropropoxy)phenyl]propanoate,
gadolinium 2,2',2"-(1 0-{1-carboxy-2-[4-(2,2,3,3-tetrafluoropropoxy) phenyl]ethyl}-1,4,7,10-tetraazacyclododecane-1,4,7-triyl)triacetate,
gadolinium 2,2',2"-(10-{(1R)-1-carboxy-2-[4-(2,2,2-trifluoroethoxy) phenyl]ethyl}-1,4,7,10-tetraazacyclododecane-1,4,7-triyl)triacetate,
gadolinium 2,2',2"-{10-[1-carboxy-2-(4-propoxyphenyl)ethyl]-1,4,7,10-tetraazacyclododecane-1,4,7-triyl}triacetate,
gadolinium (2R,2'R)-2,2'-{7-[(1S)-1-carboxy-4-{4-[2-(2-ethoxyethoxy)ethoxy]phenyl}butyl]-10-[(1S)-1-carboxylato-2-hydroxyethyl]-1,4,7,10-tetraazacyclododecane-1,4-diyl}bis(3-hydroxypropanoate) and
gadolinium (2S,2'S)-2,2'-{7-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecane-1,4-diyl}bis(3-hydroxypropanoate)
and stereoisomers, tautomers, N-oxides, hydrates, solvates, or salts thereof, or mixtures of same.

Another embodiment of the second aspect are compounds of formula (I) in the form of a sodium ($Na^+$) salt of a complex with $Gd^{3+}$, said complex with $Gd^{3+}$ selected from the group consisting of:

gadolinium 2,2',2"-(10-{1-carboxy-2-[2-(4-ethoxyphenyl)ethoxy]ethyl}-1,4,7,10-tetraazacyclododecane-1,4,7-triyl) triacetate,
gadolinium 2,2',2"-{10-[1-carboxy-2-{4-[2-(2-ethoxyethoxy)ethoxy] phenyl}ethyl]-1,4,7,10-tetraazacyclododecane-1,4,7-triyl}triacetate,
gadolinium 2,2',2"-{10-[(1S)-1-carboxy-2-{4-[2-(2-ethoxyethoxy)ethoxy] phenyl}ethyl]-1,4,7,10-tetraazacyclododecane-1,4,7-triyl}triacetate,
gadolinium 2,2',2"-{10-[(1R)-1-carboxy-2-{4-[2-(2-ethoxyethoxy)ethoxy]phenyl}ethyl]-1,4,7,10-tetraazacyclododecane-1,4,7-triyl}triacetate,
gadolinium 2-[7-(1-carboxy-2-{4-[2-(2-ethoxyethoxy)ethoxy]phenyl} ethyl)-4,10-bis(carboxylatomethyl)-1,4,7,10-tetraazacyclododecan-1-yl]propanoate,
gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]butanoate,
gadolinium (2S,2'S,2"S)-2,2',2"-{10-[(1S)-5-(4-butoxyphenyl)-1-carboxypentyl]-1,4,7,10-tetraazacyclododecane-1,4,7-triyl}tris(3-hydroxypropanoate),
gadolinium 2,2',2"-{10-[1-carboxy-2-(4-{2-[2-(2-ethoxyethoxy)ethoxy] ethoxy}phenyl)ethyl]-1,4,7,10-tetraazacyclododecane-1,4,7-triyl} triacetate,
gadolinium 2,2',2"-{10-[(1S)-1-carboxy-2-(4-{2-[2-(2-ethoxyethoxy)ethoxy]ethoxy}phenyl)ethyl]-1,4,7,10-tetraazacyclododecane-1,4,7-triyl}triacetate,
gadolinium 2,2',2"-{10-[(1R)-1-carboxy-2-(4-{2-[2-(2-ethoxyethoxy)ethoxy]ethoxy}phenyl)ethyl]-1,4,7,10-tetraazacyclododecane-1,4,7-triyl}triacetate,
gadolinium 2-{7-[1-carboxy-2-(4-{2-[2-(2-ethoxyethoxy)ethoxy]ethoxy} phenyl)ethyl]-4,10-bis(carboxylatomethyl)-1,4,7,10-tetraazacyclodo decan-1-yl}-3-methoxypropanoate,
gadolinium 2,2',2"-{10-[2-(4-butoxyphenyl)-1-carboxyethyl]-1,4,7,10-tetraazacyclododecane-1,4,7-triyl}triacetate,
gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium 2,2',2"-{10-[1-carboxy-2-{6-[2-(2-ethoxyethoxy)ethoxy] pyridin-3-yl}ethyl]-1,4,7,10-tetraazacyclododecane-1,4,7-triyl}triacetate,
gadolinium-2,2',2"-{10-[1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium (25,2'S,2"S)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium (2R,2'R,2"R)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium (2R,2'R,2"R)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),
gadolinium 2,2',2"-{10-[1-carboxy-2-{4-[2-(2-ethoxyethoxy)ethoxy] phenyl}ethyl]-1,4,7,10-tetraazacyclododecane-1,4,7-triyl}tris(3-hydroxypropanoate),
gadolinium 2,2',2"-{10-[1-carboxy-2-{5-[2-(2-ethoxyethoxy)ethoxy] pyridin-2-yl}ethyl]-1,4,7,10-tetraazacyclododecane-1,4,7-triyl}triacetate,
gadolinium 2,2',2"-{10-[1-carboxy-3-(4-propoxyphenyl)propyl]-1,4,7,10-tetraazacyclododecane-1,4,7-triyl}triacetate,
gadolinium 2,2',2"-{10-[1-carboxy-4-{4-[2-(2-ethoxyethoxy)ethoxy] phenyl}butyl]-1,4,7,10-tetraazacyclododecane-1,4,7-triyl}triacetate,
gadolinium 2,2',2"-{10-[(1S)-1-carboxy-4-{4-[2-(2-ethoxyethoxy)ethoxy] phenyl}butyl]-1,4,7,10-tetraazacyclododecane-1,4,7-triyl}triacetate,
gadolinium 2,2',2"-{10-[(1S)-1-carboxy-5-(4-ethoxyphenyl)pentyl]-1,4,7,10-tetraazacyclododecane-1,4,7-triyl}triacetate,
gadolinium 2,2',2"-{10-[1-carboxy-4-(4-ethoxyphenyl)butyl]-1,4,7,10-tetraazacyclododecane-1,4,7-triyl}triacetate,
gadolinium 2-{7-[1-carboxy-2-(4-ethoxyphenyl)ethyl]-4,10-bis(carboxylatomethyl)-1,4,7,10-tetraazacyclododecan-1-yl}pentanoate,
gadolinium 2,2',2"-{10-[(1S)-4-(4-butoxyphenyl)-1-carboxybutyl]-1,4,7,10-tetraazacyclododecane-1,4,7-triyl}triacetate,
gadolinium 2,2',2"-(10-{1-carboxy-2-[4-(2-ethoxyethoxy)phenyl]ethyl}-1,4,7,10-tetraazacyclododecane-1,4,7-triyl) triacetate,
gadolinium (2S)-2-[4,10-bis(carboxylatomethyl)-7-{1-carboxy-2-[4-(2,2,3,3-tetrafluoropropoxy)phenyl]ethyl}-1,4,7,1 0-tetraazacyclododecan-1-yl]-3-hydroxypropanoate,
gadolinium (2S,2'S,2"S)-2,2',2"-{10-[(1S)-4-(3-butoxyphenyl)-1-carboxybutyl]-1,4,7,10-tetraazacyclododecane-1,4,7-triyl}tris(3-hydroxypropanoate),
gadolinium (2S,2'S)-2,2'-{4-[(1S)-1-carboxy-4-{4-[2-(2-ethoxyethoxy) ethoxy]phenyl}butyl]-10-[(1R)-1-carboxylato-2-hydroxyethyl]-1,4,7,10-tetraazacyclododecane-1,7-diyl}bis(3-hydroxypropanoate),
gadolinium-2,2'-{4-[(1S)-1-carboxy-4-{4-[2-(2-ethoxyethoxy)ethoxy] phenyl}butyl]-10-[1-carboxylato-3-hydroxypropyl]-1,4,7,10-tetraazacyclododecane-1,7-diyl}bis(4-hydroxybutanoate),
gadolinium-2,2'-{4-[1-carboxy-2-{4-[2-(2-ethoxyethoxy)ethoxy] phenyl}ethyl]-10-[1-carboxylato-3-hydroxypropyl]-1,4,7,10-tetraazacyclododecane-1,7-diyl}bis(4-hydroxybutanoate),
gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]-3-methoxypropanoate,
gadolinium 2,2',2"-{10-[2-{3,5-bis[2-(2-ethoxyethoxy)ethoxy]phenyl}-1-carboxyethyl]-1,4,7,10-tetraazacyclododecane-1,4,7-triyl}triacetate,
gadolinium 2,2',2"-{10-[(1S)-2-(2,4-bis{2-[2-(2-ethoxyethoxy)ethoxy]ethoxy}phenyl)-1-carboxyethyl]-1,4,7,10-tetraazacyclododecane-1,4,7-triyl}triacetate,
gadolinium-2-{7-[1-carboxy-2-{4-[2-(2-ethoxyethoxy)ethoxy]phenyl} ethyl]-4,10-bis(carboxylatomethyl)-1,4,7,10-tetraazacyclododecan-1-yl}-3-hydroxypropanoate,
gadolinium-2-{7-[1-carboxy-2-(4-{2-[2-(2-ethoxyethoxy)ethoxy]ethoxy} phenyl)ethyl]-4,10-bis(carboxylatomethyl)-1,4,7,10-tetraazacyclododecan-1-yl}-3-hydroxypropanoate,
gadolinium-2-{4,10-bis(carboxylatomethyl)-7-[1-carboxypropyl]-1,4,7,10-tetraazacyclododecan-1-yl}-3-{4-[2-(2-ethoxyethoxy)ethoxy]phenyl}propanoate,
gadolinium 2,2',2"-{10-[2-(5-butoxypyridin-2-yl)-1-carboxyethyl]-1,4,7,10-tetraazacyclododecane-1,4,7-triyl}triacetate,
gadolinium-2-{4,10-bis(carboxylatomethyl)-7-[1-carboxy-2-methoxyethyl]-1,4,7,10-tetraazacyclododecan-1-yl}-3-[4-(2,2,3,3-tetrafluoropropoxy)phenyl]propanoate,

gadolinium 2,2',2"-(10-{1-carboxy-2-[4-(2,2,3,3-tetrafluoropropoxy) phenyl]ethyl}-1,4,7,10-tetraazacyclododecane-1,4,7-triyl)triacetate,
gadolinium 2,2',2"-(10-{(1R)-1-carboxy-2-[4-(2,2,2-trifluoroethoxy) phenyl]ethyl}-1,4,7,10-tetraazacyclododecane-1,4,7-triyl)triacetate,
gadolinium 2,2',2"-{10-[1-carboxy-2-(4-propoxyphenyl)ethyl]-1,4,7,10-tetraazacyclododecane-1,4,7-triyl}triacetate,
gadolinium (2R,2'R)-2,2'-{7-[(1S)-1-carboxy-4-{4-[2-(2-ethoxyethoxy)ethoxy]phenyl}butyl]-10-[(1S)-1-carboxylato-2-hydroxyethyl]-1,4,7,10-tetraazacyclododecane-1,4-diyl}bis(3-hydroxypropanoate) and
gadolinium (2S,2'S)-2,2'-{7-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecane-1,4-diyl}bis(3-hydroxypropanoate)
and stereoisomers, tautomers, N-oxides, hydrates or solvates thereof, or mixtures of same.

In accordance with another aspect, the present invention covers methods of preparing compounds of the present invention, said methods comprising the steps as described in the Experimental Section herein.

In accordance with a further aspect, the present invention covers intermediate compounds which are useful for the preparation of the compounds of general formula (I), and particularly for the preparation of the compounds of general formula (I) in the form of a complex with $Gd^{3+}$, *supra.*

Particularly, the invention covers intermediate compounds of general formula (II):

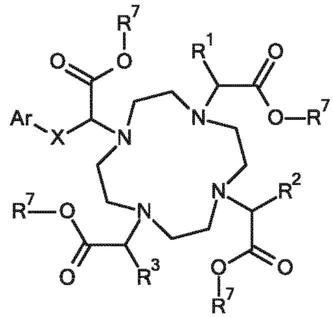

(II) ,

and stereoisomers, tautomers, N-oxides, hydrates, solvates, or salts thereof, or mixtures of same in which Ar, X, $R^1$, $R^2$ and $R^3$ are as defined for the compounds of general formula (I), *supra,* and $R^7$ represents a group selected from $C_1$-$C_4$-alkyl and benzyl.

Particularly, the invention covers intermediate compounds of general formula (III):

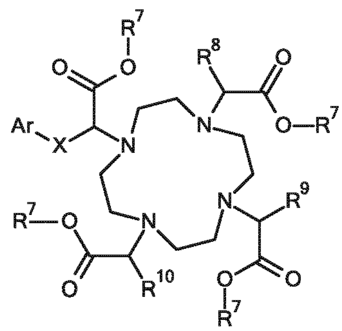

(III) ,

and stereoisomers, tautomers, N-oxides, hydrates, solvates, or salts thereof, or mixtures of same in which Ar and X are as defined for the compounds of general formula (I), *supra,* $R^7$ represents a group selected from $C_1$-$C_4$-alkyl and benzyl and $R^8$, $R^9$ and $R^{10}$ represent, independently for each occurrence, a group selected from $-CH_2O-C(CH_3)_3$, $-(CH_2)_2O-C(CH_3)_3$, $-CH_2O-CH_2Ph$ and $-((CH_2)_2O-CH_2)Ph$.

Particularly, the invention covers intermediate compounds of general formula (IV):

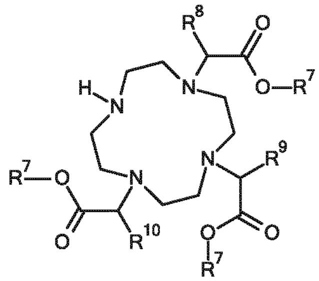

(IV) ,

and stereoisomers, tautomers, hydrates, solvates, or salts thereof, or mixtures of same in which $R^7$ represents a group selected from $C_1$-$C_4$-alkyl and benzyl and $R^8$, $R^9$ and $R^{10}$ represents, independently for each occurrence, a group selected from $-CH_2O-C(CH_3)_3$, $-(CH_2)_2O-C(CH_3)_3$, $-CH_2O-CH_2Ph$ and $-((CH_2)_2O-CH_2)Ph$.

Particularly, the invention covers intermediate compounds of general formula (IV):

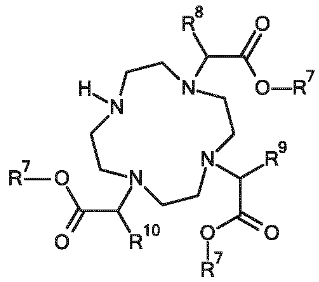

(IV) ,

and stereoisomers, tautomers, hydrates, solvates, or salts thereof, or mixtures of same in which $R^7$ represents a group selected from $C_1$-$C_4$-alkyl and benzyl and $R^8$, $R^9$ and $R^{10}$ represents, independently for each occurrence, a group selected from $C_1$-$C_3$-alkyl, $-CH_2O(CH_3)$, $-CH_2O-C(CH_3)_3$, $-(CH_2)_2O-C(CH_3)_3$, $-CH_2O-CH_2Ph$ and $-((CH_2)_2O-CH_2)Ph$.

In accordance with a further aspect, the present invention covers the use of said intermediate compounds as described above for the preparation of a compound of general formula (I) in the form of a complex with $Gd^{3+}$, as defined *supra.*

In accordance with a further aspect, the present invention covers the use of the compounds of general formula (I):

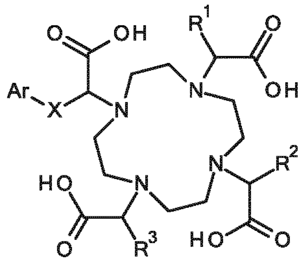

(I) ,

and stereoisomers, tautomers, N-oxides, hydrates, solvates, or salts thereof, or mixtures of same in which Ar, X, $R^1$, $R^2$ and $R^3$ are as defined for the compounds of general formula (I), *supra,* for the preparation of a compound of general formula (I) in the form of a complex with $Gd^{3+}$, as defined *supra.*

In accordance with a further aspect, the present invention covers the use of the intermediate compounds of general formula (II):

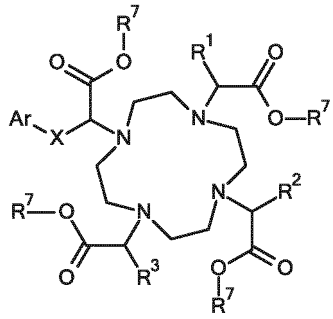

(II) ,

and stereoisomers, tautomers, N-oxides, hydrates, solvates, or salts thereof, or mixtures of same in which Ar, X, $R^1$, $R^2$ and $R^3$ are as defined for the compounds of general formula (I), *supra,* and $R^7$ represents a group selected from $C_1$-$C_4$-alkyl and benzyl, for the preparation of a compound of general formula (I) in the form of a complex with $Gd^{3+}$, as defined *supra.*

In accordance with a further aspect, the present invention covers the use of the intermediate compounds of general formula (III):

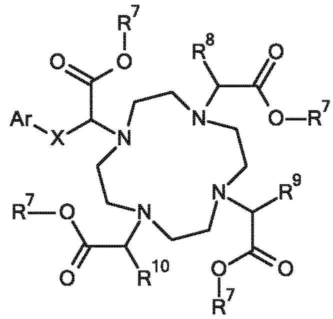

(III) ,

and stereoisomers, tautomers, N-oxides, hydrates, solvates, or salts thereof, or mixtures of same in which Ar and X are as defined for the compounds of general formula (I), *supra,* $R^7$ represents a group selected from $C_1$-$C_4$-alkyl and benzyl and $R^8$, $R^9$ and $R^{10}$ represents, independently for each occurrence, a group selected from $-CH_2O-C(CH_3)_3$, $-(CH_2)_2O-C(CH_3)_3$, $-CH_2O-CH_2Ph$ and $-((CH_2)_2O-CH_2)Ph$ for the preparation of a compound of general formula (I) in the form of a complex with $Gd^{3+}$, as defined *supra.*

In accordance with a further aspect, the present invention covers the use of the intermediate compounds of general formula (III):

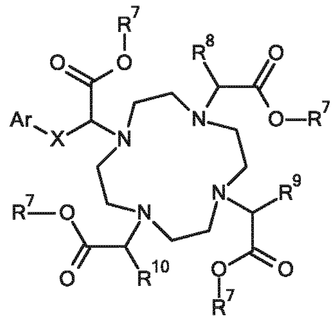

(III) ,

and stereoisomers, tautomers, N-oxides, hydrates, solvates, or salts thereof, or mixtures of same in which Ar and X are as defined for the compounds of general formula (I), *supra,* $R^7$ represents a group selected from $C_1$-$C_4$-alkyl and benzyl and $R^8$, $R^9$ and $R^{10}$ represents, independently for each occurrence, a group selected from $C_1$-$C_3$ alkyl, $-CH_2O(CH_3)$, $-CH_2O-C(CH_3)_3$, $-(CH_2)_2O-C(CH_3)_3$, $-CH_2O-CH_2Ph$ and $-((CH_2)_2O-CH_2)Ph$ for the preparation of a compound of general formula (I) in the form of a complex with $Gd^{3+}$, as defined *supra.*

In accordance with a further aspect, the present invention covers the use of the intermediate compounds of general formula (IV):

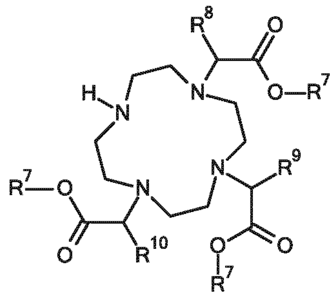

(IV) ,

and stereoisomers, tautomers, hydrates, solvates, or salts thereof, or mixtures of same in which $R^7$ represents a group selected from $C_1$-$C_4$-alkyl and benzyl and $R^8$, $R^9$ and $R^{10}$ represents, independently for each occurrence, a group selected from $-CH_2O-C(CH_3)_3$, $-(CH_2)_2O-C(CH_3)_3$, $-CH_2O-CH_2Ph$ and $-((CH_2)_2O-CH_2)Ph$ for the preparation of a compound of general formula (I) in the form of a complex with $Gd^{3+}$, as defined *supra.*

In accordance with a further aspect, the present invention covers the use of the intermediate compounds of general formula (IV):

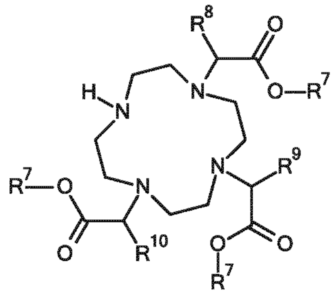

(IV) ,

and stereoisomers, tautomers, hydrates, solvates, or salts thereof, or mixtures of same in which $R^7$ represents a group selected from $C_1$-$C_4$-alkyl and benzyl and $R^8$, $R^9$ and $R^{10}$ represents, independently for each occurrence, a group selected from $C_1$-$C_3$ alkyl, $-CH_2O(CH_3)$, $-CH_2O-C(CH_3)_3$, $-(CH_2)_2O-C(CH_3)_3$, $-CH_2O-CH_2Ph$ and $-((CH_2)_2O-CH_2)Ph$ for the preparation of a compound of general formula (I) in the form of a complex with $Gd^{3+}$, as defined *supra.*

More particularly still, the present invention covers the intermediate compounds which are disclosed in the Experimental Section of this text, *infra.*

Compounds of general formula (I) in the form of a $Gd^{3+}$ complex, i.e. $Gd^{3+}$ complexes of the compounds of general formula (I) of the present invention demonstrate a valuable complex stability, solubility, uptake into hepatocytes, relaxivity and a valuable tolerability and pharmacokinetic profile, which could not have been predicted. $Gd^{3+}$-containing compounds of general formula (I) of the present invention have surprisingly been found to effectively taken up into hepatocytes and providing a high relaxivity while maintaining a pharmacokinetic, good safety and tolerability profile and it is possible therefore that said compounds be used for the contrast-enhanced MRI (CE-MRI), preferably multi-purpose MRI and liver MRI and more preferably for the detection of liver diseases like liver fibrosis, cirrhosis, metastases and differential diagnosis of focal liver lesions and functional imaging in humans and animals.

A further aspect of the invention is the use of a compound of general formula (I), *supra,* for diagnostic imaging.

A person skilled in the art would recognize that the compounds of general formula (I) of this invention can be used in combination with other MR-active metal ions instead of $Gd^{3+}$, such compounds also being encompassed by the present invention.

A further aspect of the invention is the use of a compound of general formula (I), *supra,* in the form of a complex with $Gd^{3+}$ for diagnostic imaging.

A further aspect of the invention is the use of a $Gd^{3+}$ complex of a compound of general formula (I), *supra,* for diagnostic imaging.

Preferably, the use of a compound of the invention, *i.e.* of a compound of general formula (I), *supra* and/or of a

compound of general formula (I), *supra,* in the form of a complex with $Gd^{3+}$ and/or of a $Gd^{3+}$ complex of a compound of general formula (I), *supra,* in the diagnosis is performed using magnetic resonance imaging (MRI).

A further aspect of the invention is the use of a compound of general formula (I), *supra,* for magnetic resonance imaging, preferably of the vascular, renal or of the hepatobiliary system or of the gastrointestinal tract, more preferably for magnetic resonance imaging of the liver.

A further aspect of the invention is the use of a compound of general formula (I), *supra,* in the form of a complex with $Gd^{3+}$ for magnetic resonance imaging, preferably of the vascular, renal or of the hepatobiliary system or of the gastrointestinal tract, more preferably for magnetic resonance imaging of the liver.

A further aspect of the invention is the use of a $Gd^{3+}$ complex of a compound of general formula (I), *supra,* for magnetic resonance imaging, preferably of the vascular, renal or of the hepatobiliary system or of the gastrointestinal tract, more preferably for magnetic resonance imaging of the liver.

A further aspect of the invention are compounds of general formula (I) for use in diagnostic imaging.

A further aspect of the invention are compounds of general formula (I) in the form of a complex with $Gd^{3+}$ for use in diagnostic imaging.

A further aspect of the invention are $Gd^{3+}$ complexes of compounds of general formula (I), *supra,* for use in diagnostic imaging.

A further aspect of the invention are compounds of general formula (I) for use in magnetic resonance imaging (MRI), preferably of the vascular, renal or of the hepatobiliary system or of the gastrointestinal tract, more preferably for magnetic resonance imaging of the liver.

A further aspect of the invention are compounds of general formula (I) in the form of a complex with $Gd^{3+}$ for use in magnetic resonance imaging (MRI), preferably of the vascular, renal or of the hepatobiliary system or of the gastrointestinal tract, more preferably for magnetic resonance imaging of the liver.

A further aspect of the invention are $Gd^{3+}$ complexes of compounds of general formula (I), *supra,* for use in magnetic resonance imaging (MRI), preferably of the vascular, renal or of the hepatobiliary system or of the gastrointestinal tract, more preferably for magnetic resonance imaging of the liver.

The invention also contains compounds of general formula (I) for the manufacture of diagnostic agents.

The invention also contains compounds of general formula (I) in the form of a complex with $Gd^{3+}$ for the manufacture of diagnostic agents.

A further aspect of the invention is the use of the compounds of general formula (I) or mixtures thereof for the manufacture of diagnostic agents.

A further aspect of the invention is the use of the compounds of general formula (I) in the form of a complex with $Gd^{3+}$ or mixtures thereof for the manufacture of diagnostic agents.

A further aspect of the invention is the use of the compounds of general formula (I) or mixtures thereof for the manufacture of diagnostic agents for magnetic resonance imaging (MRI).

A further aspect of the invention is the use of the compounds of general formula (I) in the form of a complex with $Gd^{3+}$ or mixtures thereof for the manufacture of diagnostic agents for magnetic resonance imaging (MRI).

A further aspect of the invention is the use of the compounds of general formula (I) or mixtures thereof for the manufacture of diagnostic agents for magnetic resonance imaging (MRI) of the vascular, vascular, renal or of the hepatobiliary system or of the gastrointestinal tract, preferably for magnetic resonance imaging of the liver.

A further aspect of the invention is the use of the compounds of general formula (I) in the form of a complex with $Gd^{3+}$ or mixtures thereof for the manufacture of diagnostic agents for magnetic resonance imaging (MRI) of the vascular, renal or of the hepatobiliary system or of the gastrointestinal tract, preferably for magnetic resonance imaging of the liver.

A further aspect of the invention is the use of $Gd^{3+}$ complexes of compounds of general formula (I), *supra,* or mixtures thereof for the manufacture of diagnostic agents for magnetic resonance imaging (MRI) of the vascular, renal or of the hepatobiliary system or of the gastrointestinal tract, preferably for magnetic resonance imaging of the liver.

A further aspect of the invention is a method of imaging body tissue in a patient, comprising the steps of administering to the patient an effective amount of one or more compounds of general formula (I) in the form of a complex with $Gd^{3+}$ in a pharmaceutically acceptable carrier, and subjecting the patient to NMR tomography.

A further aspect of the invention is a method of imaging body tissue in a patient, comprising the steps of administering to the patient an effective amount of one or more $Gd^{3+}$ complexes of the compounds of general formula (I) in a pharmaceutically acceptable carrier, and subjecting the patient to NMR tomography.

For the manufacture of diagnostic agents, for example the administration to human or animal subjects, the compounds of general formula (I) in the form of complexes with $Gd^{3+}$, i.e. $Gd^{3+}$ complexes of the compounds of general formula (I) or mixtures will conveniently be formulated together with pharmaceutical carriers or excipients. The contrast media of the invention may conveniently contain pharmaceutical formulation aids, for example stabilizers, antioxidants, pH-adjusting agents, metal scavengers, electrolytes (e.g. sodium chloride), flavors and the like. The diagnostic agents of the invention may be formulated for parenteral or enteral administration or for direct administration into body cavities. For example, parenteral formulations contain a sterile solution or suspension in a dose of 0.0001-5 mmol gadolinium/kg body

weight, preferably 0.001-0.5 mmol gadolinium/kg body weight, more preferably 0.005-0.1 mmol gadolinium/kg body weight of the compound of formula (I) according to this invention. Thus, the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

In a further aspect, the invention is directed to a method of diagnosing and health monitoring of patients. This method comprises a) administering to a human in need of such diagnosis a compound of the invention for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention, *i.e.* a compound of general formula (I) in the form of a complex with $Gd^{3+}$ for detecting the compound in the human as described above and herein, and b) measuring the signal arising from the administration of the compound to the human, preferably by magnetic resonance imaging (MRI).

In a further aspect, the invention is directed to a method of diagnosing and health monitoring of patients. This method comprises a) administering to a human in need of such diagnosis a compound of the invention, *i.e.* a $Gd^{3+}$ complex of a compound of general formula (I) for detecting the compound in the human as described above and herein, and b) measuring the signal arising from the administration of the compound to the human, preferably by magnetic resonance imaging (MRI).

## GENERAL SYNTHESIS

The compounds of general formula (I) described in this invention, including the compounds of general formula (I) in the form of a complex with $Gd^{3+}$ can be produced using the following general procedures depicted in Schemes 1 to 9 below. Unless otherwise specified, the groups $R^1$ to $R^{10}$ displayed therein have the meaning given in the description above. In case protective group chemistry is required for the introduction of one or more of the groups $R^1$, $R^2$ and/or $R^3$, the groups $R^8$, $R^9$ and/or $R^{10}$ can be employed. Thus, should no protective group chemistry be needed, the groups $R^8$, $R^9$ and $R^{10}$ equal the groups $R^1$, $R^2$ and $R^3$. In general, but not exclusively, the following groups are selected from $R^7$ = methyl, ethyl or tert-Butyl, $R^{11}$ = methyl, trifluoromethyl or para-nitrophenyl, and $R^{12}$ = benzyl.

For example, the compounds of general formula (I) described in this invention, including the compounds of general formula (I) in the form of a complex with $Gd^{3+}$, particularly when $R^1$ = $R^2$ = $R^3$ = hydrogen, can be prepared by the procedure depicted in Scheme 1.

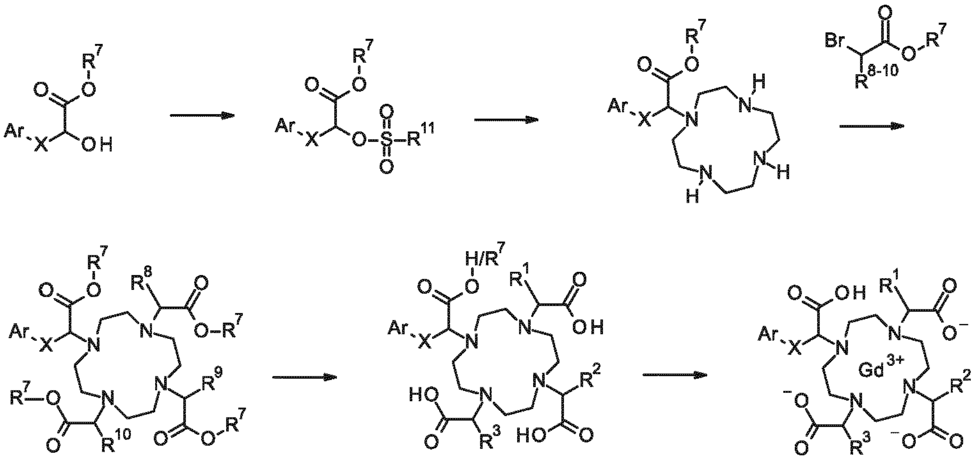

**Scheme 1.**

Alternatively, the compounds of general formula (I) described in this invention, including the compounds of general formula (I) in the form of a complex with $Gd^{3+}$, particularly when $R^1$ = $R^2$ = $R^3$ = hydrogen, can be prepared according to the procedure depicted in Scheme 2, where cyclen (1,4,7,10-tetraazacyclododecane) is first trialkylated followed by alkylation of the remaining secondary nitrogen.

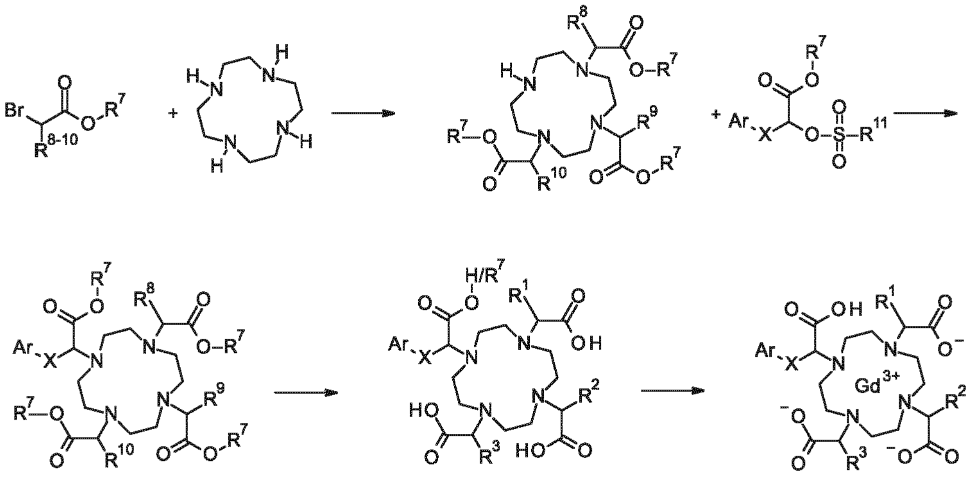

## Scheme 2.

Additionally, the compounds of general formula (I) described in this invention, including the compounds of general formula (I) in the form of a complex with $Gd^{3+}$, particularly when $R^1 = R^2 = R^3$ = hydrogen, can be prepared according to the procedure depicted in Scheme 3, where the acetic acid side chains can be introduced directly using a chloroacetic acid.

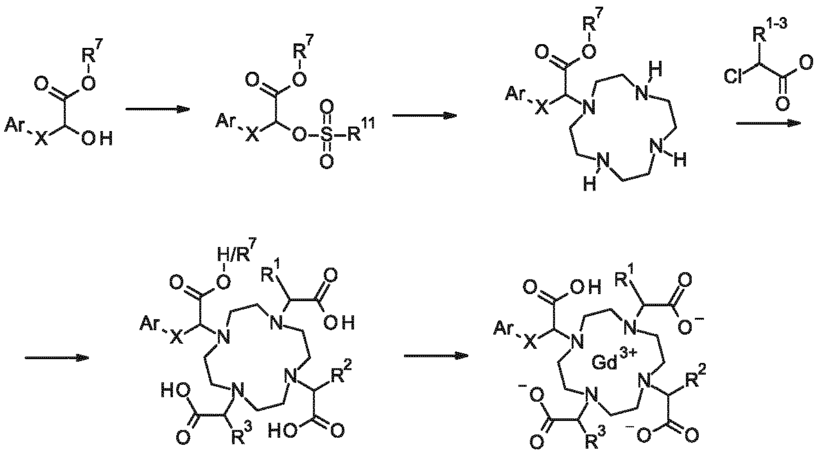

## Scheme 3

In general, Scheme 4 displays a route for the preparation of the compounds of general formula (I) described in this invention, including the compounds of general formula (I) in the form of a complex with $Gd^{3+}$. This route is particularly suited for the preparation of 1,7 disubstituted compounds, in particular when $R^2$ is different from $R^1$ and $R^3$ starting for example from the known 1,7-bis-tert-butyldiacetate cyclen, Cas No: [162148-48-3].

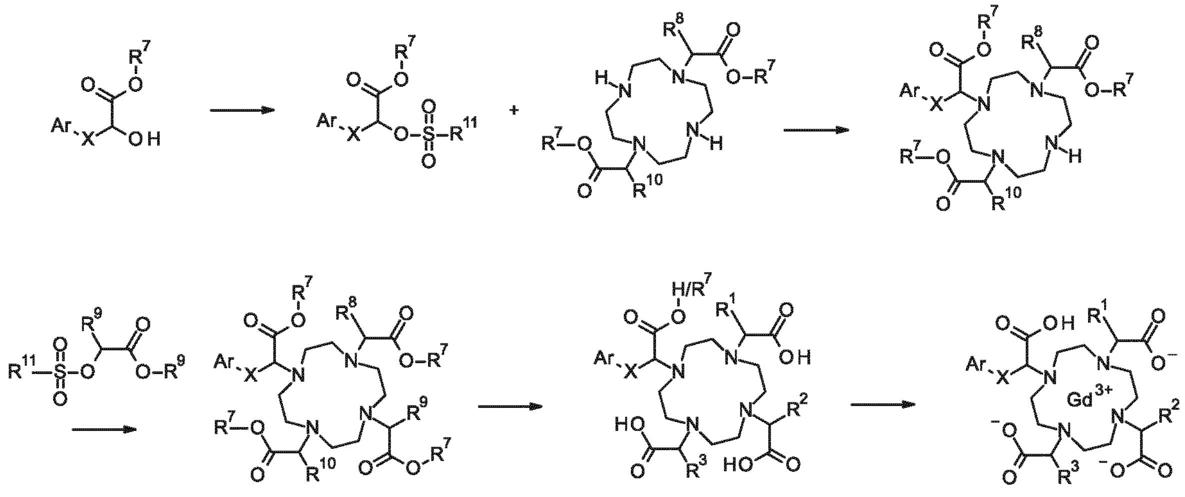

Scheme 4.

Alternatively, the sequence depicted in Scheme 5 may be employed for the preparation of the compounds of general formula (I) described in this invention, including the compounds of general formula (I) in the form of a complex with $Gd^{3+}$, particularly when $R^2$ is different from $R^1$ and $R^3$ starting from the known 1,7-bis-Cbz-protected cyclen, Cas No: [162148-45-0].

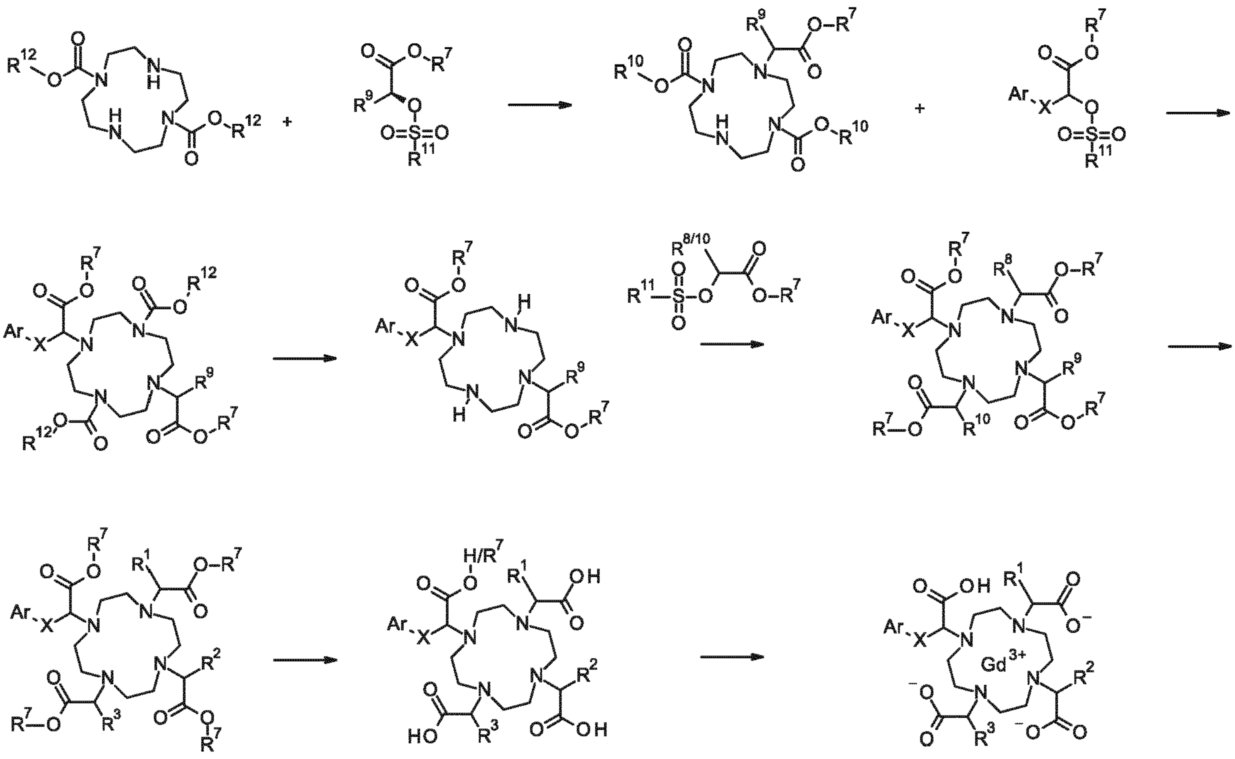

Scheme 5.

Alternatively, the sequence depicted in Scheme 6 may be employed for the preparation of the compounds of general formula (I) described in this invention, including the compounds of general formula (I) in the form of a complex with $Gd^{3+}$, particularly when $R^1$ = $R^2$ = $R^3$ = hydroxymethyl.

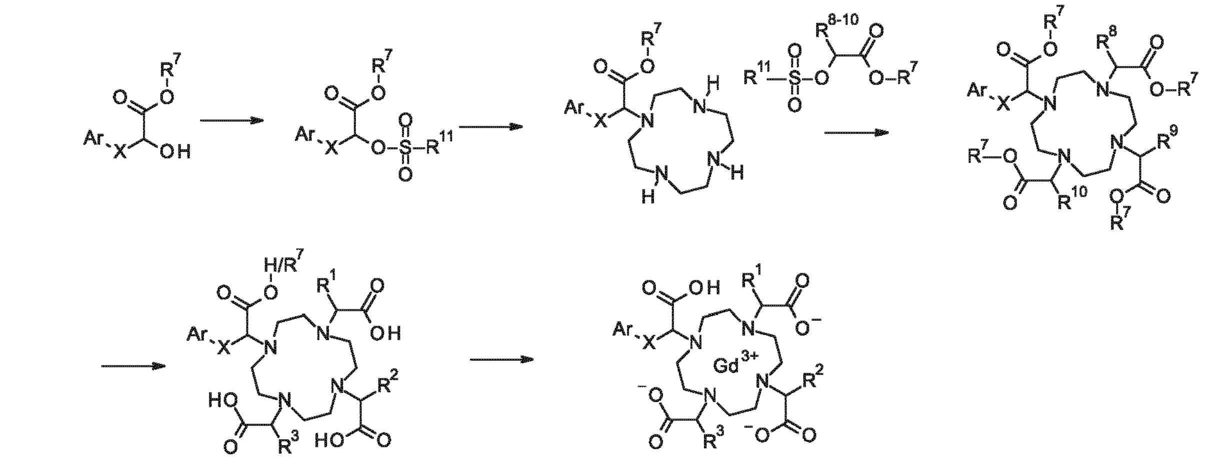

Scheme 6

Additionally, for the preparation of the compounds of general formula (I) described in this invention, including the compounds of general formula (I) in the form of a complex with $Gd^{3+}$, incorporation of an alkene side chain can be used as a point for further derivatisation as depicted in Scheme 7, particularly when $R^1 = R^2 = R^3$ = hydroxymethyl.

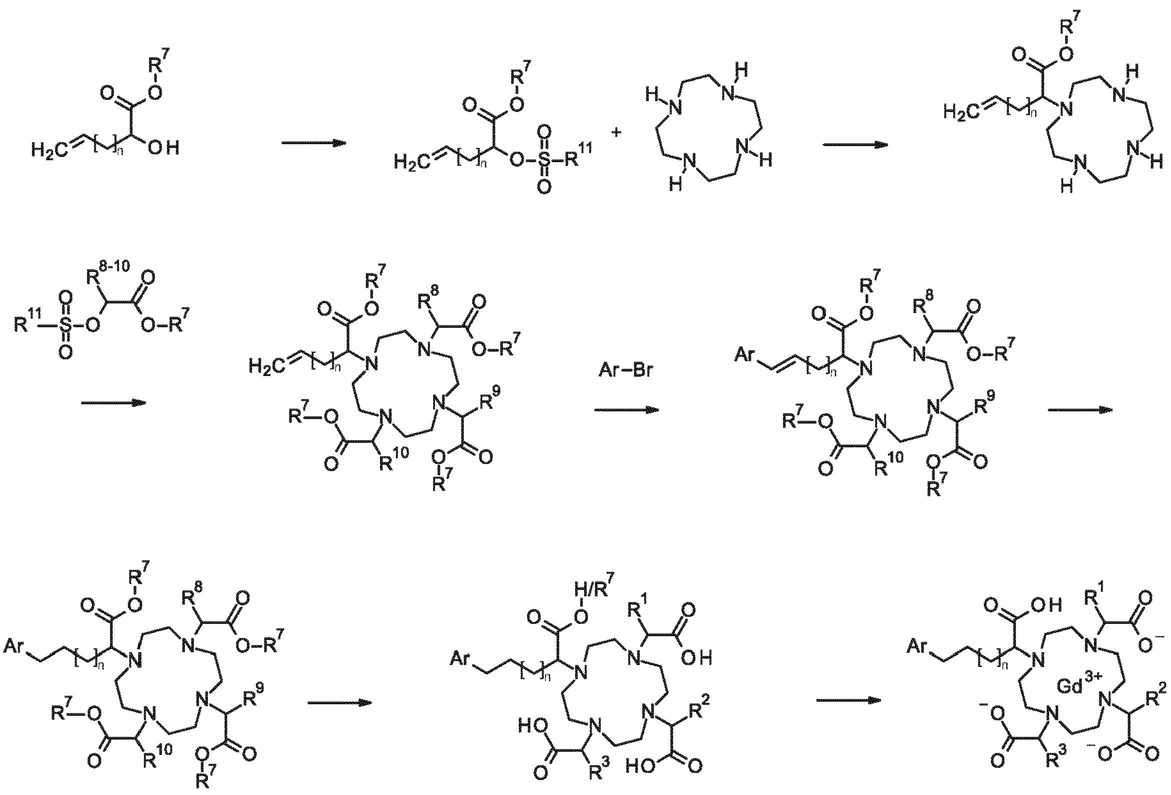


Scheme 7 (n = 0, 1, 2).

In general, the sequence depicted in Scheme 8 can be employed for the preparation of the compounds of general formula (I) described in this invention, including the compounds of general formula (I) in the form of a complex with $Gd^{3+}$, particularly when $R^1 = R^2 = R^3$ = hydroxyethyl.

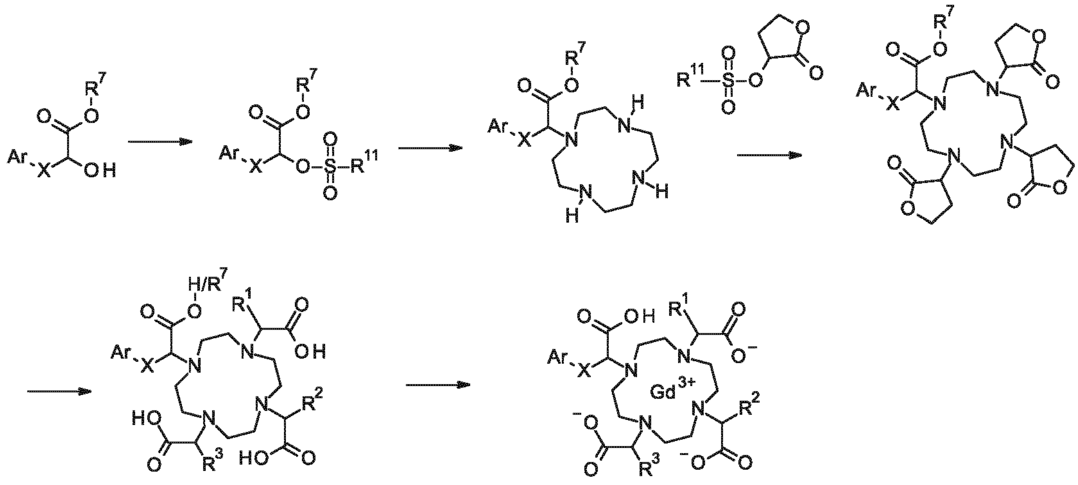

**Scheme 8.**

Finally, the sequence depicted in Scheme 9 may be used for the preparation of the compounds of general formula (I) described in this invention, including the compounds of general formula (I) in the form of a complex with $Gd^{3+}$, particularly when $R^1$ is desired to be different from $R^2$ and $R^3$, starting from the known 1,4-bis-benzyl-protected cyclen Cas No: [216101-03-0].

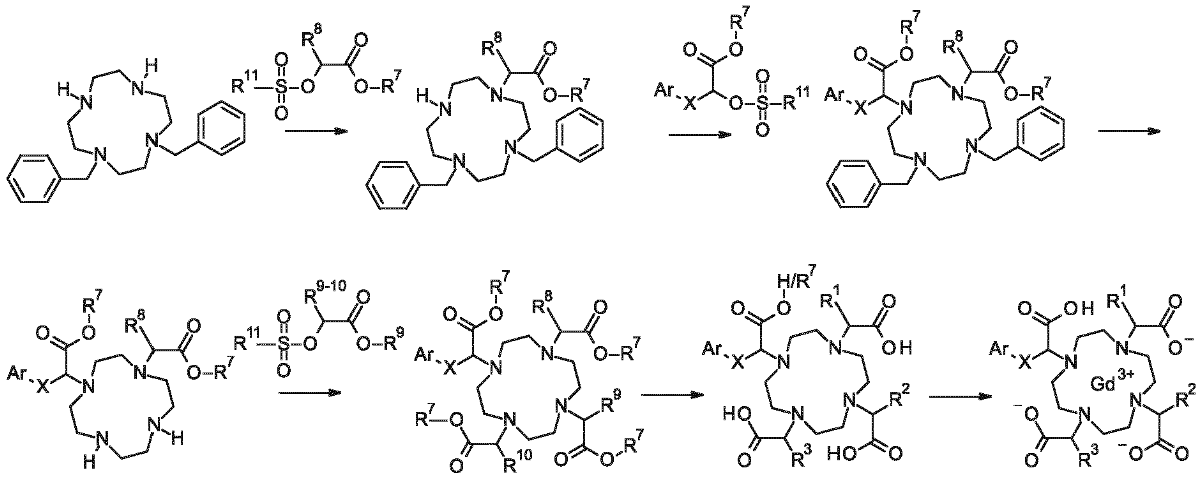

**Scheme 9.**

The schemes and procedures described above illustrate synthetic routes to the compounds of general formula (I) of the invention and are not intended to be limiting. It would be apparent to the person skilled in the art that the order of transformations as exemplified in the schemes can be modified in various ways. The order of transformations exemplified in the schemes is therefore not intended to be limiting. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

The contents of the documents which are cited herein are hereby incorporated by reference.

In accordance with an embodiment, the present invention also relates to a method of preparing a compound of general formula (I) in the form of a complex with $Gd^{3+}$, *i.e.* a $Gd^{3+}$ complex of a compound of general formula (I), as defined *supra,* said method comprising the step of allowing a compound of general formula (I):

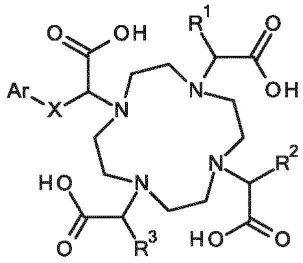

(I) ,

in which Ar, X, $R^1$, $R^2$ and $R^3$ are as defined for the compounds of general formula (I), *supra,* to react with a Gadolinium-(III)-salt,

thereby giving a compound of general formula (I), in which Ar, X, $R^1$, $R^2$ and $R^3$ are as defined for the compounds of general formula (I), *supra,* in the form of a complex with $Gd^{3+}$, *i.e.* a $Gd^{3+}$ complex of a compound of general formula (I).

In accordance with a further embodiment, the present invention also relates to a method of preparing a compound of general formula (I) in the form of a complex with $Gd^{3+}$, *i.e.* a $Gd^{3+}$ complex of a compound of general formula (I) as defined *supra,* said method comprising the step of allowing a compound of general formula (I):

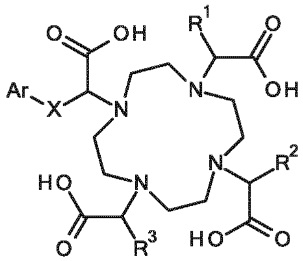

(I) ,

in which Ar, X, $R^1$, $R^2$ and $R^3$ are as defined for the compounds of general formula (I), *supra,* to react with a Gadolinium-(III)-salt, such as gadolinium oxide, gadolinium chloride, gadolinium acetate or gadolinium carbonate, thereby giving a compound of general formula (I), in which Ar, X, $R^1$, $R^2$ and $R^3$ are as defined for the compounds of general formula (I), *supra,* in the form of a complex with $Gd^{3+}$, *i.e.* a $Gd^{3+}$ complex of a compound of general formula (I).

In accordance with a further embodiment, the present invention also relates to a method of preparing a compound of general formula (I) in the form of a complex with $Gd^{3+}$, *i.e.* a $Gd^{3+}$ complex of a compound of general formula (I) as defined *supra,* said method comprising the step of allowing a compound of general formula (I):

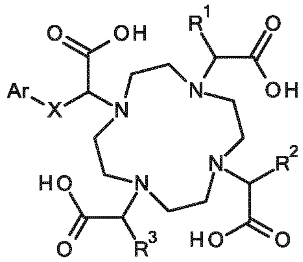

(I) ,

in which Ar, X, $R^1$, $R^2$ and $R^3$ are as defined for the compounds of general formula (I), *supra,* to react with gadolinium (III) oxide,

thereby giving a compound of general formula (I), in which Ar, X, $R^1$, $R^2$ and $R^3$ are as defined for the compounds of general formula (I), *supra,* in the form of a complex with $Gd^{3+}$, *i.e.* a $Gd^{3+}$ complex of a compound of general formula (I).

## DESCRIPTION OF THE FIGURES

**Figure 1** Chemical stability of selected Examples during heat sterilization. Normalized HPLC-ICP-MS (Gd157) signal over time before and after one-, two- and three-times autoclaving (1 bar, 121°C for 20 min). All compounds were investigated at 1 mmol/L concentration in 10 mM Tris-HCl buffer at pH 7.4.

**Figure 2** No-observed-adverse-effect level (NOAEL) in mice for exemplary compounds in relation to lipophilicity (partition coefficient log P (butanol/water, pH 7.4). The observed NOAEL (mmol Gd/kg bodyweight) denotes the level of exposure, at which no adverse effects were observed. NOAELs of reference compound 1 (RC1, Gd-EOB-DTPA) and 2 (RC2, Gd-BOPTA) are depicted at 2.5 mmol Gd/kg bw for illustration only (actually they are >2.5 mmol Gd/kg bw).

**Figure 3:** Contrast-enhanced liver MRI in healthy mice before and after contrast agent application (~10 min) compared to reference compound 1 (RC1, dose: 0.025 mmol/kg bw, Gd-EOB-DTPA) and reference compound 2 (RC2, dose: 0.050 mmol/kg bw Gd-BOPTA). The studies were performed at a 4.7T preclinical MRI scanner equipped with a dedicated transmit-receive mouse body volume coil using a T1-weighted FLASH (Fast Low Angle Shot) sequence with retrospective respiratory gating.

**Figure 4:** Contrast-Enhanced liver MRI in tumor-bearing rabbits before and 7 s, 5 min, 20 min and 40 min post contrast agent intravenous injection. Study was performed at 1.5T MR system using a T1 weighted fat-saturated 3D gradient echo sequence (Volumetric Interpolated Breath-bold Examination, VIBE) sequence covering the entire liver with 36 slices of 2 mm thickness. Immediately after detection of contrast enhancement on the pulmonary artery, 5 continuous axial dynamic scans were obtained (6s each) in which the 1st and 2nd phases corresponded to early and late arterial phases. The 7s images show high signal intensities within the aorta (white star). Exemplarily tumor slices (black arrow: VX2 tumor) are depicted as an intraindividual comparison (3-way crossover study) of Example 15 and reference compound 1 (RC1, dose: 0.025 mmol/kg bw, Gd-EOB-DTPA) and reference compound 2 (RC2, dose: 0.050 mmol/kg bw Gd-BOPTA).

**Figure 5:** Intraindividual comparison of biliary excretion of Example 15 and reference compound 1 (RC1, Gd-EOB-DTPA) in pig at 0.025 mmol Gd/kg bw. Study was performed in breath-hold at 1.5T MR clinical system using a gradient echo (VIBE) sequence (A) MRI of dynamic and late hepatobiliary phase in the liver after contrast agent application. (B). Exemplarily Maximum Intensity Projections (MIPS 2 cm) are depicted to visualize the elimination of the contrast agent into the bile (white arrow: Ductus choledochus).

## EXPERIMENTAL SECTION

| | |
|---|---|
| aq. | Aqueous |
| AUC | area under the curve |
| bw | body weight |
| CAIPIRINIA | Controlled Aliasing in Parallel Imaging Results in Higher Acceleration |
| calc. | calculated |
| $CDCl_3$ | chloroform-d |
| CGd | concentration of the compound normalized to the Gadolinium |
| $CHCl_3$ | Chloroform |
| Cltot | total clearance |
| Conc. | Concentrated |
| CPMG | Carr-Purcell-Meiboom-Gill (MRI sequence) |
| CV | Column volume(s) |
| d | day(s) |

(continued)

| | |
|---|---|
| $D_2O$ | deuterium oxide |
| DAD | diode array detection/detector |
| DCM | dichloromethane |
| DEA | diethylamine |
| DIPEA | N,N-diisopropylethylamine |
| DMSO | dimethylsulfoxide |
| DMSO-d6 | deuterated dimethylsulfoxide |
| e.e. | enantiomeric excess |
| ECCM | extracellular contrast media |
| EI | electron ionisation |
| ELSD | evaporative light scattering detection/detector |
| ESI | electrospray ionisation |
| EtOAc | ethyl acetate |
| EtOH | ethanol |
| FBS | fetal bovine serum |
| FLASH | Fast Low Angle Shot |
| $Gd_2O_3$ | Gadolinium oxide |
| h | hour(s) |
| $H_2$ | Hydrogen |
| $H_2SO_4$ | Sulfuric acid |
| HCC | hepatocellular carcinoma |
| HCl | Hydrochloric acid |
| HCOOH | formic acid |
| HPLC | high performance liquid chromatography |
| HU | Hounsfield units |
| ICP-MS | inductively coupled plasma mass spectrometry |
| IR | inversion recovery |
| IS | Internal standard |
| $K_2CO_3$ | potassium carbonate |
| $K_3PO_4$ | potassium phosphate |
| kDa | kilo Dalton |
| LCMS | liquid chromatography-mass spectroscopy |
| MeCN | acetonitrile |
| MeOH | methanol |
| min | minute(s) |
| MIPS | Maximum Intensity Projections (MRI Imaging Projection) |
| MRI | magnetic resonance imaging |
| MRT | mean residence time |
| MS | mass spectrometry |
| MsCl | Methanesulfonyl chloride |

(continued)

| | |
|---|---|
| MTBE | methyl-tert-butylether |
| $N_2$ | Nitrogen |
| $Na_2SO_4$ | sodium sulfate |
| NaCl | sodium chloride |
| $NaHCO_3$ | sodium bicarbonate |
| NaOH | sodium hydroxide |
| NMR | nuclear magnetic resonance spectroscopy: chemical shifts (δ) are given in ppm. (br = broad, d=doublet, t=triplet, q=quartet, quin=quintet, sxt= sextet, m=multiplet, mc=multiplet centrosymmetric). |
| PE | petroleum ether |
| RC | reference compound |
| Ri | (where i=1, 2) relaxation rates (1/T1,2) |
| ri | (where i=1, 2) relaxivities in L mmol-1 s-1 |
| Ri(0) | relaxation rate of the respective solvent |
| Rt | retention time |
| RT | room temperature |
| s | second(s) |
| sat. | saturated |
| $SiO_2$ | silicon dioxide |
| T | Tesla |
| t½ γ | plasma half-life, compartment V3 |
| t½ α | plasma half-life, compartment V1 |
| t½ β | plasma half-life, compartment V2 |
| T1,2 | relaxation time |
| TEA | triethylamine |
| TFA | trifluoroacetic acid |
| THF | tetrahydrofuran |
| TI | inversion time |
| TLC | thin layer chromatography |
| UPLC | ultra performance liquid chromatography |
| V1 + V2 | volume, compartments V1+V2 |
| VIBE | Volumetric Interpolated Breath-bold Examination |
| Vc (V1) | volume, central compartment V1 |
| Vd,ss | volume of distribution at steady state |
| [xxx-xx-x] | denotes Chemical Abstracts Registry Numbers |

**Materials and Instrumentation**

The chemicals used for the synthetic work were of reagent grade quality and were used as obtained.

All reagents, for which the synthesis is not described in the experimental section, are either commercially available, or are known compounds or may be formed from known compounds by known methods by a person skilled in the art.

$^1$H-NMR spectra were measured in $CDCl_3$, $D_2O$ or DMSO-$d_6$, respectively (room temperature, Bruker Avance

400 spectrometer, resonance frequency: 400.20 MHz for $^1$H or Bruker Avance 300 spectrometer, resonance frequency: 300.13 MHz for $^1$H. Chemical shifts are given in ppm relative to sodium (trimethylsilyl)propionate-$d_4$ ($D_2O$) or tetramethylsilane (DMSO-$d_6$) as external standards ($\delta$ = 0 ppm).

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartridges KP-Sil® or KP-NH® in combination with a Biotage autopurifier system (SP4® or Isolera Four®) and eluents such as gradients of hexane/ethyl acetate or DCM/methanol. Especially advantageous in some cases is the use of (preparative) reversed phase C18 columns in combination with acetonitrile/water or methanol/-water mixtures, which might be modified with acid or base as necessary. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

Compounds were analyzed and characterized by the following HPLC based analytical methods to determine characteristic retention time and mass spectrum:

## Example Compounds

**Method 1:** Instrument: Waters Acquity UPLCMS SingleQuad; Column: Acquity UPLC BEH C18 1.7 μm, 50x2.1mm; eluent A: water + 0.1 vol % formic acid (99%), eluent B: MeCN; gradient: 0-1.6min 1-99% B, 1.6-2.0min 99% B; flow 0.8 ml/min; temperature: 60°C; DAD scan: 210-400 nm.

**Method 2:** Instrument: Waters Acquity UPLCMS SingleQuad; Column: Acquity UPLC BEH C18 1.7 μm, 50x2.1mm; eluent A: water + 0.1 vol-% formic acid (99%), eluent B: MeCN; gradient: 0-1.7min 1-45% B, 1.7-1.72min 45-99% B, 1.72-2.0min 99% B; flow 0.8 ml/min; temperature: 60°C; ELSD.

**Method** 3: Instrument: Agilent 1290 UPLCMS 6230 TOF; column: BEH C 18 1.7 μm, 50x2.1mm; Eluent A: water + 0.05 % formic acid (99%); Eluent B: MeCN + 0.05 % formic acid (99%); gradient: 0-1.7 2-90% B, 1.7-2.0 90% B; flow 1.2 ml/min; temperature: 60°C; DAD scan: 190-400 nm.

**Method 4:** Instrument: Waters Acquity UPLCMS SingleQuad; Column: Acquity UPLC BEH C18 1.7 μm, 50x2.1mm; eluent A: water + 0.2 vol % aqueous ammonia (32%), eluent B: MeCN; gradient: 0-1.6min 1-99% B, 1.6-2.0min 99% B; flow 0.8 ml/min; temperature: 60°C; DAD scan: 210-400 nm.

**Method 5:** Instrument: Waters Acquity UPLCMS SingleQuad; Column: Acquity UPLC BEH C18 1.7 μm, 50x2.1mm; eluent A: water + 0.1 vol-% formic acid (99%), eluent B: MeCN; gradient: 0-1.7min 1-45% B, 1.7-1.72min 45-99% B, 1.72-2.0min 99% B; flow 0.8 ml/min; temperature: 60°C; ELSD.

**Method 6:** Instrument: Waters Acquity UPLCMS SingleQuad; Column: Acquity UPLC BEH C18 1.7 μm, 50x2.1mm; eluent A: water + 0.2 vol-% aqueous ammonia (32%), eluent B: MeCN; gradient: 0-1.7min 1-45% B, 1.7-1.72min 45-99% B, 1.72-2.0min 99% B; flow 0.8 ml/min; temperature: 60°C; ELSD.

**Method 7:** HPLC instrument type: SHIMADZU LC-20AD; column: Kinetex C18 LC Column 4.6X50mm,5um; mobile phase A: 0.0375% TFA in water (v/v), B: 0.01875% TFA in MeCN (v/v); gradient: 0.0min 0% B→ 4.2min 60% B→ 5.3min 60% B→ 5.31min 0% B→ 6.0min 0% B; flow rate: 1.5 mL/min; oven temperature: 50°C; UV detection: 220 nm & 254 nm & 215 nm.

**Method 8:** MS instrument type: SHIMADZU LCMS-2020; Kinetex EVO C18 2.1X30mm,5um; mobile phase A: 0.0375% TFA in water (v/v), B: 0.01875% TFA in MeCN (v/v); gradient: 0.0min 5% B→ 0.8min 95% B→ 1.2min 95% B→ 1.21min 5% B→ 1.55min 5% B; flow rate: 1.5 mL/min; oven temperature: 50°C; UV detection: 220 nm & 254 nm.

**Method 9:** MS instrument type: SHIMADZU LCMS-2020; column: Kinetex EVO C18 2.1X30mm,5um; mobile phase A: 0.025% $NH_3 \cdot H_2O$ in water (v/v) , B: MeCN; gradient: 0.0min 5% B→ 0.8min 95% B→ 1.2min 95% B→ 1.21min 5% B→ 1.55min 5% B; flow rate: 1.5 mL/min; oven temperature: 40°C; UV detection: 220 nm & 254 nm.

**Method 10:** MS instrument type: SHIMADZU LC-20AB; column: Kinetex EVO C18 2.1X30mm,5um; mobile phase A: 0.0375% TFA in water (v/v), B: 0.01875% TFA in MeCN (v/v); gradient: 0.0min 5% B→ 0.8min 95% B→ 1.20min 95% B→ 1.21min 5% B→ 1.55min 5% B; flow rate: 1.5 mL/min; oven temperature: 50°C; UV detection: 220 nm & 254 nm.

**Method 11:** MS instrument type: SHIMADZU LCMS-2020; Kinetex EVO C18 2.1X30mm,5um; mobile phase A: 0.0375% TFA in water (v/v), B: 0.01875% TFA in MeCN (v/v); gradient: 0.0min 5% B→ 0.8min 95% B→ 1.2min 95% B→ 1.21min 5% B→ 1.55min 5% B; flow rate: 1.5 mL/min; oven temperature: 50°C; UV detection: 220 nm & 254 nm.

**Method 12:** MS instrument type: SHIMADZU LCMS-2020; column: Kinetex EVO C18 2.1X30mm,5um; mobile phase A: 0.025% NH3·H2O in water (v/v) , B: MeCN; gradient: 0.0min 5% B→ 0.8min 95% B→ 1.2min 95% B→ 1.21min 5% B→ 1.5min 5% B; flow rate: 1.5 mL/min; oven temperature: 40°C; UV detection: 220 nm & 254 nm.

**Method 13:** Instrument: SHIMADZU LCMS-2020 SingleQuad; Column: Chromolith@Flash RP-18E 25-2 MM; eluent A: water + 0.0375 vol % TFA, eluent B: MeCN + 0.01875 vol % TFA; gradient: 0-0.8min, 5-95% B, 0.8-1.2min 95% B; flow 1.5 ml/min; temperature: 50°C; DAD: 220 nm & 254 nm.

**Intermediate 1**

methyl 3-[2-(4-ethoxyphenyl)ethoxy]-2-hydroxypropanoate

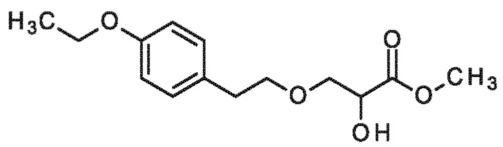

2-(4-Ethoxyphenyl)ethan-1-ol (2.99 g, 18.0 mmol, [702-23-8]) was added to a 5 ml reaction flask, methyl oxirane-2-carboxylate (1.8 ml, 22 mmol; [4538-50-5]) was then added, followed by magnesium perchlorate (1.11 g, 4.99 mmol; [10034-81-8]). The flask was sealed and stirred for 4d at 50°C, following which DCM was added, and the mixture washed twice with NaCl (sat. aq.), the organic phase was dried over $Na_2SO_4$, the mixture filtered and concentrated under reduced pressure, yielding the title compound as an oil (Intermediate 1, 4.11 g, 85 % yield).
LC-MS (Method 2): Rt = 1.01min; MS (ESIpos): m/z = 286 $[M+H]^+$.
$^1H$ NMR (400 MHz, DMSO-d6) δ ppm 1.30 (t, J=6.97 Hz, 3H), 2.70 (t, J=6.97 Hz, 2H), 3.51 - 3.64 (m, 7H), 3.97 (q, J=6.84 Hz, 3H), 4.14 - 4.25 (m, 1H), 5.54 (d, J=6.08 Hz, 1H), 6.76 - 6.87 (m, 2H), 7.02 - 7.15 (m, 2 H).

**Intermediate 2**

**Step 1**

tert-butyl (2SR,3RS)-3-{4-[2-(2-ethoxyethoxy)ethoxy]phenyl}oxirane-2-carboxylate

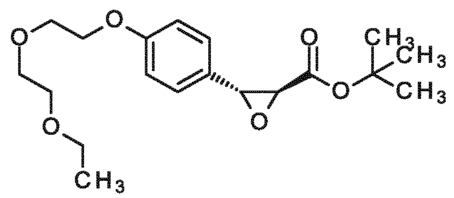

In a three-necked flask equipped with mechanical stirred and thermometer 4-[2-(2-ethoxy-ethoxy)ethoxy] benzaldehyde (30.0 g, 126 mmol, [117420-31-2]) was dissolved in THF (670 ml). The mixture was cooled to 0°C and sodium hydride (6.55 g, 60 % in mineral oil, 164 mmol) were added. *(attention: strong foaming occurred!).* After 10min, drop-wise addition of tert-butyl chloro-acetate (24 ml, 97 % purity, 160 mmol; [107-59-5]) in THF (60 ml) was started. After the addition was complete, the mixture was allowed to warm to RT and stirred overnight. The reaction was quenched by careful addition of water and extracted with EtOAc. The organic phase was dried and concentrated under reduced pressure to give 50.4 g (>100%, containing mineral oil) of the title compound as orange oil. The material was used as such in the next step.
LC-MS (Method 1): Rt = 1.35min; MS (ESIpos): m/z = 353.4 $[M+H]^+$.
A sample from an earlier experiment was characterized:
$^1H$ NMR (DMSO-$d_6$, 400 MHz): δ (ppm) 7.25-7.31 (m, 2H), 6.90-6.97 (m, 2H), 4.06-4.11 (m, 2H), 4.02 (d, *J* = 2.0 Hz, 1H), 3.70-3.75 (m, 2H), 3.67 (d, *J* = 2.0 Hz, 1H), 3.54-3.59 (m, 2H), 3.47-3.51 (m, 2H), 3.42 (q, *J* = 7.1 Hz, 2H), 1.46 (s, 8H), 1.09 (t, *J* = 7.0 Hz, 3H).

**Step 2**

tert-butyl-3-{4-[2-(2-ethoxyethoxy)ethoxy]phenyl}-2-hydroxypropanoate

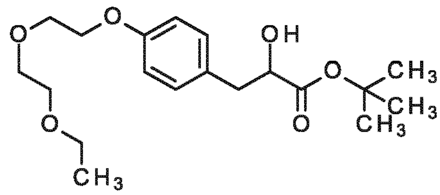

tert-butyl (2SR,3RS)-3-{4-[2-(2-ethoxyethoxy)ethoxy]phenyl}oxirane-2-carboxylate (50.4 g, 143 mmol) was dissolved in EtOAc (1000 ml) and palladium (6.01 g, 10 % on C, 5.65 mmol; [7440-05-3]) was added. The mixture was hydrogenated under RT and ambient pressure for 4h, after which 3.06 l $H_2$ (0.96 eq) had been consumed. The solvent was removed under reduced pressure, the residue taken up in the minimal amount of DCM and subjected to chromatography ($SiO_2$, 750 g SNAP-ULTRA, 250 ml/min, 254 nm, A = n-hexane, B = EtOAc, 0%B 20 CV, 0% to 50%B in 8CV, 50%B 1.7CV, 50% to100%B in 1CV, 100%B 2.5CV) to give the title compound **Intermediate 2,** (32.9 g, 65% yield) as a light yellow oil.

LC-MS (Method 1): Rt = 1.24min; MS (ESIpos): m/z = 372.5 $[M+NH_4]^+$.

$^1$H NMR ($CDCl_3$, 400 MHz): δ (ppm) 7.13-7.18 (m, 2H), 6.82-6.87 (m, 2H), 4.28 (td, *J* = 6.1, 4.8 Hz, 1H), 4.09-4.14 (m, 2H), 3.83-3.89 (m, 2H), 3.70-3.74 (m, 2H), 3.60-3.64 (m, 2H), 3.54 (q, *J* = 7.1 Hz, 2H), 3.02 (dd, *J* = 14.2, 4.8 Hz, 1H), 2.88 (dd, *J* = 14.2, 6.3 Hz, 1H), 2.82 (d, *J* = 5.8 Hz, 1H), 1.44 (s, 9H), 1.22 (t, *J* = 7.0 Hz, 3H).

The preparation was repeated several times in the same manner with similar results.

39.0 g (dissolved in 185 ml $CHCl_3$) of racemic tert-butyl 3-{4-[2-(2-ethoxyethoxy)ethoxy]phenyl}-2-hydroxypropanoate, **Intermediate 2,** was subjected to chromatography (37 x 5 ml injection) on a chiral phase (PrepCon Labomatic HPLC-1; Chiralpak IG 5μ, 250x50; A = MTBE; 100%A; 100 mL/min; 25°C; 280 nm), yielding **Enantiomer 1** and **Enantiomer 2.**

**Intermediate 3**

tert-butyl (2R)-3-{4-[2-(2-ethoxyethoxy)ethoxy]phenyl}-2-hydroxypropanoate

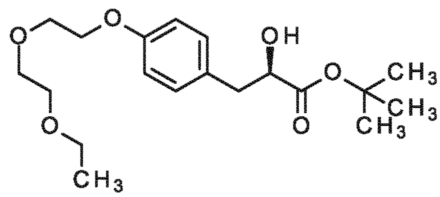

Enantiomer 1 **(Intermediate 2 - Step 2):** 18.2 g (47%), tert-butyl (2R)-3-{4-[2-(2-ethoxyethoxy)ethoxy]phenyl}-2-hydroxypropanoate **(Intermediate 3).**

LCMS: (Chiralpak IG 3μ, 100x4.6; MTBE + 0.1 vol % DEA; 1.4 ml/min; 25°C; 280 nm): Rt = 2.20min (98.5%). e.e. >99%.

Specific Rotation: $\alpha_D^{20}$ = +8.38° +/-0.28° (c=1, MeOH).

$^1$H NMR ($CDCl_3$, 400 MHz): δ (ppm) 7.12-7.17 (m, 2H), 6.82-6.86 (m, 2H), 4.27 (td, J= 6.1, 4.8 Hz, 1H), 4.09-4.13 (m, 2H), 3.82-3.87 (m, 2H), 3.70-3.74 (m, 2H), 3.59-3.63 (m, 2H), 3.54 (q, *J* = 6.9 Hz, 2H), 3.02 (dd, *J* = 14.1, 4.7 Hz, 1H), 2.87 (dd, *J* = 13.9, 6.3 Hz, 1H), 2.80 (d, *J* = 5.8 Hz, 1H), 1.44 (s, 9H), 1.21 (t, *J* = 7.1 Hz, 3H).

In comparison to many literature examples, the enantiomer with positive optical rotation was assigned the R-stereochemistry.

**Intermediate 4**

tert-butyl (2S)-3-{4-[2-(2-ethoxyethoxy)ethoxy]phenyl}-2-hydroxypropanoate

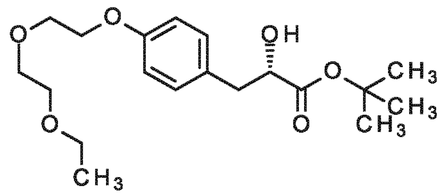

Enantiomer 2 **(Intermediate** 2 - **Step** 2): 16.9 g (43%), tert-butyl (2S)-3-{4-[2-(2-ethoxyethoxy)ethoxy]phenyl}-2-hydroxypropanoate **(Intermediate** 4).

LCMS (Chiralpak IG 3μ, 100x4.6; MTBE + 0.1 vol % DEA; 1.4 ml/min; 25°C; 280 nm): Rt = 3.86min (98.8%). e.e. = 99%.

Specific Rotation: $\alpha_D^{20}$ = -9.08° +/-0.11° (c=1, MeOH).

$^1$H NMR ($CDCl_3$, 400 MHz): δ (ppm) 7.13-7.18 (m, 2H), 6.83-6.87 (m, 2H), 4.28 (td, J= 6.1, 4.8 Hz, 1H), 4.10-4.14 (m, 2H), 3.83-3.89 (m, 2H), 3.70-3.74 (m, 2H), 3.60-3.64 (m, 2H), 3.55 (q, *J* = 7.0 Hz, 2H), 3.03 (dd, *J* = 14.2, 4.8 Hz, 1H), 2.88 (dd, *J* = 14.2, 6.3 Hz, 1H), 2.82 (d, *J* = 5.8 Hz, 1H), 1.45 (s, 9H), 1.22 (t, *J* = 7.0 Hz, 3H).

In comparison to many literature examples, the enantiomer with negative optical rotation was assigned the S-stereochemistry.

**Intermediate 5**

**Step 1**

ethyl (2SR,3RS)-3-{4-[2-(2-ethoxyethoxy)ethoxy]phenyl}oxirane-2-carboxylate

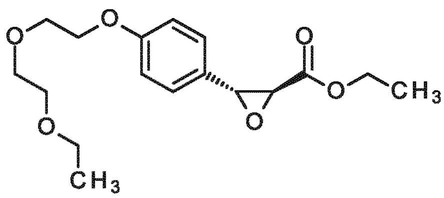

Sodium hydride (2.4 g, 60 % purity, 60 mmol; [7646-69-7]),) in THF (200 ml), was cooled to 0°C and ethyl chloroacetate (6.4 ml, 60 mmol, [105-36-2]) added dropwise under $N_2$. 4-[2-(2-Ethoxyethoxy)ethoxy]benzaldehyde (9.55 g, 40.1 mmol, [117420-31-2]) dissolved in THF (60 ml) was added and the reaction allowed to warm to RT overnight. The mixture was then added to an ice-water mixture, and the aqueous phase extracted three times with EtOAc, the organic phase washed with water and dried over $Na_2SO_4$. The product was purified using chromatography ($SiO_2$, Biotage 100g SNAP Ultra cartridge, with gradient elution (A = hexane, B = EtOAc, 0%B to 50%, over 20CV) yielding the title racemic compound (4 g, 30% yield).

LC-MS (Method 3): Rt = 1.06min; MS (ESIpos): m/z = 325.2 $[M+H]^+$.

$^1$H NMR (DMSO-$d_6$, 400 MHz): δ (ppm) 7.27-7.31 (m, 2H), 6.92-6.97 (m, 2H), 4.16-4.22 (m, 2H), 4.07-4.12 (m, 3H), 3.81 (d, *J* = 2.0 Hz, 1H), 3.69-3.76 (m, 2H), 3.54-3.59 (m, 2H), 3.47-3.50 (m, 2H), 3.42 (q, *J* = 6.8 Hz, 2H), 1.24 (t, *J* = 7.1 Hz, 3H), 1.09 (t, *J* = 7.0 Hz, 3H).

**Step 2**

ethyl-3-{4-[2-(2-ethoxyethoxy)ethoxy]phenyl}-2-hydroxypropanoate

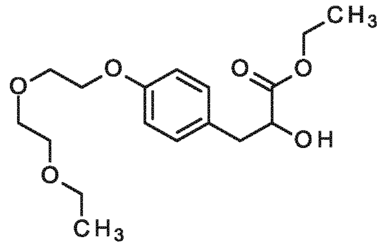

Ethyl-3-{4-[2-(2-ethoxyethoxy)ethoxy]phenyl}oxirane-2-carboxylate (4.07 g, 12.5 mmol) was dissolved in EtOH (100 ml), Pd/C (500 mg, 10 % purity, 470 μmol; [7440-05-3]), was added, and the mixture cycled between vacuum and $N_2$ three times. The mixture was then hydrogenated using 1 atm of $H_2$. After 4h, additional Pd/C (300 mg, 10 % purity, 282 μmol) was added and the mixture again stirred under $H_2$ overnight. The mixture was then filtered through a glass fiber filter and concentrated under reduced pressure. The product was purified by chromatography ($SiO_2$, Biotage, 50g SNAP Ultra cartridge, A = Hexane, B = EtOAc, 0%B to 100%B), yielding the title compound **(Intermediate 5,** 2.44 g, 60% yield).
LC-MS (Method 4): Rt = 1.02min; MS (ESIpos): m/z = 327.3 $[M+H]^+$.
$^1H$ NMR (400 MHz, DMSO-$d_6$) δ ppm 1.09 (t, J=6.97 Hz, 3H), 1.13 (t, J=7.10 Hz, 3H), 2.72 - 2.79 (m, 1H), 2.82 - 2.88 (m, 1H), 3.43 (q, J=7.01 Hz, 2H), 3.47 - 3.50 (m, 2H), 3.55 - 3.58 (m, 2H), 3.68 - 3.73 (m, 2H), 4.00 - 4.08 (m, 3H), 5.48 (d, J=6.08 Hz, 1H), 6.80 - 6.85 (m, 2H), 7.08 - 7.13 (m, 2 H).

Intermediate 6

Step 1

4-(2-ethoxyethoxy)benzaldehyde

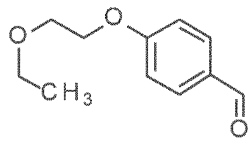

4-Fluorobenzaldehyde (5.00 g, 40.3 mmol; [459-57-4]), 2-ethoxyethan-1-ol (12 ml, 120 mmol; [110-80-5]) and cesium carbonate (15.8 g, 48.3 mmol; [534-17-8]) were added to a reaction flask containing DMF (100 ml), and the mixture was stirred at 70°C until the 4-fluorobenzaldehyde starting material was consumed (by monitoring with LCMS). Water was added, and the aqueous phase extracted with MTBE, the organic phase was washed with NaCl (sat. aq.), dried over $Na_2SO_4$, filtered and the solvent removed under reduced pressure. N-Heptane was added to the crude product, following which it was removed under reduced pressure, followed by chromatography ($SiO_2$, Biotage 100 g Ultra column, A = Hexane, B = EtOAc, 0%B to 50%B) yielding the title compound as a pale yellow crystalline solid (6.83 g, 83 % yield).
LC-MS (Method 4): Rt = 0.95min; MS (ESIpos): m/z = 195 $[M+H]^+$.
$^1H$ NMR (DMSO-$d_6$, 400 MHz): δ (ppm) 9.87 (s, 1H), 7.83-7.88 (m, 2H), 7.11-7.17 (m, 2H), 4.19-4.23 (m, 2H), 3.70-3.74 (m, 2H), 3.50 (q, J = 7.1 Hz, 2H), 1.12 (t, J = 7.1 Hz, 3H).

**Step 2**

ethyl-3-[4-(2-ethoxyethoxy)phenyl]oxirane-2-carboxylate

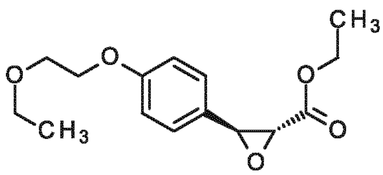

Sodium hydride (2.1 g, 60% in mineral oil, 52 mmol), was added to THF (200 ml), and ethyl chloroacetate (5.6 ml,

53 mmol) added dropwise under $N_2$. 4-(2-ethoxyethoxy)benzaldehyde (6.83 g, 35.2 mmol) was then added in THF (30 ml) and the reaction was allowed to warm to RT and stirred for 3d. The mixture was then added to an ice water mixture, and the aqueous layer extracted three times with EtOAc. The product was then purified by chromatography ($SiO_2$, Biotage, Ultra 100g column, A = Hexane, B = EtOAc, 0%B to 50%B), yielding the racemic title compound (6.60 g, 64 % yield).

LC-MS (Method 4): Rt = 1.14min; MS (ESIpos): m/z = 281.3 $[M+H]^+$.

$^1H$ NMR (DMSO-$d_6$, 400 MHz): δ (ppm) 7.27-7.31 (m, 2H), 6.92-6.97 (m, 2H), 4.15-4.24 (m, 2H), 4.06-4.10 (m, 3H), 3.81 (d, *J* = 2.0 Hz, 1H), 3.65-3.71 (m, 2H), 3.49 (q, *J* = 7.0 Hz, 2H), 1.24 (t, *J* = 7.1 Hz, 3H), 1.12 (t, *J* = 7.1 Hz, 3H).

**Step 3**

ethyl 3-[4-(2-ethoxyethoxy)phenyl]-2-hydroxypropanoate

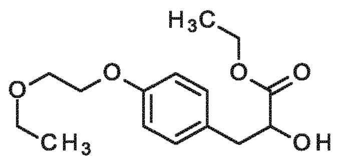

ethyl-3-[4-(2-ethoxyethoxy)phenyl]oxirane-2-carboxylate (6.60 g, 23.5 mmol) was dissolved in ethanol (110 ml), palladium (607 mg, 10 % on carbon, 571 μmol) added and the mixture hydrogenated with 1 atm of $H_2$ overnight. The mixture was then filtered through a glass fiber filter and concentrated to dryness under reduced pressure. Purification by chromatography ($SiO_2$, Biotage Ultra: 50g column, A = Hexane, B = EtOAc, 0%B to 50%B) yielded the title compound, **Intermediate 6,** as a racemate (5.39 g, 77 % yield).

$^1H$ NMR (400 MHz, DMSO-$d_6$) δ ppm 0.94 - 1.23 (m, 7H), 2.72 - 2.88 (m, 2H), 3.46 - 3.52 (m, 2H), 3.64 - 3.70 (m, 2H), 3.95 - 4.18 (m, 5H), 5.48 (d, J=6.08 Hz, 1H), 6.81 - 6.91 (m, 2H), 7.08 - 7.13 (m, 2H).

**Intermediate 7**

**Step 1**

4-{2-[2-(2-ethoxyethoxy)ethoxy]ethoxy}benzaldehyde

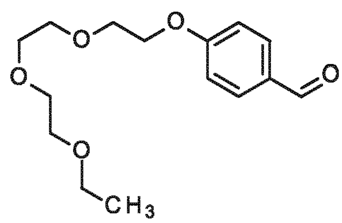

4-Fluorobenzaldehyde (9.2 ml, 85 mmol; [459-57-4]), 2-[2-(2-ethoxyethoxy)ethoxy]ethan-1-ol (24 ml, 140 mmol, [111-90-0]), and cesium carbonate (33.4 g, 102 mmol; [534-17-8]) were heated at 70°C overnight. The mixture was then added to water and the aqueous layer extracted three times with MTBE, the combined organic layers then washed with water and dried over $Na_2SO_4$, yielding the title compound 20.7 g (86 % yield).

LC-MS (Method 6): Rt = 0.99min; MS (ESIpos): m/z = 283.2 $[M+H]^+$.

$^1H$ NMR (400 MHz, DMSO-$d_6$) δ ppm 1.08 (t, J=7.10 Hz, 3H), 3.38 - 3.60 (m, 11H), 3.72 - 3.83 (m, 2H), 4.17 - 4.26 (m, 2H), 7.11 - 7.18 (m, 2H), 7.83 - 7.89 (m, 2H), 9.87 (s, 1 H).

Step 2

ethyl-3-(4-{2-[2-(2-ethoxyethoxy)ethoxy]ethoxy}phenyl)oxirane-2-carboxylate

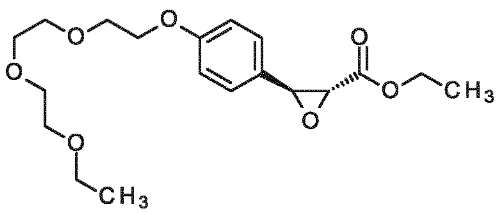

Sodium hydride (2.4 g, 60% in mineral oil, 60 mmol) was added to THF (270 ml), the mixture cooled to 0°C and ethyl chloroacetate (6.3 ml, 60 mmol) added dropwise. The solution was warmed to 15°C and 4-{2-[2-(2-ethoxyethoxy) ethoxy]ethoxy}benzaldehyde (11.3 g, 40.0 mmol) was added in a small quantity of THF dropwise. The mixture was allowed to warm to RT and stirred overnight, then it was added to an ice/water mixture, and the aqueous phase extracted three times with MTBE, the organic phase washed with NaCl (sat. aq.), dried over $Na_2SO_4$, filtered and evaporated to dryness. Purification via chromatography ($SiO_2$, Biotage Ultra 100g column, A = Hexane, B = EtOAc, 0%B to 50%B) yielded the racemic title compound (3.79 g, 24 % yield).

LC-MS (Method 4): Rt = 1.18min; MS (ESIpos): m/z = 369.1 $[M+H]^+$.

$^1H$ NMR (DMSO-$d_6$, 400 MHz): δ (ppm) 7.26-7.31 (m, 2H), 6.92-6.97 (m, 2H), 4.14-4.23 (m, 2H), 4.05-4.11 (m, 3H), 3.80 (d, *J* = 2.0 Hz, 1H), 3.70-3.75 (m, 2H), 3.55-3.59 (m, 2H), 3.47-3.55 (m, 4H), 3.38-3.47 (m, 2H), 3.41 (q, *J* = 7.0 Hz, 2H), 1.24 (t, *J* = 7.1 Hz, 3H), 1.08 (t, *J* = 7.0 Hz, 3H).

**Step** 3

ethyl 3-(4-{2-[2-(2-ethoxyethoxy)ethoxy]ethoxy}phenyl)-2-hydroxypropanoate

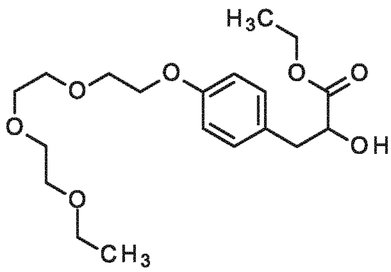

ethyl-3-(4-{2-[2-(2-ethoxyethoxy)ethoxy]ethoxy}phenyl)oxirane-2-carboxylate (3.79 g, 10.3 mmol) was dissolved in ethanol (49 ml), palladium (381 mg, 10% on carbon, 358 μmol; [7440-05-3]) was added and the mixture hydrogenated overnight at RT and ambient pressure. The reaction mixture was then filtered through a glass fiber filter and concentrated to dryness. Purification via chromatography ($SiO_2$, Biotage Ultra: 50g / A = Hexane, B = EtOAc, 0%B to 50% B) yielded the title compound, **Intermediate 7,** (2.34 g, 58 % yield).

LC-MS (Method 4): Rt = 1.04min; MS (ESIpos): m/z = 371.4 $[M+H]^+$.

$^1H$ NMR (DMSO-$d_6$, 400 MHz): δ (ppm) 7.10 (d, *J* = 7.5 Hz, 2H), 6.80-6.87 (m, 2H), 5.48 (d, *J* = 6.1 Hz, 1H), 4.15 (dt, J = 7.9, 5.7 Hz, 1H), 4.00-4.07 (m, 4H), 3.69-3.74 (m, 2H), 3.56-3.59 (m, 2H), 3.49-3.55 (m, 4H), 3.44-3.48 (m, 2H), 3.41 (q, *J* = 7.1 Hz, 2H), 2.85 (dd, J = 13.7, 5.3 Hz, 1H), 2.75 (dd, *J* = 13.7, 7.6 Hz, 1H), 1.13 (t, *J* = 7.1 Hz, 3H), 1.09 (t, *J* = 7.0 Hz, 3H).

**Intermediate 8**

**Step 1**

tert-butyl-3-(4-{2-[2-(2-ethoxyethoxy)ethoxy]ethoxy}phenyl)oxirane-2-carboxylate

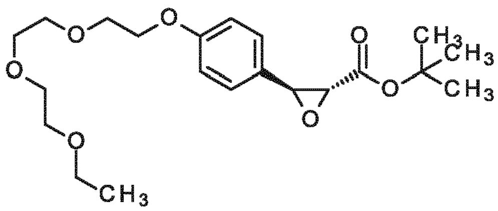

4-{2-[2-(2-ethoxyethoxy)ethoxy]ethoxy}benzaldehyde (Intermediate 7 - Step 1, 7.50 g, 26.6 mmol) was added to a flask, followed by THF (300 ml), and sodium hydride (1.49 g, 60 % in mineral oil, 37.2 mmol; [7646-69-7]) under $N_2$. The mixture was stirred at RT for 5min and then neat tert-butyl chloroacetate (5.0 ml, 35 mmol) was added at 50 μl/min at RT with reaction stirred at 250 rpm overnight. Water (20 ml) was cautiously added, and the mixture exacted with MTBE (2 x 50 ml), the organic phase was dried over $Na_2SO_4$ and the solvent removed under reduced pressure yielding the racemic compound (11.1 g, 105 % yield - containing mineral oil).

LC-MS (Method 4): Rt = 1.33min; MS (ESIpos): m/z = 419.3 $[M+Na]^+$.

$^1H$ NMR (DMSO-$d_6$, 400 MHz): δ (ppm) 7.25-7.32 (m, 2H), 6.91-6.97 (m, 2H), 4.05-4.12 (m, 2H), 4.02 (d, *J* = 1.8 Hz, 1H), 3.71-3.75 (m, 2H), 3.67 (d, *J* = 1.8 Hz, 1H), 3.55-3.59 (m, 2H), 3.48-3.55 (m, 4H), 3.44-3.47 (m, 2H), 3.41 (q, *J* = 7.0 Hz, 2H), 1.46 (s, 9H), 1.08 (t, *J* = 7.1 Hz, 3H).

The trans-stereochemistry was assigned according to previous literature (e.g. Tetrahedron 2006, 62, 10255-10270, Chemical & Pharmaceutical Bulletin (1995), 43(10), 1821-3) and the coupling constants (1.7 - 2.0 Hz) observed for the epoxide-protons.

**Step 2**

tert-butyl-3-(4-{2-[2-(2-ethoxyethoxy)ethoxy]ethoxy}phenyl)oxirane-2-carboxylate (2.6 g dissolved in 10 mL DCM/MeOH 20 x 0.5 ml injections) was separated into its enantiomeric components via chiral HPLC (PrepCon Labomatic HPLC-3; YMC Cellulose SC 5μ, 250x50; A = hexane + 0.1 vol % DEA; B = ethanol + 0.1 vol % DEA; 20%B; 120 ml/min; 25°C; 254 nm).

Enantiomeric purity was determined using the following method (Waters Alliance 2695; YMC Cellulose SC 3μ, 100x4.6; A = hexane + 0.1 vol % DEA; B = ethanol; 20%B; 1.4 ml/min; 25°C; 254 nm), Enantiomer 1: Rt = 2.68min, enantiomer 2: $R_t$ = 3.29min.

Both enantiomers were obtained after concentration of the combined product fractions, and removal of the solvent under reduced pressure.

tert-butyl (2R,3S)-3-(4-{2-[2-(2-ethoxyethoxy)ethoxy]ethoxy}phenyl)oxirane-2-carboxylate

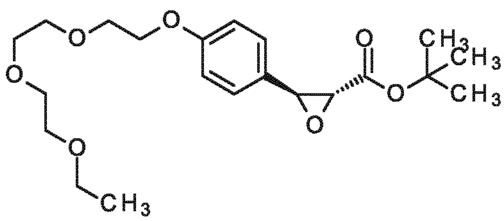

Enantiomer 1 (930 mg, 100% e.e.).

HPLC: Rt = 2.68min.

Specific rotation: +124° (c=1, MeOH, 20°C, 589 nm).

$^1H$ NMR (400 MHz, DMSO-$d_6$) δ ppm 1.08 (t, 3H), 1.46 (s, 8H), 3.38 - 3.55 (m, 8H), 3.55 - 3.59 (m, 2H), 3.67 (d, 1H), 3.71 - 3.75 (m, 2H), 4.02 (d, 1H), 4.07 - 4.11 (m, 2H), 6.87 - 7.01 (m, 2H), 7.25 - 7.31 (m, 2H).

tert-butyl (2S,3R)-3-(4-{2-[2-(2-ethoxyethoxy)ethoxy]ethoxy}phenyl)oxirane-2-carboxylate

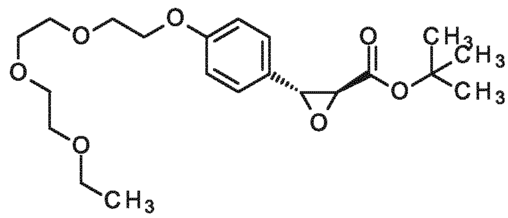

Enantiomer 2 (1040 mg, 100% e.e.)

LC-MS: Rt = 3.29min.

Specific rotation: -110° (c=1, MeOH, 20°C, 589 nm).

$^1$H NMR (400 MHz, DMSO-$d_6$) δ ppm 1.08 (t, 3H), 1.46 (s, 8H), 3.38 - 3.55 (m, 8H), 3.55 - 3.59 (m, 2H), 3.67 (d, 1H), 3.71 - 3.75 (m, 2H), 4.02 (d, 1H), 4.07 - 4.11 (m, 2H), 6.87 - 7.01 (m, 2H), 7.25 - 7.31 (m, 2 H).

Step 3

tert-butyl (2R)-3-(4-{2-[2-(2-ethoxyethoxy)ethoxy]ethoxy}phenyl)-2-hydroxypropanoate

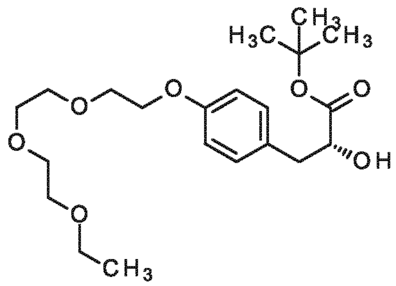

tert-butyl (2R,3S)-3-(4-{2-[2-(2-ethoxyethoxy)ethoxy]ethoxy}phenyl)oxirane-2-carboxylate **(Enantiomer 1, Step 2, 930** mg, 2.35 mmol) was dissolved in EtOAc (11 ml), palladium (87.0 mg, 10 % on carbon, 81.7 μmol; [7440-05-3]) added, the mixture cycled between vacuum and $N_2$ three times, following which it was placed under an atmosphere of $H_2$ (1 atm) overnight. The mixture was filtered through a glass fiber filter and concentrated to dryness under reduced pressure, yielding 812 mg (87%) of the title compound, **Intermediate** 8. The stereochemistry of the alcohol is assigned as (R) based on the positive optical rotation with reference to the series of closely related literature examples.

Specific Rotation: +7.5° (c=1, MeOH, 20°C, 589 nm).

LC-MS (Method 4): Rt = 1.20min; MS (ESIpos): m/z = 416.2 $[M+NH_4]^+$.

$^1$H NMR (400 MHz, DMSO-$d_6$) δ ppm 1.09 (t, 3H), 1.34 (s, 9H), 2.70 - 2.85 (m, 2H), 3.39 - 3.59 (m, 10H), 3.68 - 3.75 (m, 2H), 4.01 - 4.07 (m, 3H), 5.31 (d, 1H), 6.81 - 6.85 (m, 2H), 7.09 - 7.14 (m, 2 H).

Intermediate 9

Step 1

tert-butyl (2S)-3-(4-{2-[2-(2-ethoxyethoxy)ethoxy]ethoxy}phenyl)-2-hydroxypropanoate

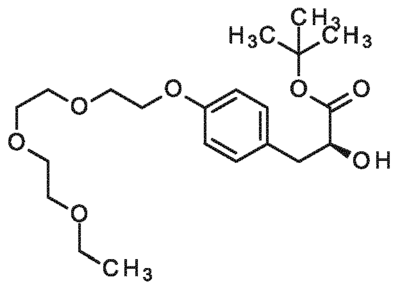

tert-butyl (2S,3R)-3-(4-{2-[2-(2-ethoxyethoxy)ethoxy]ethoxy}phenyl)oxirane-2-carboxylate, **Enantiomer 2, Step 2 of Intermediate** 8) (1.04 g, 2.62 mmol) was dissolved in EtOAc (13 ml), palladium (97.2 mg, 10 % on carbon, 91.4 μmol; [7440-05-3]) was added and the mixture cycled between $N_2$ and vacuum three times before the mixture was hydrogenated under 1 atm of $H_2$ overnight. The mixture was filtered through a glass fiber filter and concentrated to dryness under reduced pressure, yielding the title compound **(Intermediate 9,** 749 mg, 72%). The stereochemistry of the alcohol is assigned as (S) based on the negative optical rotation with reference to the series of closely related literature examples.

Specific Rotation: -6.8° (c=1, MeOH, 20°C, 589 nm).

LC-MS (Method 4): $R_t$ = 1.20min; MS (ESIpos): m/z = 416.2 $[M+NH_4]^+$.

$^1$H NMR (400 MHz, DMSO-$d_6$) δ ppm 1.09 (t, 3H), 1.17 (s, 1H), 1.34 (s, 9H), 2.52 - 2.55 (m, 1H), 2.70 - 2.85 (m, 2H), 3.39 - 3.59 (m, 10H), 3.68 - 3.75 (m, 2H), 4.00 - 4.07 (m, 3H), 5.31 (d, 1H), 6.80 - 6.86 (m, 2H), 7.09 - 7.14 (m, 2 H).

**Intermediate** 10

**Step 1**

methyl-3-(4-butoxyphenyl)oxirane-2-carboxylate

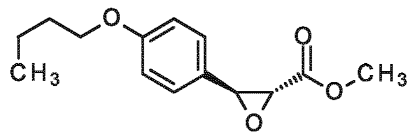

Sodium hydride (5.05 g, 60% purity, 126.2 mmol), was added to THF (190 ml), the mixture was cooled to 0°C and under $N_2$. Methyl chloroacetate (3.7 ml, 42 mmol) was added and the mixture was stirred for 10min, following which 4-butoxybenzaldehyde (4.8 ml, 28 mmol [123-11-5]) was added. After reaction overnight, the mixture was brought to 0°C and HCl (0.5 M, aq.) added, followed by extraction of the mixture with MTBE, drying of the organic phase over $Na_2SO_4$, filtration and concentration under reduced pressure, yielding the racemic title compound (9.80 g), which was used in the next step without further purification.

LC-MS (Method 4): Rt = 1.33min; MS (ESIpos): m/z = 251.2 $[M+H]^+$.

**Step 2**

methyl 3-(4-butoxyphenyl)-2-hydroxypropanoate

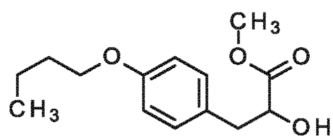

Methyl 3-(4-butoxyphenyl)oxirane-2-carboxylate (9.80 g, 27.4 mmol) was dissolved in ethanol (130 ml), palladium (714 mg, 10 % on carbon, 671 μmol) was added and the mixture cycled between $N_2$ and vacuum three times before the mixture was hydrogenated under 1 atm of $H_2$ overnight. The mixture was filtered through a glass fiber filter and concentrated under reduced pressure, with purification using chromatography ($SiO_2$, Biotage, Ultra 100g column, A =

Hexane, B = EtOAc, 0%B to 100%B) yielding the title compound **(Intermediate 10,** 1.20 g, 17% yield calc, from step 1).

LC-MS (Method 4): Rt = 1.18min; MS (ESIpos): m/z = 270.2 $[M+NH_4]^+$.

$^1H$ NMR (DMSO-$d_6$, 400 MHz): δ (ppm) 7.05-7.12 (m, 2H), 6.78-6.84 (m, 2H), 5.51 (d, *J* = 6.1 Hz, 1H), 4.17 (ddd, J = 7.9, 6.1, 5.1 Hz, 1H), 3.91 (t, *J* = 6.5 Hz, 2H), 3.59 (s, 3H), 2.86 (dd, J = 13.7, 5.1 Hz, 1H), 2.74 (dd, J = 13.7, 7.9 Hz, 1H), 1.63-1.71 (m, 2H), 1.36-1.48 (m, 2H), 0.92 (t, *J* = 7.4 Hz, 3H).

**Intermediate 11**

**Step 1**

6-[2-(2-ethoxyethoxy)ethoxy]pyridine-3-carbaldehyde

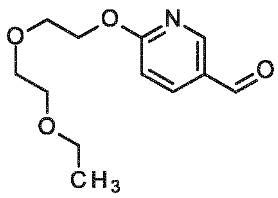

6-bromopyridine-3-carbaldehyde (7.00 g, 37.6 mmol, [ 149806-06-4]), 2-(2-ethoxyethoxy) ethanol (10 ml, 75 mmol, [111-90-0]) and cesium carbonate (14.7 g, 45.2 mmol; [534-17-8]) were added to a reaction flask and the mixture stirred overnight at 70°C. The mixture was added to water, the extracted with MTBE (3x), the combined organic phases washed once with water, dried over $Na_2SO_4$, filtered and concentrated under reduced pressure. Purification by chromatography ($SiO_2$, Biotage, Ultra 100g column / A = Hexane, B = EtOAc, 0%B to 100%B) yielded the title compound (6.17 g, 69 % yield).

$^1H$ NMR (DMSO-$d_6$, 400 MHz): δ (ppm) 9.96 (s, 1H), 8.75 (dd, *J* = 2.4, 0.6 Hz, 1H), 8.12 (dd, *J* = 8.7, 2.4 Hz, 1H), 7.01 (d, *J* = 8.9 Hz, 1H), 4.47-4.52 (m, 2H), 3.74-3.79 (m, 2H), 3.55-3.58 (m, 2H), 3.38-3.49 (m, 6H), 1.08 (t, *J* = 7.0 Hz, 3H).

**Step 2**

tert-butyl-3-{6-[2-(2-ethoxyethoxy)ethoxy]pyridin-3-yl}oxirane-2-carboxylate

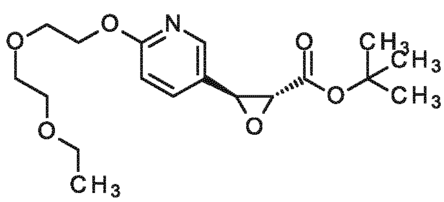

6-[2-(2-ethoxyethoxy)ethoxy]pyridine-3-carbaldehyde (6.17 g, 25.8 mmol) was dissolved in THF (290 ml), and at RT under $N_2$, sodium hydride (1.44 g, 60 % purity, 36.1 mmol; [7646-69-7]) was added portion wise. The mixture was stirred for 5min, following which tert-butyl chloroacetate (4.8 ml, 34 mmol) was added dropwise. The reaction was stirred overnight, following which it was added to an ice-water mixture, and the aqueous phase extracted with EtOAc (3 x), the organic phase washed with NaCl (sat. aq.), dried over $Na_2SO_4$, filtered and the solvent removed under reduced pressure. Purification by chromatography ($SiO_2$, Biotage, Ultra 100g column / A = Hexane, B = EtOAc, 0%B to 100%B) yielded the racemic title compound (2.35 g, 23%).

LC-MS (Method 4): $R_t$ = 1.25min; MS (ESIpos): m/z =354.3 $[M+H]^+$.

$^1H$ NMR (DMSO-$d_6$, 400 MHz): δ (ppm) 8.25 (d, *J* = 2.3 Hz, 1H), 7.62 (dd, *J* = 8.6, 2.5 Hz, 1H), 6.83 (d, *J* = 8.9 Hz, 1H), 4.35-4.38 (m, 2H), 4.11 (d, *J* = 1.8 Hz, 1H), 3.82 (d, *J* = 2.0 Hz, 1H), 3.72 (dd, *J* = 5.4, 3.9 Hz, 2H), 3.54-3.57 (m, 2H), 3.46-3.49 (m, 2H), 3.41 (q, *J* = 6.9 Hz, 2H), 1.46 (s, 9H), 1.08 (t, *J* = 7.1 Hz, 3H).

**Step 3**

tert-butyl 3-{6-[2-(2-ethoxyethoxy)ethoxy]pyridin-3-yl}-2-hydroxypropanoate

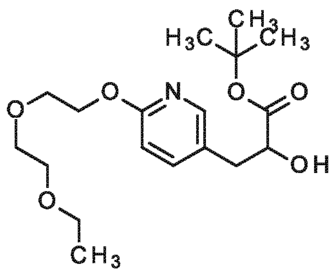

tert-Butyl 3-{6-[2-(2-ethoxyethoxy)ethoxy]pyridin-3-yl}oxirane-2-carboxylate (2.35 g, 6.65 mmol) was dissolved in EtOAc (32 ml), palladium (247 mg, 10% on carbon, 232 μmol; [7440-05-3]) was added and the mixture cycled between $N_2$ and vacuum three times before the mixture was hydrogenated under 1 atm of $H_2$ overnight. The mixture was filtered through a glass fiber filter and concentrated to dryness under reduced pressure yielding the title compound, **(Intermediate 11**, 2.13 g, 90%) with a purity of ca. 80% (H-NMR).

LC-MS (Method 4): $R_t$ = 1.11min; MS (ESIpos): m/z = 356.2 $[M+H]^+$.

$^1$H NMR (DMSO-$d_6$, 400 MHz): δ (ppm) 7.95 (d, *J* = 1.8 Hz, 1H), 7.58 (dd, *J* = 8.5, 2.4 Hz, 1H), 6.74 (d, *J* = 8.4 Hz, 1H), 4.29-4.34 (m, 2H), 4.08 (dd, *J* = 7.2, 5.7 Hz, 1H), 3.71 (dd, *J* = 5.3, 4.1 Hz, 2H), 3.38-3.57 (m, 6H), 2.83 (dd, *J* = 13.9, 5.3 Hz, 1H), 2.75 (dd, *J* = 13.9, 7.4 Hz, 1H), 1.35 (s, 9H), 1.09 (t, *J* = 7.0 Hz, 3H).

**Intermediate 12**

**Step 1**

(5R)-2,2-dimethyl-5-(prop-2-en-1-yl)-1,3-dioxolan-4-one

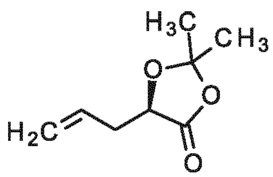

To a solution of (2R)-2-hydroxypent-4-enoic acid (35.0 g, 301 mmol, [413622-10-3]) in acetone (700 ml) were added pyridinium p-toluene sulfonate (37.9 g, 151 mmol) and 2,2-dimethoxypropane (251 g, 2.41 mol) at room temperature. The mixture was stirred at 60°C for 3h. The mixture was concentrated to give a residue. It was combined with the residue of an identical experiment (35 g). The combined residues were diluted with EtOAc and filtered through a pad of Celite. The filtrate was concentrated to give a residue. The residue was purified by column chromatography ($SiO_2$, 1000 mesh, petroleum ether: EtOAc = 1: 0, then 20: 1) to give (5R)-5-allyl-2,2-dimethyl-1,3-dioxolan-4-one (51.0 g, 327 mmol, 54%) as yellow oil.

$^1$H NMR (400 MHz, DMSO-*$d_6$*): δ [ppm] = 5.84-5.67 (m, 1H), 5.21-5.08 (m, 2H), 4.73-4.67 (m, 1H), 2.60-2.51 (m, 1H), 2.46-2.34 (m, 1H), 1.54 (s, 3H), 1.52 (s, 3H).

**Step 2**

(5R)-5-[(2 E)-3-{4-[2-(2-ethoxyethoxy)ethoxy]phenyl}prop-2-en-1-yl]-2,2-dimethyl-1, 3-dioxolan-4-one

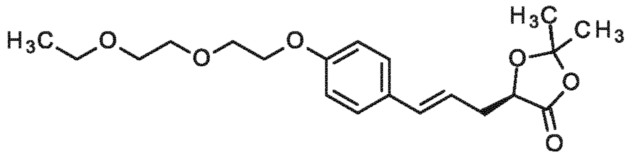

A mixture of (5R)-2,2-dimethyl-5-(prop-2-en-1-yl)-1,3-dioxolan-4-one (25.0 g, 160 mmol), 1-[2-(2-ethoxyethoxy) ethoxy]-4-iodobenzene (53.8 g, 160 mmol, [2305345-75-7]), palladium(II) acetate (3.59 g, 16.0 mmol), tri-2-tolylphosphine (4.87 g, 16.0 mmol) and DIPEA (70 ml, 400 mmol) in MeCN (500 ml) was stirred at 80°C for 12h. The mixture was concentrated to give a residue. It was combined with the residues of two identical experiments (25 g and 3 g). The combined residues were diluted with EtOAc and washed with $NH_4Cl$ (sat. aq.). The organic phase was washed with NaCl (sat. aq.), dried over anhydrous $Na_2SO_4$ filtered and concentrated. The residue was purified by flash column chromatography ($SiO_2$, petroleum ether: EtOAc = 1: 0 to 10: 1, then 10: 1) to give (5R)-5-[(2E)-3-{4-[2-(2-ethoxyethoxy)ethoxy] phenyl}prop-2-en-1-yl]-2,2-dimethyl-1,3-dioxolan-4-one (64.0 g, 176 mmol, 73%) as yellow oil.

LC-MS (Method 13): $R_t$ = 0.951min; MS (ESIpos): m/z = 365.2 $[M+H]^+$.

$^1H$ NMR (400 MHz, DMSO-$d_6$): δ [ppm] = 7.32 (d, *J* = 8.8 Hz, 2H), 6.90 (d, *J* = 8.8 Hz, 2H), 6.46 (d, *J* = 16 Hz, 1H), 6.12-5.99 (m, 1H), 4.81-4.73 (m, 1H), 4.13-4.05 (m, 2H), 3.79-3.69 (m, 2H), 3.63-3.55 (m, 2H), 3.53-3.47 (m, 2H). 3.43 (q, *J* = 6.8 Hz, 2H). 2.78-2.65 (m, 1H), 2.60-2.52(m, 1H), 1.55 (s, 3H), 1.55 (s, 3H), 1.10 (t, *J* = 7.2 Hz, 3H).

**Step 3**

(5R)-5-(3-{4-[2-(2-ethoxyethoxy)ethoxy]phenyl}propyl)-2,2-dimethyl-1,3-dioxolan-4-one

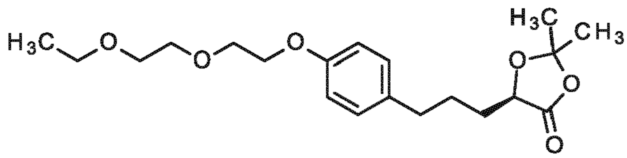

To a solution of (5R)-5-[(2E)-3-{4-[2-(2-ethoxyethoxy)ethoxy]phenyl}prop-2-en-1-yl]-2,2-dimethyl-1,3-dioxolan-4-one (59.0 g, 162 mmol) in THF (1.2 l) was added palladium (2.9 g, 10% on carbon, 5.54 mmol) at RT. The mixture was stirred at RT under $H_2$ atmosphere (15 psi) for 12h. The mixture was filtered, and the filtrate was concentrated to give the title compound (60.0 g) as a yellow oil.

LC-MS (Method 13): $R_t$ = 0.987min; MS (ESIpos): m/z = 367.1 $[M+H]^+$.

**Step 4**

methyl (2R)-5-{4-[2-(2-ethoxyethoxy)ethoxy]phenyl}-2-hydroxypentanoate

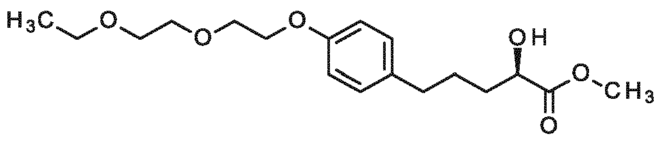

To a solution of (5R)-5-(3-{4-[2-(2-ethoxyethoxy)ethoxy]phenyl}propyl)-2,2-dimethyl-1,3-dioxolan-4-one (60.0 g, 164 mmol) in MeOH (600 ml) was 4-toluenesulfonic acid (2.82 g, 16.4 mmol) at RT. The mixture was stirred at RT for 12h. The mixture was combined with an earlier experiment (5.2 g), concentrated and diluted with EtOAc. The organic phase was washed with $NaHCO_3$ (sat. aq.) and NaCl (sat. aq.). The organic phase was dried over anhydrous $Na_2SO_4$, filtered and concentrated. The residue was purified by reverse phase column (Agela HP1000; Welch Ultimate XB_C18 100*400mm 20-40 μm; 200 ml/min; A = water (0.1% formic acid), B = MeCN; B%: 10%B-45B% in 35min, 50%B 20min; RT; UV 220/254 nm) to give the title compound **(Intermediate 12,** 48.4 g, 142 mmol, 80%) as a yellow oil.

LC-MS (Method 13): Rt = 0.630min; MS (ESIpos): m/z = 341.3 $[M+H]^+$.

LC-MS (Method 3): Rt = 1.00min (91% DAD); MS (ESIpos): m/z = 341 $[M + H]^+$.

Chiral HPLC (Thermo Fisher UltiMate 3000; YMC Cellulose SB 3μ, 100x4.6; A = hexane + 0.1 vol % DEA; B = ethanol; 10%B; 1.4 ml/min; 25°C; 280 nm): Rt = 2.85min (4.94%), Rt = 3.57min (85.21%). e.e. = 89%.

Specific Rotation: $\alpha_D^{20}$ = -7.46° +/- 0.58° (c=1, $CHCl_3$).

$^1$H NMR ($CDCL_3$, 400 MHz): δ (ppm) 7.05-7.10 (m, 2H), 6.82-6.86 (m, 2H), 4.20 (ddd, *J* = 7.0, 5.7, 4.1 Hz, 1H), 4.12 (dd, *J* = 5.6, 4.3 Hz, 2H), 3.86 (dd, *J* = 5.1, 4.1 Hz, 2H), 3.77 (s, 3H), 3.71-3.74 (m, 2H), 3.60-3.64 (m, 2H), 3.54 (q, *J* = 7.1 Hz, 2H), 2.74 (d, *J* = 5.6 Hz, 1H), 2.53-2.64 (m, 2H), 1.74-1.86 (m, 2H), 1.61-1.73 (m, 2H), 1.22 (t, *J* = 7.1 Hz, 3H).

$^{13}$C NMR (101MHz, $CDCL_3$) δ = 175.67, 156.96, 134.13, 129.21 (2C), 114.49 (2C), 70.87, 70.30, 69.85, 69.80, 67.42, 66.69, 52.53, 34.53, 33.84, 26.68, 15.15.

**Intermediate 13**

<u>**Step 1**</u>

2-(2-ethoxyethoxy)ethyl methanesulfonate

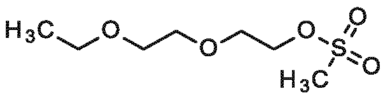

A solution of 2-(2-ethoxyethoxy)ethan-1-ol (10 ml, 75 mmol) in THF (200 ml) was treated with TEA (25 ml, 180 mmol) and was cooled to 0°C, followed by the dropwise addition of MsCl (6.9 ml, 89 mmol). The mixture was stirred at RT for 16h, then poured into an aqueous solution of $NaHCO_3$, then it was extracted with EtOAc three times, the organic layers were washed with NaCl (sat. aq.) and dried over $Na_2SO_4$, with concentration under reduced pressure yielding the title compound (15.1 g, 95 % yield).

$^1$H NMR (400 MHz, DMSO-d6) δ ppm 1.10 (t, J=6.97 Hz, 3H), 3.18 (s, 3H), 3.43 (q, J=7.01 Hz, 2H), 3.46 - 3.51 (m, 2H), 3.53 - 3.58 (m, 2H), 3.63 - 3.70 (m, 2H), 4.28 - 4.33 (m, 2 H).

<u>**Step 2**</u>

3-{4-[2-(2-ethoxyethoxy)ethoxy]phenyl}propan-1-ol

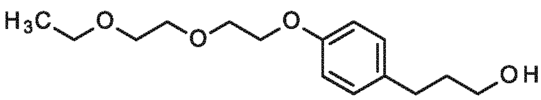

A solution of 4-(3-hydroxypropyl)phenol (2.80 g, 18.4 mmol, [10210-17-0]) and cesium carbonate (5.99 g, 18.4 mmol; [534-17-8]) in DMF (40 ml) was stirred at RT for 30min. To this mixture, 2-(2-ethoxyethoxy)ethyl methanesulfonate (3.90 g, 18.4 mmol) was added and the mixture stirred at 60°C for 16h. Water was added to the reaction mixture and the mixture was extracted with MTBE (3x). The organic layers were washed with sat. NaCl (aq.), dried over $Na_2SO_4$, and concentrated under reduced pressure yielding the title compound (4.90 g, (92% purity), 99 % yield).

LC-MS (Method 1): Rt = 0.97min; MS (ESIpos): m/z = 269.3 $[M+H]^+$.

$^1$H NMR (400 MHz, DMSO-$d_6$) δ ppm 1.09 (t, J=6.97 Hz, 3H), 1.61 - 1.70 (m, 2H), 2.50 - 2.53 (m, 2H), 3.35 - 3.45 (m, 4H), 3.46 - 3.50 (m, 2H), 3.55 - 3.59 (m, 2H), 3.69 - 3.75 (m, 2H), 4.00 - 4.06 (m, 2H), 4.44 (t, J=5.20 Hz, 1H), 6.80 - 6.86 (m, 2H), 7.05 - 7.11 (m, 2H).

<u>**Step 3**</u>

3-{4-[2-(2-ethoxyethoxy)ethoxy]phenyl}propanal

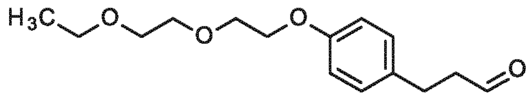

A solution of 3-{4-[2-(2-ethoxyethoxy)ethoxy]phenyl}propan-1-ol (4.90 g, 18.3 mmol) in DCM (120 ml) was treated with DMSO (32 ml), TEA (12 ml, 88 mmol;) and pyridine sulfur trioxide complex (8.72 g, 54.8 mmol; [26412-87-3]) sequentially and the mixture was stirred at RT for 16h. The reaction mixture was poured into a mixture of saturated $NH_4Cl$ and ice, and the aqueous layer extracted with DCM (3x), the organic layers combined and washed with NaCl (sat. aq.) dried over $Na_2SO_4$, filtered and concentrated under reduced pressure. The crude product was purified by chromatography ($SiO_2$, Biotage Ultra 100g column, A = Hexane, B = EtOAc, gradient) yielding the title compound (4.60 g, 95 % yield).

LC-MS (Method 1): $R_t$ = 1.05min; MS (ESIpos): m/z = 267.3 $[M+H]^+$.

$^1H$ NMR (400 MHz, DMSO-$d_6$) δ ppm 1.09 (t, J=7.10 Hz, 3H), 2.68 - 2.74 (m, 2H), 2.76 - 2.83 (m, 2H), 3.42 (q, J=7.10 Hz, 2H), 3.47 - 3.51 (m, 2H), 3.53 - 3.59 (m, 2H), 3.68 - 3.73 (m, 2H), 3.99 - 4.09 (m, 2H), 6.78 - 6.89 (m, 2H), 7.08 - 7.19 (m, 2H), 9.67 - 9.73 (m, 1 H).

**Step 4**

ethyl (2EZ)-2-(acetyloxy)-5-{4-[2-(2-ethoxyethoxy)ethoxy]phenyl}pent-2-enoate

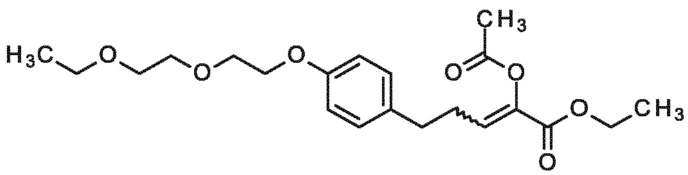

To lithium chloride (1.12 g, 26.5 mmol; [7447-41-8]) was added a solution of ethyl (acetyloxy)(diethoxyphosphoryl)acetate (7.47 g, 26.5 mmol, [162246-77-7]) in THF (30 ml). The mixture was stirred at RT for 1h. The mixture was cooled to 0°C in an ice-water bath and 1,1,3,3-tetramethylguanidine (4.4 ml, 35 mmol; [80-70-6]) was added dropwise and the mixture was stirred for 20min. A solution of 3-{4-[2-(2-ethoxyethoxy)ethoxy]phenyl}propanal (4.70 g, 17.6 mmol) in THF (30 ml) was added, the mixture stirred for another 10min. The ice-water bath was removed, and the mixture stirred for another 1h at RT. The reaction mixture was quenched by addition of sat. $NH_4Cl$ (aq.), the layers separated, and the aqueous layer extracted with DCM (3x). The combined organic layers were dried with $Na_2SO_4$, filtered and concentrated under reduced pressure. Purification by chromatography ($SiO_2$, Biotage, Ultra 50g column, eluent: A = Hexane, B = EtOAc, gradient elution) yielded the title compound as an E:Z mixture (4.65 g ,67 % yield).

LC-MS (Method 1): Rt = 1.30min; MS (ESIpos): m/z = 395.4 $[M+H]^+$.

$^1H$ NMR (DMSO-$d_6$, 400 MHz): δ (ppm) 7.08-7.14 (m, 2H), 6.85 (d, *J* = 8.6 Hz, 2H), 6.50 (t, *J* = 7.6 Hz, 0.3H), 6.01 (t, *J* = 7.6 Hz, 0.7H), 4.09-4.16 (m, 2H), 4.02-4.06 (m, 2H), 3.69-3.74 (m, 2H), 3.55-3.59 (m, 2H), 3.47-3.50 (m, 2H), 3.42 (q, *J* = 6.9 Hz, 2H), 2.71-2.81 (m, 1.4H), 2.60-2.68 (m, 2H), 2.38 (q, *J* = 7.6 Hz, 0.6H), 2.20 (s, 1H), 2.13 (s, 2H), 1.15-1.21 (m, 3H), 1.09 (t, *J* = 7.0 Hz, 3H). The sample is a 7:3 mixture of the E/Z-isomers and contains EtOAc.

**Step 5**

ethyl 5-{4-[2-(2-ethoxyethoxy)ethoxy]phenyl}-2-oxopentanoate

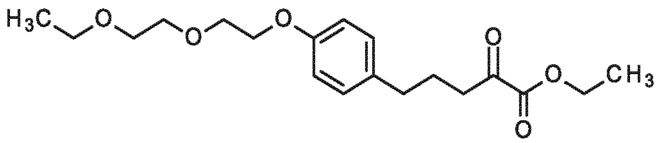

Ethyl (2EZ)-2-(acetyloxy)-5-{4-[2-(2-ethoxyethoxy)ethoxy]phenyl}pent-2-enoate (4.65 g, 11.8 mmol) was dissolved in ethanol (150 ml) and $H_2SO_4$ (conc., 5.0 ml, 94 mmol; [7664-93-9]) was added. The reaction mixture was stirred at reflux for 16h, following which the mixture was concentrated under reduced pressure, the residue dissolved in EtOAc, washed with $NaHCO_3$ (sat. aq.), dried over $Na_2SO_4$, filtered and concentrated under reduced pressure, yielding the title

compound (2.40 g ,58 % yield).

LC-MS (Method 1): $R_t$ = 1.26min; MS (ESIpos): m/z = 370.4 $[M + H_2O + H]^+$.

$^1H$ NMR (400 MHz, DMSO-$d_6$) δ ppm 1.09 (t, J=7.10 Hz, 3H), 1.25 (t, J=7.10 Hz, 3H), 1.76 (quin, J=7.41 Hz, 2H), 1.72 - 1.72 (m, 1H), 2.50 - 2.54 (m, 2H), 2.79 (t, J=7.35 Hz, 2H), 3.42 (q, J=7.10 Hz, 2H), 3.46 - 3.50 (m, 2H), 3.54 - 3.59 (m, 2H), 3.67 - 3.74 (m, 2H), 4.01 - 4.06 (m, 2H), 4.20 (q, J=7.10 Hz, 2H), 6.82 - 6.88 (m, 2H), 7.06 - 7.11 (m, 2 H).

**Step 6**

ethyl-5-{4-[2-(2-ethoxyethoxy)ethoxy]phenyl}-2-hydroxypentanoate

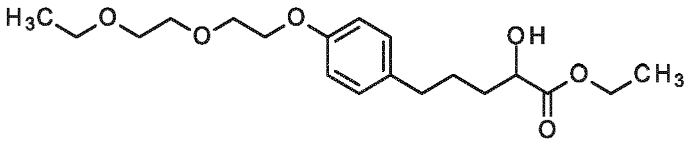

A solution of ethyl 5-{4-[2-(2-ethoxyethoxy)ethoxy]phenyl}-2-oxopentanoate (2.40 g, 6.81 mmol) in ethanol (100 ml) was treated with Pd/C (269 mg, 10 % purity, 252 μmol; [7440-05-3]) and flushed with $N_2$ followed by vacuum three times. The mixture was stirred under an atmosphere of $H_2$ at RT for 96h. The reaction mixture was filtered using a glass fiber filter and concentrated under reduced pressure, yielding the racemic title compound **(Intermediate 13,** 1.80 g (75 % yield).

LC-MS (Method 1): Rt = 1.15min; MS (ESIpos): m/z = 355.4 $[M+H]^+$.

$^1H$ NMR (400 MHz, DMSO-$d_6$) δ ppm 1.09 (t, J=7.10 Hz, 3H), 1.17 (t, J=7.10 Hz, 3H), 1.50 - 1.64 (m, 4H), 2.46 - 2.53 (m, 2 H, coalescence with solvent signal) 3.42 (q, J=7.01 Hz, 2H), 3.47 - 3.50 (m, 2H), 3.54 - 3.60 (m, 2H), 3.69 - 3.74 (m, 2H), 3.96 - 4.11 (m, 5H), 5.32 (d, J=6.08 Hz, 1H), 6.79 - 6.87 (m, 2H), 7.04 - 7.10 (m, 2 H).

**Intermediate 14**

ethyl (2S)-5-{4-[2-(2-ethoxyethoxy)ethoxy]phenyl}-2-hydroxypentanoate

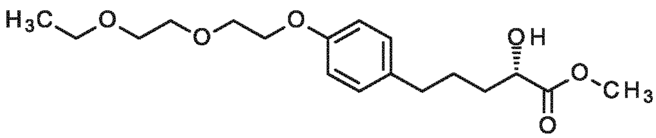

Racemic ethyl-5-{4-[2-(2-ethoxyethoxy)ethoxy]phenyl}-2-hydroxypentanoate **(Intermediate 13,** 3480 mg, dissolved in 25 ml DCM) was separated into its two enantiomeric components using the following method (PrepCon Labomatic HPLC-1; Column: YMC Cellulose SB 10μ, 250x50; A = hexane/ 0.1 vol % DEA; B = ethanol; 15%B; 100 mL/min; 280 nm; 25 × 1 ml injections).

Analytical method for e.e. determination: (Thermo Fisher UltiMate 3000; YMC Cellulose SB 3μ, 100x4.6; A = hexane/0.1 vol % DEA; B = ethanol; 10%B; 1.4 ml/min; 25°C, 280 nm). Rt Enantiomer 1 = 3.03min: $R_t$ Enantiomer 2 = 3.58min.

**Enantiomer 2:** ethyl (2S)-5-{4-[2-(2-ethoxyethoxy)ethoxy]phenyl}-2-hydroxypentanoate was obtained **(Intermediate 14,** 1600 mg) after combination of the collected fractions and concentration under reduced pressure at 45°C. The stereochemistry of the alcohol was assigned by correlation of retention times via chiral HPLC to compounds produced from chiral starting materials.

HPLC (above analytical method): Rt = 3.58min.

Specific rotation: -1.6° ± 0.17 (CHCL3, 20°C, 589 nm).

LC-MS (Method 1): Rt = 1.15min; MS (ESIpos): m/z = 355.4 $[M+H]^+$.

$^1H$ NMR (400 MHz, DMSO-$d_6$) δ ppm 1.09 (t, 3H), 1.17 (t, 3H), 1.48 - 1.64 (m, 4H), 1.91 (s, 2H), 3.42 (q, 2H), 3.46 - 3.50 (m, 2H), 3.54 - 3.59 (m, 2H), 3.68 - 3.74 (m, 2H), 3.97 - 4.11 (m, 5H), 5.32 (d, 1H), 6.84 (d, 2H), 7.07 (d, 2H).

**Intermediate 15**

ethyl (2R)-5-{4-[2-(2-ethoxyethoxy)ethoxy]phenyl}-2-hydroxypentanoate

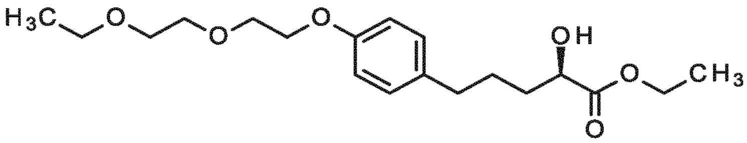

**Enantiomer 1, Intermediate 14:** ethyl (2R)-5-{4-[2-(2-ethoxyethoxy)ethoxy]phenyl}-2-hydroxypentanoate was obtained **(Intermediate 15,** 1560 mg) after combination of the collected fractions and concentration under reduced pressure at 45°C. The stereochemistry of the alcohol is assigned as (*R*) by correlation of retention times via chiral HPLC to compounds produced from chiral starting materials.
HPLC (above analytical method): Rt = 3.03min.
Specific rotation: 0.71° ± 0.4 (CHCL3, 20°C, 589 nm).
LC-MS (Method 1): $R_t$ = 1.15min; MS (ESIpos): m/z = 355.4 $[M+H]^+$.
$^1H$ NMR (400 MHz, DMSO-$d_6$) δ ppm 1.09 (t, 3H) 1.17 (t, 3H) 1.48 - 1.64 (m, 4H) 1.91 (s, 2H) 3.42 (q, 2H) 3.46 - 3.50 (m, 2H) 3.54 - 3.59 (m, 2H) 3.68 - 3.74 (m, 2H) 3.97 - 4.11 (m, 5H) 5.32 (d, 1H) 6.84 (d, 2H) 7.07 (d, 2H).

**Intermediate 16**

**Step 1**

2-phenyl-1,3-dioxan-4-yl]methanol

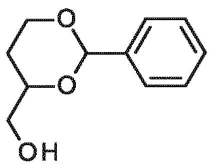

Racemic t-butane-1,2,4-triol (9.3 ml, 100 mmol; [3068-00-6]) was dissolved in DMF (220 ml) under argon. Tetrafluoroboronic acid-diethyl ether complex (1.4 ml, 10 mmol; [67969-82-8]) was added to the stirred mixture followed by the dropwise addition of (dimethoxymethyl)benzene (16 ml, 100 mmol; [1125-88-8]), and the mixture was stirred overnight at RT. The mixture was neutralized with $NaHCO_3$ (solid) and then concentrated under reduced pressure. The crude mixture was diluted with DCM and filtrated through Celite, and the filtrate concentrated under reduced pressure (24.5 g). Purification by chromatography ($SiO_2$, Biotage; 340 g Ultra; A = Hexane, B = EtOAc, 0%B to 100%B; 150 ml/min) yielded the title compound as a mixture of diastereomers (16.0 g, 75 % yield).
LC-MS (Method 3): $R_t$ = 0.67min; MS (ESIpos): m/z = 195.2 $[M+H]^+$.
$^1H$ NMR (400 MHz, DMSO-$d_6$) δ ppm 1.48 - 1.55 (m, 1H) 1.57 - 1.68 (m, 1H) 3.36 - 3.52 (m, 2H) 3.83 - 3.95 (m, 2H) 4.13 - 4.21 (m, 1H) 4.72 - 4.79 (m, 1H) 5.52 (s, 1H) 7.31 - 7.47 (m, 1H).

**Step 2**

tert-butyl 2-phenyl-1,3-dioxane-4-carboxylate

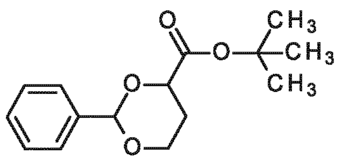

Chromium (VI) oxide (12.0 g, 120 mmol; [1333-82-0]) was added to a mixture of DCM:DMF (400 ml, 4:1). Pyridine (26 ml) was added and the mixture was stirred at RT for 30min. 2-Methylpropan-2-ol (77 ml, 800 mmol) and acetic anhydride (30 ml, 320 mmol; [108-24-7]) were added. [2-phenyl-1,3-dioxan-4-yl]methanol (7.80 g, 40.2 mmol) dissolved in DCM:DMF (180 ml, 4:1) was added dropwise over 90min and the mixture stirred overnight. The mixture was cooled to 0-5°C, and solid $NaHCO_3$ (54.0 g, 643 mmol) and ethanol (38 ml) were added and the mixture was stirred for 30min. Toluene was added, and the mixture concentrated under reduced pressure. The mixture was then dissolved in EtOAc, filtered and the filter cake washed with EtOAc. Purification by chromatography ($SiO_2$, Biotage 120 g Ultra; A = Hexane, B = EtOAc, C = MeOH, 100%A to 100%B, followed by 0%C to 15% C) yielded the title compound as a mixture of diastereomers (5.53 g, 51 % yield).

LC-MS (Method 6): Rt = 1.25min; MS (ESIpos): m/z = 282.3 $[M+NH_4]^+$.

$^1H$ NMR (400 MHz, DMSO-$d_6$) δ ppm 1.43 (s, 9H) 1.73 - 1.92 (m, 2H) 3.91 - 4.05 (m, 1H) 4.13 - 4.23 (m, 1H) 4.50 (dd, 1H) 5.56 - 5.62 (s, 1H) 7.32 - 7.46 (m, 5H).

**Step 3**

tert-butyl-4-(benzyloxy)-2-hydroxybutanoate

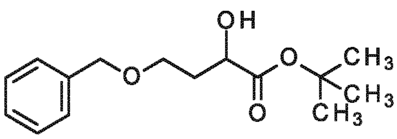

Triethyl silane (17 ml, 100 mmol, [617-86-7]) was added to DCM (110 ml), followed by the addition of racemic tert-butyl-2-phenyl-1,3-dioxane-4-carboxylate (5.53 g, 20.9 mmol). The mixture was cooled to -5°C and TFA (8.1 ml, 100 mmol; [76-05-1]) added dropwise. The mixture was then brought to RT and stirred for 90min. A solution of $NaHCO_3$ (sat. aq.) was added, until a basic pH was obtained, the layers separated, and the organic phase dried over $Na_2SO_4$. Purification by chromatography ($SiO_2$, Biotage, 100g Ultra; A = Hexane, B = EtOAc, 0%B to 35%B, 120ml/min) yielded the title compound (2.16 g, 37 % yield).

LC-MS (Method 6): Rt = 1.18min; MS (ESIpos): m/z = 211.2 $[M + H - C_4H_8]^+$.

$^1H$ NMR (400 MHz, OMSO-$d_6$) δ ppm 1.39 (s, 9H) 1.64 - 1.77 (m, 1H) 1.90 (m, 1H) 3.40 - 3.58 (m, 1H) 3.99 (m, 1H) 4.44 (s, 1H) 5.23 (d, 2H) 7.15 - 7.55 (m, 5H).

**Step 4**

tert-butyl (2R)-4-(benzyloxy)-2-[(methanesulfonyl)oxy]butanoate

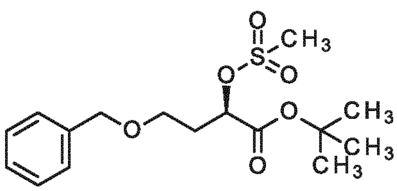

Racemic tert-butyl-4-(benzyloxy)-2-hydroxybutanoate (2.16 g, 8.11 mmol), TEA (2.3 ml, 16 mmol;) and THF (110 ml) were stirred at 0-5°C. Under an atmosphere of $N_2$, MsCl (690 µl, 8.9 mmol; [124-63-0]) was added, the mixture allowed to warm to RT and stirred overnight. The mixture was added to $NaHCO_3$ (50 % sat. aq.), and the mixture extracted with MTBE (3x), the organic solutions combined, washed with a NaCl solution (sat. aq.), dried over $Na_2SO_4$, filtered and concentrated under reduced pressure yielding the title compound **(Intermediate 16,** 2.87 g, 92 % yield).

LC-MS (Method 6): $R_t$ = 1.29min; MS (ESIpos): m/z = 362.3 $[M+NH_4]^+$.

$^1H$ NMR (400 MHz, OMSO-$d_6$) δ ppm 1.41 (s, 9H), 1.93 - 2.20 (m, 2H), 3.53 (dd, 2H), 4.47 (s, 2H), 4.98 (dd, 1H), 7.24 - 7.40 (m, 5H).

**Intermediate 17**

**Step 1**

5-[2-(2-ethoxyethoxy)ethoxy]pyridine-2-carbaldehyde

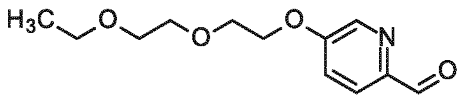

A mixture of 5-fluoropyridine-2-carbaldehyde (5.00 g, 40.0 mmol, [ 31181-88-1]) and 2-(2-ethoxyethoxy)ethan-1-ol (16 ml, 120 mmol) was treated with cesium carbonate (15.6 g, 48.0 mmol; [534-17-8]), and the mixture was stirred at 70°C for 18h. Water was added to the reaction mixture and the mixture was extracted with MTBE three times, the organic layers were washed with NaCl solution (sat. aq.) and dried over $Na_2SO_4$, filtered, and concentrated under reduced pressure. The crude product was purified by chromatography ($SiO_2$, Biotage Ultra 50g column eluent: A = Hexane, B = EtOAc, gradient elution) yielding the title compound (1.99 g, 21 % yield).

LC-MS (Method 1): Rt = 0.85min; MS (ESIpos): m/z = 240.1 $[M+H]^+$.

$^1$H NMR (400 MHz, OMSO-$d_6$) δ ppm 1.08 (t, J=6.97 Hz, 3H), 3.42 (q, J=6.93 Hz, 2H), 3.46 - 3.50 (m, 2H), 3.56 - 3.61 (m, 2H), 3.76 - 3.81 (m, 2H), 4.29 - 4.33 (m, 2H), 7.59 - 7.62 (m, 1H), 7.93 (d, J=8.62 Hz, 1H), 8.51 (d, J=2.79 Hz, 1H), 9.89 (s, 1 H).

**Step 2**

tert-butyl-3-{5-[2-(2-ethoxyethoxy)ethoxy]pyridin-2-yl}oxirane-2-carboxylate

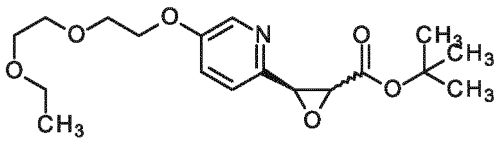

A solution of 5-[2-(2-ethoxyethoxy)ethoxy]pyridine-2-carbaldehyde (2.00 g, 8.36 mmol) in THF (100 ml) was treated portion wise with sodium hydride (468 mg, 60 % purity, 11.7 mmol; [7646-69-7]), the mixture stirred for 5min, then tert-butyl chloroacetate (1.6 ml, 11 mmol) was added dropwise, the mixture stirred at RT for 16h. The reaction mixture was poured onto a mixture of ice and water, it was extracted with EtOAc three times, the organic layers were washed with NaCl (sat. aq.) and dried over $Na_2SO_4$, then concentrated under reduced pressure. The crude product was purified by chromatography (Biotage Sfär 50g column eluent: A = Hexane, B = EtOAc, gradient elution) yielding the cis and trans forms of the product of the title compound (title compound "trans" - 550 mg (19%) + title compound "cis" - 1250 mg (42%).

Title compound "trans".

LC-MS (Method 4): Rt = 1.19min; MS (ESIpos): m/z = 354.1 $[M+H]^+$.

$^1$H NMR (DMSO-$d_6$, 500 MHz): δ (ppm) 8.28 (dd, *J* = 2.5, 1.0 Hz, 1H), 7.41-7.46 (m, 2H), 4.17-4.21 (m, 2H), 4.09 (d, *J* = 1.9 Hz, 1H), 3.82 (d, *J* = 1.9 Hz, 1H), 3.73-3.77 (m, 2H), 3.56-3.59 (m, 2H), 3.47-3.50 (m, 2H), 3.42 (q, *J* = 7.0 Hz, 2H), 1.46 (s, 9H), 1.09 (t, *J* = 7.0 Hz, 3H).

$^{13}$C NMR (DMSO-$d_6$, 126 MHz): δ (ppm) 166.8, 154.9, 145.2, 137.7, 122.6, 121.5, 82.0, 69.9, 69.1, 68.7, 67.6, 65.5, 56.8, 54.7, 27.5 (3C), 15.0.

Title compound "cis".

LC-MS (Method 4): $R_t$ = 1.13min; MS (354.1): m/z = $[M+H]^+$.

$^1$H NMR (DMSO-$d_6$, 400 MHz): δ (ppm) 8.25 (d, *J* = 2.5 Hz, 1H), 7.43 (dd, *J* = 8.6, 2.8 Hz, 1H), 7.28 (d, *J* = 8.6 Hz, 1H), 4.28 (d, *J* = 4.8 Hz, 1H), 4.14-4.18 (m, 2H), 3.91 (d, *J* = 4.8 Hz, 1H), 3.71-3.75 (m, 2H), 3.55-3.58 (m, 2H), 3.46-3.50 (m, 2H), 3.42 (q, *J* = 7.0 Hz, 2H), 1.19 (s, 9H), 1.09 (t, *J* = 7.1 Hz, 3H).

$^{13}$C NMR (DMSO-$d_6$, 126 MHz): δ (ppm) 165.4, 154.5, 145.1, 137.1, 121.8, 121.0, 81.6, 70.0, 69.2, 68.8, 67.7, 65.6, 56.7, 55.2, 27.4 (3C), 15.1.

**Step 3**

tert-butyl-3-{5-[2-(2-ethoxyethoxy)ethoxy]pyridin-2-yl}-2-hydroxypropanoate

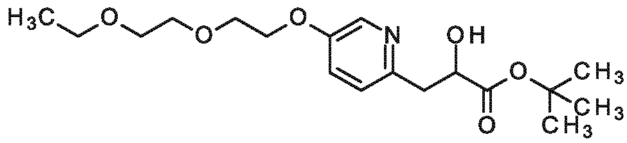

A solution of racemic tert-butyl-3-{5-[2-(2-ethoxyethoxy)ethoxy]pyridin-2-yl}oxirane-2-carboxylate (1.25 g, 3.54 mmol) in EtOAc (15 ml) was treated with palladium/C (131 mg, 10 % purity, 123 μmol; [7440-05-3]) and the mixture cycled between $N_2$ and vacuum three times. The mixture was stirred under a $H_2$ atmosphere (1 atm) at RT for 72h. The reaction mixture was filtered using a glass fiber filter and concentrated under reduced pressure. Purification by chromatography ($SiO_2$, Biotage ULTRA 25g column eluent: A = Hexane, B = EtOAc, gradient elution), yielded the title racemic title compound **(Intermediate 17,** 1.05 g, 84 % yield).

LC-MS (Method 1): $R_t$ = 0.84min; MS (ESIpos): m/z = 356.2 $[M+H]^+$.

$^1H$ NMR (DMSO-$d_6$, 400 MHz): δ (ppm) 8.18 (d, *J* = 2.8 Hz, 1H), 7.31 (dd, *J* = 8.6, 3.0 Hz, 1H), 7.18 (d, *J* = 8.6 Hz, 1H), 5.36 (d, *J* = 6.3 Hz, 1H), 4.24 (dt, *J* = 8.0, 5.9 Hz, 1H), 4.10-4.16 (m, 2H), 3.71-3.76 (m, 2H), 3.54-3.60 (m, 2H), 3.47-3.50 (m, 2H), 3.42 (q, *J* = 6.8 Hz, 2H), 2.97 (dd, *J* = 13.7, 5.3 Hz, 1H), 2.87 (dd, *J* = 13.9, 8.1 Hz, 1H), 1.35 (s, 9H), 1.09 (t, *J* = 7.1 Hz, 3H).

**Intermediate 18**

**Step 1**

(3E)-2-oxo-4-(4-propoxyphenyl)but-3-enoic acid

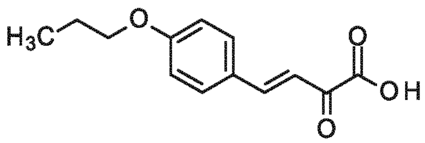

Under an atmosphere of $N_2$, to a mixture of 4-propoxybenzaldehyde (5.00 g, 30.4 mmol, [5736-85-6]) in MeOH (4 ml), was added 2-oxopropanoic acid (2.68 g, 30.4 mmol; [127-17-3]). To this mixture was added a mixture of potassium hydroxide in MeOH (25 weight % solution, 45.7 mmol) while maintaining the reaction temperature below 15°C. A small portion of additional MeOH was added and the mixture allowed to warm to RT and stirred overnight. The mixture was concentrated under reduced pressure, added to a HCl (1.5 M aq.) solution and the aqueous mixture extracted with EtOAc. The organic phases were combined, washed with a NaCl solution (sat. aq.), dried over $Na_2SO_4$ and concentrated under reduced pressure yielding the crude title compound (3.24 g, (80% purity), 45 % yield). The large J-coupling value for the alkene signals suggests assignment of the (E) configuration.

LC-MS (Method 1): $R_t$ = 1.06min; MS (ESIpos): m/z = 235.1 $[M+H]^+$.

$^1H$ NMR (400 MHz, OMSO-$d_6$) δ ppm 0.98 (t, J=7.48 Hz, 3H), 1.68 - 1.79 (m, 2H), 3.97 - 4.04 (m, 2H), 6.97 - 7.03 (m, 2H), 7.13 (d, J=16.22 Hz, 1H), 7.70 (d, J=16.22 Hz, 1H), 7.74 - 7.80 (m, 2H).

**Step 2**

2-hydroxy-4-(4-propoxyphenyl)butanoic acid

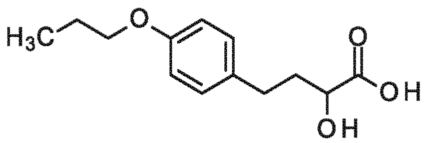

(3E)-2-xo-4-(4-propoxyphenyl)but-3-enoic acid (6.89 g, 29.4 mmol) was dissolved in ethanol (200 ml), Pd/C (1.56 g, 10 % purity, 1.47 mmol; [7440-05-3]) added, the mixture cycled between $N_2$ and vacuum three times and hydrogenated (1 atm $H_2$) overnight. The mixture was filtered through a glass fiber filter, and concentrated under reduced pressure, following which chromatography ($SiO_2$, Biotage, Ultra 100g column, A = Hexane, B = EtOAc, 0%B to 100%B) yielded the racemic title compound (6.50 g, 93 % yield).

LC-MS (Method 1): Rt = 1.01min; MS (ESIneg): m/z = 237 $[M-H]^-$.

$^1H$ NMR (400 MHz, DMSO-$d_6$) δ ppm 0.96 (t, J=7.48 Hz, 3H), 1.64 - 1.92 (m, 4H), 2.54 - 2.62 (m, 2H), 2.58 (t, J=7.86 Hz, 2H), 3.87 (t, J=6.46 Hz, 3H), 5.20 - 5.27 (br. s, 1H), 6.80 - 6.85 (m, 2H), 7.05 - 7.11 (m, 2H), 12.21 - 12.58 (br. s, 1 H).

methyl-2-hydroxy-4-(4-propoxyphenyl)butanoate

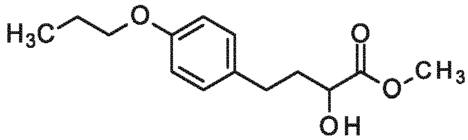

Racemic 2-hydroxy-4-(4-propoxyphenyl)butanoic acid (6.50 g, 27.3 mmol) was dissolved in MeOH (50 ml) and stirred with a drop of conc. $H_2SO_4$ at RT overnight. The mixture was concentrated under reduced pressure, $NaHCO_3$ (sat. aq.) was added, the mixture extracted with EtOAc, dried over $Na_2SO_4$ and concentrated under reduced pressure yielding the title compound **(Intermediate 18,** 3.85 g, 56 % yield).

LC-MS (Method 1): Rt = 1.14min; MS (ESIpos): m/z = 254 $[M+H]^+$.

1H-NMR (400MHz, DMSO-$d_6$): δ [ppm]= 0.96 (t, 3H), 1.65 - 1.90 (m, 4H), 2.52 - 2.60 (m, 2H), 3.61 (s, 3H), 3.87 (t, 2H), 3.94 - 4.04 (m, 1H), 5.47 (d, 1H), 6.79 - 6.86 (m, 2H), 7.05 - 7.11 (m, 2H).

**Intermediate 19**

2-oxooxolan-3-yl methanesulfonate

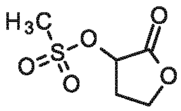

3-hydroxyoxolan-2-one (2.50 g, 24.5 mmol, [19444-84-9]), THF (82 ml) and TEA (7.5 ml, 54 mmol;) were stirred at 0°C under $N_2$. MsCl (2.1 ml, 27 mmol; [124-63-0]) was added dropwise, and the mixture stirred for 30min at 0°C followed by stirring overnight at RT. The mixture was filtered, and the filtrate concentrated under reduced pressure, affording the title compound **(Intermediate 19,** 5.15 g, 82 % yield) also containing some TEA hydrochloride salt.

$^1H$ NMR (400 MHz, DMSO-$d_6$) δ ppm 2.39 (m, 1H), 2.73 (dddd, J=12.71, 8.40, 6.15, 1.90 Hz, 1H), 3.33 (s, 3H), 4.26 (ddd, J=10.14, 8.74, 6.21 Hz, 1H), 4.43 (td, J=8.93, 1.90 Hz, 1H), 5.55 (dd, J=9.76, 8.49 Hz, 1H).

**Intermediate 20**

**Step 1**

(2S)-2-(tert-butoxycarbonylamino)-5-(4-ethoxyphenyl)pentanoate

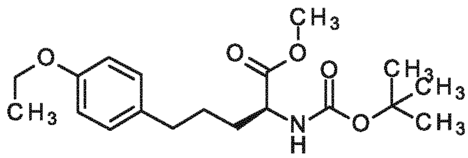

To a solution of methyl (2S)-2-[[(1,1-dimethylethoxy)carbonyl]amino]-4-pentenoate (60.0 g, 261 mmol, [89985-87-59]) in THF (600 mL) was added 9-BBN (0.50 M, 1.05 L, [280-64-8]) at 0°C for 30min. The reaction mixture was stirred at 20°C for 1 h. Then $K_3PO_4$ (3.00 M, aq., 87.2 mL) was added to the reaction, followed by addition to a mixture of 4-Bromophenetole (57.8 g, 287 mmol, [588-96-5]), $Pd(OAc)_2$ (2.94 g, 13.0 mmol, [3375-31-3]) and SPhos (10.7 g, 26.2 mmol, [657408-07-6]) in DMF (900 mL) at 20°C. After the reaction mixture was stirred at 60°C for 12 h, KCl (195 g, 2.62 mol) and sat. $NaHCO_3$ (1000 mL) was added and stirred at 20°C for 1 h. TLC (PE : EtOAc = 5 : 1) showed the starting material ($R_f$ = 0.8) was consumed and a large new spot ($R_f$ = 0.4) was detected. The crude reaction mixture was worked up together with an earlier experiment (74 g, 323 mmol) methyl (2S)-2-[[(1,1-dimethylethoxy)carbonyl]amino]-4-pentenoate. The combined reaction mixture was poured into water (4.0 L) and extracted with EtOAc (1.0 × 3). The combined organic phase was washed with NaCl (sat. aq. 1.0 L × 3), dried over $Na_2SO_4$, filtered and concentrated under reduced pressure to give a residue. The crude product was purified by chromatography ($SiO_2$, A = PE, B = EtOAc, A:B = 30:1 to 20:1) to give the title compound (162 g, 79%) as a yellow oil.

LC-MS (Method 7): Rt = 0.824min, m/z = 252 $[M + H - C_4H_8 - CO_2]^+$.

$^1$H NMR : (400 MHz $CDCl_3$) δ 7.04-7.06 (m, 2H), 6.80-6.82 (m, 2H), 3.99 (dd, *J* = 14, 14 Hz, 2H), 3.72 (s, 3H), 2.55-2.57 (m, 2H), 1.84-1.87 (m, 4H), 1.63-1.66 (m, 7H), 1.44 (s, 9H), 1.38-1.42 (m, 3H).

**Step 2**

(2S)-2-amino-5-(4-ethoxyphenyl)pentanoic acid hydrochloride salt

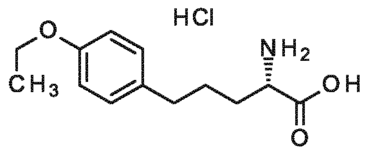

To a solution of the methyl (2S)-2-(tert-butoxycarbonylamino)-5-(4-ethoxyphenyl)pentanoate (95.0 g, 270 mmol) was added aq. HCl (6.00 M, 2.25 L) at 20°C. The reaction mixture was stirred at 100°C for 3 h. The reaction mixture was concentrated under reduced pressure to give the title compound (128 g, >100%) as a yellow solid.

LC-MS: (Method 8): $R_t$ = 0.513min, MS = 238 $[M+H]^+$.

$^1$H NMR: EW16671-49-P1B1, 400 MHz MeOD) δ 7.08-7.11 (m, 2H), 6.81-6.83 (m, 2H), 3.94-4.02 (m, 3H), 2.61-2.64 (m, 2H), 1.72-1.94 (m, 4H), 1.34-1.38 (m, 3H).

**Step 3**

(2S)-5-(4-ethoxyphenyl)-2-hydroxy-pentanoic acid

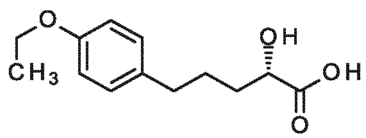

To a solution of (2S)-2-amino-5-(4-ethoxyphenyl)pentanoic acid hydrochloride salt (32.0 g, 117 mmol) in $H_2SO_4$ (0.50 M, aq., 320 mL) and THF (320 mL) was added $NaNO_2$ (23.4 g, 338 mmol, 160 mL) at 0°C for 3 h. The reaction mixture was stirred at 0°C for 2h and stirred at 20°C for 12 h. The reaction mixture combined with another identical preparation (30 g) and the combined reaction mixture was extracted with EtOAc (1.0 L × 3). The combined organic phase was washed with sat. NaCl (500 mL × 3), dried over $Na_2SO_4$, filtered and concentrated under reduced pressure to give (2S)-5-(4-ethoxyphenyl)-2-hydroxy-pentanoic acid (66.0 g, >100%) as yellow oil.

LC-MS: (Method 9): $R_t$ = 0.178min, = 237 $[M-H]^-$.

**Step 4**

methyl (2S)-5-(4-ethoxyphenyl)-2-hydroxypentanoate

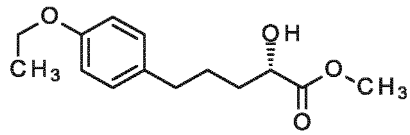

To a solution of (2S)-5-(4-ethoxyphenyl)-2-hydroxy-pentanoic acid (28.0 g, 117 mmol) in DCM (60.0 mL) and MeOH (30.0 mL) was added $TMSCHN_2$ (2.00 M, 235 mL, [18107-18-1]) dropwise at 0°C for 30min. The reaction mixture was stirred at 20°C for 2 h. TLC (PE : EtOAc = 3 : 1) showed that the starting material ($R_f$= 0.25) was consumed completely and a large new spot ($R_f$ = 0.6) was detected. The reaction mixture was combined with an identical reaction (10 g). To the combined reaction mixture was added $CH_3COOH$ (50 mL) dropwise. The mixture was quenched with aq. 10% $NaHCO_3$ (500 mL) and extracted with DCM (500 mL × 3). The combined organic phase was washed with sat. NaCl (500 mL × 3), dried over $Na_2SO_4$, filtered and concentrated under reduced pressure. The crude product was purified by column chromatography ($SiO_2$, A = PE, B = EtOAc, A:B = 30:1 to 20:1) to give the title compound **(Intermediate 20,** 19.0 g, 75.3 mmol, 47% yield) as yellow oil.
LC-MS: (Method 10): $R_t$ = 0.850min, = 253 $[M+H]^+$.
SFC: (Chiralpak AD-3 50×4.6mm I.D., 3μm, A = $CO_2$, B = MeOH(0.05%DEA); 5% B to 40% B; 3mL/min; DAD; 35°C; 100Bar): $R_t$ = 1.10min (100%).
$^1$H NMR : (400 MHz $CDCl_3$) δ 7.07-7.09 (m, 2H), 6.81-6.84 (m, 2H), 4.20-4.21 (m, 1H), 3.99-4.04 (m, 2H), 3.77 (s, 3H), 2.74 (d, *J* = 6Hz, 1H), 2.57-2.62 (m, 2H), 1.63-1.84 (m, 4H), 1.42 (t, *J* = 6.8 Hz, 3H).

**Intermediate 21**

ethyl 3-(4-ethoxyphenyl)-2-hydroxypropanoate

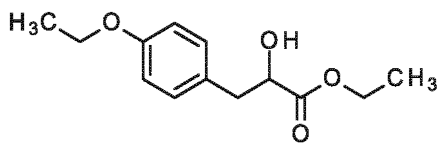

ethyl 2-hydroxy-3-(4-hydroxyphenyl)propanoate (2.50 g, 11.9 mmol, [62517-34-4]) was dissolved in DMF (86 ml) and cesium carbonate (4.26 g, 13.1 mmol; [534-17-8]) was added. The mixture was placed under $N_2$ and cooled to 0°C. Iodoethane (1.24 ml, 15.5 mmol; [75-03-6]) was added dropwise and the reaction stirred at RT overnight. NaCl (3.1 M, aq.) solution was added and the mixture extracted with MTBE (3x). The combined organic phases were washed with NaCl (sat. aq.), dried over $Na_2SO_4$ and concentrated under reduced pressure. The raw product obtained was purified by chromatography ($SiO_2$, A = Hexane, B = EtOAc, 0%B to 100%B) to give the title compound **(Intermediate 21,** 1.19 g, 40 % yield).
LC-MS (Method 4): Rt = 1.04min; MS (ESIpos): m/z = 239 $[M + H]^+$.
$^1$H NMR (DMSO-$d_6$, 400 MHz): δ (ppm) 7.06-7.13 (m, 2H), 6.73-6.88 (m, 2H), 5.48 (d, *J* = 6.1 Hz, 1H), 4.15 (dt, *J* = 7.8, 5.7 Hz, 1H), 4.04 (q, *J* = 7.1 Hz, 2H), 3.97 (q, *J* = 6.8 Hz, 2H), 2.85 (dd, *J* = 13.7, 5.3 Hz, 1H), 2.75 (dd, *J* = 13.7, 7.6 Hz, 1H), 1.30 (t, *J* = 7.0 Hz, 3H), 1.13 (t, *J* = 7.1 Hz, 3H).

**Intermediate 22**

**Step 1**

methyl (2R)-[5-[4-butoxy]phenyl]-2-[[(1,1-dimethylethoxy)carbonyl]amino]-pentanoate

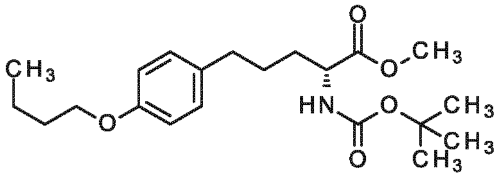

To a solution of methyl (2R)-2-[[(1,1-dimethylethoxy)carbonyl]amino]-4-pentenoate (20.1 g, 88 mmol, [150652-96-3]) in THF (200 mL) was added 9-BBN (0.50 M, 349 mL) at 0°C. The reaction mixture was stirred at 16°C for 1h. Then $K_3PO_4$ (3 M, 29.2 mL) was added, followed by addition to a mixture of 1-bromo-4-butoxybenzene (20.0 g, 87.3 mmol, [39969-57-8]), $Pd(OAc)_2$ (980 mg, 4.37 mmol), SPhos (3.60 g, 8.77 mmol, [657408-07-6]) in DMF (500 mL). To the reaction mixture after stirring under $N_2$ at 60°C for 18h, aq. $NaHCO_3$ (1.00 L) and potassium chloride (651 g, 8.73 mol) was added and stirred for 2h at 16°C. The reaction mixture was poured into water (1.00 L) and extracted with EtOAc (500 mL × 3). The combined organic phase was washed with NaCl (sat. aq.) (500 mL × 3), dried over $Na_2SO_4$, filtered and concentrated under reduced pressure to give the title compound (60.0 g, crude) as yellow oil.

LC-MS (Method 12): Rt = 1.177min, MS: 379.24 $[M+H]^+$.

**Step 2**

(2R)-2-amino-[5-[4-butoxy]phenyl]-pentanoic acid

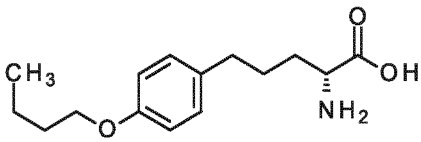

To methyl (2R)-[5-[4-butoxy]phenyl]-2-[[(1,1-dimethylethoxy)carbonyl]amino]-pentanoate (74.0 g, 195 mmol) was added HCl (6 M, aq., 1.63 L) at 16°C. The reaction mixture was stirred at 100°C for 12h. The reaction mixture was concentrated under reduced pressure to give the title compound (49.0 g, 185 mmol, 94.70% yield) as white solid.

$^1H$ NMR : (400 MHz, $CDCl_3$) $\delta$ 7.10 (d, $J$ = 8.8 Hz, 2H), 6.82 (d, $J$ = 8.8 Hz, 2H), 3.96 - 3.91 (m, 2H), 2.64 - 2.60(m, 2H), 1.75 - 1.70 (m, 6H), 1.52 - 1.36 (m, 2H), 1.00 - 0.96 (m, 2H).

**Step 3**

(2R)-[5-[4-butoxy]phenyl]-2-hydroxy-pentanoic acid

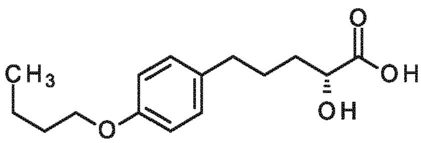

To a solution of (2R)-2-amino-[5-[4-butoxy]phenyl]-pentanoic acid (13.0 g, 49.0 mmol) in THF (130 mL) and $H_2SO_4$ (0.50 M, 130 mL) was added $NaNO_2$ (9.50 g, 138 mmol) in $H_2O$ (65.0 mL) at 0°C for 3h, then stirred for 12h at 20°C. The reaction mixture was concentrated under reduced pressure to give (2R)-[5-[4-butoxy]phenyl]-2-hydroxy-pentanoic acid (10.0 g, crude) as a white solid.

LCMS (Method 11): $R_t$ = 1.19min, MS = 265.1 $[M-H]^-$.

**Step 4**

methyl (2R)-5-(4-butoxyphenyl)-2-hydroxypentanoate

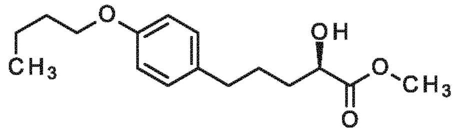

To a solution of (2R)-[5-[4-butoxy]phenyl]-2-hydroxy-pentanoic acid (33.0 g, 124 mmol) in DCM/MeOH (3/1, 440 mL) was added $TMSCHN_2$ (55.0 g, 482 mmol, [18107-18-1]) at -10°C and stirred for 12h at 20°C. TLC (PE: EA=20:1) showed the acid to be consumed completely. The reaction mixture was concentrated under reduced pressure to give a residue. The crude product was purified by prep-HPLC (Phenomenex Luna C18 250 × 80mm × 10 um; A = [water(0.05% HCl), B = MeCN, 45B% to 75%B in 19min) to give the title compound **(Intermediate 22, 7.00** g, 25.0 mmol, 20% yield) as yellow oil.

LC-MS (Method 7): Rt = 0.931min, MS: 281.1 $[M+H]^+$.

SFC: (Chiralpak AD-3 50×4.6mm I.D., 3μm; A = $CO_2$, B = MeOH (0.05%DEA); 5%B to 40%B, 3mL/min; DAD; 35°C; 100Bar): $R_t$ = 1.20min (94%), $R_t$ = 1.27min (6%); e.e. = 88%.

Specific rotation: $\alpha_D^{20}$ = -5.4° +/- 0.24° (c=1, $CHCl_3$).

$^1H$ NMR (400 MHz, $CDCl_3$) δ 7.04-7.10 (m, 2H), 6.78-6.84 (m, 2H), 4.20 (dd, J=4.06, 7.10 Hz, 1H), 3.93 (t, J=6.59 Hz, 2H), 3.77 (s, 3H), 2.75 (br, s, 1H), 2.51-2.64 (m, 2H), 1.60-1.86 (m, 6H), 1.48 (qt, J=7.40, 7.60 Hz, 2H), 0.97 (t, J=7.48 Hz, 3H).

$^{13}C$ NMR (101 MHz, $CDCL_3$) δ 175.7, 157.3, 133.7, 129.2 (2C), 114.3 (2C), 70.3, 67.7, 52.5, 34.5, 33.9, 31.4, 26.7, 19.3, 13.9.

**Intermediate 23**

**Step 1**

ethyl (2RS,3SR)-3-[4-(2,2,3,3-tetrafluoropropoxy)phenyl]oxirane-2-carboxylate

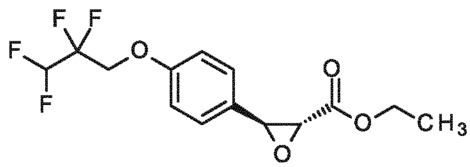

Sodium hydride (1.78 g, 60% in mineral oil, 44.5 mmol) was suspended in THF (190 ml), placed under $N_2$ and cooled to 0°C. Ethyl chloroacetate (4.7 ml, 44 mmol) was added dropwise followed by addition of 4-(2,2,3,3-tetrafluoropropoxy)benzaldehyde (7.00 g, 29.6 mmol, [103962-17-0]) in THF (10 ml). The reaction was stirred at RT overnight. The reaction was poured in ice water and was extracted with EtOAc (3 x). The combined organic phases were washed with NaCl (sat. aq.), dried over $Na_2SO_4$ and concentrated under reduced pressure. Chromatography ($SiO_2$, SNAP Ultra 100 g, A = Hexane, B = EtOAc, 0%B to 50%B in 30min, 80 ml/min,) gave 5.21 g (52%) of the title compound.

LC-MS (Method 4): $R_t$ = 1.23min; MS (ESIpos): m/z = 323.1 $[M + H]^+$.

$^1H$ NMR (DMSO-$d_6$, 400 MHz): δ (ppm) 7.32-7.37 (m, 2H), 7.00-7.12 (m, 2H), 6.68 (tt, $^2J_{HF}$ = 51.7, $^3J_{HF}$ = 5.6 Hz, 1H), 4.60 (t, $^3J_{HF}$ = 13.4 Hz, 2H), 4.15-4.24 (m, 2H), 4.12 (d, $J$ = 1.8 Hz, 1H), 3.82 (d, $J$ = 1.8 Hz, 1H), 1.24 (t, $J$ = 7.1 Hz, 3H).

$^{19}F$ NMR (DMSO-$d_6$, 377 MHz): δ (ppm) -125.27 (tq, $J$ = 13.7, 5.7 Hz, 1F), -139.69 (dt, $J$ = 52.4, 5.3 Hz, 2F).

$^{13}C$ NMR (DMSO-$d_6$, 101 MHz): δ (ppm) 167.9, 157.8, 128.4, 128.0 (2C), 115.2 (2C), 115.0 (tt, $^1J_{CF}$=248.8, $^2J_{CF}$=26.3 Hz), 109.3(tt, $^1J_{CF}$=247.8, $^2J_{CF}$=32.7 Hz), 64.7 (t, $^2J_{CF}$=27.6 Hz), 61.3, 56.8, 55.7, 14.0.

**Step 2**

ethyl 2-hydroxy-3-[4-(2,2,3,3-tetrafluoropropoxy)phenyl]propanoate

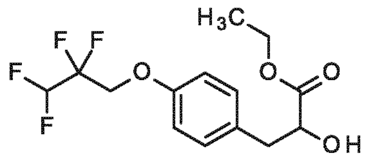

Ethyl 3-[4-(2,2,3,3-tetrafluoropropoxy)phenyl]oxirane-2-carboxylate (5.21 g, 16.2 mmol) was dissolved in ethanol (73 ml) and palladium (417 mg, 10 % on carbon, 392 μmol) was added. The mixture was hydrogenated at RT and ambient pressure overnight. The catalyst was filtered off and the filtrate concentrated under reduced pressure. The residue was purified by chromatography ($SiO_2$, SNAP Ultra 50 g, 40min/min, A= n-hexane, B = EtOAc, 0%B to 50%B in 30min) to give the title compound **(Intermediate 23,** 4.42 g, 80%).

$^{19}$F NMR (DMSO-$d_6$, 377 MHz): δ (ppm) -125.32 (ttd, J = 13.8, 5.7, 5.4 Hz, 2F), -139.78 (dt, J = 52.6, 5.7 Hz, 2F).

$^1$H NMR (DMSO-$d_6$, 400 MHz): δ (ppm) 7.14-7.18 (m, 2H), 6.92-6.97 (m, 2H), 6.67 (tt, *J* = 52.0, 5.6 Hz, 1H), 5.51 (d, *J* = 6.1 Hz, 1H), 4.53 (t, *J* = 13.4 Hz, 2H), 4.17 (dt, *J* = 7.8, 5.6 Hz, 1H), 4.05 (q, *J* = 7.1 Hz, 2H), 2.88 (dd, *J* = 13.7, 5.3 Hz, 1H), 2.78 (dd, *J* = 13.7, 7.9 Hz, 1H), 1.14 (t, *J* = 7.1 Hz, 3H).

$^{13}$C NMR (DMSO-$d_6$, 101 MHz): δ (ppm) 173.5, 155.9, 131.1, 130.5 (2C), 115.2 (tt, $^1J_{CF}$ =249.2, $^2J_{CF}$ 26.3 Hz), 114.5 (2C), 109.3 (tt, $^1J_{CF}$ =247.8, $^2J_{CF}$ 32.8 Hz), 71.3, 64.7 (t, $^2J_{CF}$ =27.6 Hz), 60.0, 39.2, 14.1.

**Intermediate 24**

**Step 1**

methyl (2R)-2-hydroxypent-4-enoate

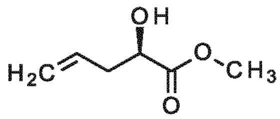

To a solution of copper(I) bromide dimethyl sulfide complex (20.1 g, 98.0 mmol; [54678-23-8]) in THF (400 ml) was added bromido(ethenyl)magnesium (240 ml, 240 mmol, 1 M in THF; [1826-67-1]) at -70°C. The mixture was stirred at -70°C for 0.5h. Then to the above mixture was added a solution of methyl (2R)-oxirane-2-carboxylate (20.0 g, 196 mmol; [111058-32-3]) in THF (200 ml) at -70°C. The mixture was stirred at -70°C for 1h. The mixture was quenched with $NH_4Cl$ (sat. aq.) and extracted with EtOAc. The organic phase was dried over anhydrous $Na_2SO_4$, filtered and concentrated to give a residue. The residue was purified by flash column chromatography ($SiO_2$, A = PE, B = EtOAc, 1 : 0 to 9 : 1, then 9 : 1 A: B) to give methyl (2R)-2-hydroxypent-4-enoate (6.30 g, 25% yield) as yellow oil.

$^1$H NMR (400 MHz, OMSO-$d_6$) δ [ppm] = 5.87-5.69 (m, 1H), 5.54-5.43 (m, 1H), 5.11-4.99 (m, 2H), 4.15-4.06 (m, 1H), 3.62 (s, 3H), 2.45-2.25 (m, 2H).

**Step 2**

methyl (2R,4E)-5-(3-butoxyphenyl)-2-hydroxypent-4-enoate

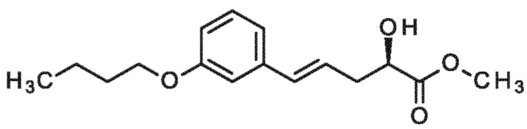

A mixture of 1-butoxy-3-iodobenzene (12.7 g, 46.1 mmol; [103030-54-2]), methyl (2R)-2-hydroxypent-4-enoate (6.00 g, 46.1 mmol), palladium(II)diacetate (1.04 g, 4.61 mmol; [3375-31-3]), tri-2-tolylphosphine (1.40 g, 4.61 mmol; [6163-58-2]) and DIPEA (20 ml, 120 mmol; [7087-68-5]) in MeCN (150 ml) was stirred at 80°C for 12h. TLC (PE : EtOAc = 2 :1) showed the iodide to be consumed and one new main spot was formed. The mixture was concentrated and diluted with EtOAc. The mixture was washed with $NH_4Cl$ (sat. aq.) solution and NaCl (sat. aq.). The organic phase was dried over anhydrous $Na_2SO_4$ filtered and concentrated to give a residue. The residue was purified by flash column chromatography

($SiO_2$, PE : EtOAc = 10 : 1, to 4 : 1, then 4 : 1) to give the title compound as a yellow oil. The material was combined with the material from a preceding experiment: 6.80 g (50 % yield).

LCMS: (Method 13): $R_t$ = 1.001min; MS (ESIpos): m/z = 279.2 $[M+H]^+$.

$^1$H-NMR (400 MHz, DMSO-$d_6$) δ [ppm] = 7.20 (t, *J* = 8.0 Hz, 1H), 6.95-6.88 (m, 2H), 6.80-6.74 (m, 1H), 6.44-6.36 (m, 1H), 6.30-6.19 (m, 1H), 5.57 (d, *J* = 6.0 Hz, 1H), 4.22-4.15 (m, 1H), 3.95 (t, *J* = 6.4 Hz, 2H), 3.63 (s, 3H), 2.60-2.52 (m, 1H), 2.48-2.37 (m, 1H), 1.75-1.61 (m, 2H), 1.50-1.37 (m, 2H), 0.93 (t, *J* = 7.6 Hz, 3H).

**Step 3**

methyl (2R)-5-(3-butoxyphenyl)-2-hydroxypentanoate

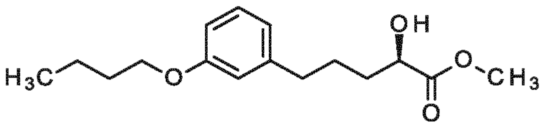

To a solution of methyl (2R,4E)-5-(3-butoxyphenyl)-2-hydroxypent-4-enoate (6.30 g, 22.6 mmol) in methanol was added palladium (10% on carbon) at room temperature. The mixture was stirred at room temperature under $H_2$ atmosphere (15 Psi) for 5 h. The mixture was filtered and combined with the pilot experiment. The combined filtrates were concentrated under reduced pressure to give the title compound **(Intermediate 24,** 5.80 g, 82 % yield) as a yellow oil.

LCMS: (Method 9): $R_t$ = 0.997min; MS (ESIpos): m/z = 281.2 $[M+H]^+$.

Specific Rotation: $\alpha_D^{20}$ = -6.54° +/- 0.16° (c=1, $CHCl_3$).

$^1$H NMR ($CDCl_3$, 400MHz): δ (ppm) 7.15-7.20 (m, 1H), 6.70-6.77 (m, 3H), 4.21 (dd, J=7.1, 3.8 Hz, 1H), 3.95 (t, J=6.6 Hz, 2H), 3.77 (s, 3H), 2.55-2.68 (m, 2H), 1.61-1.88 (m, 6H), 1.44-1.55 (m, 2H), 0.98 (t, J=7.4 Hz, 3H).

$^{13}$C NMR (CHLOROFORM-d, 101MHz): δ (ppm) 175.6, 159.2, 143.4, 129.2, 120.6, 114.8, 111.6, 70.3, 67.5, 52.5, 35.5, 33.9, 31.4, 26.4, 19.3, 13.9.

**Intermediate 25**

**Step 1**

Ethyl-2-hydroxy-3-methoxypropanoate

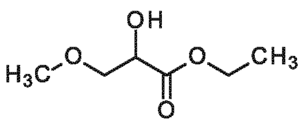

Ethyl oxirane-2-carboxylate (2.5 g, 21.5 mmol, [4660-80-4]) was dissolved in MeOH (1 ml, 32 mmol) in a crimp sealable vial. Magnesium triflate (1.74 g, 5.38 mmol; [60871-83-2]) was added, the vial crimped shut and the mixture stirred at 40°C until consumption of the starting materials (16h) had occurred. The mixture was filtered and purified by chromatography ($SiO_2$, A = Hexane, B = EtOAc, gradient elution), yielding the title compound (771 mg, 24% yield).

$^1$H NMR (400 MHz, DMSO-$d_6$) δ ppm 1.19 (t, 3H), 3.25 (s, 3H), 3.50 (dd, 2H), 4.10 (q, 2H), 4.17 (dt, 1H), 5.51 (d, 1 H).

**Step 2**

ethyl-2-[(methanesulfonyl)oxy]-3-methoxypropanoate

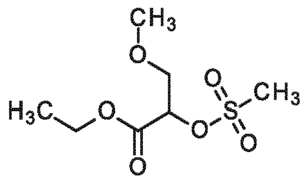

Racemic ethyl-2-hydroxy-3-methoxypropanoate (771 mg, 5.20 mmol) and TEA (1.6 ml, 11 mmol;) were added to a solution of THF (15 ml) and the mixture cooled to 0-5°C. Under $N_2$, MsCl (440 μl, 5.7 mmol; [124-63-0]) was added dropwise, the mixture warmed to RT and stirred for 3h. The mixture was added to aqueous $NaHCO_3$ solution (50% saturation at RT), followed by extraction with MTBE (3 x), the organic phases combined and washed with NaCl (sat. aq.) (2x), dried over $Na_2SO_4$, filtered and concentrated under reduced pressure yielding the title compound **(Intermediate 25,** 780 mg, 63 % yield).

$^1H$ NMR (400 MHz, DMSO-$d_6$) δ ppm 1.21 (t, J=7.10 Hz, 3H), 3.26 (s, 3H), 3.29 (s, 3H), 3.64 - 3.74 (m, 1H), 3.75 - 3.82 (m, 1H), 4.11 - 4.27 (m, 2H), 5.26 - 5.34 (m, 1H).

**Intermediate 26**

**Step 1**

methyl (2R)-3-tert-butoxy-2-hydroxypropanoate

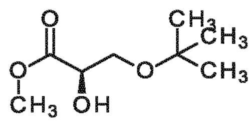

Into a three-necked round bottom flask equipped with mechanical stirrer were placed 2-methylpropan-2-ol (370 ml, 3.9 mol) and magnesium trifluormethanesulfonate (79.0 g, 245 mmol; [60871-83-2]) and EtOAc (370 ml) were added. The reaction was placed under $N_2$ and brought to 50°C (measured in flask). Then methyl (2R)-oxirane-2-carboxylate (21 ml, 240 mmol, [111058-32-3]) were added dropwise and the mixture was stirred at 60°C for 3.5d. After cooling to RT, EtOAc was added. The reaction mixture was washed twice with NaCl (3.1 M aq.) and the organic phase concentrated under reduced pressure. The residue was co-distilled with toluene 3 times to remove unreacted methyl glycidate. The resulting raw product was purified by chromatography (Isolera LS, Sfär 350 g, A = Hexane, B = DCM, C = EtOAc, A = 3CV, A to B in 5 CV, B 4 CV, B to 50%C in 6CV). The interesting fractions were pooled, concentrated under reduced pressure and co-distilled with toluene once to give the title compound 37.3 g (68 as a clear oil.

$^1H$ NMR ($CDCl_3$, 400 MHz): δ (ppm) 3.77 (s, 3H), 3.60-3.66 (m, 2H), 1.15 (s, 9H).

**Step 2**

methyl (2R)-3-tert-butoxy-2-[(trifluoromethanesulfonyl)oxy]propanoate

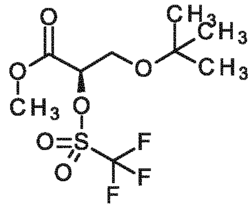

methyl (2R)-3-tert-butoxy-2-hydroxypropanoate (5.00 g, 28.4 mmol) in DCM (4.0 ml) was cooled to -70°C under $N_2$ and lutidine (4.0 ml, 34 mmol) was added. Trifluoromethanesulfonic anhydride (30 ml, 1.0 M in DCM, 30 mmol) was added dropwise, after which the mixture was stirred at -70°C for 2.5 h. Then the mixture was allowed to warm to -20°C and was quenched with ice-water. The phases were separated, and the aqueous phase extracted with DCM. The combined organic phases filtered through a water repellent filter and concentrated under reduced pressure. the title compound

**Intermediate 26** (9.45 g (>100%)), which was used without further purification.

Specific rotation: $\alpha_D^{20}$ = +20.9° +/- 0.2° (c=1, $CHCl_3$).

$^{19}F$ NMR ($CDCl_3$, 471 MHz): δ (ppm) -75.86 (s, 1F).

$^1H$ NMR ($CDCl_3$, 500 MHz): δ (ppm) 5.22 (dd, *J* = 6.9, 2.8 Hz, 1H), 3.86 (s, 3H), 3.85 (dd, *J* = 11.0, 2.8 Hz, 1H), 3.80 (dd, *J* = 11.0, 6.9 Hz, 1H), 1.20 (s, 9H). Residual lutidine triflate is easily quantified: δ (ppm) 8.04 (t, *J* = 7.9 Hz, 0.11 H), 7.41 (d, *J* = 7.9 Hz, 0.21 H), 2.82 (s, 0.66H); -79.62 (s, 0.1 F).

**Intermediate 27**

**Step 1**

ethyl-3-tert-butoxy-2-hydroxypropanoate

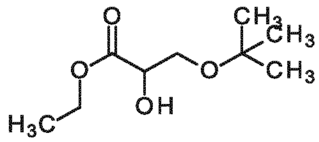

Due to safety concerns, the reaction was split into 3 equal parts: 2-methylpropan-2-ol (8.2 ml, 120 mmol, [75-65-0]), ethyl oxirane-2-carboxylate (15.6 g 134 mmol, [4660-80-4]) and magnesium perchlorate (7.53 g, 33.7 mmol, [10034-81-8]) were placed in equal portions in three crimp sealed vials, sealed and stirred at 50°C for 48h. The three batches were combined, diluted with DCM, then washed with $NH_4Cl$ (sat. aq.) followed by NaCl (sat. aq.). The organic layer was dried over $Na_2SO_4$ and concentrated under reduced pressure, affording the title racemic compound (12.0 g, 52 % yield).

$^1H$ NMR (400 MHz, DMSO-$d_6$) δ ppm 1.09 (s, 9H), 1.19 (t, 3H), 3.46 (dd, 2H), 4.02 - 4.15 (m, 3H), 5.35 (d, 1H).

**Step 2**

ethyl-3-tert-butoxy-2-[(trifluoromethanesulfonyl)oxy]propanoate

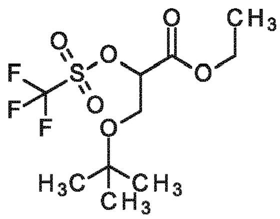

A solution of ethyl-3-tert-butoxy-2-hydroxypropanoate (12.0 g, 63.1 mmol) in DCM (220 ml) was cooled to -60°C, then 2,6-dimethylpyridin (8.8 ml, 75.7 mmol, [108-48-5]) was added and the vessel flushed with $N_2$. Trifluoromethanesulfonic anhydride (1M in DCM, 66.2 mmol, 358-23-6) was added dropwise to the mixture under $N_2$ and it was stirred at -60°C for 3h. The mixture was warmed up to 0°C, then extracted with water and HCl (1M, aq.), dried over $Na_2SO_4$ and the organic layer was concentrated under reduced pressure yielding the title compound **(Intermediate 27,** 19.9 g, 98 % yield). The product was stored at -20°C.

$^1H$ NMR (400 MHz, OMSO-$d_6$) δ ppm 1.15 (s, 9H), 1.25 (t, 3H), 3.74 - 3.87 (m, 2H), 4.19 - 4.33 (m, 2H), 5.42 (m, 1 H).

$^{19}F$ NMR (377 MHz, DMSO-*d*$_6$) δ ppm -78.13 (s, 3 F).

**Intermediate 28**

**Step 1**

methyl (2S)-3-tert-butoxy-2-hydroxypropanoate

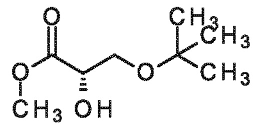

2-methylpropan-2-ol (2.1 ml, 22 mmol), methyl (2S)-oxirane-2-carboxylate (2.1 ml, 24 mmol, [ 118712-39-3]) and magnesium perchlorate (1.37 g, 6.1 mmol, [10034-81-8]) were added together and was stirred at 50°C for 48h. The mixture was treated with DCM, then washed with $NH_4Cl$ (sat. aq.) and NaCl (sat. aq.). The organic layer was dried over $Na_2SO_4$ and concentrated under reduced pressure yielding the title compound (2.70 g, 70 % yield).

Specific Rotation: 10.5° ($CHCl_3$, 20°C, 589 nm).

$^1H$ NMR (400 MHz, OMSO-$d_6$) δ ppm 1.09 (s, 9H), 3.46 (d, J=5.07 Hz, 2H), 3.62 (s, 3H), 4.10 (dt, J=6.27, 4.97 Hz, 1H), 5.40 (d, J=6.59 Hz, 1 H).

**Step 2**

methyl (2S)-3-tert-butoxy-2-[(trifluoromethanesulfonyl)oxy]propanoate

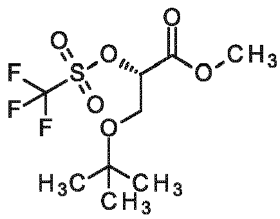

A solution of methyl (2S)-3-tert-butoxy-2-hydroxypropanoate (2.70 g, 15.3 mmol) in DCM (54 ml) was cooled to -60°C, then 2,6-dimethylpyridin (2.1 ml, 18.4 mmol, [108-48-5]) was added and the reaction vessel flushed with $N_2$. Trifluoromethanesulfonic anhydride (1M in DCM, 16.1 mmol, [358-23-6]) was added dropwise to the mixture under $N_2$ and the mixture was stirred at -60°C for 3h. The mixture was warmed up to 0°C, then extracted with water and HCl (1M, aq.), the organic layer dried over $Na_2SO_4$, filtered and concentrated under reduced pressure yielding the title compound 3.66 g **(Intermediate 28,** 77 % yield).

$^1H$ NMR (DMSO-$d_6$, 400 MHz): δ (ppm) 4.10 (t, *J* = 4.9 Hz, 1H), 3.62 (s, 3H), 3.46 (d, *J* = 5.1 Hz, 2H), 1.09 (s, 9H).

**Example 1**

**Step 1**

methyl 3-[2-(4-ethoxyphenyl)ethoxy]-2-[(methanesulfonyl)oxy]propanoate

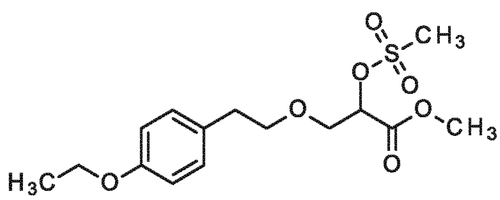

Methyl-3-[2-(4-ethoxyphenyl)ethoxy]-2-hydroxypropanoate **(Intermediate 1,** 1.61 g, 6.02 mmol) and TEA (2.1 ml, 15 mmol; [121-44-8]) in THF (40 ml) were cooled to 0-5°C under $N_2$. MsCl (700 μl, 9.0 mmol; [124-63-0]) was added dropwise, and the mixture was allowed to warm to RT, and it was stirred overnight. The mixture was added to $NaHCO_3$ (50% sat. aq.) and it was extracted with EtOAc (3x), the organic phase washed with NaCl (sat. aq.), dried over $Na_2SO_4$, filtered and concentrated under reduced pressure, yielding the title compound (1.79 g, 86 % yield).

LC-MS (Method 1): $R_t$ = 1.17min; MS (ESIpos): m/z = 364 $[M+H]^+$.

1H-NMR (400MHz, DMSO-$d_6$): δ [ppm]= 1.30 (t, 3H), 2.71 (t, 2H), 3.22 (s, 3H), 3.52 - 3.67 (m, 2H), 3.69 (s, 3H), 3.75 - 3.87 (m, 2H), 3.97 (q, 2H), 5.31 - 5.33 (m, 1H), 6.80 - 6.82 (m, 2H) 7.09 - 7.12 (m, 2H).

## Step 2

methyl 3-[2-(4-ethoxyphenyl)ethoxy]-2-[4,7,10-tris(2-tert-butoxy-2-oxoethyl)-1,4,7,10-tetraazacyclododecan-1-yl]propanoate

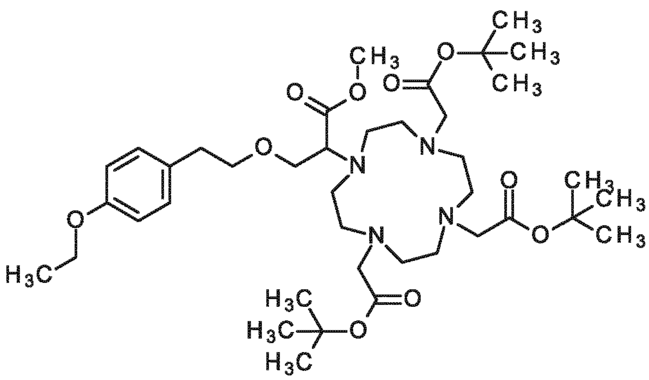

tri-tert-butyl 2,2',2"-(1,4,7,10-tetraazacyclododecane-1,4,7-triyl)triacetate (6.52 g, 12.7 mmol, [122555-91-3]), methyl 3-[2-(4-ethoxyphenyl)ethoxy]-2-[(methanesulfonyl)oxy] propanoate (6.58 g, 19.0 mmol), $K_2CO_3$ (2.63 g, 19.0 mmol) and MeCN (44 ml) were added to a reaction vessel and heated at 80°C overnight. The mixture was filtered, and solid washed with MeCN, the combined organic phases concentrated under reduced pressure yielding the crude product (12,57 g).

LC-MS (Method 2): Rt = 1.18min; MS (ESIpos): m/z = 765.5 $[M+H]^+$.

## Step 3

2,2',2"-(10-{3-[2-(4-ethoxyphenyl)ethoxy]-1-methoxy-1-oxopropan-2-yl}-1,4,7,10-tetraazacyclododecane-1,4,7-triyl) triacetic acid

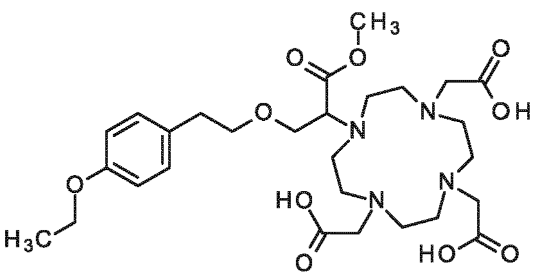

Crude product methyl 3-[2-(4-ethoxyphenyl)ethoxy]-2-[4,7,10-tris(2-tert-butoxy-2-oxoethyl)-1,4,7,10-tetraazacyclododecan-1-yl]propanoate (15.1 g, 19.7 mmol) and formic acid (320 ml) were heated at 50°C for 2h, thereafter the temperature was increased to 70°C for 1h. The mixture was concentrated under reduced pressure yielding the crude product (17,3 g). Purification by RP chromatography (2 × injection of crude mixture in 50% MeOH water, Biotage C18 120 g, A = water/1% formic acid, B = MeOH/1% formic acid, 0%B to 50% in 15CV) yielded the product, containing

approximately 1 equivalent of formic acid (3.38 g, 29% yield).

LC-MS (Method 2): $R_t$ = 0.68min; MS (ESIpos): m/z = 597.3 $[M+H]^+$.

$^1H$ NMR (400 MHz, OMSO-$d_6$) δ ppm 1.30 (t, 3H) 2.67 - 2.73 (m, 4H) 2.80 - 3.14 (m, 14H) 3.41 - 3.54 (m, 8H) 3.57 (s, 3H) 3.73 - 3.81 (m, 3H) 3.97 (q, 2H) 6.80 (dd, 2H) 7.11 (dd, 2H).

**Step 4**

gadolinium 2,2',2"-(10-{1-carboxy-2-[2-(4-ethoxyphenyl)ethoxy]ethyl}-1,4,7,10-tetraazacyclododecane-1,4,7-triyl)triacetate

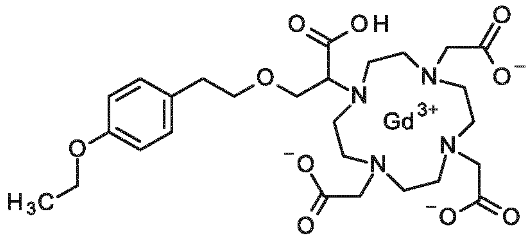

2,2',2"-(10-{3-[2-(4-ethoxyphenyl)ethoxy]-1-methoxy-1-oxopropan-2-yl}-1,4,7,10-tetraazacyclododecane-1,4,7-triyl)triacetic acid (990 mg, 1.66 mmol) and $Gd_2O_3$ (259 mg, 713 μmol) were added to a reaction vessel, water (16 ml) was added and the mixture heated for 18h at 100°C. The pH of the mixture was adjusted to 5 using NaOH (1M, aq.) and 2g Chelex100™ (sodium-form, washed) was added the mixture stirred for 2h at RT. Xylenol-orange test was performed indicating the mixture was free from uncomplexed gadolinium. The mixture was filtered, following which purification by RP-chromatography (Biotage, SNAP Ultra C18 120 g, 50 ml/min, A = water, B = MeCN, 5%B to 50%B in 15C) yielded the title compound (1.41 g, containing water, 115 % yield).

LC-MS (Method 2): $R_t$ = 0.71min; MS (ESIpos): m/z = 738 $[M+H]^+$.

LC-MS (Method 3): Rt = 0.69min; MS (ESIpos): m/z = 369.7 $[M+2H]^{++}$, 738.3 $[M+H]^+$, 747.3 $[2M+2H+H2O]^{++}$. The isotope pattern observed conforms to a gadolinium complex.

**Example 2**

**Step 1**

tert-butyl-3-{4-[2-(2-ethoxyethoxy)ethoxy]phenyl}-2-[(methanesulfonyl)oxy]propanoate

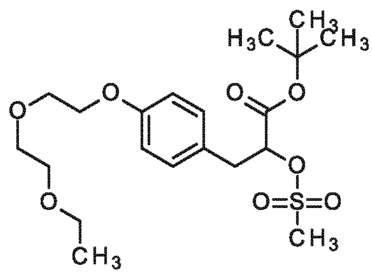

tert-butyl-3-{4-[2-(2-ethoxyethoxy)ethoxy]phenyl}-2-hydroxypropanoate **(Intermediate 2,** 9.68 g, 27.3 mmol) was added to THF (91 ml) under $N_2$, followed by TEA (8.4 ml, 60 mmol; [121-44-8]) and the mixture cooled to 0-5°C. MsCl (2.3 ml, 30 mmol; [124-63-0]) was added dropwise, following which the reaction was warmed to RT and stirred for 3h. The mixture was added to $NaHCO_3$ (50% sat. aq.) and the aqueous layer extracted with EtOAc (3x), the combined organic phases were washed with NaCl (sat. aq. 2x), dried over $Na_2SO_4$, filtered and concentrated under reduced pressure, yielding the title compound (11.7 g, 94 % yield).

LC-MS (Method 1): $R_t$ = 1.26min; MS (ESIpos): m/z = 450.4 $[M+NH_4]^+$.

$^1H$ NMR (DMSO-$d_6$, 400 MHz): δ (ppm) 7.16-7.22 (m, $J$ = 8.6 Hz, 2H), 6.84-6.92 (m, 2H), 5.11 (dd, $J$ = 7.4, 5.8 Hz, 1H), 4.00-4.09 (m, 2H), 3.69-3.74 (m, 2H), 3.55-3.59 (m, 2H), 3.47-3.50 (m, 2H), 3.42 (q, $J$ = 7.0 Hz, 2H), 3.02 (s, 3H),

2.98-3.11 (m, 2H), 1.36 (s, 9H), 1.09 (t, *J* = 7.0 Hz, 3H).

**Step 2**

tert-butyl-3-{4-[2-(2-ethoxyethoxy)ethoxy]phenyl}-2-(1,4,7,10-tetraazacyclododecan-1-yl)propanoate

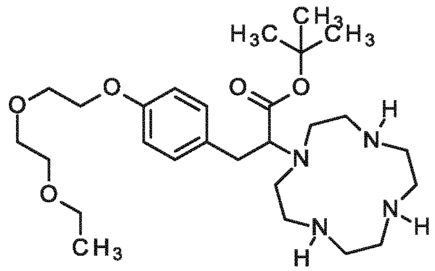

Racemic tert-butyl-3-{4-[2-(2-ethoxyethoxy)ethoxy]phenyl}-2-[(methanesulfonyl)oxy]propanoate (38.5 g, 88.9 mmol), 1,4,7,10-tetraazacyclododecane (18.4 g, 107 mmol) and MeCN (220 ml) were heated under $N_2$ at 70°C overnight. The mixture was filtered and the crude product (45.2 g) used without further purification.

LC-MS (Method 2): Rt = 0.63min; MS (ESIpos): m/z = 509.4 $[M+H]^+$.

**Step 3**

tert-butyl-3-{4-[2-(2-ethoxyethoxy)ethoxy]phenyl}-2-[4,7,10-tris(2-tert-butoxy-2-oxoethyl)-1,4,7,10-tetraazacyclododecan-1-yl]propanoate

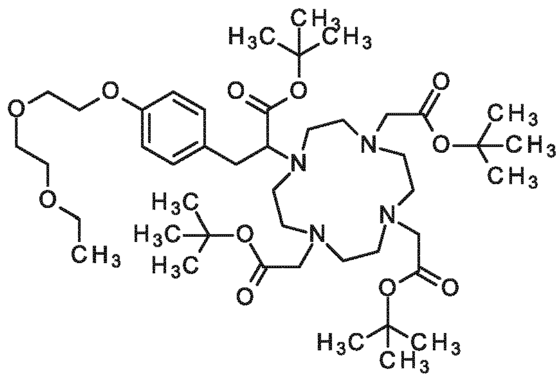

tert-butyl-3-{4-[2-(2-ethoxyethoxy)ethoxy]phenyl}-2-(1,4,7,10-tetraazacyclododecan-1-yl)propanoate (45.0 g, 88.5 mmol) was dissolved in MeCN (450 ml) under $N_2$. DIPEA (77 ml, 440 mmol; [7087-68-5]) was added followed by the dropwise addition of tert-butyl bromoacetate (46 ml, 310 mmol) dissolved in MeCN (15 ml). The mixture was stirred for 5h at 60°C. The mixture was concentrated under reduced pressure, the residue dissolved in EtOAc and washed with $NaHCO_3$ (50% sat. aq.), followed by NaCl (sat. aq.), dried over $Na_2SO_4$, filtered and the solvent removed under reduced pressure yielding the crude title compound (88.5 g).

LC-MS (Method 2): Rt = 1.17min; MS (ESIpos): m/z = 851.4 $[M+H]^+$.

**Step 4**

3-{4-[2-(2-ethoxyethoxy)ethoxy]phenyl}-2-[4,7,10-tris(carboxymethyl)-1,4,7,10-tetraazacyclododecan-1-yl]propanoic acid

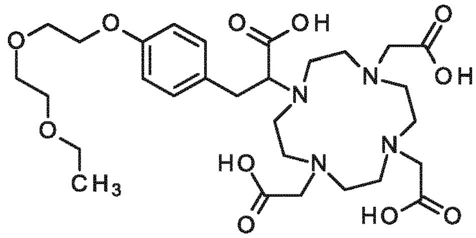

Crude racemic tert-butyl-3-{4-[2-(2-ethoxyethoxy)ethoxy]phenyl}-2-[4,7,10-tris(2-tert-butoxy-2-oxoethyl)-1,4,7,10-tetraazacyclododecan-1-yl]propanoate (88.5 g, 83.2 mmol) was stirred with formic acid (530 ml, 14 mol; [64-18-6]) at 70°C for 4h. The mixture was concentrated under reduced pressure and purified using RP-chromatography (the compound was spilt into two batches, Biotage C18 Ultra 400g, 70 ml/min, A = water, B = MeCN, 0%B to 50%B in 30min, 100%B 10min) yielding the title compound (24.8 g, 48 % yield over 4 steps) and a further fraction (8.4 g, maximum 16 % yield) of lower purity product.

LC-MS (Method 2): $R_t$ = 0.59min; MS (ESIpos): m/z = 627.3 $[M+H]^+$.

$^1H$ NMR (400 MHz, OMSO-$d_6$) δ ppm 1.09 (t, 3H) 2.67 - 3.1 (m, 15H) 3.35 - 3.61 (m, 12H) 3.64 - 3.79 (m, 2H) 3.92 - 4.12 (m, 2H) 6.79 - 6.83 (m, 2H) 7.14 - 7.25 (m, 2H).

$^{13}C$ NMR (DMSO-$d_6$, 101 MHz): δ (ppm) 172.3, 171.5 (br), 169.9 (br, 2C), 157.2, 130.8 (2C), 130.6, 114.4 (2C), 70.2, 69.5, 69.3, 67.2, 65.9, 65.3 (br), 55.4 (br), 55.2 (br, 2C), 52.0 (br, 2C), 51.7 (br, 2C), 49.3 (br, 2C), 47.1 (br, 2C), 33.1 (br), 15.4.

**Step 5**

gadolinium 2,2',2''-{10-[1-carboxy-2-{4-[2-(2-ethoxyethoxy)ethoxy]phenyl}ethyl]-1,4,7,10-tetraazacyclododecane-1,4,7-triyl}triacetate

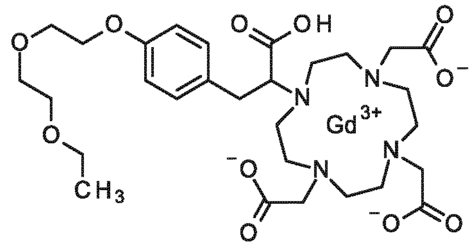

Racemic 3-{4-[2-(2-ethoxyethoxy)ethoxy]phenyl}-2-[4,7,10-tris(carboxymethyl)-1,4,7,10-tetra-azacyclododecan-1-yl]propanoic acid (24.8 g, 39.5 mmol), water (310 ml) and $Gd_2O_3$ (6.45 g, 17.8 mmol, [12064-62-9]) were heated and stirred at 105°C for 4h. Chelex100™ (sodium-form, washed, 10 g) was added and the pH adjusted to 5 using NaOH (aq. solution, 1M) and stirring was continued until the solution tested gadolinium free. The mixture was filtered and then purified by RP-chromatography (mixture was spilt into two equal portions, C18 Biotage 120g, 40 ml/min, A = water, B = MeCN, 0%B to 50%B in 25min, 100%B 10min) after which combination of the corresponding product fractions yielded the title compound (16.0 g, 52 % yield) and a second fraction of lower purity product fraction (11 g, 84% purity, 29 % yield).

LC-MS (Method 3): Rt = 0.59min; MS (ESIpos): m/z = 391.7 $[M+2H]^{++}$, 782.4 $[M+H]^+$. The isotope pattern observed conforms to a gadolinium complex.

**Example 3**

**Step 1**

tert-butyl (2R)-3-{4-[2-(2-ethoxyethoxy)ethoxy]phenyl}-2-[(methanesulfonyl)oxy]propanoate

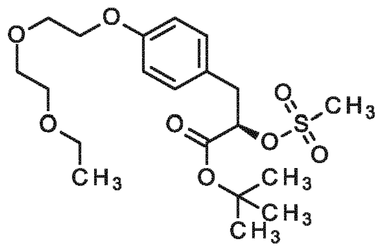

**Intermediate 3,** (2R)-3-{4-[2-(2-ethoxyethoxy)ethoxy]phenyl}-2-hydroxypropanoate (9.20 g, 26.0 mmol) was dissolved in 2-methyl-tetrahydrofuran and DIPEA (14 ml, 78 mmol; [7087-68-5]) was added. The mixture was cooled to 10°C and MsCl (6.0 ml, 78 mmol) was added dropwise. The reaction was allowed to warm to RT and stirred overnight. MTBE (400 ml) was added and the reaction extracted with water (5 × 200 ml), sat. $NaHCO_3$ (2 × 200 ml) and NaCl (sat.aq., 200 ml) and dried over $Na_2SO_4$. The solvent was removed under reduced pressure to give 11.5 g (>100%) of the title compound as orange oil, which was used as such in the next step.

LC-MS (Method 2): Rt = 1.30min; MS (ESIpos): m/z = 450.5 $[M+NH_4]^+$.

$^1H$ NMR ($CDCl_3$, 400 MHz): δ (ppm) 7.13-7.17 (m, 2H), 6.84-6.88 (m, 2H), 4.99 (dd, J = 8.4, 4.6 Hz, 1H), 4.11 (dd, J = 5.8, 5.1 Hz, 2H), 3.85 (dd, J = 5.4, 4.2 Hz, 2H), 3.68-3.73 (m, 2H), 3.60-3.63 (m, 2H), 3.53 (q, J = 7.0 Hz, 2H), 3.17 (dd, J = 14.4, 4.3 Hz, 1H), 3.05 (dd, J = 14.4, 8.4 Hz, 1H), 2.83 (s, 3H), 1.44 (s, 9H), 1.21 (t, J = 7.1 Hz, 3H).

**Step 2**

tert-butyl (2S)-3-{4-[2-(2-ethoxyethoxy)ethoxy]phenyl}-2-(1,4,7,10-tetraazacyclododecan-1-yl)propanoate

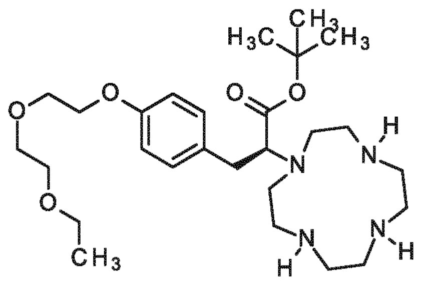

1,4,7,10-tetraazacyclododecane (5.50 g, 31.9 mmol, 1.2 eq) was dissolved in MeCN and cesium carbonate (8.66 g, 26.6 mmol) added. The mixture was heated to 50°C and a solution of tert-butyl (2R)-3-{4-[2-(2-ethoxyethoxy)ethoxy] phenyl}-2-[(methanesulfonyl)oxy] propanoate (11.5 g, 26.6 mmol) in MeCN (10 ml) was added. The mixture was then heated to 70°C overnight. LCMS (Method 1): $R_t$ = 0.76min (74% ELSD, m/z 509) indicated the reaction to be complete. The solids were filtered off, washed with MeCN and the combined filtrates were concentrated under reduced pressure. 13.3 g of tert-butyl (2S)-3-{4-[2-(2-ethoxyethoxy)ethoxy]phenyl}-2-(1,4,7,10-tetraazacyclododecan-1-yl) propanoate were obtained as orange oil, which was used as such in the next step.

LC-MS (Method 2): $R_t$ = 0.76min; MS (ESIpos): m/z = 509.6 $[M+H^+]^+$.

**Step 3**

tert-butyl (2S)-3-{4-[2-(2-ethoxyethoxy)ethoxy]phenyl}-2-[4,7,10-tris(2-tert-butoxy-2-oxoethyl)-1,4,7,10-tetraazacyclododecan-1-yl]propanoate

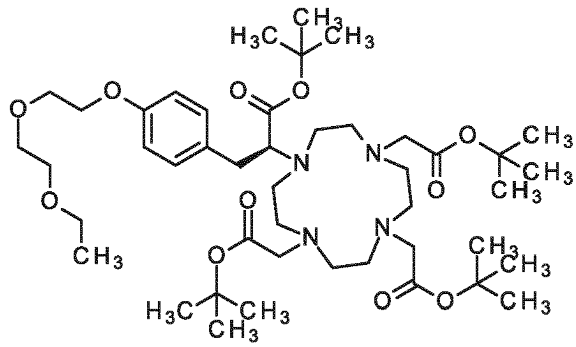

tert-butyl (2S)-3-{4-[2-(2-ethoxyethoxy)ethoxy]phenyl}-2-(1,4,7,10-tetraazacyclododecan-1-yl)propanoate (13.3 g, 26.1 mmol) was dissolved in MeCN (220 ml) and placed under $N_2$. $K_2CO_3$ (11.9 g, 86.3 mmol) was added and the mixture heated to 50°C. tert-butyl bromoacetate (11.7 ml, 15.6 mmol) was added and the mixture stirred at 50°C overnight. The solids were filtered off and the filtrate concentrated under reduced pressure. The residue was taken up in DCM (500 ml) and extracted with water (1 × 200 ml). The organic phase was dried and concentrated under reduced pressure to give the title compound (24.1 g) as orange-brown oil, which was used as such in the next step.

LC-MS (Method 2): $R_t$ = 1.35min; MS (ESIpos): m/z = 852.3 $[M+H]^+$.

**Step 4**

(2S)-3-{4-[2-(2-ethoxyethoxy)ethoxy]phenyl}-2-[4,7,10-tris(carboxymethyl)-1,4,7,10-tetraazacyclododecan-1-yl]propanoic acid

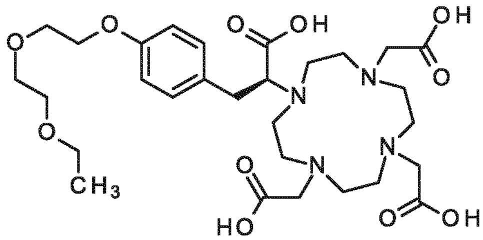

tert-butyl (2S)-3-{4-[2-(2-ethoxyethoxy)ethoxy]phenyl}-2-[4,7,10-tris(2-tert-butoxy-2-oxoethyl)-1,4,7,10-tetraazacyclododecan-1-yl]propanoate (24.1 g, 28.3 mmol) was dissolved in formic acid (160 ml) and stirred at 70°C for 4h and overnight at RT. LCMS (Method 2): Rt = 0.58min (82% ELSD) indicated the reaction to be complete. Formic acid was removed under reduced pressure and the residue suspended in toluene, which was removed under reduced pressure. The toluene procedure was repeated three times, during which the residue became more solid and was obtained as foam. The 22.2 g raw material thus obtained was dissolved in water / MeCN 98 / 2 (1.2 g remained as residue, which was filtered off) and purified by RP-chromatography in 5 × 20 ml portions (SNAP C18 120g, 50 ml/min, 205 nm. A = water/0.1% TFA, B = MeCN, 0% to 28% B in 6.8 CV, 48%B to 100%B 1CV, 100% B 1.2 CV) Interesting fractions were pooled, and lyophilized to give 4.9 g of the title compound.

Another batch of **Intermediate 3** (9.0 g) was taken through steps 1 to 4 in an identical fashion. The material obtained (5.6 g) was identical, combined with the 4.9 g obtained above. After drying (10 mbar) 9.5 g (30%, calc. from step 1) of (2S)-3-{4-[2-(2-ethoxyethoxy) ethoxy] phenyl}-2-[4,7,10-tris(carboxymethyl)-1,4,7,10-tetraazacyclododecan-1-yl]propanoic acid was obtained.

LC-MS (Method 2): Rt = 0.58min; MS (ESIpos): m/z = 627.7 $[M+H]^+$.

**Step 5**

gadolinium 2,2',2"-{10-[(1S)-1-carboxy-2-{4-[2-(2-ethoxyethoxy)ethoxy]phenyl}ethyl]-1,4,7,10-tetraazacyclododecane-1,4,7-triyl}triacetate

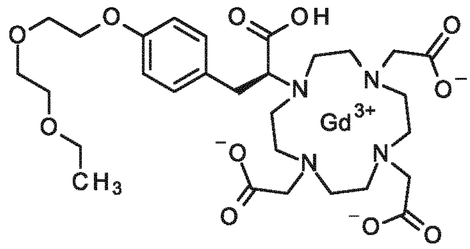

(2S)-3-{4-[2-(2-ethoxyethoxy)ethoxy]phenyl}-2-[4,7,10-tris(carboxymethyl)-1,4,7,10-tetraaza-cyclododecan-1-yl]propanoic acid (9.10 g, 14.5 mmol) was dissolved in water (120 ml) and $Gd_2O_3$ (2.37 g, 6.53 mmol, 0.9 eq) added. The pH was adjusted to 4 (formic acid) and the mixture stirred at 100°C for 8h. Chelex100™ (sodium form, ca 10 g) was added and the mixture stirred overnight at RT, after which the Xylenol-orange test indicated the absence of free gadolinium. The pH was adjusted to 8 (25% ammonium hydroxide) and the whole mixture loaded in an empty Biotage cartridge and subjected directly to RP-chromatography (SNAP C18 400g, 100 ml/min, 220 nm. A = water, B = MeCN, 0%B 5CV, 0% to 15% B in 4.4CV, 15%B 0.3CV, 15% to 16%B in 0.3CV, 16%B 1.3CV, 16%B to 50%B in 10.2CV) Interesting fractions were pooled, lyophilized and dried at 50°C to give 7.44 g (63% of the title compound as white, fluffy solid.

Specific Rotation: $\alpha_D^{20}$ = +14.42° +/- 0.21° (c=1, $H_2O$).

Specific Rotation: $\alpha_D^{20}$ = +12.16° +/- 0.25° (c=1, MeOH).

LC-MS (Method 2): $R_t$ = 0.68min; MS (ESIpos): m/z = 782 $[M+H]^+$.

LC-MS (Method 3): $R_t$ = 0.60min (100% DAD); MS (ESIpos): m/z = 391 $[M + 2H]^{++}$, 782 $[M + H]^+$ and some $[2M + 2H]^{++}$, 791 $[2M + H_2O + 2H]^{++}$. The observed isotope pattern conforms to a gadolinium complex.

## Example 4

### Step 1

tert-butyl (2S)-3-{4-[2-(2-ethoxyethoxy)ethoxy]phenyl}-2-[(methylsulfonyl)oxy]propanoate

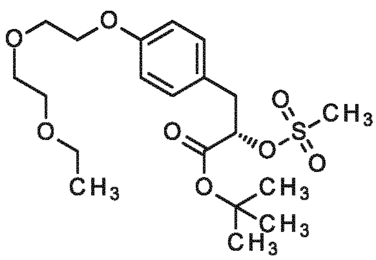

tert-butyl (2S)-3-{4-[2-(2-ethoxyethoxy)ethoxy]phenyl}-2-hydroxypropanoate **(Intermediate 4,** 650 mg, 1.83 mmol) and TEA (560 μl, 4.0 mmol; [121-44-8]) were stirred in THF (6.1 ml) under an atmosphere of $N_2$. The mixture was cooled 0-5°C and MsCl (160 μl, 2.0 mmol; [124-63-0]) added dropwise. The mixture was warmed to RT, stirred for 3h, after which it was added to a solution of $NaHCO_3$ (50% sat. aq.), and the mixture extracted with MTBE (3x), the combined organic phases washed with a solution of NaCl (sat. aq.), dried over $Na_2SO_4$, filtered and concentrated under reduced pressure, yielding the title compound (831 mg, 100 % yield).

LC-MS (Method 4): $R_t$ = 1.29min; MS (ESIpos): m/z = 450.4 $[M+NH_4^+]^+$.

$^1$H NMR (400 MHz, DMSO-$d_6$) δ ppm 1.09 (t, J=6.97 Hz, 3H) 1.36 (s, 9H) 2.97 - 3.10 (m, 5H) 3.42 (q, J=6.84 Hz, 2H) 3.46 - 3.50 (m, 2H) 3.54 - 3.61 (m, 2H) 3.67 - 3.82 (m, 2H) 4.00 - 4.15 (m, 2H) 5.04 - 5.15 (m, 1H) 6.84 - 6.93 (m, 2H) 7.14 - 7.25 (m, 2H).

### Step 2

tert-butyl (2R)-3-{4-[2-(2-ethoxyethoxy)ethoxy]phenyl}-2-(1,4,7,10-tetraazacyclododecan-1-yl)propanoate

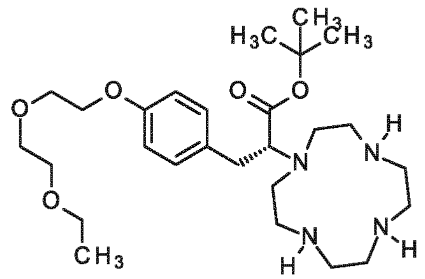

tert-butyl (2S)-3-{4-[2-(2-ethoxyethoxy)ethoxy]phenyl}-2-[(methanesulfonyl)oxy]propanoate (830 mg, 1.92 mmol) and 1,4,7,10-tetraazacyclododecane (397 mg, 2.31 mmol) were stirred in MeCN (17 ml) at 55°C overnight. The temperature was then raised to 70°C and the reaction stirred for further 18h. The mixture was then concentrated under reduced pressure, affording the crude product (977 mg, 80% product by ELSD).

LC-MS (Method 2): Rt = 0.66min; MS (ESIpos): m/z = 509.2 $[M+H]^+$.

**Step 3**

tert-butyl (2R*)-3-{4-[2-(2-ethoxyethoxy)ethoxy]phenyl}-2-[4,7,10-tris(2-tert-butoxy-2-oxoethyl)-1,4,7,10-tetraazacyclododecan-1-yl]propanoate

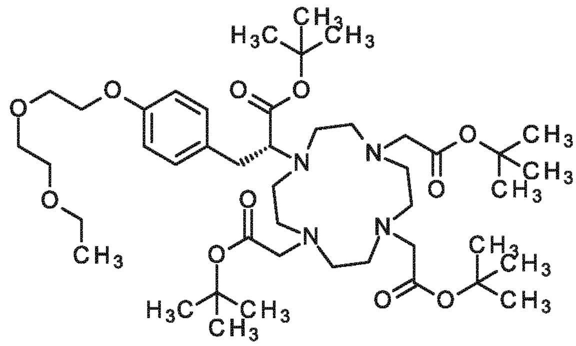

Crude product, (2R)-3-{4-[2-(2-ethoxyethoxy)ethoxy]phenyl}-2-(1,4,7,10-tetraazacyclo-dodecan-1-yl)propanoate (977 mg, 1.92 mmol), was stirred in MeCN (20 ml) under $N_2$. DIPEA (1.7 ml, 9.6 mmol; [7087-68-5]) was added, following by the dropwise addition of tert-butyl bromoacetate (990 μl, 6.7 mmol) dissolved in MeCN (0.33 ml) and the mixture heated to 60°C for 5h. The mixture was then concentrated under reduced pressure, the residue dissolved in EtOAc, washed with a solution of $NaHCO_3$ (50% sat. aq.) followed by washing with NaCl (sat. aq.), dried over $Na_2SO_4$, filtered and the mixture concentrated under reduced pressure affording the crude product (2.19 g).

LC-MS (Method 2): $R_t$ = 1.15min; MS (ESIpos): m/z = 851.4 $[M+H]^+$.

**Step 4**

(2R)-3-{4-[2-(2-ethoxyethoxy)ethoxy]phenyl}-2-[4,7,10-tris(carboxymethyl)-1,4,7,10-tetraaza-cyclododecan-1-yl]propanoic acid

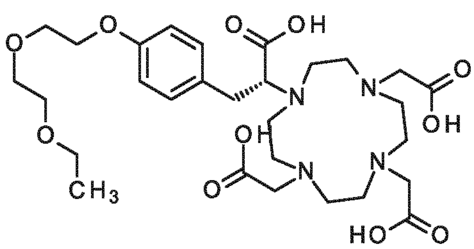

Crude product tert-butyl (2R)-3-{4-[2-(2-ethoxyethoxy)ethoxy]phenyl}-2-[4,7,10-tris(2-tert-butoxy-2-oxoethyl)-1,4,7,10-tetraazacyclododecan-1-yl]propanoate (2.19 g, 2.57 mmol) was heated with formic acid (33 ml) for 4h at 70°C. The mixture was then concentrated under reduced pressure, with purification by RP-chromatography (Biotage C18 60g, 50 ml/min, A = water, B = MeCN, 0%B to 50%B in 30min, 100%B 10min,) yielding the title compound (286 mg, 18 % yield over 4 steps).

Specific Rotation: 5.6° (MeOH, 20°C, 589 nm).

LC-MS (Method 2): $R_t$ = 0.58min; MS (ESIpos): m/z = 627.1 $[M+H]^+$.

$^1$H NMR (DMSO-$d_6$, 400 MHz): δ (ppm) 7.20 (d, *J* = 8.6 Hz, 2H), 6.82 (d, *J* = 8.6 Hz, 2H), 4.01-4.06 (m, 2H), 3.69-3.73 (m, 2H), 3.36-3.61 (m, 14H), 2.63-3.10 (m, 17H), 1.09 (t, *J* = 7.0 Hz, 3H).

$^{13}$C NMR (DMSO-$d_6$, 101 MHz): δ (ppm) 171.8, 170.9, 169.4 (br, 2C), 156.6, 130.2 (2C), 130.1, 113.8 (2C), 69.7, 69.0, 68.7, 66.7, 65.3, 64.8 (br), 54.9 (br), 54.7 (br, 2C), 51.4 (br, 2C), 51.1 (br, 2C), 48.8 (br, 2C), 46.5 (br, 2C), 32.5 (br), 14.9.

**Step 5**

gadolinium 2,2'', 2''-{10-[(1R)-1-carboxy-2-{4-[2-(2-ethoxyethoxy)ethoxy]phenyl}ethyl]-1,4,7,10-tetraazacyclododecane-1,4,7-triyl}triacetate

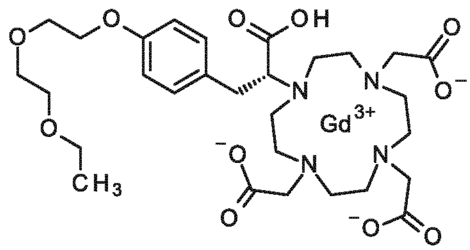

(2R)-3-{4-[2-(2-ethoxyethoxy)ethoxy]phenyl}-2-[4,7,10-tris(carboxymethyl)-1,4,7,10-tetraaza-cyclododecan-1-yl]propanoic acid (286 mg, 456 μmol), $Gd_2O_3$ (74.4 mg, 205 μmol) and water (4.7 ml) were stirred overnight in a sealed vial at 105°C. Thereafter the mixture was warmed to 120°C for further 5h. Chelex100™ (approximately 5 g, sodium form, washed), was added and the pH adjusted to 5 using NaOH (1M, aq.) and the mixture stirred for 1h. The mixture was filtered, the concentrated to dryness, thereafter it was purified by RP-chromatography (Biotage C18 30g, 25 ml/min, A = water, B = MeCN, 0%B to 50%B in 25min, 100%B 10min) yielding the title compound (267 mg, 75 % yield).

Specific Rotation: -14.1° (c=1, MeOH, 20°C, 589 nm.

LC-MS (Method 2): $R_t$ = 0.66min; MS (ESIpos): m/z = 782.3 $[M+H^+]^+$.

LC-MS (Method 3): $R_t$ = 0.62min; MS (ESIpos): m/z = 391.7 $[M+2H^+]^{+2}$ , 782.3 $[M+H^+]^+$, 790 $[2M+2H]^+ + H_2O]^{++}$.

**Example 5**

**Step 1**

ethyl-3-{4-[2-(2-ethoxyethoxy)ethoxy]phenyl}-2-[(methanesulfonyl)oxy]propanoate

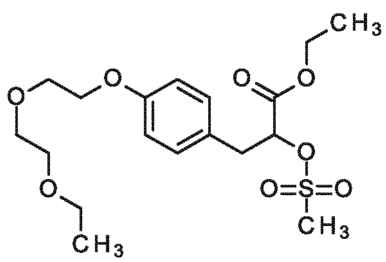

Ethyl-3-{4-[2-(2-ethoxyethoxy)ethoxy]phenyl}-2-hydroxypropanoate **(Intermediate 5,** 2.44 g, 7.48 mmol) and TEA (2.3 ml, 16 mmol; [121-44-8]) were stirred in THF (25 ml) under $N_2$ at 0-5°C. MsCl (640 μl, 8.2 mmol; [124-63-0]) was

added dropwise to the mixture and stirring was continued for 3h. The mixture was then added to a solution of $NaHCO_3$ (50% sat. aq.), and the aqueous layer extracted with MTBE (3x), the combined organic layers washed with a solution of NaCl (sat. aq.), dried over $Na_2SO_4$, filtered and concentrated under reduced pressure yielding the title compound (2.82 g, 93 % yield).

LC-MS (Method 4): $R_t$ = 1.14min; MS (ESIpos): m/z = 422.3 $[M+NH_4^+]^+$.

$^1H$ NMR (400 MHz, DMSO-$d_6$) δ ppm 1.09 (t, 3H) 1.16 (t, 3H) 3.00 - 3.14 (m, 5H) 3.38 - 3.51 (m, 4H) 3.54 - 3.59 (m, 2H) 3.69 - 3.75 (m, 2H) 4.05 (dd, 2H) 4.13 (q, 2H) 5.26 (dd, 1H) 6.84 - 6.91 (m, 2H) 7.12 - 7.21 (m, 2H).

## Step 2

ethyl-2-[4,10-bis(2-tert-butoxy-2-oxoethyl)-1,4,7,10-tetraazacyclododecan-1-yl]-3-{4-[2-(2-ethoxyethoxy)ethoxy]phenyl}propanoate

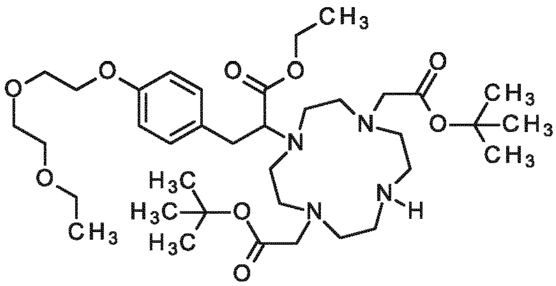

di-tert-butyl 2,2'-(1,4,7,10-tetraazacyclododecane-1,7-diyl)diacetate (1.86 g, 4.65 mmol, [162148-48-3]), $K_2CO_3$ (0.71 g, 5.1 mmol), racemic 3-{4-[2-(2-ethoxyethoxy)ethoxy] phenyl}-2-[(methanesulfonyl)oxy]propanoate (1.88 g, 4.65 mmol) and MeCN (22 ml) were stirred at 70°C for 3d. The mixture was filtered, the solid washed with MeCN and the filtrates combined and concentrated under reduced pressure affording the crude product (3.30 g).

## Step 3

methyl-2-[(methanesulfonyl)oxy]propanoate

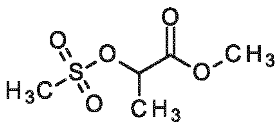

Methyl-2-hydroxypropanoate (5.00 g, 48.0 mmol), TEA (15 ml, 110 mmol; [121-44-8]) and THF (50 ml) were stirred at 0-5°C. Under an atmosphere of $N_2$, MsCl (4.1 ml, 53 mmol; [124-63-0]) was added dropwise, following which the mixture was brought to RT and stirred for 3h. The mixture was then added to a solution of $NaHCO_3$ (50% sat. aq.), it was extracted with MTBE (3x), the combined organic phases washed with NaCl (sat. aq. 2x), dried over $Na_2SO_4$, filtered and concentrated under reduced pressure, affording the title compound (7.17 g, 82 % yield).

$^1H$ NMR (DMSO-$d_6$, 400 MHz): δ (ppm) 5.21 (q, $J$ = 6.9 Hz, 1H), 3.72 (s, 3H), 3.25 (s, 3H), 1.48 (d, $J$ = 7.1 Hz, 3H).

## Step 4

ethyl-2-{4,10-bis(2-tert-butoxy-2-oxoethyl)-7-[1-methoxy-1-oxopropan-2-yl]-1,4,7,10-tetraazacyclododecan-1-yl}-3-{4-[2-(2-ethoxyethoxy)ethoxy]phenyl}propanoate

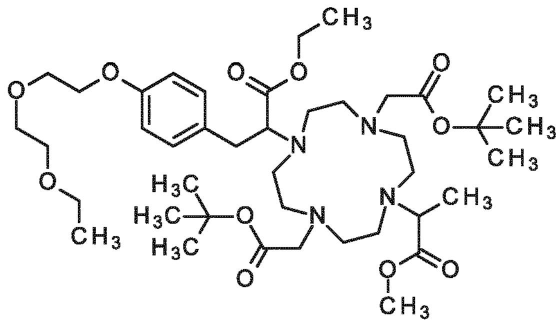

Racemic ethyl-2-[4,10-bis(2-tert-butoxy-2-oxoethyl)-1,4,7,10-tetraazacyclododecan-1-yl]-3-{4-[2-(2-ethoxyethoxy)ethoxy]phenyl}propanoate, **Step 2,** (1.10 g, 1.55 mmol), methyl-2-[(methanesulfonyl)oxy]propanoate (339 mg, 1.86 mmol), $K_2CO_3$ (472 mg, 3.41 mmol) in MeCN (2.8 ml) were stirred together at 55°C overnight. The temperature was then raised to 80°C for additional 6h. The mixture was then filtered, the solid washed with EtOH, the organic phases combined and concentrated under reduced pressure, affording the crude product as a mixture of diastereomers (1.14 g).

LC-MS (Method 2): $R_t$ = 1.11 - 1.17min; MS (ESIpos): m/z = 795.9 $[M+H^+]^+$.

**Step 5**

2,2'-{4-[1-ethoxy-3-{4-[2-(2-ethoxyethoxy)ethoxy]phenyl}-1-oxopropan-2-yl]-10-[1-methoxy-1-oxopropan-2-yl]-1,4,7,10-tetraazacyclododecane-1,7-diyl}diacetic acid

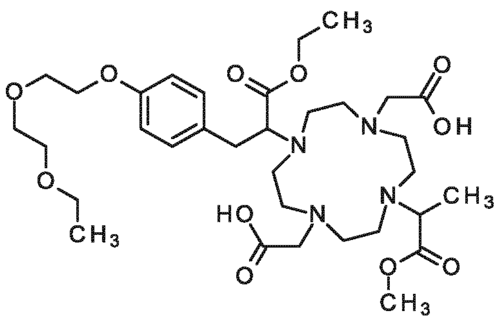

Ethyl-2-{4,10-bis(2-tert-butoxy-2-oxoethyl)-7-[1-methoxy-1-oxopropan-2-yl]-1,4,7,10-tetraaza-cyclododecan-1-yl}-3-{4-[2-(2-ethoxyethoxy)ethoxy]phenyl}propanoate (1.14 g, 1.44 mmol) and formic acid (15 ml, 400 mmol; [64-18-6]) were stirred overnight at 60°C, followed by further 6h at 70°C. The mixture was concentrated under reduced pressure. Purification by RP-chromatography (Biotage C18 Ultra 60g, 50 ml/min, A = water/0,5% formic acid; B = EtOH/+0,5% formic acid; 5%B 1CV, 5%B to 40 B 15CV) yielded the title compound as a mixture of diastereomers (250 mg, 95 % purity, 24 % yield).

LC-MS (Method 2): Rt = 0.72min; MS (ESIpos): m/z = 683.7 $[M+H^+]^+$.

$^1$H NMR (DMSO-$d_6$, 400 MHz): $\delta$ (ppm) 7.12-7.27 (m, 2H), 6.76-6.86 (m, 2H), 3.91-4.08 (m, 4H), 3.39-3.82 (m, 16H), 2.55-3.27 (m, 18H), 0.99-1.28 (m, 9H).

**Step 6**

gadolinium 2-[7-(1-carboxy-2-{4-[2-(2-ethoxyethoxy)ethoxy]phenyl}ethyl)-4,10-bis(carboxylatomethyl)-1,4,7,10-tetraazacyclododecan-1-yl]propanoate

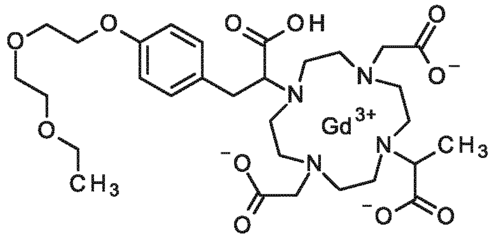

2,2'-{4-[-1-Ethoxy-3-{4-[2-(2-ethoxyethoxy)ethoxy]phenyl}-1-oxopropan-2-yl]-10-[1-methoxy-1-oxopropan-2-yl]-1,4,7,10-tetraazacyclododecane-1,7-diyl}diacetic acid (250 mg, 366 μmol), $Gd_2O_3$ (59.7 mg, 165 μmol) in water (5 ml) were stirred for 18h at 105°C in a crimp sealed reaction vessel, following which the temperature was raised to 120°C for an additional 18h. The mixture was treated with Chelex100™ (sodium form, washed, approximately 5 g), and stirred for 1h, following which it was filtered, concentrated under reduced pressure and purified by RP-chromatography (Biotage SNAP Ultra C18 30g, 25 ml/min, A = water, B = MeCN, 0%B to 50% in 25min, 100%B 10min) yielding two diastereomers. Diastereomer 1 (40 mg, 13 % yield) and Diastereomer 2 (130 mg, 42 % yield).

Diastereomer 1:

LC-MS (Method 3): $R_t$ = 0.61min; MS (ESIpos): m/z = 398.6 $[M+2H]^{++}$, 796.4 $[M+H^+]^+$. Isotope patterns match theoretical values for corresponding gadolinium complex.

**Example 6**

gadolinium 2-[7-(1-carboxy-2-{4-[2-(2-ethoxyethoxy)ethoxy]phenyl}ethyl)-4,10-bis(carboxylatomethyl)-1,4,7,10-tetraazacyclododecan-1-yl]propanoate

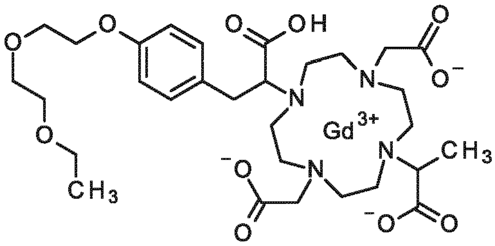

Diastereomer 2 of Example 5 - 130 mg (95 % purity).

LC-MS (Method 3): $R_t$ = 0.63min; MS (ESIpos): m/z = 398.7 $[M+2H]^{++}$, 796.4 $[M+H^+]^+$. Isotope patterns match theoretical values for corresponding gadolinium complex.

**Example 7**

**Step 1**

ethyl-3-[4-(2-ethoxyethoxy)phenyl]-2-[(methanesulfonyl)oxy]propanoate

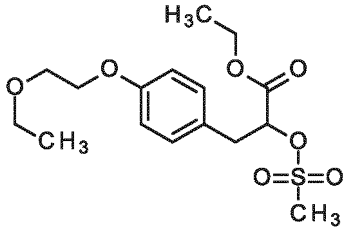

Ethyl-3-[4-(2-ethoxyethoxy)phenyl]-2-hydroxypropanoate (5.39 g, 19.1 mmol, **Intermediate 6),** and TEA (5.9 ml, 42 mmol; [121-44-8]) were stirred in THF (56 ml) under $N_2$ at 0-5°C. MsCl (1.6 ml, 21 mmol; [124-63-0]) was added dropwise to the mixture and stirring was continued for 3h. The mixture was then added to a solution of $NaHCO_3$ (50% sat. aq.), and the aqueous layer extracted with MTBE (3x), the combined organic layers washed with a solution of NaCl (sat. aq.), dried over $Na_2SO_4$, filtered and concentrated under reduced pressure yielding the title compound (6.96 g, 90 % purity, 91 % yield).

LC-MS (Method 4): $R_t$ = 1.15min; MS (ESIpos): m/z = 378.3 $[M+NH_4^+]^+$.

$^1H$ NMR (400 MHz, DMSO-$d_6$) δ ppm 1.09 - 1.18 (m, 6H) 3.02 (s, 3H) 3.04 - 3.10 (m, 2H) 3.49 (q, 2H) 3.64 - 3.70 (m, 2H) 4.02 - 4.07 (m, 2H) 4.13 (q, 2H) 5.26 (dd, 1H) 6.85 - 6.90 (m, 2H) 7.14 - 7.19 (m, 2H).

**Step 2**

ethyl-2-[4,10-bis(2-tert-butoxy-2-oxoethyl)-1,4,7,10-tetraazacyclododecan-1-yl]-3-[4-(2-ethoxyethoxy)phenyl]propanoate

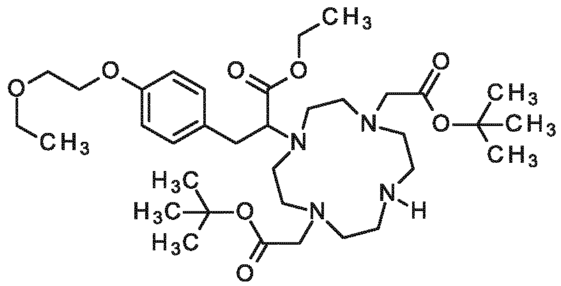

di-tert-butyl 2,2'-(1,4,7,10-tetraazacyclododecane-1,7-diyl)diacetate (.51 g, 11.2 mmol, [162148-48-3]), racemic ethyl-3-[4-(2-ethoxyethoxy)phenyl]-2-[(methanesulfonyl)oxy]propanoate (4.46 g, 12.4 mmol) and $K_2CO_3$ (3.42 g, 24.8 mmol) in MeCN (31 ml) were stirred at 80°C for 24h. The mixture was filtered, the solid washed with EtOH, the organic phases combined and concentrated under reduced pressure to afford the crude title compound (8.40 g, 57% product by ELSD detection).

LC-MS (Method 2): $R_t$ = 1.26min; MS (ESIpos): m/z = 665.8 $[M+H^+]^+$.

**Step 3**

ethyl-2-[(methanesulfonyl)oxy]butanoate

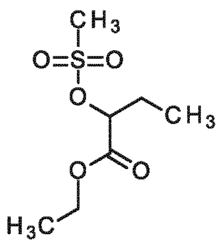

Ethyl-2-hydroxybutanoate (5.00 g, 37.8 mmol, [52089-54-0]) and TEA (12 ml, 83 mmol; [121-44-8]) were stirred in THF (110 ml) under $N_2$ at 0-5°C. MsCl (3.2 ml, 42 mmol; [124-63-0]) was added dropwise to the mixture and stirring was continued for 3h. The mixture was then added to a solution of $NaHCO_3$ (50% sat. aq.), and the aqueous layer extracted with MTBE (3x), the combined organic layers washed with NaCl (sat. aq.), dried over $Na_2SO_4$, filtered and concentrated under reduced pressure yielding the title compound (8.21 g, 103 % yield).

$^1H$ NMR (400 MHz, OMSO-$d_6$) δ ppm 0.93 (t, 3H) 1.22 (t, 3H) 1.71 - 1.95 (m, 2H) 3.25 (s, 3H) 4.19 (dtt, 2H) 5.05 (dd, 1H).

**Step 4**

ethyl-2-[4,10-bis(2-tert-butoxy-2-oxoethyl)-7-{(2SR)-1-ethoxy-3-[4-(2-ethoxyethoxy)phenyl]-1-oxopropan-2-yl}-1,4,7,10-tetraazacyclododecan-1-yl]butanoate

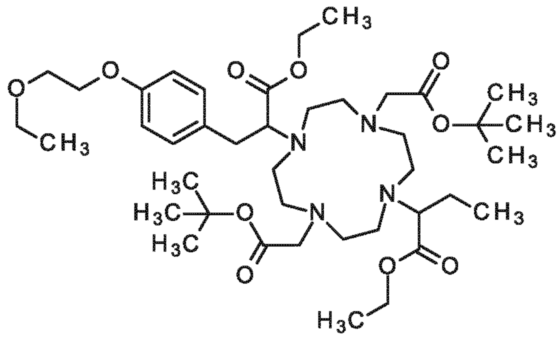

Ethyl-2-[4,10-bis(2-tert-butoxy-2-oxoethyl)-1,4,7,10-tetraazacyclododecan-1-yl]-3-[4-(2-ethoxyethoxy)phenyl] propanoate, Step 2, (4.20 g, 6.32 mmol), ethyl-2-[(methanesulfonyl)oxy] butanoate (2.12 g, 10.1 mmol), $K_2CO_3$ (1.75 g, 12.6 mmol) in MeCN (21 ml) were stirred for 24h at 80°C. The mixture was then filtered, the solid washed with EtOH, the organic phases combined and concentrated under reduced pressure affording the crude title compound (6.50 g).

LC-MS (Method 2): $R_t$ = 1.28min; MS (ESIpos): m/z = 779.9 $[M+H]^+$.

**Step 5**

2,2'-(4-{1-ethoxy-3-[4-(2-ethoxyethoxy)phenyl]-1-oxopropan-2-yl}-10-[(2SR)-1-ethoxy-1-oxobutan-2-yl]-1,4,7,10-tetraazacyclododecane-1,7-diyl)diacetic acid

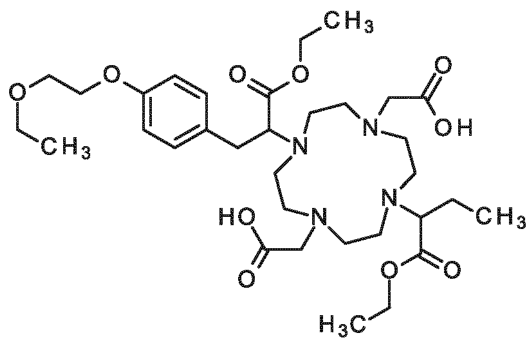

Ethyl-2-[4,10-bis(2-tert-butoxy-2-oxoethyl)-7-{1-ethoxy-3-[4-(2-ethoxyethoxy)phenyl]-1-oxopropan-2-yl}-1,4,7,10-tetraazacyclododecan-1-yl]butanoate (6.50 g, 8.34 mmol) and formic acid (110 ml) were stirred together overnight at 70°C. The mixture was concentrated under reduced pressure, and RP-chromatography (Biotage SNAP Ultra C18 120g, 50ml/min, A = water, B = MeCN, 0%B to 50%B in 30min, 100%B 10min) yielded the title compound as a mixture of diastereomers (1.21 g, 95 % purity, 22 % yield over 5 steps).

LC-MS (Method 2): Rt = 0.76min; MS (ESIpos): m/z = 665.7 $[M+H]^+$.

$^1H$ NMR (DMSO-$d_6$, 600 MHz): δ (ppm) 7.17-7.24 (m, 2H), 6.82 (d, *J* = 7.8 Hz, 2H), 4.07-4.13 (m, 2H), 3.94-4.04 (m, 4H), 3.74 (dd, *J* = 9.4, 6.4 Hz, 0.6H), 3.66 (t, *J* = 4.7 Hz, 2H), 3.63 (br dd, *J* = 9.0, 6.4 Hz, 0.5H), 3.48 (q, *J* = 6.9 Hz, 2H), 3.31-3.41 (m, 4H), 2.58-3.28 (m, 19H), 1.64-1.78 (m, 1H), 1.53-1.63 (m, 1H), 1.18-1.23 (m, 3H), 1.03-1.13 (m, 6H), 0.81-0.89 (m, 3H).

$^{13}C$ NMR (DMSO-$d_6$, 151 MHz): δ (ppm) 171.5/171.4, 170.7/170.7, 167.9/168.7 (br. 2C), 156.7, 130.3/130.2 (2C), 129.4/129.5, 113.8/113.8 (2C), 68.1, 66.7, 65.4, 65.3/65.2, 64.1 (br), 59.7/59.7, 59.6/59.7, 55.3/54.4 (2C), 52.4/52.7 (2C), 52.0 (br, 2C), 45.9/45.7 (2C), 45.5 (br, 2C), 33.5/33.3, 21.5/21.3, 14.8, 14.0, 13.9, 13.8, 10.7/10.7.

**Step 6**

gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]butanoate

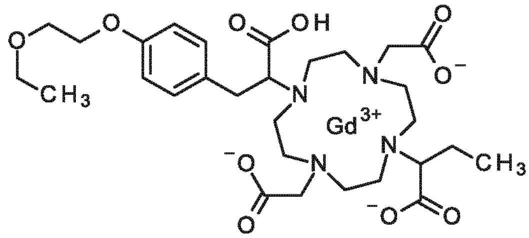

2,2'-(4-{1-ethoxy-3-[4-(2-ethoxyethoxy)phenyl]-1-oxopropan-2-yl}-10-[1-ethoxy-1-oxobutan-2-yl]-1,4,7,10-tetraazacyclododecane-1,7-diyl)diacetic acid (1.21 g, 1.81 mmol), $Gd_2O_3$ (296 mg, 817 μmol) and water were stirred together and heated at 105°C for 24h in a crimp sealed vessel, following which the mixture was heated at 120°C for an additional 6h. The mixture was treated with Chelex100™ (sodium form, washed, approximately 5 g), stirred for 1h, following which it was filtered, concentrated under reduced pressure and purified by RP-chromatography (Biotage SNAP Ultra C18 60g, 50 ml/min, A = water, B = MeCN, 0%B to 50%B in 25min, 100%B 10min) yielding the title compounds as diastereomeric mixtures. Fraction 1 **(Example 7**, 575 mg, 41 % yield) and Fraction 2 (74 mg, 5 % yield).

Fraction 1

LC-MS (Method 3): $R_t$ = 0.58min (24% DAD) and 0.63min (74% DAD); MS (ESIpos): m/z = 383.7 $[M+2H]^{++}$, 766.4 $[M+H]^+$. Isotope patterns match theoretical values for corresponding gadolinium complex.

**Example 8**

gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]butanoate

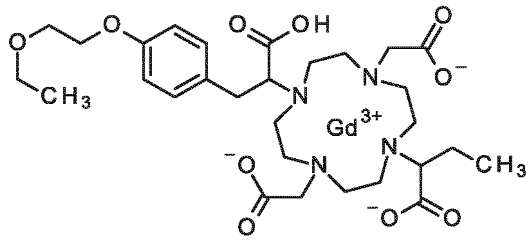

**Fraction 2 - Step 6 Example 7**

LC-MS (Method 3): Rt = 0.63min (100% DAD); MS (ESIpos): m/z = 383.7 $[M+2H]^{++}$, 766.4 $[M+H]^+$. Isotope patterns match theoretical values for corresponding gadolinium complex.

**Example 9**

**Step 1**

methyl (2R)-2-hydroxyhex-5-enoate

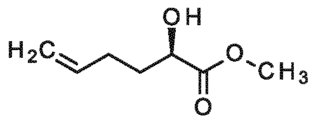

A mixture of copper(I) bromide-dimethyl sulfide (16.8 g, 81.8 mmol; CAS-RN: [54678-23-8]) in THF (80 mL) was cooled to -70°C and treated dropwise with chlorido(prop-2-en-1-yl)magnesium (150 mmol, 74 mL of 2.0 M solution in THF; CAS-RN: [2622-05-1]) keeping the temperature between -70°C to -60°C. It was stirred at -70°C for additional 30min upon which a solution of methyl (2R)-oxirane-2-carboxylate (16.7 g, 164 mmol; CAS-RN: [111058-32-3]) in THF (70 mL) was added dropwise and stirring at -70°C continued for 30min. The reaction mixture was gradually warmed to RT and quenched with $NH_4Cl$ (sat. aq.). The reaction mixture was extracted with EtOAc, the organic layer washed twice with water and dried with $Na_2SO_4$. The mixture was filtered and carefully concentrated under reduced pressure (volatile material) to give the title compound (22.3 g, 100%).

LC-MS (Method 3): $R_t$ = 0.61min; MS (ESIpos): m/z = 145.1 $[M+H]^+$.

$^1H$ NMR (400 MHz, OMSO-$d_6$) δ ppm 2.05 - 2.11 (m, 2H), 2.85 (dd, 1H), 2.96 (dd, 1H), 3.69 (s, 3H), 4.01 - 4.07 (m, 1H), 4.94 - 5.05 (m, 2H), 5.41 (d, 1H), 5.75 - 5.85 (m, 1H).

**Step 2**

methyl (2R)-2-[(4-nitrobenzene-1-sulfonyl)oxy]hex-5-enoate

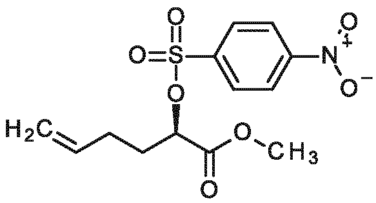

A solution of crude methyl (2R)-2-hydroxyhex-5-enoate (16.3 g, 113 mmol) in dry toluene (210 mL) was cooled to 0°C and treated with 4-nitrobenzene-1-sulfonyl chloride (27.6 g, 125 mmol) and TEA (32 mL, 227 mmol; [121-44-8]). The reaction mixture was warmed to RT and stirred overnight. The mixture was concentrated under reduced pressure, the residue taken up with EtOAc, the formed precipitate filtered off (discarded) and washed with EtOAc. The filtrate was washed with water and the aqueous layer extracted with EtOAc. The combined organic layers were washed with sat. NaCl, dried with $Na_2SO_4$, filtered and concentrated under reduced pressure. The obtained crude material was purified by Biotage Isolera™ chromatography (SNAP Si 340 g, eluting with hexane-EtOAc, 0:1 to 7:3) to give the title compound (7.2 g, 18%).

LC-MS (Method 3): $R_t$ = 1.17min; MS (ESIpos): m/z = 330.0 $[M+H]^+$.

$^1H$ NMR (400 MHz, OMSO-$d_6$) δ ppm 1.84 - 1.92 (m, 2H), 1.95 - 2.02 (m, 2H), 3.61 (s, 3H), 4.89 - 4.96 (m, 2H), 5.11 (t, 1H), 5.64 - 5.74 (m, 1H), 8.20 - 8.24 (m, 2H), 8.45 - 8.49 (m, 2H).

**Step 3**

methyl (2S)-2-(1,4,7,10-tetraazacyclododecan-1-yl)hex-5-enoate

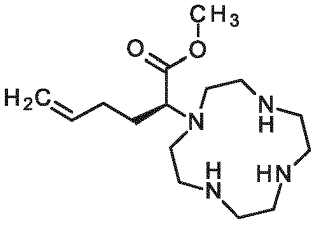

A solution of 1,4,7,10-tetraazacyclododecane (3.45 g, 20.0 mmol) in MeCN (25 mL) was treated at RT with a

solution of methyl (2R)-2-[(4-nitrobenzene-1-sulfonyl)oxy]hex-5-enoate (3.3 g, 10 mmol) in MeCN (15 mL) and $K_2CO_3$ (1.38 g, 10.0 mmol). The reaction mixture was stirred at 60°C for 7h and subsequently at RT overnight. The solid material was filtered off, washed with MeCN and the filtrate concentrated under reduced pressure. The obtained residue was taken up with EtOAc, washed with NaOH (0.1 M aq.), NaCl (sat. aq.), dried with $Na_2SO_4$, filtered and concentrated under reduced pressure to give the crude title compound (2.6 g, 39%).

Specific rotation: $[\alpha]_D^{20}$ = -46.6° +/- 0.24° (c = 1, $CHCl_3$).

LC-MS (Method 3): Rt = 0.19min; MS (ESIpos): m/z = 299.1 $[M+H]^+$, 150.1 $[M+2H]^{2+}$.

$^1H$ NMR (400 MHz, DMSO-$d_6$) δ ppm 1.57 - 1.75 (m, 2H), 2.01 - 2.22 (m, 2H), 2.26 - 2.35 (m, 5H), 2.41 - 2.44 (m, 3H), 2.54 - 2.57 (m, 2H), 2.60 - 2.65 (m, 4H), 2.73 - 2.78 (m, 2H), 3.35 - 3.37 (m, 1H), 3.61 (s, 3H), 4.96 - 5.07 (m, 2H), 5.74 - 5.84 (m, 1H).

**Step 4**

benzyl (2R)-3-(benzyloxy)-2-hydroxypropanoate

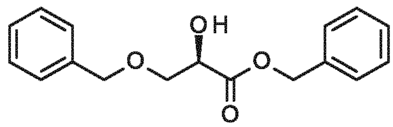

A solution of (2R)-3-(benzyloxy)-2-hydroxypropanoic acid (2.00 g, 10.2 mmol; [130111-08-9]) in phenylmethanol (1.3 mL, 12 mmol; [100-51-6]) was treated with $H_2SO_4$ (conc. 54 µL, 1.0 mmol) at RT upon which it was warmed to 50°C and stirred overnight. The reaction mixture was cooled to RT and diluted with DCM. The organic layer was washed with $NaHCO_3$ (sat. aq.), concentrated under reduced pressure and the crude material subjected to Biotage Isolera™ chromatography (SNAP Si 100 g, eluting with DCM-hexane 7:3 followed by DCM-MeOH 8:2) to give the title compound (1.75 g, 57%).

Specific rotation: $[\alpha]_D^{20}$ = 21.4° +/- 0.58° (c = 1, $CHCl_3$).

LC-MS (Method 3): Rt = 1.10min; MS (ESIpos): m/z = 304.1 $[M+H_2O]^+$.

$^1H$ NMR (400 MHz, DMSO-$d_6$) δ ppm 3.65 (dq, 2H), 4.30 - 4.33 (m, 1H), 4.46 (d, 1H), 4.52 (d, 1H), 5.13 (d, 1H), 5.17 (d, 1H), 5.66 (d, 1H), 7.26 - 7.35 (m, 10H).

**Step 5**

benzyl (2R)-3-(benzyloxy)-2-[(trifluoromethanesulfonyl)oxy]propanoate

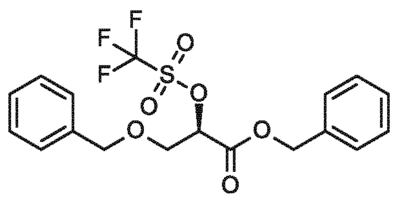

A solution of benzyl (2R)-3-(benzyloxy)-2-hydroxypropanoate (870 mg, 3.04 mmol) in DCM (10 mL) was cooled to -70°C and dropwise treated with 2,6-dimethylpyridine (150 mmol, 410 µL; [108-48-5]) and subsequently with trifluoromethanesulfonic anhydride (570 µL, 3.3 mmol; [358-23-6]). It was stirred at -70°C for 2h, then warmed to -40°C and stirred for 2h and finally warmed to -20°C and stirring continued for 1h. The reaction mixture was diluted with MTBE at 0°C, the formed precipitate filtered off (discarded) and washed with MTBE. The filtrate was washed with water and 0.1 M aqueous HCl, dried with $Na_2SO_4$, filtered and concentrated under reduced pressure. The obtained crude title compound (1.3 g) was used in the next step without further purification.

LC-MS (Method 3): $R_t$ = 1.46min; MS (ESIpos): m/z = 436.2 $[M+H_2O]^+$.

$^1H$ NMR (400 MHz, DMSO-$d_6$) δ ppm 3.95 (dq, 2H), 4.46 (d, 1H), 4.59 (d, 1H), 5.27 (d, 1H), 5.34 (d, 1H), 5.55 - 5.57 (m, 1H), 7.22 - 7.25 (m, 2H), 7.31 - 7.39 (m, 8H).

$^{19}F$ NMR (377 MHz, DMSO-$d_6$) δ [ppm]: -78.12 (s, 3F).

**Step 6**

methyl (2S)-2-{4,7,10-tris[(2S)-1,3-bis(benzyloxy)-1-oxopropan-2-yl]-1,4,7,10-tetraazacyclododecan-1-yl}hex-5-enoate

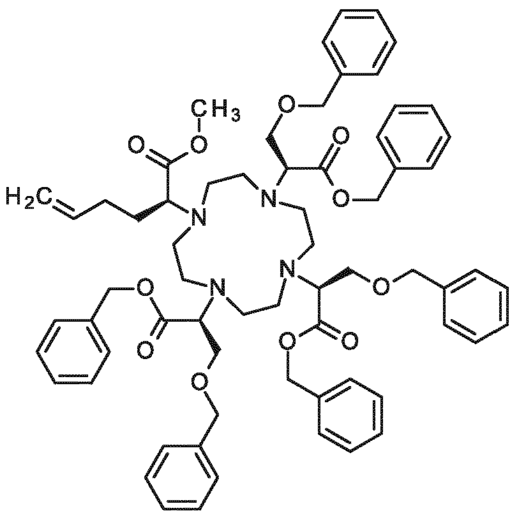

A suspension of 1 methyl (2S)-2-(1,4,7,10-tetraazacyclododecan-1-yl)hex-5-enoate, **Step 3** (206 mg, 690 μmol) in MeCN (5 mL) was treated at RT with DIPEA (600 μL, 3.5 mmol; [7087-68-5]) and dropwise with a solution of crude benzyl (2R)-3-(benzyloxy)-2-[(trifluoromethanesulfonyl)oxy]propanoate (1.3 g, 3.1 mmol) in MeCN (25 mL). The reaction mixture was stirred at 70°C overnight, cooled to RT and diluted with water. The mixture was extracted with DCM (2x), the combined organic layers washed with $Na_2CO_3$ (sat. aq.), filtered with a hydrophobic filter and concentrated under reduced pressure to give the crude title compound (1.08 g) which was used in the next step without further purification.
LC-MS (Method 3): Rt = 1.40/1.46min; MS (ESIpos): m/z = 1103.8 $[M+H]^+$, 552.4 $[M+2H]^{2+}$.

**Step 7**

methyl (2S,5E)-6-(4-butoxyphenyl)-2-{4,7,10-tris[(2S)-1,3-bis(benzyloxy)-1-oxopropan-2-yl]-1,4,7,10-tetraazacyclododecan-1-yl}hex-5-enoate

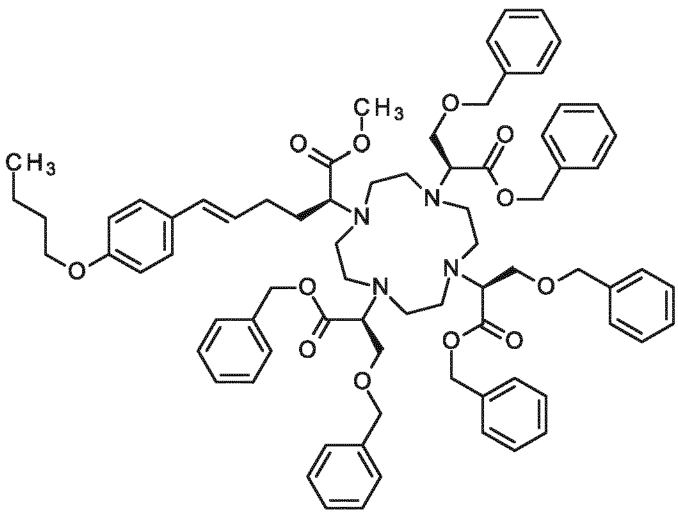

A mixture of crude methyl (2S)-2-{4,7,10-tris[(2S)-1,3-bis(benzyloxy)-1-oxopropan-2-yl]-1,4,7,10-tetraazacyclododecan-1-yl}hex-5-enoate (1.00 g, 906 µmol) and 1-bromo-4-butoxy-benzene (170 µL, 1.0 mmol; [39969-57-8]) in MeCN (90 mL) was degassed under reduced pressure and put under argon. TEA (2.5 mL, 18 mol; [121-44-8]), tri-o-tolylphosphine (99 mg, 330 µmol; [6163-58-2]) and palladium(II) acetate (24 mg, 110 µmol; [3375-31-3]) were added and the mixture was degassed under reduced pressure and put under argon again. The reaction mixture was stirred at 80°C overnight upon which another amount of 1-bromo-4-butoxybenzene (77 µL, 0.45 mmol) and palladium(II) acetate (24 mg, 110 µmol) were added and stirring continued for a further 24h. The mixture was cooled to RT, filtrated over Celite™ and the filter cake washed with MeCN. The filtrate was concentrated under reduced pressure, the residue taken up with DCM and washed with water. The organic layer was dried with $Na_2SO_4$, filtered, concentrated under reduced pressure and the crude material subjected to Biotage Isolera™ chromatography (SNAP Si - 100 g, eluting with DCM-hexane 1:1 followed by DCM-MeOH 85:15) to give the title compound (866 mg, 46%).

LC-MS (Method 3): Rt = 1.57min; MS (ESIpos): m/z = 626.5 $[M+2H]^{2+}$.

Step 8

(2S,2'S,2"S)-2,2',2"-{10-[(2S)-6-(4-butoxyphenyl)-1-methoxy-1-oxohexan-2-yl]-1,4,7,10-tetraazacyclododecane-1,4,7-triyl}tris(3-hydroxypropanoic acid)

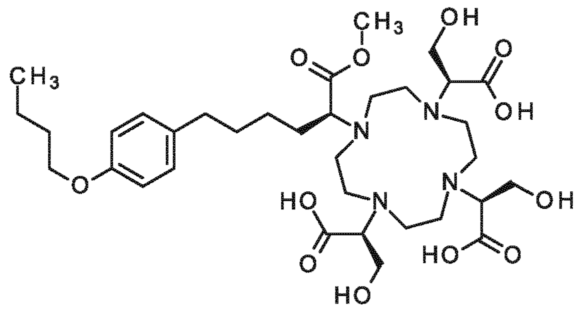

Methyl (2S,5E)-6-(4-butoxyphenyl)-2-{4,7,10-tris[(2S)-1,3-bis(benzyloxy)-1-oxopropan-2-yl]-1,4,7,10-tetraazacyclododecan-1-yl}hex-5-enoate (850 mg, 679 µmol) was placed into a steel autoclave under argon atmosphere and dissolved in a 3:1 mixture of MeOH and THF (28 mL). Pd on charcoal (180 mg, 10% purity, 170 µmol, 0.25 eq.; [7440-05-3]) was added and the reaction mixture heated at 80°C under a $H_2$ pressure of 40 bar for 30h yielding a maximum pressure of 42 bar. As the conversion was not complete another amount of Pd on charcoal (180 mg, 10% purity, 170 µmol, 0.25 eq.) was added and the mixture heated at 80°C under a $H_2$ pressure of 40 bar for another 40h. The autoclave was cooled to RT, the pressure was released, and the reaction mixture was filtrated. The filter cake was washed with MeOH (20 mL) and THF (20 mL) and the filtrate concentrated under reduced pressure to give the crude title compound (672 mg) which was used in the next step without further purification.

LC-MS (Method 3): Rt = 0.92min; MS (ESIpos): ): m/z = 713.6 $[M+H]^+$, 357.3 $[M+2H]^{2+}$.

Step 9

(2S)-6-(4-butoxyphenyl)-2-{4,7,10-tris[(1S)-1-carboxy-2-hydroxyethyl]-1,4,7,10-tetraazacyclododecan-1-yl}hexanoic acid

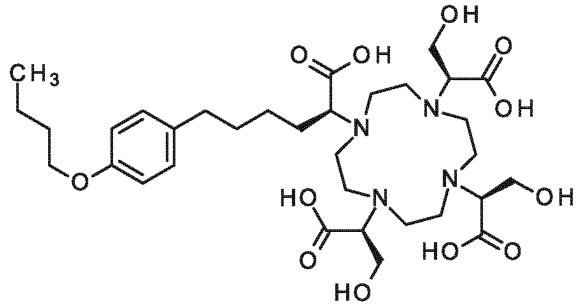

A solution of crude (2S,2'S,2"S)-2,2',2"-{10-[(2S)-6-(4-butoxyphenyl)-1-methoxy-1-oxohexan-2-yl]-1,4,7,10-tetraazacyclododecane-1,4,7-triyl}tris(3-hydroxypropanoic acid) (670 mg, 940 μmol) in THF (3 mL) was treated with NaOH (2 M. aq. 5.0 eq., 2.4 mL, 4.7 mmol) at RT overnight. The reaction mixture was adjusted to pH 7 by addition of HCl (2 M aq.) and concentrated under reduced pressure. The resulting aqueous layer was washed with DCM (2x), concentrated to a volume of 4 mL and subjected to Biotage Isolera™ chromatography (SNAP Si-C18 - 60 g, eluting with water-MeCN 1:0 to 0:1) to give the title compound (7.3 mg).

LC-MS (Method 3): Rt = 0.87min; MS (ESIpos): m/z = 699.5 $[M+H]^+$, 350.4 $[M+2H]^{2+}$.

Step **10**

gadolinium (2S,2'S,2"S)-2,2',2"-{10-[(1S)-5-(4-butoxyphenyl)-1-carboxypentyl]-1,4,7,10-tetraazacyclododecane-1,4,7-triyl}tris(3-hydroxypropanoate)

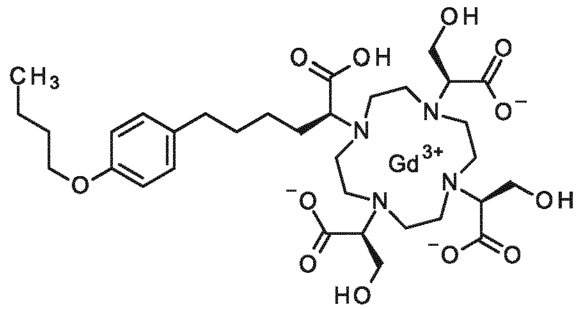

A solution of (2S)-6-(4-butoxyphenyl)-2-{4,7,10-tris[(1S)-1-carboxy-2-hydroxyethyl]-1,4,7,10-tetraazacyclododecan-1-yl}hexanoic acid (7.3 mg, 10 μmol) in water (1 mL) was treated with $Gd_2O_3$ (0.49 eq., 1.9 mg, 5.1 μmol; [12064-62-9]) and stirred at 105°C for 44h. The reaction mixture was cooled to RT, treated with an excess of Chelex100™ (sodium form, washed) chelating resin (pH was adjusted by addition of formic acid from pH 9 to 5), the resin filtered off and the filtrate lyophilized to give the title compound (5.1 mg, 52%).

LC-MS (Method 3): Rt = 0.96-0.99min; MS (ESIpos): m/z = 854.4 $[M+H]^+$, 427.7 $[M+2H]^{2+}$.

**Example 10**

**Step 1**

ethyl-3-(4-{2-[2-(2-ethoxyethoxy)ethoxy]ethoxy}phenyl)-2-[(methanesulfonyl)oxy]propanoate

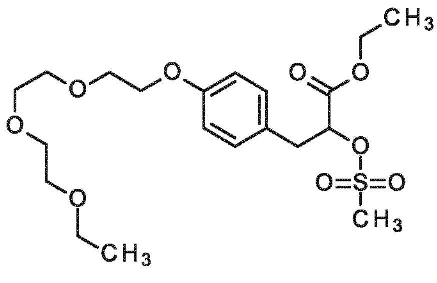

Racemic ethyl-3-(4-{2-[2-(2-ethoxyethoxy)ethoxy]ethoxy}phenyl)-2-hydroxypropanoate (**Intermediate 7**, 2.34 g, 6.32 mmol) and TEA (1.9 ml, 14 mmol; [121-44-8]) were stirred in THF (21 ml) under $N_2$ at 0-5°C. MsCl (540 μl, 6.9 mmol; [124-63-0]) was added dropwise to the mixture and stirring was continued for 3h. The mixture was then added to a solution of $NaHCO_3$ (50% sat. aq.), and the aqueous layer extracted with MTBE (3x), the combined organic layers washed with a solution of NaCl (sat. aq.), dried over $Na_2SO_4$, filtered and concentrated under reduced pressure yielding the crude title compound (2.97 g, 99% yield).

LC-MS (Method 4): Rt = 1.17min; MS (ESIpos): m/z = 466.2 $[M+H]^+$.

$^1$H NMR (400 MHz, DMSO-d6) δ ppm 1.09 (t, 3H) 1.36 (s, 9H) 3.00 - 3.08 (m, 4H) 3.38 - 3.48 (m, 2H) 3.44 - 3.47 (m, 2H) 3.49 - 3.55 (m, 4H) 3.56 - 3.59 (m, 2H) 3.69 - 3.77 (m, 2H) 4.03 - 4.08 (m, 2H) 5.05 - 5.16 (m, 1H) 6.84 - 6.94 (m, 2H) 7.14 - 7.25 (m, 2H).

**Step 2**

ethyl-3-(4-{2-[2-(2-ethoxyethoxy)ethoxy]ethoxy}phenyl)-2-(1,4,7,10-tetraazacyclododecan-1-yl)propanoate

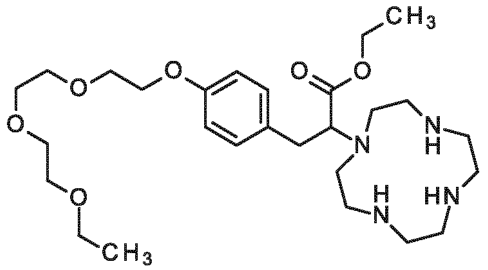

1,4,7,10-tetraazacyclododecane (384 mg, 2.23 mmol), racemic ethyl-3-(4-{2-[2-(2-ethoxy ethoxy)ethoxy] ethoxy}phenyl)-2-[(methanesulfonyl)oxy]propanoate (1.00 g, 2.23 mmol), $K_2CO_3$ (308 mg, 2.2 mmol) and MeCN (6.2 ml) were stirred under $N_2$ at 55°C for 24h. The mixture was filtered, the filter cake washed with EtOH, and organic filtrates combined and concentrated under reduced pressure yielding the crude title compound as a racemate (1.46 g, 61% product by ELSD).

LC-MS (Method 2): Rt = 0.55min; MS (ESIpos): m/z = 525.3 $[M+H]^+$.

**Step 3**

ethyl-3-(4-{2-[2-(2-ethoxyethoxy)ethoxy]ethoxy}phenyl)-2-[4,7,10-tris(2-tert-butoxy-2-oxoethyl)-1,4,7,10-tetraazacyclododecan-1-yl]propanoate

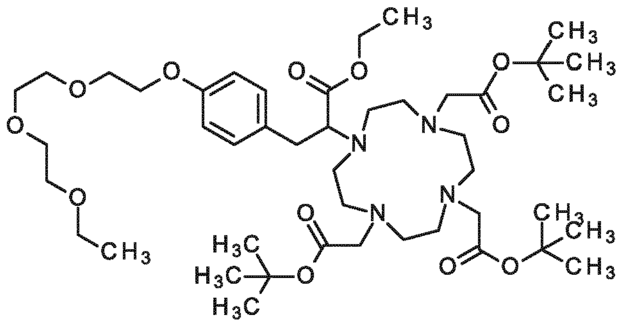

Racemic ethyl-3-(4-{2-[2-(2-ethoxyethoxy)ethoxy]ethoxy}phenyl)-2-(1,4,7,10-tetraazacyclodo-decan-1-yl)propanoate (1.40 g, 2.67 mmol) was dissolved MeCN (13 ml), $K_2CO_3$ (3.13 g, 22.7 mmol; [584-08-7]) was added. Under an atmosphere of $N_2$ tert-butyl bromoacetate (1.4 ml, 9.3 mmol, [ 5292-43-3]) in MeCN (3.8 ml) was added dropwise and the mixture was stirred overnight at RT. The mixture was filtered, the filter cake washed with EtOH, the combined organic phases were concentrated under reduced pressure affording the crude title compound (2.40 g, 104 % yield).

LC-MS (Method 2): Rt = 1.13min; MS (ESIpos): m/z = 867 $[M+H]^+$.

**Step 4**

2,2',2''-{10-[1-ethoxy-3-(4-{2-[2-(2-ethoxyethoxy)ethoxy]ethoxy}phenyl)-1-oxopropan-2-yl]-1,4,7,10-tetraazacyclododecane-1,4,7-triyl}triacetic acid

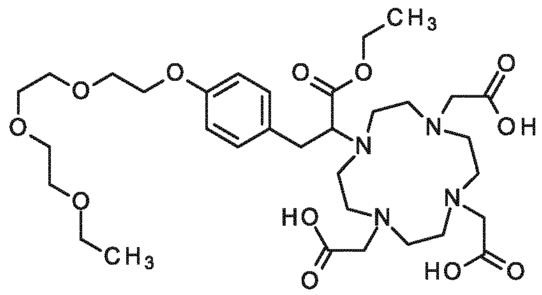

Racemic ethyl-3-(4-{2-[2-(2-ethoxyethoxy)ethoxy]ethoxy}phenyl)-2-[4,7,10-tris(2-tert-butoxy-2-oxoethyl)-1,4,7,10-tetraazacyclododecan-1-yl]propanoate (2.30 g, 2.65 mmol) was stirred in formic acid (15 ml, 400 mmol; [64-18-6]) overnight at 50°C. The solvent was removed under reduced pressure and the title compound was purified by RP-chromatography (Biotage SNAP Ultra C18 60 g, A = water, B = MeCN, 0%B to 100% B in 12 CV, 50 ml/min), yielding the racemate (614 mg, 33 % yield over 4 steps).

LC-MS (Method 2): Rt = 0.67min; MS (ESIpos): m/z = 700 $[M+H]^+$.

$^1$H NMR ($D_2O$, 400 MHz): δ (ppm) 7.31 (br d, *J* = 8.4 Hz, 2H), 6.95 (d, *J* = 8.4 Hz, 2H), 4.26 (br dd, *J* = 10.0, 4.7 Hz, 1H), 4.15-4.22 (m, 2H), 3.91-4.10 (m, 4H), 3.81-3.89 (m, 4H), 3.52-3.75 (m, 13H), 2.74-3.51 (m, 17H), 1.15 (t, *J* = 7.1 Hz, 3H), 1.03 (t, *J* = 7.1 Hz, 3H).

$^{13}$C NMR ($D_2O$, 101 MHz): δ (ppm) 174.7, 173.0, 170.1, 169.5, 156.9, 130.8 (2C), 129.8, 115.0 (2C), 69.8, 69.7, 69.6, 69.1, 69.0, 67.3, 66.7, 62.0 (2C), 56.8, 56.4, 52.9, 52.5, 51.1 (2C), 49.9, 49.0 (br), 46.7 (br), 45.8, 44.2, 34.2 (br), 14.1, 13.4.

**Step 5**

gadolinium 2,2", 2"-{10-[1-carboxy-2-(4-{2-[2-(2-ethoxyethoxy)ethoxy]ethoxy}phenyl)ethyl]-1,4,7,10-tetraazacyclododecane-1,4,7-triyl}triacetate

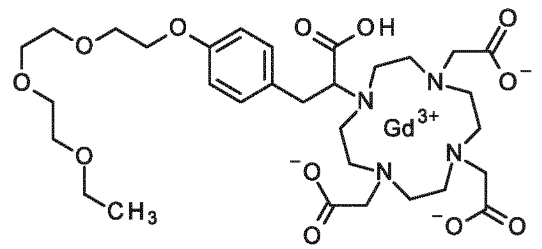

Racemic 2,2',2"-{10-[1-ethoxy-3-(4-{2-[2-(2-ethoxyethoxy)ethoxy]ethoxy}phenyl)-1-oxopropan-2-yl]-1,4,7,10-tetraazacyclododecane-1,4,7-triyl}triacetic acid (600 mg, 859 μmol), $Gd_2O_3$ (140 mg, 386 μmol) and water (8.8 ml) were stirred at 120°C for 24h. Chelex100™ was added (sodium form, washed, approximately 5 g), the pH brought to 5 using NaOH (1M, aq.) and the mixture stirred for 1h. Filtration and purification using RP C18 chromatography (Biotage SNAP Ultra C18 60g, 50 ml/min, A = water, B = MeCN, 0%B to 50%B in 25min, 100%B 10min) yielded the racemic title compound (607 mg, 95 % purity, 81 % yield).

LC-MS (Method 3): $R_t$ = 0.62min; MS (ESIpos): m/z = 413.6 $[M+2H]^{++}$, 826.3 $[M+H]^+$. Isotope patterns match theoretical values for corresponding gadolinium complex.

**Example 11**

**Step 1**

tert-butyl (2R)-3-(4-{2-[2-(2-ethoxyethoxy)ethoxy]ethoxy}phenyl)-2-[(methylsulfonyl)oxy] propanoate

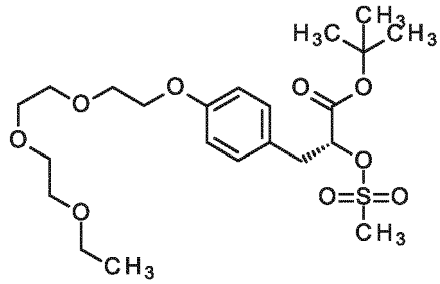

tert-butyl (2R)-3-(4-{2-[2-(2-ethoxyethoxy)ethoxy]ethoxy}phenyl)-2-hydroxypropanoate (**Intermediate 8,** 811 mg, 2.04 mmol) and TEA (620 µl, 4.5 mmol; [121-44-8]) were stirred in THF (6.8 ml) at 0 - 5 °C under an atmosphere of $N_2$, MsCl (170 µl, 2.2 mmol; [124-63-0]) was added dropwise to the mixture and stirring was continued for 3h. The mixture was then added to a solution of $NaHCO_3$ (50% sat. aq.), and the aqueous layer extracted with MTBE (3x), the combined organic layers washed with a solution of NaCl (sat. aq.), dried over $Na_2SO_4$, filtered and concentrated under reduced pressure yielding the crude title compound (1.02 g, 95 % purity, 100 % yield).

LC-MS (Method 4): Rt = 1.27min; MS (ESIpos): m/z = 494.2 $[M+NH_4]^+$.

$^1H$ NMR (400 MHz, DMSO-$d_6$) δ ppm 1.09 (t, 3H) 1.36 (s, 9H) 2.97 - 3.11 (m, 5H) 3.37 - 3.47 (m, 4H) 3.48 - 3.55 (m, 4H) 3.55 - 3.60 (m, 2H) 3.68 - 3.75 (m, 2H) 4.03 - 4.08 (m, 2H) 5.08 - 5.14 (m, 1H) 6.84 - 6.92 (m, 2H) 7.16 - 7.21 (m, 2H).

## Step 2

tert-butyl (2S)-3-(4-{2-[2-(2-ethoxyethoxy)ethoxy]ethoxy}phenyl)-2-(1,4,7,10-tetraazacyclodo-decan-1-yl)propanoate

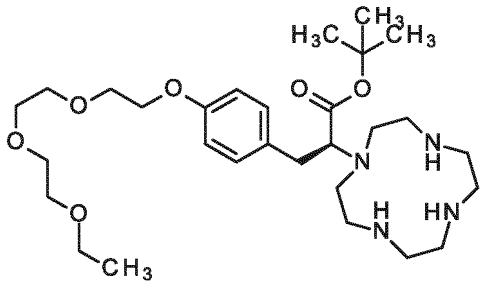

tert-butyl (2R)-3-(4-{2-[2-(2-ethoxyethoxy)ethoxy]ethoxy}phenyl)-2-[(methanesulfonyl)oxy] propanoate (1.02 g, 2.14 mmol) and 1,4,7,10-tetraazacyclododecane (402 mg, 2.33 mmol) were heated and stirred overnight at 55°C under $N_2$ in MeCN (18 ml), followed by 6h at 65°C followed by further 18h at 70°C. The mixture was then concentrated under reduced pressure affording the crude product (1.18 g, 67% product by ELSD).

LC-MS (Method 5): Rt = 0.69min; MS (ESIpos): m/z = 553.9 $[M+NH_4]^+$.

## Step 3

tert-butyl (2S)-3-(4-{2-[2-(2-ethoxyethoxy)ethoxy]ethoxy}phenyl)-2-[4,7,10-tris(2-tert-butoxy-2-oxoethyl)-1,4,7,10-tetra-azacyclododecan-1-yl]propanoate

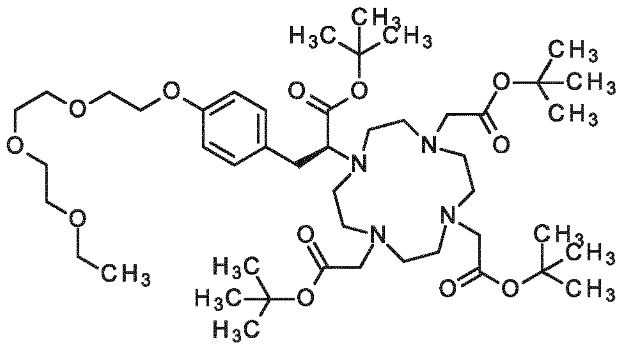

tert-butyl (2S)-3-(4-{2-[2-(2-ethoxyethoxy)ethoxy]ethoxy}phenyl)-2-(1,4,7,10-tetraazacyclododecan-1-yl)propanoate (1.18 g, 2.13 mmol) and MeCN (22 ml) were stirred under $N_2$. DIPEA (1.9 ml, 11 mmol; [7087-68-5]) was added, followed by tert-butyl bromoacetate (1.1 ml, 7.5 mmol) in MeCN (370 µl) and the mixture stirred at 60°C for 5h. The mixture was concentrated under reduced pressure. The residue was then dissolved in EtOAc and the organic phase washed with water, $NaHCO_3$ (aq.), NaCl (sat. aq.), dried over $Na_2SO_4$, filtered and concentrated under reduced pressure affording the crude product (2.87 g, 41% product by ELSD).

LC-MS (Method 2): Rt = 1.17min; MS (ESIpos): m/z = 895.4 $[M+H]^+$.

__Step__ **4**

(2S)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid

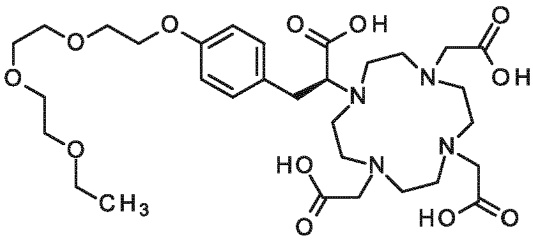

tert-butyl (25)-3-(4-{2-[2-(2-ethoxyethoxy)ethoxy]ethoxy}phenyl)-2-[4,7,10-tris(2-tert-butoxy-2-oxoethyl)-1,4,7,10-tetraazacyclododecan-1-yl]propanoate (2.87 g, 3.21 mmol) was stirred at 70°C for 4h in formic acid (41 ml). The mixture was then concentrated under reduced pressure, and purified by RP-Chromatography (Biotage C18 / 60g, 50 ml/min, A = water, B = MeCN, 0%B to 50%B in 30min, 100%B 10min) yielding the title compound (337 mg, 95 % purity, 16 % yield).

Specific Rotation: -2.4° ± 0.3 (C=1, MeOH, 20°C, 589 nm).

LC-MS (Method 2): Rt = 0.62min; MS (ESIpos): m/z = 671.3 $[M+H]^+$.

$^1$H NMR (DMSO-$d_6$, 400 MHz): δ (ppm) 7.20 (br d, *J* = 8.4 Hz, 2H), 6.82 (d, *J* = 8.6 Hz, 2H), 3.99-4.07 (m, 2H), 3.68-3.75 (m, 2H), 3.25-3.64 (m, 18H), 2.54-3.19 (m, 18H), 1.09 (t, *J* = 7.0 Hz, 3H).

$^{13}$C NMR (DMSO-$d_6$, 101 MHz): δ (ppm) 172.1, 171.3, 169.7 (br, 2C), 156.9, 130.5 (2C), 130.4, 114.1 (2C), 69.9, 69.9, 69.8, 69.2, 69.0, 67.0, 65.6, 65.1 (br), 55.2 (br), 54.9 (br, 2C), 51.7 (br, 2C), 51.5 (br, 2C), 49.1 (br, 2C), 46.8 (br, 2C), 32.8 (br), 15.2.

__Step 5__

gadolinium 2,2',2"-{10-[(1S)-1-carboxy-2-(4-{2-[2-(2-ethoxyethoxy)ethoxy]ethoxy}phenyl)ethyl]-1,4,7,10-tetraazacyclododecane-1,4,7-triyl}triacetate

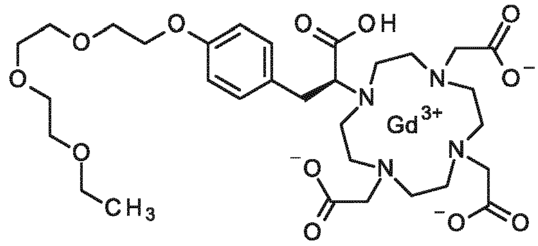

(2S)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid (339 mg, 505 μmol), $Gd_2O_3$ (82.4 mg, 227 μmol) and water (5.2 ml), were stirred at 105°C overnight in a crimp sealed vial, followed by a further 5h of stirring at 120°C. Chelex100™ was added (sodium form, washed, approximately 5 g), the pH brought to 5 using NaOH (1M, aq.) and the mixture stirred for 1h. Filtration and purification using RP-chromatography (Biotage C18 30g, 25 ml/min, A = water, B = MeCN, 0%B to 50%B in 25min, 100%B 10min) yielded the title compound (307 mg, 95 % purity, 74 % yield).

Specific Rotation: 14.9° (c=1, MeOH, 20°C, 589 nm).

LC-MS (Method 3): Rt = 0.64min; MS (ESIpos): m/z = m/z = 413.6 $[M+2H]^{++}$, 826.4 $[M+H]^{+}$. Isotope patterns match theoretical values for corresponding gadolinium complex.

## Example 12

### Step 1

(2R)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid

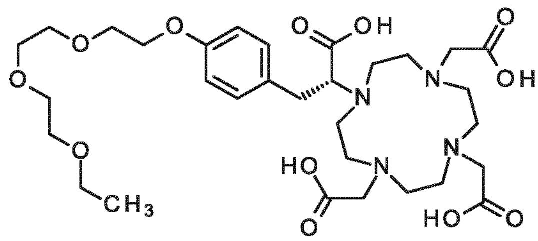

(2R)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid was produced analogously to (2S)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid (**Example 11** - **Step 4**) from tert-butyl (2S)-3-(4-{2-[2-(2-ethoxyethoxy)ethoxy]ethoxy}phenyl)-2-hydroxypropanoate (**Intermediate 9**) yielding the title compound (310 mg, 16 % total yield over the 4 steps).

Specific Rotation: 1.7° ± 0.53 (c=1, MeOH, 20°C, 589 nm).

LC-MS (Method 2): Rt = 0.61min; MS (ESIpos): m/z = 671.3 $[M+H]^{+}$.

$^{1}H$ NMR (DMSO-$d_6$, 400 MHz): δ (ppm) 7.11-7.23 (m, 2H), 6.82 (d, *J* = 8.6 Hz, 2H), 3.99-4.07 (m, 2H), 3.68-3.75 (m, 2H), 3.36-3.62 (m, 17H), 2.53-3.14 (m, 18H), 1.09 (t, *J* = 7.0 Hz, 3H).

$^{13}C$ NMR (DMSO-$d_6$, 101 MHz): δ (ppm) 172.1 (br), 171.3 (br), 169.7 (br, 2C), 156.9, 130.5 (2C), 130.4, 114.1 (2C), 69.9, 69.9, 69.8, 69.2, 69.0, 67.0, 65.6, 65.1 (br), 55.2 (br), 54.9 (br, 2C), 51.7 (br, 2C), 51.5 (br, 2C), 49.1 (br, 2C), 46.8 (br, 2C), 32.8 (br), 15.2.

### Step 2

gadolinium 2,2',2"-{10-[(1R)-1-carboxy-2-(4-{2-[2-(2-ethoxyethoxy)ethoxy]ethoxy}phenyl)ethyl]-1,4,7,10-tetraazacyclododecane-1,4,7-triyl}triacetate

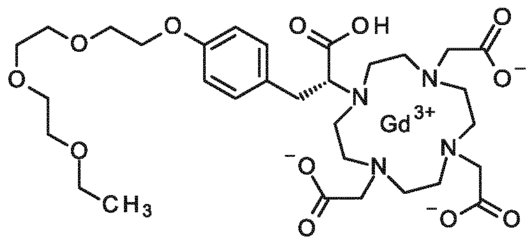

(2R)-3-(4-{2-[2-(2-ethoxyethoxy)ethoxy]ethoxy}phenyl)-2-[4,7,10-tris(carboxymethyl)-1,4,7,10-tetraazacyclododecan-1-yl]propanoic acid (305 mg, 455 μmol), $Gd_2O_3$ (74.2 mg, 205 μmol) and water (4.6 ml), were stirred at 105°C overnight in a crimp sealed vial, followed by a further 5h of stirring at 120°C. Chelex100™ was added (sodium form, washed, approximately 5 g), the pH brought to 5 using NaOH (1M, aq.) and the mixture stirred for 1h. Filtration and purification using RP-chromatography (Biotage C18 30 g, 25 ml/min, A = water, B = MeCN, 0%B to 50%B in 25min, 100%B 10min) yielded the title compound (292 mg, 100 % purity, 78 % yield).
Specific Rotation: -13.7° (c=1, MeOH, 20°C, 589 nm).
LC-MS (Method 3): Rt = 0.64min; MS (ESIpos): m/z = 413.7 $[M+2H]^{++}$, 826.4 $[M+H]^+$. Isotope patterns match theoretical values for corresponding gadolinium complex.

**Example 13**

**Step 1**

ethyl-3-(4-{2-[2-(2-ethoxyethoxy)ethoxy]ethoxy}phenyl)-2-[(methanesulfonyl)oxy]propanoate

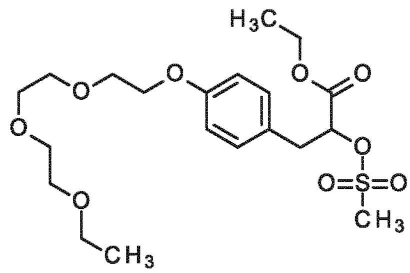

Racemic ethyl-3-(4-{2-[2-(2-ethoxyethoxy)ethoxy]ethoxy}phenyl)-2-hydroxypropanoate , **Intermediate** 7, 2.34 g, 6.32 mmol) and TEA (1.9 ml, 14 mmol; [121-44-8]) were stirred in THF (21 ml) at 0-5°C. Under an atmosphere of $N_2$, MsCl (540 μl, 6.9 mmol; [124-63-0]) was added dropwise, the mixture was warmed to RT and stirred for 3h. The mixture was added to $NaHCO_3$ (50% sat. aq.), followed by extraction with MTBE (3x), the organic phases were combined and washed with NaCl solution (sat. aq. 2x), dried over $Na_2SO_4$ and concentrated under reduced pressure affording the title compound (2.97 g 95 % purity, quantitative).
LC-MS (Method 4): $R_t$ = 1.17min; MS (ESIpos): m/z = 466.2 $[M+NH_4]^+$.

**Step 2**

ethyl 2-[4,10-bis(2-tert-butoxy-2-oxoethyl)-1,4,7,10-tetraazacyclododecan-1-yl]-3-(4-{2-[2-(2-ethoxyethoxy)ethoxy]ethoxy}phenyl)propanoate

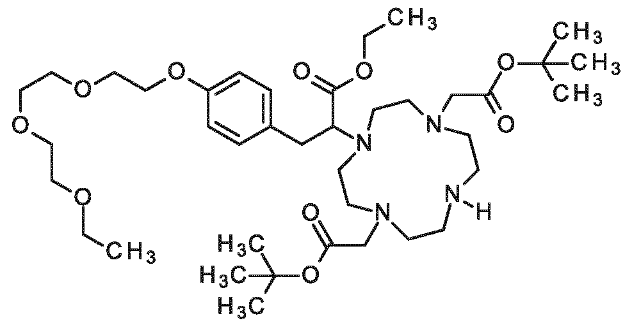

Racemic ethyl-3-(4-{2-[2-(2-ethoxyethoxy)ethoxy]ethoxy}phenyl)-2-[(methanesulfonyl)oxy] propanoate (2.79 g, 6.22 mmol), di-tert-butyl 2,2'-(1,4,7,10-tetraazacyclododecane-1,7-diyl)diacetate (2.27 g, 5.65 mmol, [162148-48-3]), $K_2CO_3$ (1.56 g, 11.3 mmol) and MeCN (51 ml) were stirred together in a reaction vessel at 70°C under $N_2$ for 4d. The mixture was then filtered, concentrated under reduced pressure affording the crude title compound (3.60 g, 50% product by ESLD detection).

LC-MS (Method 2): Rt = 1.25min; MS (ESIpos): m/z = 753.8 $[M+H]^+$.

## Step 3

ethyl-2-{4,10-bis(2-tert-butoxy-2-oxoethyl)-7-[1-ethoxy-3-(4-{2-[2-(2-ethoxyethoxy)ethoxy] ethoxy}phenyl)-1-oxopropan-2-yl]-1,4,7,10-tetraazacyclododecan-1-yl}-3-methoxypropanoate

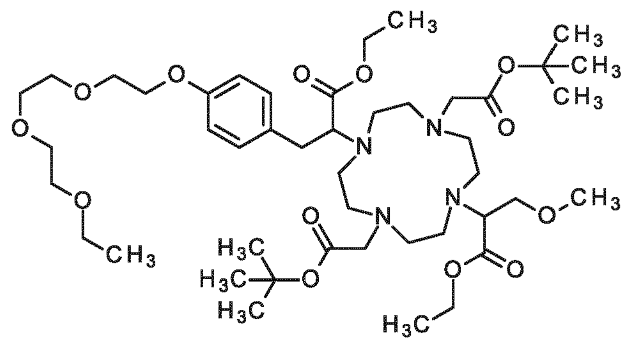

ethyl-2-[4,10-bis(2-tert-butoxy-2-oxoethyl)-1,4,7,10-tetraazacyclododecan-1-yl]-3-(4-{2-[2-(2-ethoxyethoxy) ethoxy]ethoxy}phenyl)propanoate (1.80 g, 2.39 mmol), ethyl-2-[(methanesulfonyl)oxy]-3-methoxypropanoate (**Intermediate 25,** 1.24 g, 5.50 mmol), $K_2CO_3$ (661 mg, 4.78 mmol; [584-08-7]) and MeCN (9 ml), were stirred for 24h at 80°C. The mixture was filtered, the filter cake washed with EtOH, the organic layers combined and concentrated under reduced pressure affording the crude title compound as a mixture of diastereomers (2.74 g).

LC-MS (Method 2): Rt = 1.10 - 1.17min; MS (ESIpos): m/z = 884.0 $[M+H]^+$.

## Step 5

2,2'-{4-[1-ethoxy-3-(4-{2-[2-(2-ethoxyethoxy)ethoxy]ethoxy}phenyl)-1-oxopropan-2-yl]-10-[1-ethoxy-3-methoxy-1-oxopropan-2-yl]-1,4,7,10-tetraazacyclododecane-1,7-diyl}diacetic acid

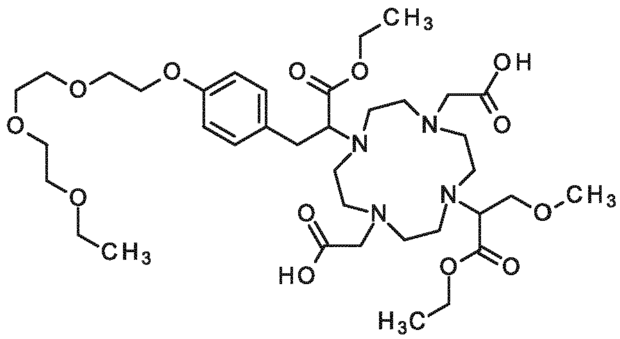

Crude ethyl-2-{4,10-bis(2-tert-butoxy-2-oxoethyl)-7-[1-ethoxy-3-(4-{2-[2-(2-ethoxyethoxy) ethoxy]ethoxy}phenyl)-1-oxopropan-2-yl]-1,4,7,10-tetraazacyclododecan-1-yl}-3-methoxy-propanoate (2.73 g, 3.09 mmol) was stirred with formic acid (40 ml) for 4h at 70°C. The mixture was then concentrated under reduced pressure followed by purification with RP-chromatography (Biotage, SNAP C18 50g, A = water, B = MeCN, 0%B to 50%B in 30min, 100%B 10min) yielding the title compound as a mixture of diastereomers (229 mg, 10 % yield over 5 steps).
LC-MS (Method 2): Rt = 0.76min; MS (ESIpos): m/z = 771.8 $[M+H]^+$.
$^1H$ NMR (DMSO-$d_6$, 400 MHz): δ (ppm) 7.10-7.31 (m, 2H), 6.76-6.86 (m, 2H), 3.61-4.16 (m, 12H), 3.37-3.60 (m, 14H), 3.21 (s, 3H), 2.53-3.13 (m, 18H), 0.97-1.25 (m, 9H).

**Step 6**

gadolinium 2-{7-[1-carboxy-2-(4-{2-[2-(2-ethoxyethoxy)ethoxy]ethoxy}phenyl)ethyl]-4,10-bis(carboxylatomethyl)-1,4,7,10-tetraazacyclododecan-1-yl}-3-methoxypropanoate

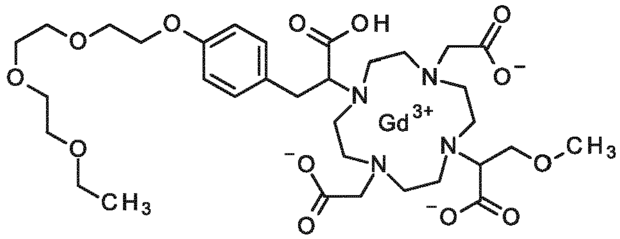

The diastereomeric mixture, 2,2'-{4-[1-ethoxy-3-(4-{2-[2-(2-ethoxyethoxy)ethoxy]ethoxy} phenyl)-1-oxopropan-2-yl]-10-[1-ethoxy-3-methoxy-1-oxopropan-2-yl]-1,4,7,10-tetraazacyclododecane-1,7-diyl}diacetic acid (220 mg, 285 μmol), $Gd_2O_3$ (46.6 mg, 128 μmol) and water (2.9 ml) were stirred for 24h at 120°C. Chelex100™ was added (sodium form, washed, approximately 5 g), the pH brought to 5 using NaOH (1M, aq.) and the mixture stirred for 1h. Filtration and purification using RP-chromatography (Biotage SNAP Ultra C18 30g, 25 ml/min, A = water, B = MeCN, 0%B to 50%B in 25min, 100% B 10min) yielded the racemic title diastereomeric mixture (45.1 mg, 18 % yield).
LC-MS (Method 3): Rt = 0.65min (diastereomer 1, 72% UV), 0.69min (diastereomer 2, 27 % UV); MS (ESIpos): m/z = 435.7 $[M+2H]^{++}$ ; 870.5 $[M+H]^+$. Isotope patterns match theoretical values for corresponding gadolinium complex.

**Example 14**

**Step 1**

methyl-3-(4-butoxyphenyl)-2-[(methanesulfonyl)oxy]propanoate

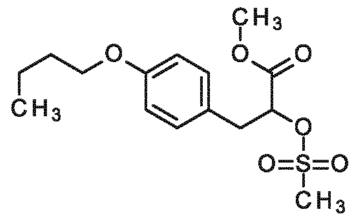

Racemic methyl-3-(4-butoxyphenyl)-2-hydroxypropanoate (**Intermediate 10,** 919 mg, 3.64 mmol), TEA (1.1 ml, 8.0 mmol; [121-44-8]) and THF (11 ml) were stirred at 0-5°C under an atmosphere of $N_2$. MsCl (310 µl, 4.0 mmol; [124-63-0]) was added dropwise, the mixture was warmed to RT and stirred for 3h. The mixture was added to $NaHCO_3$ (50% sat. aq.), followed by extraction with MTBE (3x), the organic phases were combined and washed with NaCl solution (sat. aq. 2x), dried over $Na_2SO_4$ and concentrated under reduced pressure affording the title compound (1.25 g, 99 % yield).

LC-MS (Method 4): Rt = 1.29min; MS (ESIpos): m/z = 348.2 $[M+NH_4]^+$.

$^1H$ NMR (400 MHz, DMSO-$d_6$) δ ppm 0.92 (t, 3H) 1.35 - 1.47 (m, 2H) 1.63 - 1.71 (m, 2H) 2.93 - 3.17 (m, 5H) 3.68 (s, 3H) 3.83 - 3.95 (t, 2H) 5.29 (m, 1H) 6.86 (d, 2H) 7.15 (d, 2H).

**Step 2**

methyl-3-(4-butoxyphenyl)-2-(1,4,7,10-tetraazacyclododecan-1-yl)propanoate

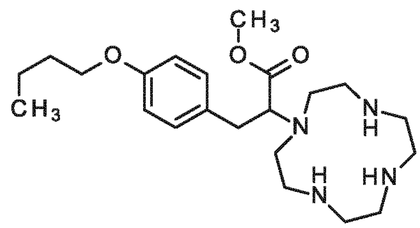

1,4,7,10-tetraazacyclododecane (1.30 g, 7.57 mmol) was dissolved in MeCN (28 ml), $K_2CO_3$ (836 mg, 6.1 mmol) was added, and racemic methyl-3-(4-butoxyphenyl)-2-[(methanesulfonyl)oxy]propanoate (2.00 g, 6.05 mmol) in MeCN (8.0 ml) was added and the mixture stirred overnight at 55°C. The mixture was filtered, the filter cake washed with EtOH, the organic filtrates combined and concentrated under reduced pressure yielding the crude title compound (2.49 g). The presence of the corresponding ethyl ester was also detected in the crude product.

LC-MS (Method 2): $R_t$ = 0.67min; MS (ESIpos): m/z = 407.3 $[M+H]^+$.

**Step 3**

methyl-3-(4-butoxyphenyl)-2-[4,7,10-tris(2-tert-butoxy-2-oxoethyl)-1,4,7,10-tetraazacyclododecan-1-yl]propanoate

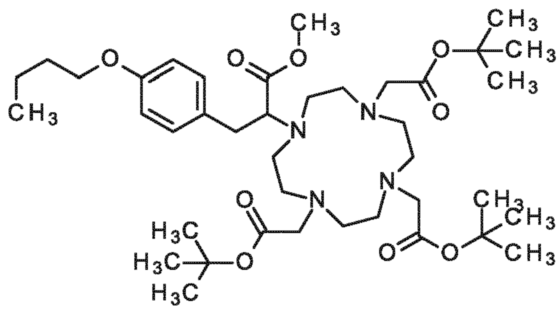

Crude methyl-3-(4-butoxyphenyl)-2-(1,4,7,10-tetraazacyclododecan-1-yl)propanoate (2.49 g, 6.12 mmol) was dissolved in MeCN (24 ml), $K_2CO_3$ (7.19 g, 52.1 mmol; [584-08-7]) was added, and under an atmosphere of $N_2$, tert-butyl

bromoacetate (3.2 ml, 21 mmol) dissolved in MeCN (7.9 ml) was added dropwise. The mixture was stirred overnight at RT, the mixture filtered, the filter cake washed with EtOH and the combined organic fractions concentrated under reduce pressure yielding the crude title compound (5.12 g). The presence of the corresponding ethyl ester was also detected in the crude product, presumably through extended contact with ethanol during the workup.

LC-MS (Method 2): Rt = 1.38min; MS (ESIpos): m/z = 749.5 $[M+H]^+$.

**Step 4**

2,2',2"-{10-[3-(4-butoxyphenyl)-1-ethoxy-1-oxopropan-2-yl]-1,4,7,10-tetraazacyclododecane-1,4,7-triyl}triacetic acid

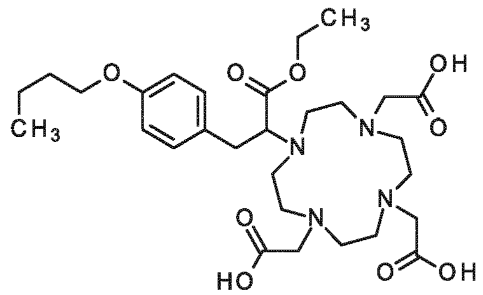

Crude methyl-3-(4-butoxyphenyl)-2-[4,7,10-tris(2-tert-butoxy-2-oxoethyl)-1,4,7,10-tetraaza-cyclododecan-1-yl]propanoate (5.12 g, 6.84 mmol, also containing the corresponding ethyl ester) was stirred with formic acid (88 ml) at 70°C for 4h and the mixture concentrated under reduced pressure. Purification using RP-chromatography (Biotage Ultra C18 60g, 50 ml/min, A = water, B = MeCN, 0%B to 50%B in 25min, 100%B 10min) yielded the title compound (262 mg, 6 % yield over 4 steps).

LC-MS (Method 2): Rt = 0.79min; MS (ESIpos): m/z = 595.3 $[M+H]^+$.

$^{13}C$ NMR (DMSO-$d_6$, 101 MHz): δ (ppm) 171.1, 171.0 (br), 169.7 (br, 2C), 157.3, 130.5 (2C), 129.6, 114.1 (2C), 67.0, 64.7 (br), 59.9, 55.2 (br), 55.0 (br, 2C), 51.6 (br, 2C), 51.3 (br, 2C), 49.1 (br, 2C), 46.8 (br, 2C), 33.7 (br), 30.9, 18.8, 14.2, 13.8.

**Step 5**

gadolinium 2,2',2"-{10-[2-(4-butoxyphenyl)-1-carboxyethyl]-1,4,7,10-tetraazacyclododecane-1,4,7-triyl}triacetate

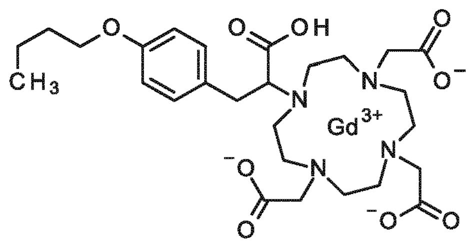

Racemic 2,2',2"-{10-[3-(4-butoxyphenyl)-1-ethoxy-1-oxopropan-2-yl]-1,4,7,10-tetraazacyclododecane-1,4,7-triyl}triacetic acid (253 mg, 425 μmol), $Gd_2O_3$ (69.4 mg, 191 μmol) and water (4.3 ml) were stirred at 105°C for 3d. Chelex100™ was added (sodium form, washed, approximately 5 g), the pH brought to 5 using NaOH (1M, aq.) and the mixture stirred for 1h showing the mixture was gadolinium free. Filtration and purification using RP-chromatography (Biotage Ultra C18 12 g, 12 ml/min, A = water, B = MeCN, 0%B to 50%B in 25min, 100%B 10min) yielded the title compound (196 mg, 64%).

LC-MS (Method 3): Rt = 0.75min; MS (ESIpos): m/z = 361.7 $[M+2H]^{++}$, 722.3 $[M+H]^+$. Isotope patterns match theoretical values for corresponding gadolinium complex.

**Example 15**

Step 1

methyl (2R)-5-{4-[2-(2-ethoxyethoxy)ethoxy]phenyl}-2-[(trifluoromethanesulfonyl) oxy]pentanoate

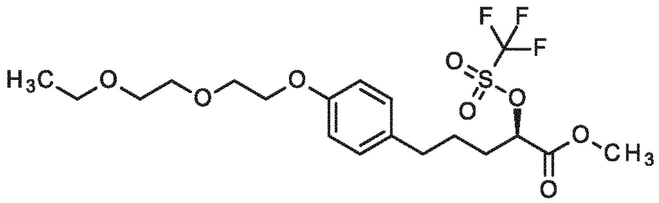

**Intermediate 12,** methyl (2R)-5-{4-[2-(2-ethoxyethoxy)ethoxy]phenyl}-2-hydroxypentanoate, (5.00 g, 14.7 mmol) was dissolved in DCM (50 ml), placed under $N_2$ and cooled to -60°C. Lutidine (2.1 ml, 18 mmol; [108-48-5]) was added followed by dropwise addition of trifluoromethanesulfonic anhydride (15 ml, 1.0 M in DCM, 15 mmol; [358-23-6]) at -60°C. The reaction was stirred for 2h and then allowed to warm to ca. 8°C. Diethyl ether was added to precipitate the lutidine triflate, filtered and the filter cake washed with diethyl ether. The combined filtrates were extracted with water and HCl (1 M, aq.), dried ($Na_2SO_4$) and concentrated under reduced pressure. 6.97 g (85%) of the title compound were obtained as reddish oil, which was used directly in the next step.
$^{19}F$ NMR ($CDCl_3$, 377 MHz): δ (ppm) -76.04 (s).
$^{1}H$ NMR ($CDCl_3$, 400 MHz): δ (ppm) 7.03-7.07 (m, 2H), 6.82-6.87 (m, 2H), 5.12 (t, *J* = 6.1 Hz, 1H), 4.10-4.14 (m, 2H), 3.86 (dd, *J* = 5.6, 4.3 Hz, 2H), 3.82 (s, 3H), 3.70-3.74 (m, 2H), 3.60-3.63 (m, 2H), 3.54 (q, *J* = 6.9 Hz, 2H), 2.56-2.65 (mc, 2H), 1.95-2.03 (m, 2H), 1.70-1.79 (m, 2H), 1.21 (t, *J* = 7.0 Hz, 3H). The sample contains 1.1 eq (15%) diethyl ether: 3.48 (q, *J* = 7.1 Hz, 0.48H), 1.21 (br t, *J* = 7.0 Hz, 0.69H).

**Step 2**

methyl (2S)-5-{4-[2-(2-ethoxyethoxy)ethoxy]phenyl}-2-(1,4,7,10-tetraazacyclododecan-1-yl)pentanoate

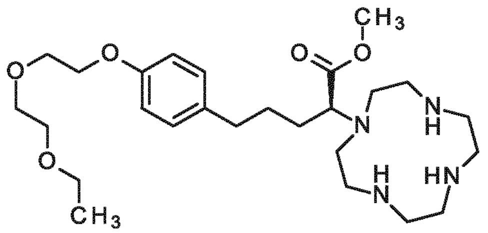

1,4,7,10-tetraazacyclododecane (10.1 g, 58.8 mmol, ca. 5 eq) was dissolved in chloroform (100 ml), DIPEA (2.0 ml, 12 mmol; [7087-68-5]) and a solution of methyl (2R)-5-{4-[2-(2-ethoxyethoxy)ethoxy]phenyl}-2-[(trifluoromethanesulfonyl)oxy]pentanoate (6.94 g) in chloroform (25 ml) added. The reaction was stirred overnight at RT. The mixture was extracted with water (3 x) to remove excess cyclen and sat. NaCl, dried ($Na_2SO_4$) and concentrated under reduced pressure to give the title compound (7,55 g) as an orange-brown oil, part of which was used directly in the next step.
LC-MS (Method 2): $R_t$ = 0.72min; MS (ESIpos): m/z = 496 $[M+H]^+$.

**Step 3**

methyl (2S)-5-{4-[2-(2-ethoxyethoxy)ethoxy]phenyl}-2-{4,7,10-tris[(2S)-3-tert-butoxy-1-methoxy-1-oxopropan-2-yl]-1,4,7,10-tetraazacyclododecan-1-yl}pentanoate

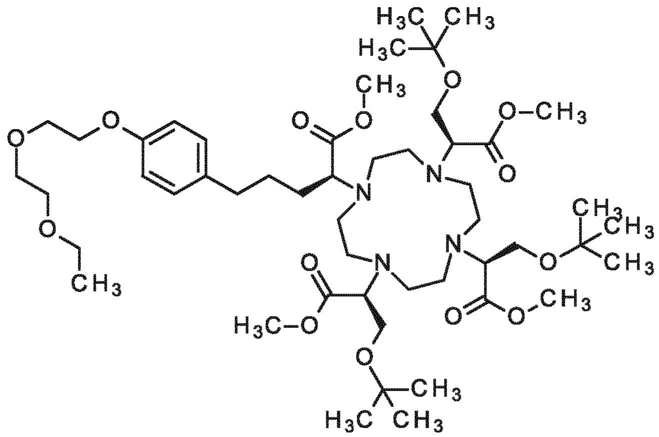

methyl (2S)-5-{4-[2-(2-ethoxyethoxy)ethoxy]phenyl}-2-(1,4,7,10-tetraazacyclododecan-1-yl) pentanoate (5.81 g, 11.7 mmol) was dissolved in MeCN (150 ml) and $K_2CO_3$ (5.68 g, 41.1 mmol) added. A solution of **Intermediate 26,** methyl (2R)-3-tert-butoxy-2-[(trifluoromethanesulfonyl)oxy]propanoate (13.3 g, 90 % purity, 38.8 mmol), in MeCN (50 ml) was added dropwise and the mixture stirred overnight at RT. The mixture was filtrated, the filter cake washed (MeCN) and the combined filtrates concentrated under reduced pressure. The residue obtained, 30,90 g, was absorbed to Isolute™ and purified by RP-chromatography (SNAP C18 400 g, 100 ml/min, 254 nm, A = water/0.1% formic acid, B = MeCN, 10% B 6CV, 10% to 41% B in 8.8CV, 41% B 1.7CV, 41% to 80% B in 11CV, 80%B 2.9CV). After lyophilization, two fractions of the title compound were obtained.

Fraction 1: 2.38 g

LC-MS (Method 2): Rt = 1.37min; MS (ESIpos): m/z = 970 $[M+H]^+$.

Fraction 2: 5.76 g

LC-MS (Method 2): $R_t$ = 1.24min; MS (ESIpos): m/z = 970 $[M+H]^+$.

Specific Rotation: $\alpha_D^{20}$ = +14.60° +/- 0.18° (c=1, MeOH).

LC-MS (Method 3): Rt = 1.17min (91% DAD); MS (ESIpos): m/z = 485 $[M + 2H]^{++}$, 970 $[M + H]^+$.

$^1$H NMR ($CDCl_3$, 400 MHz): δ (ppm) 8.39 (s, 0.3H, formiate), 7.01-7.08 (m, 2H), 6.82 (d, *J* = 7.5 Hz, 2H), 4.07-4.13 (m, 2H), 3.48-3.95 (m, 30H), 2.49-3.43 (m, 18H), 1.49-1.88 (m, 4H), 1.09-1.25 (m, 30H).

$^{13}$C NMR ($CDCl_3$, 101 MHz): δ (ppm) 173.4 (br), 171.9 (br, 3C), 157.5, 134.3, 129.6 (2C), 115.0 (2C), 74.4, 74.2 (2C), 71.3, 70.3, 70.2, 67.8, 67.1, 63.9 (br), 63.2 (br, 3C), 60.0 (br, 2C), 59.7 (br), 52.5, 52.2 (2C), 52.1 (br, 8C), 51.2, 35.2, 29.2, 27.8 (6C), 27.7 (3C), 15.6. Some signals are very broad, one C in the propylene-chain is not observed.

The combined yield calculated from step 1 is 74%.

**Step 4**

methyl (2S)-5-{4-[2-(2-ethoxyethoxy)ethoxy]phenyl}-2-{4,7,10-tris[(2S)-3-hydroxy-1-methoxy-1-oxopropan-2-yl]-1,4,7,10-tetraazacyclododecan-1-yl}pentanoate

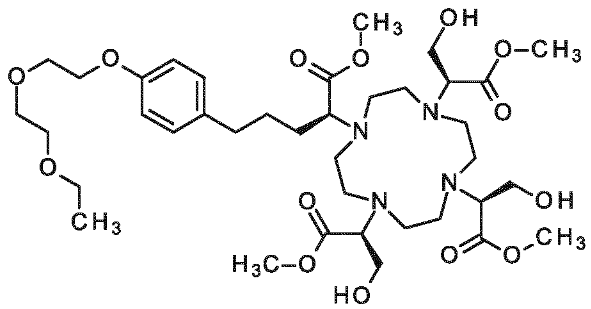

methyl (2S)-5-{4-[2-(2-ethoxyethoxy)ethoxy]phenyl}-2-{4,7,10-tris[(2S)-3-tert-butoxy-1-methoxy -1-oxopropan-2-yl]-1,4,7,10-tetraazacyclododecan-1-yl}pentanoate (2.38 g, 2.46 mmol) was dissolved in 1,4-dioxane (60 ml) and HCl (9.8 ml, 4 M in dioxane, 39.2 mmol) added. The mixture was stirred at 80°C for 4h and then evaporated to dryness under reduced pressure. The raw product, 2,13 g of methyl (2S)-5-{4-[2-(2-ethoxyethoxy)ethoxy]phenyl}-2-{4,7,10-tris [(2S)-3-hydroxy-1-methoxy-1-oxopropan-2-yl]-1,4,7,10-tetraazacyclododecan-1-yl}pentanoate, was used as such in the next step.

**Step 5**

(2S)-5-{4-[2-(2-ethoxyethoxy)ethoxy]phenyl}-2-{4,7,10-tris[(1S)-1-carboxy-2-hydroxyethyl]-1,4,7,10-tetraazacyclododecan-1-yl}pentanoic acid

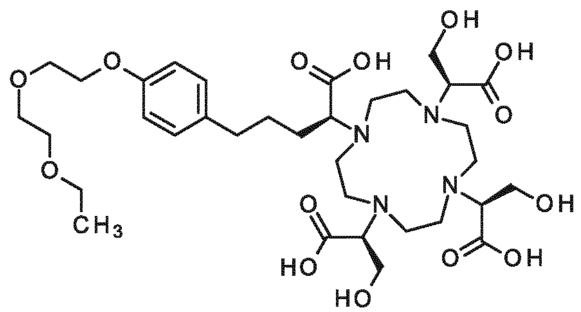

methyl (2S)-5-{4-[2-(2-ethoxyethoxy)ethoxy]phenyl}-2-{4,7,10-tris[(2S)-3-hydroxy-1-methoxy-1-oxopropan-2-yl]-1,4,7,10-tetraazacyclododecan-1-yl}pentanoate (2.13 g, 2.66 mmol) was dissolved in THF (13 ml) and a solution of lithium hydroxide (382 mg, 16.0 mmol) water (13 ml) was added. After stirring at RT overnight, the reaction was nearly complete. (*The presence of one methyl ester does negatively affect the complexation step,* as *the ester is hydrolyzed under the complexation conditions used. Therefore, complete saponification is not mandatory at this point.*) The reaction mixture was evaporated to dryness under reduced pressure to give 2.36 g raw material, which was used in the next reaction.

**Step 6**

gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)

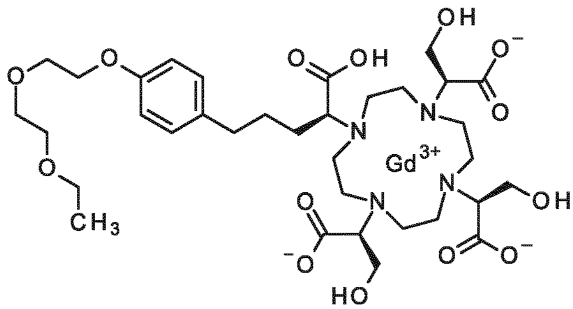

(2S)-5-{4-[2-(2-ethoxyethoxy)ethoxy]phenyl}-2-{4,7,10-tris[(1S)-1-carboxy-2-hydroxyethyl]-1,4,7,10-tetraazacyclododecan-1-yl}pentanoic acid (5.50 g, 7.38 mmol) was dissolved in water (400 ml) and the pH adjusted to 4 with formic acid. $Gd_2O_3$ (1.20 g, 3.32 mmol, 0.9 eq) was added and the mixture stirred at 100°C for 18h and overnight at RT and further 4h at 100°C. Chelex100™ (sodium form, ca. 10 g) were added and the mixture stirred 2d at RT, after which the Xylenol-orange test indicated the absence of free gadolinium. The pH of the solution was adjusted to 7 by addition of ammonium hydroxide (25%) and reduced to about 200 ml under reduced pressure. (*Fraction 2 of step 3 was taken through steps 4 to 6 in the same way. Although the retention times of the two fractions were different, they behaved in the same in the subsequent reactions and were combined at this point.*) The combined reactions (including the resin) were transferred to an empty Biotage cartridge and directly subjected to RP-chromatography (SNAP C18 400g, 100 ml/min, 254 nm. A = water, B = MeCN, 0% B 5CV, 0% to 45% B in 14CV). Interesting fractions were combined and lyophilized to give two fractions of gadolinium (2S,2'S,2"S)-2,2',2"-{10-[(1S)-1-carboxy-4-{4-[2-(2-ethoxyethoxy)ethoxy] phenyl} butyl]-1,4,7,10-tetraazacyclododecane-1,4,7-triyl}tris(3-hydroxypropanoate):

Fraction 1: 2.49 g

Specific rotation: $\alpha_D^{20}$ = +15.95° +/- 0.17° (c=0.84, $H_2O$).

LC-MS (Method 3): Rt = 0.73min (100% DAD); MS (ESIpos): m/z = 450 $[M + 2H]^{++}$, 900 $[M + H]^+$ and some $[2M + 2H]^{++}$; 909 $[2M + H_2O + 2H]^{++}$. The observed isotope pattern conforms to structure.

UPLC-MS (Method 2): Rt = 0.78min (100% ELSD), 0.75min (100% UV 220 nm) MS (ESIpos): m/z = 900 $[M + H]^+$.

Fraction 2: 1.99 g

Specific rotation: $\alpha_D^{20}$ = +16.38° +/- 0.35° (c=0.84, $H_2O$).

LC-MS (Method 3): Rt = 0.73min (100% DAD); MS (ESIpos): m/z = 450 $[M + 2H]^{++}$, 900 $[M + H]^+$ and some $[2M + 2H]^{++}$; 909 $[2M + H_2O + 2H]^{++}$. The observed isotope pattern conforms to structure.

UPLC-MS (Method 2): $R_t$ = 0.78min (100% ELSD), 0.75min (94% UV 220 nm) 0.80min (6%, UV, 220 nm), both peaks MS (ESIpos): m/z = 900 $[M + H]^+$.

Fraction 2 contains some stereoisomer, probably through a racemization event at one of the preceding steps. The combined yield from step 3 is 42%).

**Example 16**

**Step 1**

tert-butyl 3-{6-[2-(2-ethoxyethoxy)ethoxy]pyridin-3-yl}-2-[(methylsulfonyl)oxy]propanoate

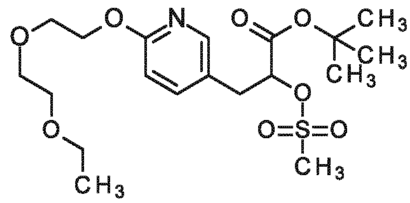

tert-butyl-3-{6-[2-(2-ethoxyethoxy)ethoxy]pyridin-3-yl}-2-hydroxypropanoate (Intermediate 11, 2.13 g, 5.99 mmol), TEA (1.8 ml, 13 mmol; [121-44-8]) and THF (20 ml) were stirred at 0-5°C under an atmosphere of $N_2$. MsCl (510 μl, 6.6 mmol; [124-63-0]) was added dropwise, the mixture was warmed to RT and stirred for 3h. The mixture was added to $NaHCO_3$ (50% sat. aq.), followed by extraction with MTBE (3x), the organic phases were combined and washed with NaCl solution (sat. aq. 2x), dried over $Na_2SO_4$ and concentrated under reduced pressure affording the title compound (2.55 g). The sample contains about 20% of an impurity and was used as such in the next steps.

LC-MS (Method 4): $R_t$ = 1.21min; MS (ESIpos): m/z = 434.2 $[M+H]^+$.

$^1H$ NMR (DMSO-$d_6$, 400 MHz): δ (ppm) 8.02 (d, *J* = 2.0 Hz, 1H), 7.65 (dd, J = 8.6, 2.5 Hz, 1H), 6.79 (d, *J* = 8.4 Hz, 1H), 5.16-5.20 (m, 1H), 4.33 (br dd, *J* = 5.3, 4.1 Hz, 2H), 3.69-3.73 (m, 2H), 3.53-3.57 (m, 2H), 3.45-3.50 (m, 2H), 3.42 (q, *J* = 7.1 Hz, 2H), 3.11 (s, 3H), 3.04-3.10 (m, 2H), 1.36 (d, *J* = 0.5 Hz, 9H), 1.09 (t, *J* = 7.0 Hz, 3H).

**Step 2**

tert-butyl-3-{6-[2-(2-ethoxyethoxy)ethoxy]pyridin-3-yl}-2-(1,4,7,10-tetraazacyclododecan-1-yl)propanoate

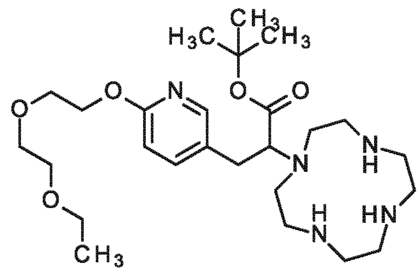

Racemic tert-butyl-3-{6-[2-(2-ethoxyethoxy)ethoxy]pyridin-3-yl}-2-[(methanesulfonyl)oxy] propanoate (1.27 g, 2.93 mmol), 1,4,7,10-tetraazacyclododecane (551 mg, 3.20 mmol), and MeCN (24 ml) were stirred overnight under $N_2$ at 55°C, followed by heating and stirring for 2d at 70°C. The mixture was concentrated under reduced pressure yielding the crude product (1.49 g).

LC-MS (Method 2): Rt = 0.65min; MS (ESIpos): m/z = 510.3 $[M+H]^+$.

**Step 3**

tert-butyl-3-{6-[2-(2-ethoxyethoxy)ethoxy]pyridin-3-yl}-2-[4,7,10-tris(2-tert-butoxy-2-oxoethyl)-1,4,7,10-tetraazacyclododecan-1-yl]propanoate

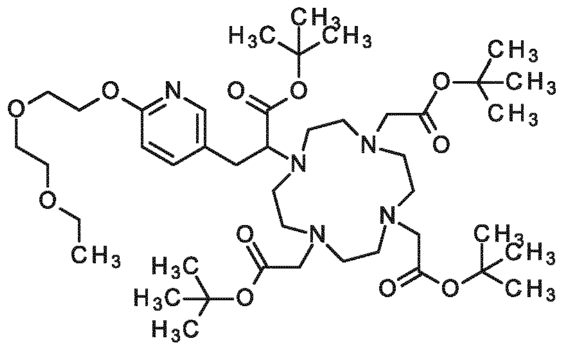

Racemic tert-butyl-3-{6-[2-(2-ethoxyethoxy)ethoxy]pyridin-3-yl}-2-(1,4,7,10-tetraazacyclodo-decan-1-yl)propanoate (1.49 g, 2.92 mmol) was stirred in MeCN (30 ml) under an atmosphere of $N_2$. DIPEA (2.5 ml, 15 mmol; [7087-68-5]) was added, followed by tert-butyl bromoacetate (1.5 ml, 10 mmol) in MeCN (500 μl). The mixture was stirred for 5h at 60°C and then concentrated under reduced pressure. EtOAc was added, the organic phase was washed with $NaHCO_3$ solution (50% sat. aq.), NaCl (sat. aq.), dried over $Na_2SO_4$, filtered and concentrated under reduced pressure yielding the crude title compound (3.50 g).

LC-MS (Method 2): $R_t$ = 1.12min; MS (ESIpos): m/z = 852.4 $[M+H]^+$.

**Step 4**

3-{6-[2-(2-ethoxyethoxy)ethoxy]pyridin-3-yl}-2-[4,7,10-tris(carboxymethyl)-1,4,7,10-tetraazacyclododecan-1-yl]propanoic acid

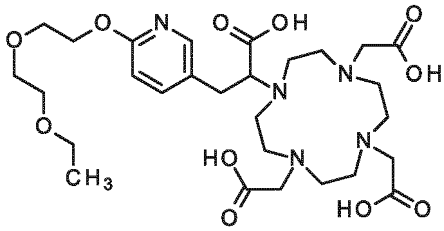

Racemic tert-butyl-3-{6-[2-(2-ethoxyethoxy)ethoxy]pyridin-3-yl}-2-[4,7,10-tris(2-tert-butoxy-2-oxoethyl)-1,4,7,10-tetraazacyclododecan-1-yl]propanoate (12.6 g, 14.8 mmol) was stirred at 70°C for 4h in formic acid (53 ml), thereafter the mixture was concentrated under reduced pressure. Purification using RP-chromatography (Biotage C18 Ultra 120g, 50 ml/min, A = water, B = MeCN, 0%B to 50%B in 25min, 100%B 10min) yielded the title compound (458 mg, 5% yield over 4 steps).

LC-MS (Method 2): Rt = 0.50min; MS (ESIpos): m/z = 628.9 $[M+H]^+$.

$^1$H NMR (DMSO-$d_6$, 400 MHz): δ (ppm) 8.04 (d, *J* = 2.0 Hz, 1H), 7.71 (dd, *J* = 8.6, 2.3 Hz, 1H), 6.72 (d, *J* = 8.4 Hz, 1H), 4.27-4.36 (m, 2H), 3.69-3.75 (m, 2H), 3.38-3.62 (m, 15H), 2.81-3.09 (m, 14H), 2.60-2.71 (m, 2H), 1.09 (t, *J* = 7.0 Hz, 3H).

$^{13}$C NMR (DMSO-$d_6$, 101 MHz): δ (ppm) 172.0, 170.9 (br), 169.6 (br, 2C), 161.8, 147.3, 140.6, 126.9, 110.0, 69.9, 69.2, 68.9, 65.6, 64.7, 64.6 (br), 55.2 (br, 3C), 51.4 (br, 2C), 51.1 (br, 2C), 49.3 (br, 2C), 46.9 (br, 2C), 30.2 (br), 15.2.

**Step 5**

gadolinium 2,2',2"-{10-[1-carboxy-2-{6-[2-(2-ethoxyethoxy)ethoxy]pyridin-3-yl}ethyl]-1,4,7,10-tetraazacyclododecane-1,4,7-triyl}triacetate

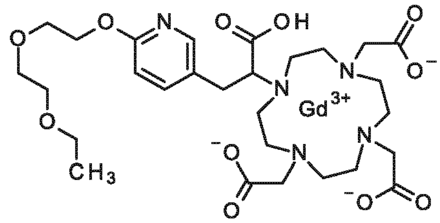

Racemic-3-{6-[2-(2-ethoxyethoxy)ethoxy]pyridin-3-yl}-2-[4,7,10-tris(carboxymethyl)-1,4,7,10-tetraazacyclododecan-1-yl]propanoic acid (458 mg, 730 μmol), $Gd_2O_3$ (119 mg, 328 μmol) and water (7.4 ml) were heated for 24h at 105°C, thereafter the mixture was heated for 5h at 120°C. Chelex100™ was added (sodium form, washed, approximately 5 g), the pH brought to 5 using NaOH (1M, aq.) and the mixture stirred for 1h showing the mixture was gadolinium free. Filtration and purification using RP-chromatography (Biotage Ultra C18 30g, 25 ml/min, A = water, B = MeCN, 0%B to 50% B in 25min, 100%B 0min) yielded the title compound (355 mg, 62 %).

LC-MS (Method 3): Rt = 0.50min; MS (ESIpos): m/z = 392.2 $[M+2H]^{++}$ ; 783.4 $[M+H]^{+}$. Isotope patterns match theoretical values for corresponding gadolinium complex.

**Example 17**

gadolinium-2,2',2"-{10-[1-carboxy-4-{4-[2-(2-ethoxyethoxy)ethoxy]phenyl}butyl]-1,4,7,10-tetraazacyclododecane-1,4,7-triyl}tris(3-hydroxypropanoate)

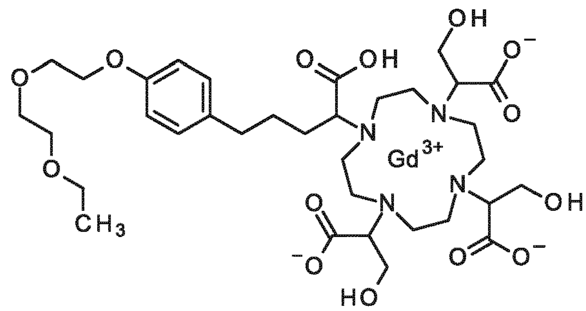

The title compound was produced as a mixture of stereoisomers from racemic ethyl-5-{4-[2-(2-ethoxyethoxy) ethoxy]phenyl}-2-hydroxypentanoate (**Intermediate 13**), and racemic ethyl - 3-tert-butoxy-2-{[(trifluoromethyl)sulfonyl] oxy}propanoate (**Intermediate 27**) using the methods described herein.

LC-MS (Method 3): $R_t$ = 0.66 - 0.74min; MS (ESIpos): m/z = 450.6 $[M+2H]^{++}$, 900.2 $[M+H]^{+}$. Isotope patterns match theoretical values for corresponding gadolinium complex.

**Example 18**

gadolinium (2S,2'S,2"S)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)

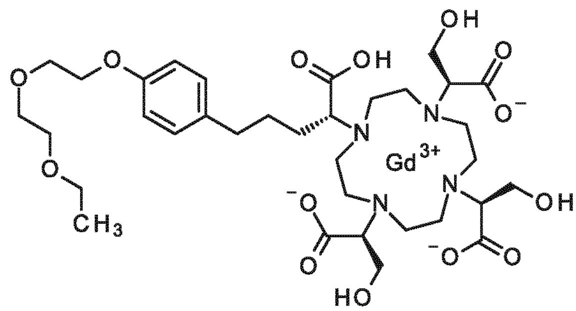

The title compound was produced from ethyl (2S)-5-{4-[2-(2-ethoxyethoxy)ethoxy]phenyl}-2-hydroxypentanoate (**Intermediate 14**) and methyl (2R)-3-tert-butoxy-2-{[(trifluoromethyl)-sulfonyl]oxy}propanoate (**Intermediate 26**).

Specific Rotation: -18.5° ± 0.1 (c=1, $H_2O$, 20°C, 589 nm).

LC-MS (Method 3): $R_t$ = 0.65min; MS (ESIpos): m/z = 450.6 $[M+2H]^{++}$ ; 900.3 $[M+H]^+$. Isotope patterns match theoretical values for corresponding gadolinium complex.

## Example 19

gadolinium (2R,2'R,2"R)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)

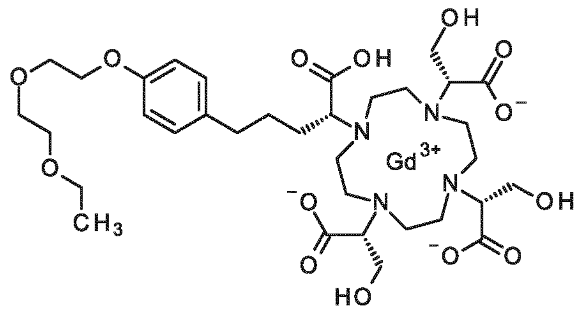

The title compound was produced using the methods detailed herein from ethyl (2S)-5-{4-[2-(2-ethoxyethoxy) ethoxy]phenyl}-2-hydroxypentanoate (**Intermediate 14**) and methyl (2S)-3-tert-butoxy-2-{[(trifluoromethyl)sulfonyl]oxy} propanoate (**Intermediate 28**).

Specific Rotation: -12.6° ± 0.14 ($H_2O$, 20°C, 589 nm).

LC-MS (Method 3): $R_t$ = 0.71min; MS (ESIpos): m/z = 450.6 $[M+2H]^{++}$ ; 900.3 $[M+H]^+$ ; 908.8 $[2M+H_2O+2H]^{++}$. Isotope patterns match theoretical values for corresponding gadolinium complex.

## Example 20

gadolinium (2R,2'R,2"R)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate)

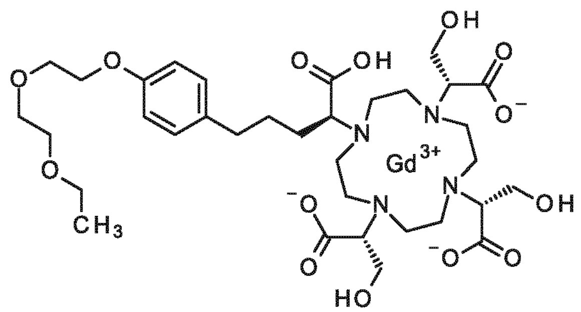

The title compound was produced using the methods detailed herein from ethyl (2R)-5-{4-[2-(2-ethoxyethoxy) ethoxy]phenyl}-2-hydroxypentanoate (**Intermediate 15**) and methyl (2S)-3-tert-butoxy-2-{[(trifluoromethyl)sulfonyl]oxy} propanoate (**Intermediate 28**).

Specific Rotation: 18.5° ± 0.1 (c=1, $H_2O$, 20°C, 589 nm).

LC-MS (Method 3): $R_t$ = 0.65min; MS (ESIpos): m/z = 450.8 $[M+2H]^{++}$ ; 900.5 $[M+H]^+$ ; 908.8 $[2M+H_2O+2H]^{++}$. Isotope patterns match theoretical values for corresponding gadolinium complex.

**Example 21**

gadolinium-2,2',2"-{10-[(1S)-1-carboxy-4-{4-[2-(2-ethoxyethoxy)ethoxy]phenyl}butyl]-1,4,7,10-tetraazacyclododecane-1,4,7-triyl}tris(3-hydroxypropanoate)

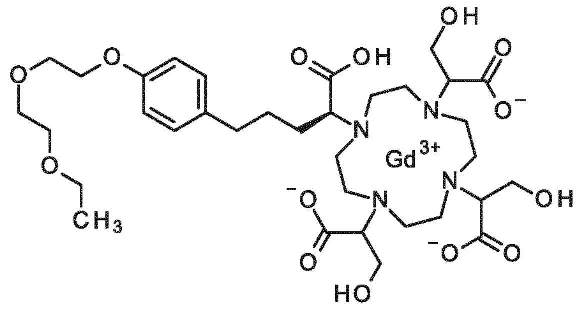

The title compound was produced using the methods detailed herein from ethyl (2R)-5-{4-[2-(2-ethoxyethoxy) ethoxy]phenyl}-2-hydroxypentanoate (**Intermediate 15**) and racemic methyl-3-tert-butoxy-2-{[(trifluoromethyl)sulfonyl] oxy}propanoate (**Intermediate 27**).

Specific Rotation: 5.97° ± 0.16 (c=1, $H_2O$, 20°C, 589 nm)

LC-MS (Method 3): Rt = 0.65 - 0.72min; MS (ESIpos): m/z = 450.8 $[M+2H]^{++}$, 900.5 $[M+H]^{+}$ . Isotope patterns match theoretical values for corresponding gadolinium complex.

**Example 22**

**Step 1**

triethyl 2,2',2"-{10-[1-tert-butoxy-3-{4-[2-(2-ethoxyethoxy)ethoxy]phenyl}-1-oxopropan-2-yl]-1,4,7,10-tetraazacyclododecane-1,4,7-triylltris(3-tert-butoxypropanoate)

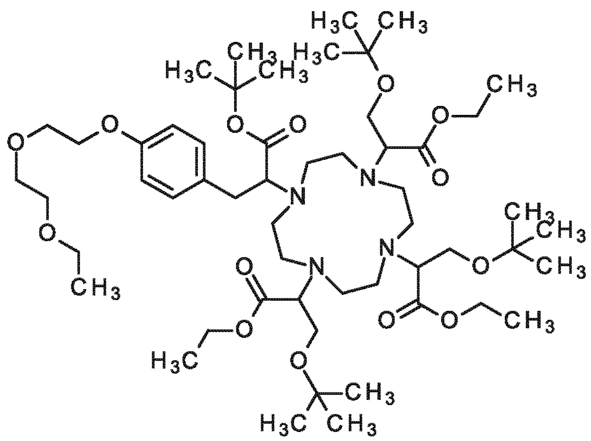

tert-butyl 3-{4-[2-(2-ethoxyethoxy)ethoxy]phenyl}-2-(1,4,7,10-tetraazacyclododecan-1-yl)-propanoate, **Example 2 - Step 2,** (1.80 g, 3.54 mmol) was dissolved in MeCN (30 ml) and $K_2CO_3$ (1.71 g, 12.4 mmol) added. A solution of ethyl 3-tert-butoxy-2-[(trifluoromethanesulfonyl)oxy]propanoate, **Intermediate 27,** (3.76 g, 11.7 mmol) in MeCN (20 ml) was added dropwise at RT and the mixture stirred for 20h. The reaction mixture was concentrated under reduced pressure. The residue was dissolved in DCM, then treated with water and extracted with DCM (2 x). The combined organic layers were washed with sat. NaCl solution, filtered using a water-repellent filter and concentrated under reduced pressure. The raw product (3.88 g) was used in the next reaction.

LC-MS (Method 2): Rt = 1.53min; MS (ESlpos): m/z = 1026.1 $[M+H]^+$.

**Step 2**

3-{ 4-[2-(2-ethoxyethoxy)ethoxy]phenyl}-2-{4, 7,1 0-tris[1-ethoxy-3-hydroxy-1-oxopropan-2-yl]-1,4,7,10-tetraazacyclododecan-1-yl}propanoic acid

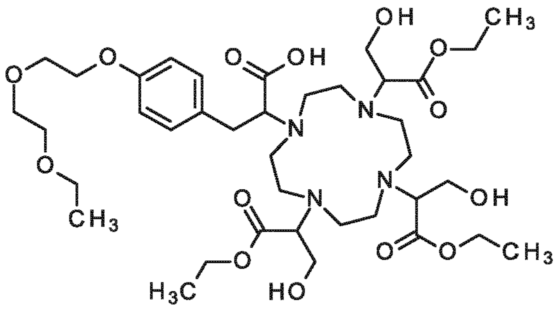

triethyl 2,2',2"-{10-[1-tert-butoxy-3-{4-[2-(2-ethoxyethoxy)ethoxy]phenyl}-1-oxopropan-2-yl]-1,4,7,10-tetraazacyclododecane-1,4,7-triyl}tris(3-tert-butoxypropanoate) (3.63 g, 3.54 mmol) was dissolved in 1,4-dioxane (34 ml), some drops of water and HCl (14 ml, 4.0 M in dioxane, 57 mmol) were added. The mixture was stirred at 80°C for 6h and was concentrated under reduced pressure yielding 2.84 g raw product as mixture of stereoisomers, which was used in the next step.

LC-MS (Method 2): $R_t$ = 0.77 - 0.85min; MS (ESlpos): m/z = 801.1 $[M+H]^+$.

**Step 3**

2,2',2"-{10-[1-carboxy-2-{4-[2-(2-ethoxyethoxy)ethoxy]phenyl}ethyl]-1,4,7,10-tetraazacyclododecane-1,4,7-triyl}tris(3-hydroxypropanoic acid)

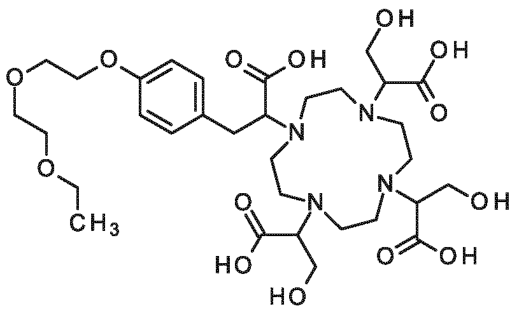

3-{4-[2-(2-ethoxyethoxy)ethoxy]phenyl}-2-{4,7,10-tris[1-ethoxy-3-hydroxy-1-oxopropan-2-yl]-1,4,7,10-tetraazacyclododecan-1-yl}propanoic acid (2.85 g, 3.56 mmol) was dissolved in THF (15 ml) and a solution of lithium hydroxide (682 mg, 28.5 mmol) in water (15 ml) added. The mixture was stirred at RT for 48h concentrated under reduced pressure. The residue extracted with MeOH/EtOH (1:3) and the extract concentrated under reduced pressure. 2.25 g raw product were used in the next step.

LC-MS (Method 2): Rt = 0.60min; MS (ESlpos): m/z = 717.7 $[M+H]^+$.

**Step 4**

gadolinium 2,2',2"-{10-[1-carboxy-2-{4-[2-(2-ethoxyethoxy)ethoxy]phenyl}ethyl]-1,4,7,10-tetraazacyclododecane-1,4,7-triyl}tris(3-hydroxypropanoate)

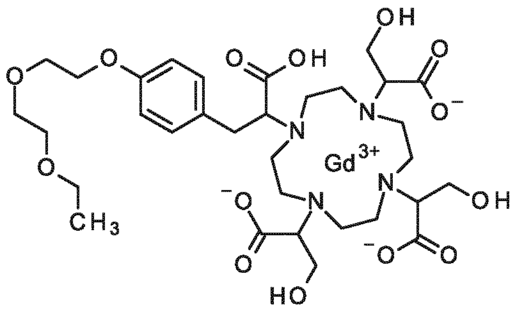

2,2',2"-{10-[1-carboxy-2-{4-[2-(2-ethoxyethoxy)ethoxy]phenyl}ethyl]-1,4,7,10-tetraazacyclododecane-1,4,7-triyl}tris(3-hydroxypropanoic acid (2.25 g, 3.14 mmol) was dissolved in water (27 ml) and $Gd_2O_3$ (512 mg, 1.41 mmol, 0.9 eq) added. The mixture was stirred at 105°C for 21h and treated with Chelex100™ (sodium form) at pH 5 for 72h. The mixture was filtered, and the filtrate loaded directly on the column (SNAP ULTRA C18 120g, 50 ml/min, 254 nm, A = water, B = MeCN, 0%B to 50%B in 10 CV, 100%B 5 CV). Interesting fractions were pooled and lyophilized to give 220 mg (3.5% from step 1) of the title compound.

LC-MS (Method 3): Rt = 0.57 - 0.60min (100% DAD); MS (ESIpos): m/z = 436 $[M + 2H]^{++}$, 872 $[M + H]^{+}$ and some $[2M + 2H]^{++}$. The observed isotope pattern conforms to structure.

## Example 23

### Step 1

tert-butyl 3-{5-[2-(2-ethoxyethoxy)ethoxy]pyridin-2-yl}-2-[(methanesulfonyl)oxy]propanoate

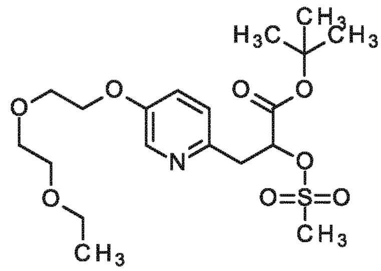

tert-butyl 3-{5-[2-(2-ethoxyethoxy)ethoxy]pyridin-2-yl}-2-hydroxypropanoate, **Intermediate 17,** (1.05 g, 2.95 mmol) was dissolved in THF (10 ml), TEA (990 μl, 7.1 mmol) added and cooled to 0°C. MsCl (270 μl, 3.5 mmol) was added and the mixture stirred at RT for 1h. The reaction mixture was poured on 0.6M $NaHCO_3$ and was extracted with EtOAc (3 x). The combined organic layers were washed with sat. NaCl, filtered using a water-repellent filter and concentrated under reduced pressure. The residue obtained (1.15 g) was without further purification.

LC-MS (Method 1): Rt = 1.08min; MS (ESIpos): m/z = 434.0 $[M+H]^+$.

$^1H$ NMR (DMSO-$d_6$, 500 MHz): δ (ppm) 8.23 (d, *J* = 2.8 Hz, 1H), 7.37 (dd, *J* = 8.5, 2.8 Hz, 1H), 7.28 (d, *J* = 8.5 Hz, 1H), 5.26 (dd, *J* = 7.6, 5.7 Hz, 1H), 4.13-4.16 (m, 2H), 3.72-3.75 (m, 2H), 3.57 (dd, *J* = 5.7, 3.8 Hz, 2H), 3.47-3.49 (m, 2H), 3.42 (q, *J* = 6.9 Hz, 2H), 3.16-3.26 (m, 2H), 3.02 (s, 3H), 1.39 (s, 9H), 1.09 (t, *J* = 7.1 Hz, 3H).

### Step 2

tert-butyl 3-{5-[2-(2-ethoxyethoxy)ethoxy]pyridin-2-yl}-2-(1,4,7,10-tetraazacyclododecan-1-yl)-propanoate

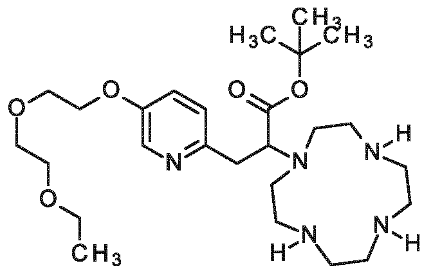

tert-butyl 3-{5-[2-(2-ethoxyethoxy)ethoxy]pyridin-2-yl}-2-[(methanesulfonyl)oxy]propanoate (1.15 g, 2.65 mmol) was dissolved in MeCN (15 ml), 1,4,7,10-tetraazacyclododecane (548 mg, 3.18 mmol) added and the mixture stirred at 70°C for 20h. The solvent was removed under reduced pressure and the raw product (1.35 g) directly used in the next step.

LC-MS (Method 2): Rt = 0.65min (63% ELSD); MS (ESIpos): m/z = 510 $[M + H]^+$.

**Step 3**

tert-butyl 3-{5-[2-(2-ethoxyethoxy)ethoxy]pyridin-2-yl}-2-[4,7,10-tris(2-tert-butoxy-2-oxoethyl)-1,4,7,10-tetraazacyclododecan-1-yl]propanoate

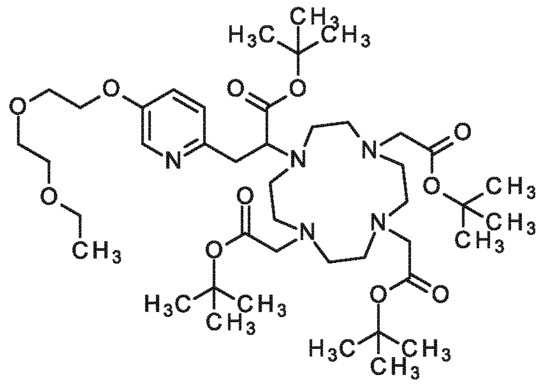

tert-butyl 3-{5-[2-(2-ethoxyethoxy)ethoxy]pyridin-2-yl}-2-(1,4,7,10-tetraazacyclododecan-1-yl)-propanoate (1.35 g, 2.65 mmol) was dissolved in MeCN (15 ml), DIPEA (2.3 ml, 13 mmol) and a solution of tert-butyl bromoacetate (1.4 ml, 9.3 mmol) in MeCN (0.70 ml) added. The mixture was stirred at 60°C for 20h after which the mixture was concentrated under reduced pressure. The residue was dissolved in EtOAc, washed with $NaHCO_3$ (0.6M aq.) and NaCl (sat. aq.). The organic phase was filtered using a water-repellent filter and concentrated under reduced pressure. Crude product 3.20 g was used without further purification.

LC-MS (Method 2): Rt = 1.20min; MS (ESIpos): m/z = 852.4 $[M+H^+]$.

**Step 4**

3-{5-[2-(2-ethoxyethoxy)ethoxy]pyridin-2-yl}-2-[4,7,10-tris(carboxymethyl)-1,4,7,10-tetraazacyclododecan-1-yl]propanoic acid

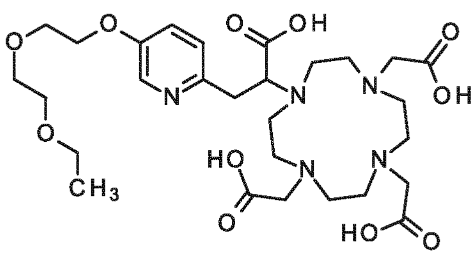

tert-butyl 3-{5-[2-(2-ethoxyethoxy)ethoxy]pyridin-2-yl}-2-[4,7,10-tris(2-tert-butoxy-2-oxoethyl)-1,4,7,10-tetraazacyclododecan-1-yl]propanoate (3.20 g, 3.76 mmol) was dissolved in formic acid (24 ml, 640 mmol) and stirred at 70°C for 20h. Th reaction mixture was concentrated under reduced pressure and the residue obtained purified by RP-chromatography. (SNAP ULTRA C18 30g, 50 ml/min, 254 nm, A = water, B = MeCN, 0% B 3CV, 0% to 50%B in 15 CV, 100%B 5 CV). Interesting fractions were combined and lyophilized to give 730 mg (39% calc. from step 1) of the title compound.

LC-MS (Method 2): Rt = 0.45min; MS (ESIpos): m/z = 628 $[M + H]^+$.

## Step 5

gadolinium 2,2',2"-{10-[1-carboxy-2-{5-[2-(2-ethoxyethoxy)ethoxy]pyridin-2-yl}ethyl]-1,4,7,10-tetraazacyclododecane-1,4,7-triyl}triacetate

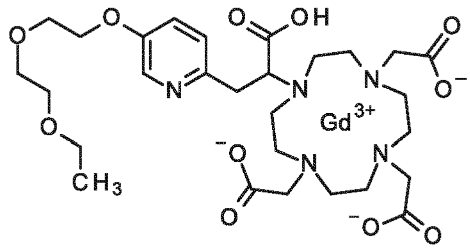

(2S)-3-{5-[2-(2-ethoxyethoxy)ethoxy]pyridin-2-yl}-2-[4,7,10-tris(carboxymethyl)-1,4,7,10-tetra-azacyclododecan-1-yl]propanoic acid (730 mg, 1.16 mmol, 0.9 eq) was dissolved in water (10 ml) and $Gd_2O_3$ (190 mg, 523 μmol) added. The mixture was stirred at 105°C for 21h. The reaction mixture was treated with Chelex100™ (sodium form) and stirred at pH 5 for 72h. The mixture was filtered and the filtrate loaded directly on a Biotage column for purification (SNAP ULTRA C18 30g, 25 ml/min, 254 nm, A = water, B = MeCN, 0% B 2CV, 0% to 50%B in 15 CV, 100%B 5 CV). The interesting fractions were combined and lyophilized to give 320 mg (35%) of the title compound.

LC-MS (Method 2): $R_t$ = 0.49min; MS (ESIpos): m/z = 782 $[M + H]^+$.

LC-MS (Method 3): $R_t$ = 0.38min (100% DAD); MS (ESIpos): m/z = 392 $[M + 2H]^+$, 522 $[2M + 3H]^{3+}$, 783 $[M + H]^+$ and some $[2M + 2H]^{++}$. The isotope pattern conforms to a gadolinium complex.

## Example 24

## Step 1

methyl 2-[(methanesulfonyl)oxy]-4-(4-propoxyphenyl)butanoate

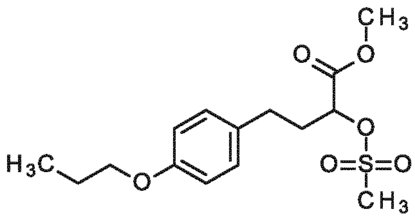

methyl 2-hydroxy-4-(4-propoxyphenyl)butanoate, (Intermediate 18, 2.00 g, 7.93 mmol) was dissolved in THF, TEA (2.7 ml, 19 mmol) added and cooled to 0°C. MsCl (1.09 g, 9.51 mmol; [124-63-0]) was added dropwise and the mixture stirred at RT overnight. $NaHCO_3$ (0.6 M aq.) was added and the mixture extracted with MTBE. The combined organic phases were dried ($Na_2SO_4$) and concentrated under reduced pressure. The title compound 2.48 g was used without further purification.

LC-MS (Method 4): Rt = 1.28min; MS (ESIpos): m/z = 331 $[M+H]^+$.

$^1H$ NMR (DMSO-$d_6$, 400 MHz): δ (ppm) 7.09-7.15 (m, 2H), 6.81-6.87 (m, 2H), 5.08 (dd, *J* = 7.6, 4.6 Hz, 1H), 3.88 (t, *J* = 6.6 Hz, 2H), 3.70 (s, 3H), 3.27 (s, 3H), 2.58-2.65 (m, 2H), 2.00-2.15 (m, 2H), 1.70 (sxt, *J* = 7.0 Hz, 2H), 0.96 (t, *J* = 7.5

Hz, 3H).

**Step 2**

methyl 4-(4-propoxyphenyl)-2-(1,4,7,10-tetraazacyclododecan-1-yl)butanoate

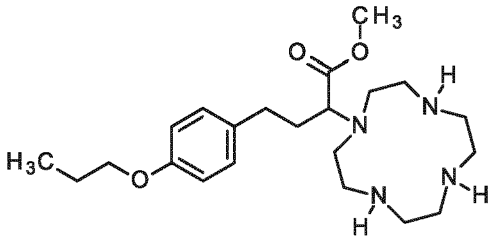

1,4,7,10-tetraazacyclododecane (1.61 g, 9.37 mmol) was dissolved in MeCN (30 ml) and $K_2CO_3$ (1.04 g, 7.50 mmol) added. A solution of methyl 2-[(methanesulfonyl)oxy]-4-(4-propoxyphenyl)butanoate (2.48 g, 7.50 mmol) in MeCN (9.3 ml) was added dropwise and the mixture stirred at 55°C overnight. The mixture was filtrated, washed with EtOH and the combined filtrated concentrated under reduced pressure. 3.10 g (96% calc. from step 1) of the title compound were obtained, which was used as such in the next step.
LC-MS (Method 2): $R_t$ = 0.77min; MS (ESIpos): m/z = 407 $[M+H]^+$.
$^1H$ NMR (DMSO-$d_6$, 400 MHz): δ (ppm) 7.08-7.14 (m, 2H), 6.80-6.85 (m, 2H), 3.87 (t, *J* = 6.6 Hz, 2H), 3.61 (s, 3H), 2.51-2.81 (m, 12H), 2.40-2.49 (m, 3H), 2.28-2.37 (m, 4H), 1.65-1.91 (m, 4H), 0.96 (t, *J* = 7.4 Hz, 3H).

**Step 3**

methyl 4-(4-propoxyphenyl)-2-[4,7,10-tris(2-tert-butoxy-2-oxoethyl)-1,4,7,10-tetraazacyclododecan-1-yl]butanoate

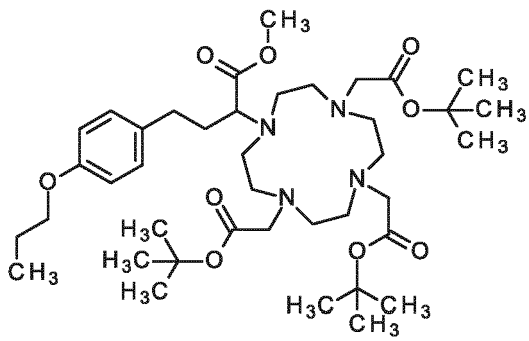

Methyl 4-(4-propoxyphenyl)-2-(1,4,7,10-tetraazacyclododecan-1-yl)butanoate (1.55 g, 3.81 mmol) was dissolved in MeCN (15 ml), placed under $N_2$ and $K_2CO_3$ (4.48 g, 32.4 mmol) added. A solution of tert-butyl bromoacetate (2.0 ml, 13 mmol; [5292-43-3]) in MeCN (10 ml) was added dropwise and the mixture stirred at RT overnight. The mixture was filtered, the filter cake washed with EtOH and the combined filtrates concentrated under reduced pressure. The raw material (2.85 g) was used without further purification.
LC-MS (Method 2): Rt = 1.24min; MS (ESIpos): m/z = 750.3 $[M+H]^+$.

**Step 4**

2,2',2"-{10-[1-methoxy-1-oxo-4-(4-propoxyphenyl)butan-2-yl]-1,4,7,10-tetraazacyclododecane-1,4,7-triyl}triacetic acid

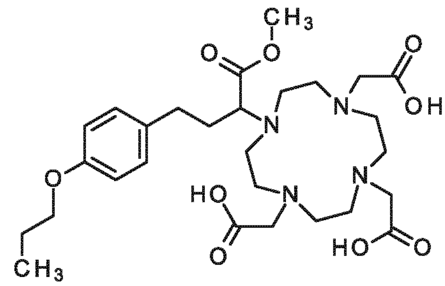

methyl 4-(4-propoxyphenyl)-2-[4,7,10-tris(2-tert-butoxy-2-oxoethyl)-1,4,7,10-tetraazacyclododecan-1-yl]butanoate (2.85 g, 3.80 mmol) was dissolved in formic acid (50 ml) and heated to 70°C overnight. The mixture was concentrated under reduced pressure and the residue obtained was columned on C18 Biotage using MeCN and Water (0 - 100 %). Two fractions of the title compound were obtained:

Fraction 1: 620 mg (1.07 mmol, 28% calc. from step 3)

LC-MS (Method 2): Rt = 0.72min (100%, ELSD); MS (ESIpos): m/z = 582 $[M+H]^+$.

$^1H$ NMR (DMSO-$d_6$, 400 MHz): δ (ppm) 7.07-7.13 (m, *J* = 8.6 Hz, 2H), 6.77-6.83 (m, *J* = 8.6 Hz, 2H), 3.86 (t, *J* = 6.5 Hz, 2H), 3.62 (s, 3H), 3.34-3.52 (m, 6H), 2.76-3.11 (m, 13H), 2.40-2.61 (m, 6H), 1.78-1.99 (m, 2H), 1.70 (sxt, *J* = 7.0 Hz, 2H), 0.96 (t, *J* = 7.4 Hz, 3H).

$^{13}C$ NMR (DMSO-$d_6$, 101 MHz): δ (ppm) 172.4, 170.8 (br), 170.0 (br, 2C), 156.9, 133.0, 129.3, 114.3, 68.8, 62.3 (br), 55.4 (br), 54.9 (br, 2C), 51.6 (br, 2C), 51.3, 51.2 (br, 2C), 49.0 (br, 2C), 46.7 (br, 2C), 31.2, 30.3, 22.1, 10.5.

Fraction 2: 500 mg (0.86 mmol, 23% calc. from step 3)

LC-MS (Method 2): $R_t$ = 0.71min (94%, ELSD); MS (ESIpos): m/z = 582 $[M+H]^+$.

**Step 5**

gadolinium 2,2',2"-{10-[1-carboxy-3-(4-propoxyphenyl)propyl]-1,4,7,10-tetraazacyclododecane-1,4,7-triyl}triacetate

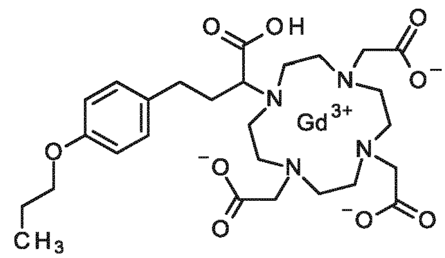

2,2',2"-{10-[1-methoxy-1-oxo-4-(4-propoxyphenyl)butan-2-yl]-1,4,7,10-tetraazacyclododecane-1,4,7-triyl}triacetic acid (620 mg, 1.07 mmol) was dissolved in water (10.9 ml) and $Gd_2O_3$ (174 mg, 0.48 mmol, 0.9 eq.) added. The mixture was stirred at 100°C for 2d. Chelex100™ (sodium form, ca. 5 g) was added and the mixture stirred for 1h at RT and pH 5. The mixture was filtered, and the filtrate concentrated under reduced pressure. Fraction 2 from the previous step was treated in the same manner and the raw product obtained was found to be identical. Both raw products were combined and purified by RP-chromatography (SNAP C18 60g, 50 ml/min, A = water, B = MeCN, 0% B 5min, 0%B to 50%B in 25min, 100% B 10min). Interesting fractions were combined and lyophilized to give 1.01 g (1.04 mmol, 66%) of the title compound.

LC-MS (Method 3): $R_t$ = 0.71min (100% DAD); MS (ESIpos): m/z = 361 $[M + 2H]^{++}$, 722 $[M + H]^+$ and some $[2M + 2H]^{++}$, 731 $[2M + H_2O + 2H]^{++}$. The isotope pattern conforms to a gadolinium complex.

**Example 25**

**Step 1**

ethyl 5-{4-[2-(2-ethoxyethoxy)ethoxy]phenyl}-2-[(methanesulfonyl)oxy]pentanoate

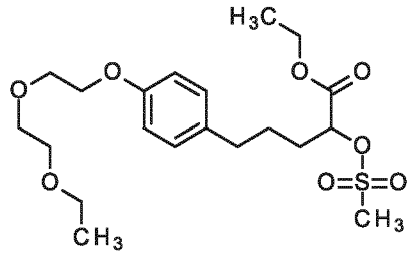

ethyl 5-{4-[2-(2-ethoxyethoxy)ethoxy]phenyl}-2-hydroxypentanoate, Intermediate 13, (700 mg, 1.97 mmol) was dissolved in THF (15 ml), TEA (660 µl, 4.7 mmol) added and cooled to 0°C. Dropwise addition of MsCl (180 µl, 2.4 mmol) was followed by stirring at RT overnight. The reaction mixture was poured on $NaHCO_3$ (0.6 M, aq.) and extracted with EtOAc (3 x). The combined organic phases were washed with NaCl (sat. aq.), dried over $Na_2SO_4$, filtered using a water-repellent filter and concentrated under reduced pressure. The obtained title compound, 790 mg was used without further purification.
LC-MS (Method 4): $R_t$ = 1.25min; MS (ESIpos): m/z = 433 $[M+H]^+$.
$^1H$ NMR (DMSO-$d_6$, 400 MHz): δ (ppm) 7.09 (d, *J* = 8.6 Hz, 2H), 6.85 (d, *J* = 7.2 Hz, 2H), 5.09 (dd, *J* = 7.5, 4.7 Hz, 1H), 4.12-4.21 (m, 2H), 4.04 (dd, *J* = 5.4, 3.9 Hz, 2H), 3.71 (dd, *J* = 5.4, 3.9 Hz, 2H), 3.54-3.59 (m, 2H), 3.46-3.50 (m, 2H), 3.42 (q, *J* = 7.0 Hz, 2H), 3.24 (s, 3H), 2.51-2.64 (m, 2H), 1.70-1.85 (m, 2H), 1.53-1.70 (m, 2H), 1.19 (t, *J* = 7.2 Hz, 3H), 1.09 (t, *J* = 7.0 Hz, 3H).

**Step 2**

ethyl 5-{4-[2-(2-ethoxyethoxy)ethoxy]phenyl}-2-(1,4,7,10-tetraazacyclododecan-1-yl)-pentanoate

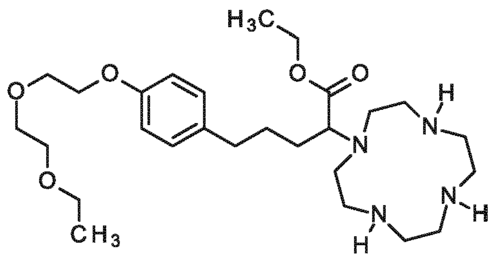

ethyl 5-{4-[2-(2-ethoxyethoxy)ethoxy]phenyl}-2-[(methanesulfonyl)oxy]pentanoate (790 mg, 1.83 mmol) was dissolved in MeCN (30 ml), placed under $N_2$ and 1,4,7,10-tetraazacyclododecane (535 mg, 3.10 mmol) was added. The mixture was stirred at 55°C overnight. The solvent was removed under reduced pressure, the residue dissolved in DCM (100 ml) and stirred with NaOH solution (1N, aq. 60ml) for 15min. The layers were separated, the organic layer washed with water (2 x 50 ml) and sat. NaCl (1 x 50 ml), dried over $Na_2SO_4$ and concentrated under reduced pressure. 829 mg of the title compound were obtained.
LC-MS (Method 2): Rt = 0.70min; MS (ESIpos): m/z = 510 $[M+H]^+$.
$^1H$ NMR ($CDCl_3$, 400 MHz): δ (ppm) 7.06-7.10 (m, 2H), 6.79-6.84 (m, 2H), 4.08-4.19 (m, 4H), 3.85 (dd, *J* = 5.4, 4.4 Hz, 2H), 3.70-3.74 (m, 2H), 3.60-3.63 (m, 2H), 3.54 (q, *J* = 7.0 Hz, 2H), 3.32-3.40 (m, 1H), 2.39-2.98 (m, 18H), 1.60-1.84 (m, 4H), 1.26 (t, *J* = 7.1 Hz, 3H), 1.21 (t, *J* = 7.1 Hz, 3H).

**Step 3**

ethyl 5-{4-[2-(2-ethoxyethoxy)ethoxy]phenyl}-2-[4,7,10-tris(2-tert-butoxy-2-oxoethyl)-1,4,7,10-tetraazacyclododecan-1-yl]pentanoate

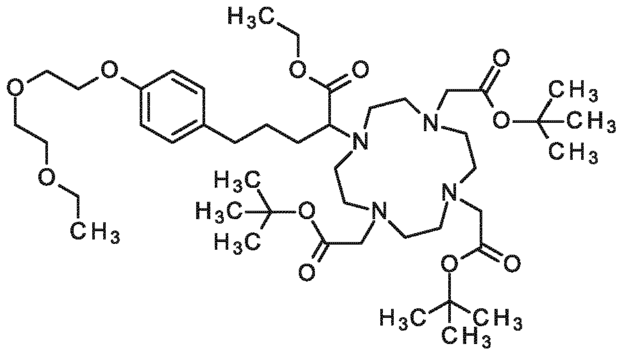

ethyl 5-{4-[2-(2-ethoxyethoxy)ethoxy]phenyl}-2-(1,4,7,10-tetraazacyclododecan-1-yl)-pentanoate (829 mg, 1.63 mmol) was dissolved in MeCN (9.2 ml) and DIPEA (1.4 ml, 8.2 mmol) was added, followed by tert-butyl bromoacetate (600 µl, 4.1 mmol). The mixture was stirred at 60°C for 20h. The reaction mixture was concentrated under reduced pressure. The residue was dissolved in EtOAc, washed with $NaHCO_3$ (0.6M, aq.) solution and NaCl (sat. aq.). The organic phase was dried over $Na_2SO_4$ and concentrated under reduced pressure. 1.40 g (>100%) of the title compound was obtained, which was used without further purification.

LC-MS (Method 2): Rt = 1.25min; MS (ESIpos): m/z = 852.0 $[M+H]^+$.

**Step 4**

2,2',2''-{10-[1-ethoxy-5-{4-[2-(2-ethoxyethoxy)ethoxy]phenyl}-1-oxopentan-2-yl]-1,4,7,10-tetraazacyclododecane-1,4,7-triyl}triacetic acid

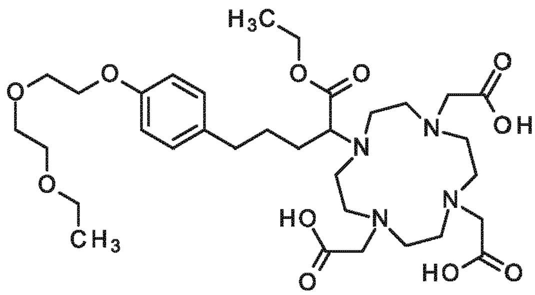

ethyl 5-{4-[2-(2-ethoxyethoxy)ethoxy]phenyl}-2-[4,7,10-tris(2-tert-butoxy-2-oxoethyl)-1,4,7,10-tetraazacyclododecan-1-yl]pentanoate (1.40 g, 1.64 mmol) was dissolved in formic acid (11 ml) and stirred at 70°C for 20h. The solvent was removed under reduced pressure and the residue purified by RP-chromatography (SNAP Ultra C18 30g, 25 ml/min, 254 nm, A = water, B = MeCN, 0%B to 55%B in 10 CV, 100%B5 CV). Interesting fractions were combined and lyophilized to give 490 mg (0.72 mmol, 37% calc. from step 1) of the title compound.

LC-MS (Method 2): Rt = 0.74min; MS (ESIpos): m/z = 683 $[M + H]^+$.

$^1H$ NMR (DMSO-$d_6$, 400 MHz): δ (ppm) 7.08 (d, *J* = 7.5 Hz, 2H), 6.80-6.87 (m, 2H), 4.02-4.10 (m, 4H), 3.69-3.73 (m), 3.55-3.59 (m), 3.34-3.51 (m), 2.76-3.11 (m, 16H), 2.40-2.55 (m, 2H), 1.34-1.72 (m, 4H), 1.17 (t, *J* = 7.1 Hz, 3H), 1.09 (t, *J* = 7.1 Hz, 3H). A very strong water signal prevents the integration between 3-5 ppm.

**Step 5**

gadolinium 2,2',2"-{10-[1-carboxy-4-{4-[2-(2-ethoxyethoxy)ethoxy]phenyl}butyl]-1,4,7,10-tetraazacyclododecane-1,4,7-triyl}triacetate

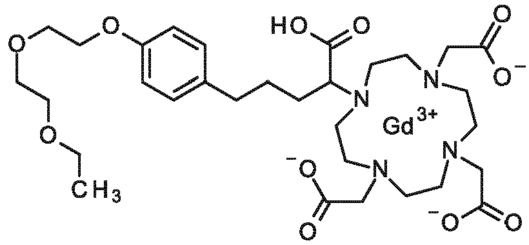

2,2',2"-{10-[1-ethoxy-5-{4-[2-(2-ethoxyethoxy)ethoxy]phenyl}-1-oxopentan-2-yl]-1,4,7,10-tetraazacyclododecane-1,4,7-triyl}triacetic acid (490 mg, 718 µmol) was dissolved in water (6.3 ml) and $Gd_2O_3$ (117 mg, 323 µmol, 0.90 eq) added. After heating at 100°C for 48h, the mixture was treated at RT with Chelex100™ (sodium form, ca. 5 g) at pH 5 for 72h, after which the xylenol-orange test indicated the absence of free gadolinium. The mixture (including the resin) was loaded into an empty Biotage-cartridge and subjected to RP-Chromatography (SNAP Ultra C18 30g, 25 ml/min, 254 nm, A = water, B = MeCN, 0%B to 55%B in 10 CV, 100%B5 CV). Interesting fractions were combined and lyophilized to give 430 mg (74%) of the title compound.
LC-MS (Method 2): Rt = 0.72min; MS (ESIpos): m/z = 809 $[M + H]^+$.
LC-MS (Method 3): Rt = 0.71min (100% DAD); MS (ESIpos): m/z = 808 $[M + H]^+$. The isotope pattern conforms to a gadolinium complex.

**Example 26**

**Step 1**

methyl (2R)-5-{4-[2-(2-ethoxyethoxy)ethoxy]phenyl}-2-[(methanesulfonyl)oxy]pentanoate

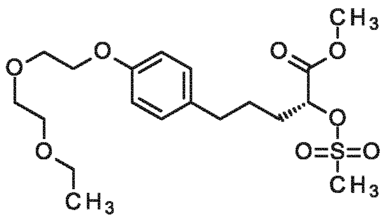

A mixture of methyl (2R)-5-{4-[2-(2-ethoxyethoxy)ethoxy]phenyl}-2-hydroxypentanoate, **Intermediate 12,** (2.50 g, 7.34 mmol) and TEA (2.3 mL, 16 mmol; [121-44-8]) in THF (25 mL) was cooled to 0°C and dropwise treated with MsCl (630 µL, 8.1 mmol; [124-63-0]). The reaction mixture was stirred at 0°C for 30min and then gradually warmed to RT under $N_2$ with stirring overnight. The mixture was added to aqueous $NaHCO_3$ solution and the aqueous layer was extracted with MTBE (3x). The combined organic layers were washed with sat. NaCl, dried with $Na_2SO_4$, filtered and concentrated under reduced pressure. The obtained material was subjected to Biotage Isolera™ chromatography (SNAP Si - 25 g, eluting with hexane-EtOAc 1:0 to 4:6) to give the title compound (1.7 g, 53%).
Specific rotation: $[\alpha]_D^{20}$ = 16.2° +/- 0.20° (c = 1, $CHCl_3$).
LC-MS (Method 6): Rt = 1.18min; MS (ESIpos): m/z = 436 $[M+NH_4]^+$.
$^1H$ NMR (400 MHz, DMSO-$d_6$) δ ppm 1.09 (t, 3H), 1.56 - 1.68 (m, 2H), 1.70 - 1.86 (m, 2H), 2.53 - 2.60 (m, 2H), 3.24 (s, 3H), 3.43 (q, 2H), 3.47 - 3.50 (m, 2H), 3.56 - 3.58 (m, 2H), 3.70 - 3.73 (m, 5H), 4.03 - 4.05 (m, 2H), 5.10 - 5.13 (m, 1H), 6.83 - 6.87 (m, 2H), 7.07 - 7.11 (m, 2H).

**Step 2**

methyl (2S)-5-{4-[2-(2-ethoxyethoxy)ethoxy]phenyl}-2-(1,4,7,10-tetraazacyclododecan-1-yl)pentanoate

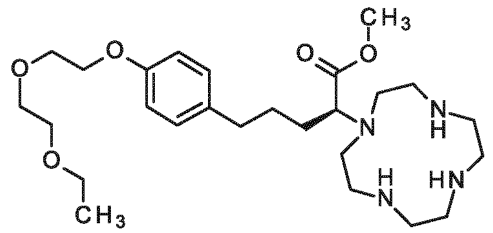

A solution of 1,4,7,10-tetraazacyclododecane (1.00g, 5.80mmol) in MeCN (6mL) was treated at RT with a solution of methyl (2R)-5-{4-[2-(2-ethoxyethoxy) ethoxy]phenyl}-2-[(methanesulfonyl)oxy]pentanoate (1.2 g, 2.9 mmol) in MeCN (5 mL). The reaction mixture was stirred at 70°C for 7h and subsequently at RT overnight. The solid material was filtered off, washed with MeCN and the filtrate concentrated under reduced pressure. The obtained residue was taken up with EtOAc, washed with NaOH (0.1 M aq.) and NaCl (sat. aq.), dried with $Na_2SO_4$, filtered and concentrated under reduced pressure to give the crude title compound (1.19 g, 37%) which was used in the next step without further purification.

Specific rotation: $[\alpha]_D^{20}$ = -30.2° +/- 0.15° (c = 1, $CHCl_3$).

LC-MS (Method 2): $R_t$ = 0.61min; MS (ESIpos): m/z = 495 $[M+H]^+$.

$^1$H NMR (400 MHz, DMSO-$d_6$) δ ppm 1.09 (t, 3H), 1.50 - 1.76 (m, 4H), 2.27 - 2.43 (m, 7H), 2.54 - 2.67 (m, 9H), 2.72 - 2.77 (m, 2H), 3.38 - 3.45 (m, 3H), 3.47 - 3.50 (m, 2H), 3.56 - 3.60 (m, 5H), 3.70 - 3.72 (m, 2H), 4.02 - 4.05 (m, 2H), 6.82 - 6.86 (m, 2H), 7.08 - 7.12 (m, 2H).

**Step 3**

methyl (2S)-5-{4-[2-(2-ethoxyethoxy)ethoxy]phenyl}-2-[4,7,10-tris(2-tert-butoxy-2-oxoethyl)-1,4,7,10-tetraazacyclododecan-1-yl]pentanoate

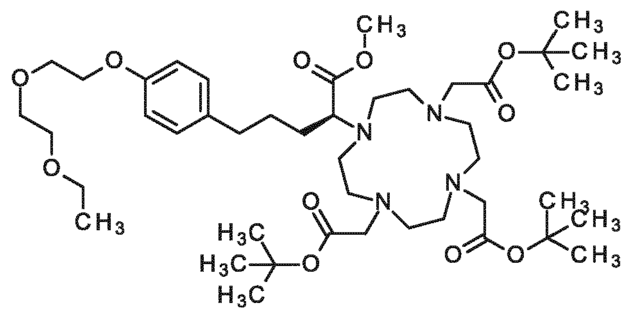

A solution of methyl (2S)-5-{4-[2-(2-ethoxyethoxy)ethoxy]phenyl}-2-(1,4,7,10-tetraazacyclododecan-1-yl)pentanoate (250 mg, 455 μmol) in MeCN (3 mL) was treated at RT with tert-butyl bromoacetate (230 μL, 1.5 mmol; [5292-43-3]) and $K_2CO_3$ (220 mg, 1.6 mmol; [584-08-7]) and stirred at RT overnight. Due to incomplete conversion, additional tert-butyl bromoacetate (0.7 eq., 50 μL, 0.3 mmol) was added, and the mixture stirred overnight at RT. The solid material was filtered off, washed with MeCN and the filtrate concentrated under reduced pressure to give the crude title compound (356 mg) which was used in the next step without further purification.

LC-MS (Method 3): Rt = 1.08min; MS (ESIpos): m/z = 837.6 $[M+H]^+$, 419.3 $[M+2H]^{2+}$.

Specific rotation: $[\alpha]_D^{20}$ = -7.3° +/- 0.48° (c = 1, $CHCl_3$).

$^1$H NMR (DMSO-$d_6$, 400 MHz): δ (ppm) 7.02-7.12 (m, 2H), 6.79-6.87 (m, 2H), 4.02-4.05 (m, 2H), 3.69-3.75 (m, 2H), 3.52-3.65 (m, 5H), 3.46-3.50 (m, 2H), 3.42 (q, *J* = 7.1 Hz, 2H), 3.03-3.30 (m, 7H), 2.55-2.81 (m, 10H), 1.49-1.73 (m, 4H), 1.38-1.44 (m, 28H), 1.09 (t, *J* = 7.0 Hz, 3H). 4 cyclen-H are hidden under the strong solvent signal.

**Step 4**

(2S)-5-{4-[2-(2-ethoxyethoxy)ethoxy]phenyl}-2-[4,7,10-tris(2-tert-butoxy-2-oxoethyl)-1,4,7,10-tetraazacyclododecan-1-yl]pentanoic acid

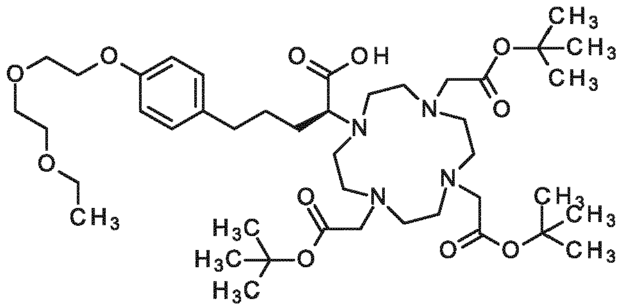

A solution of crude methyl (2S)-5-{4-[2-(2-ethoxyethoxy)ethoxy]phenyl}-2-[4,7,10-tris(2-tert-butoxy-2-oxoethyl)-1,4,7,10-tetraazacyclododecan-1-yl]pentanoate (350 mg, 420 μmol) in THF (3 mL) was treated with 2 M aqueous NaOH (2 mL, 4 mmol) at RT for 4d . The reaction mixture was adjusted to pH 7 by addition of HCl (2 M aq.) and extracted with EtOAc (3x). The combined organic layers were dried with $Na_2SO_4$, filtered and concentrated under reduced pressure to give the crude title compound (55 mg) which was used in the next step without further purification.

LC-MS (Method 3): Rt = 1.04min; MS (ESIpos): m/z = 823.6 $[M+H]^+$, 412.3 $[M+2H]^{2+}$.

**Step 5**

(2S)-5-{4-[2-(2-ethoxyethoxy)ethoxy]phenyl}-2-[4,7,10-tris(carboxymethyl)-1,4,7,10-tetraazacyclododecan-1-yl]pentanoic acid

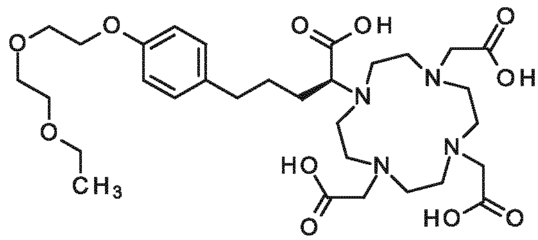

A solution of crude (2S)-5-{4-[2-(2-ethoxyethoxy)ethoxy]phenyl}-2-[4,7,10-tris(2-tert-butoxy-2-oxoethyl)-1,4,7,10-tetraazacyclododecan-1-yl]pentanoic acid (55 mg, 67 μmol) in formic acid (740 μL, 20 mmol; [64-18-6]) was stirred at 55°C for 3h followed by RT overnight. The reaction mixture was concentrated under reduced pressure and the obtained residue taken up with water. The aqueous layer was washed with MTBE and concentrated under reduced pressure to give the crude title compound (44 mg) which was used in the next step without further purification.

LC-MS (Method 2): $R_t$ = 0.69min; MS (ESIpos): m/z = 655 $[M+H]^+$.

**Step 6**

gadolinium 2,2',2"-{10-[(1S)-1-carboxy-4-{4-[2-(2-ethoxyethoxy)ethoxy]phenyl}butyl]-1,4,7,10-tetraazacyclododecane-1,4,7-triyl}triacetate

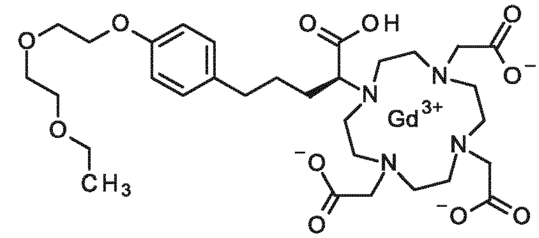

A solution of crude (2S)-5-{4-[2-(2-ethoxyethoxy)ethoxy]phenyl}-2-[4,7,10-tris(carboxymethyl)-1,4,7,10-tetraazacyclododecan-1-yl]pentanoic acid (44 mg, 67 μmol) in water (5 mL) was treated with $Gd_2O_3$ (0.49 eq., 12 mg, 33 μmol; [12064-62-9]) and stirred at 105°C overnight. The reaction mixture was cooled to RT, treated with an excess of Chelex100™ (sodium form, washed) chelating resin (pH was adjusted by addition of formic acid to 4-5), the resin filtered off and the filtrate purified by preparative HPLC to give the title compound (3.4 mg, 0.6% yield over 5 steps).

LC-MS (Method 3): $R_t$ = 0.73min; MS (ESIpos): ): m/z = 810.4 $[M+H]^+$, m/2z = 405.7 $[M+H]^{2+}$.

## Example 27

### Step 1

methyl (2R)-2-hydroxyhex-5-enoate

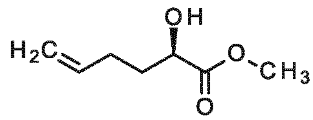

Copper(I)bromide dimethyl sulfide (16.2 g, 78.0 mmol; [54678-23-8]) together with THF (150 ml) were cooled to -70°C under $N_2$. Allyl magnesiumchloride (74 ml, 2.0 M in THF, 150 mmol; [2622-05-1]) was added dropwise and the mixture stirred at -70°C for 30min after the end of the addition. A solution of methyl (2R)-oxirane-2-carboxylate (17.2 g, 164 mmol; [111058-32-3]) in THF (150 ml) was added dropwise at -70°C. After the addition the mixture was stirred for 30min and then allowed to warm to -5°C. The reaction was quenched at this temperature by addition of sat. ammonium chloride and the mixture extracted with EtOAc. The combined organic phases were washed with water (2 x), dried over $Na_2SO_4$ and carefully concentrated under reduced pressure (*low boiling point of the product!*). The raw product (18.1 g) was combined with the raw product of a previous experiment (4.26 g from 7.0 g glycidate) and distilled at 4.1 to 4.4 mbar.

| | | |
|---|---|---|
| Fraction 1: | 90 - 100°C | 2.04 g (26% product, 74 % methyl glycidate by H-NMR) |
| Fraction 2: | 100 - 105°C | 9.30 g (74% product, 8% methyl glycidate, 18% unknown impurity) |
| Fraction 3: | 110 - 130°C | 1.88 g (50% Product, 50% unknown impurity) |

A sample from a small-scale experiment was purified by chromatography (Interchim 120 g, A = DCM, B = Ethyl acetate, 0% B, 2.5 CV, 0% to 20% B in 12.5 CV).

$^1H$ NMR ($CDCl_3$, 400MHz): δ (ppm) 5.81 (ddt, J=17.0, 10.3, 6.6 Hz, 1H), 5.06 (dq, J=17.1, 1.7 Hz, 1H), 4.99 (ddt, J=10.3, 2.0, 1.0 Hz, 1H), 4.20 (ddd, J=7.9, 5.6, 4.1 Hz, 1H), 3.77-3.80 (m, 3H), 2.78 (d, J=5.8 Hz, 1H), 2.11-2.27 (m, 2H), 1.89 (dddd, J=13.7, 8.9, 7.3, 4.1 Hz, 1H), 1.73 (dddd, J=13.7, 9.0, 7.8, 5.8 Hz, 1H).

$^{13}C$ NMR ($CDCl_3$, 101MHz): δ (ppm) 175.7, 137.4, 115.5, 69.8, 52.5, 33.5, 28.9.

The main fraction was used as such in the next step.

### Step 2

methyl (2R)-2-[(4-nitrobenzene-1-sulfonyl)oxy]hex-5-enoate

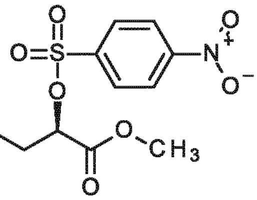

methyl (2R)-2-hydroxyhex-5-enoate (9.10 g, 63.1 mmol) was dissolved in dry toluene (120 ml), cooled to 0°C, TEA (18 ml, 130 mmol) and 4-nitrobenzene-1-sulfonyl chloride (15.4 g, 69.4 mmol) were added at this temperature. The reaction was allowed to warm to RT and stirred overnight. The solvent was removed under reduced pressure and the residue treated with EtOAc. The white precipitate formed (triethylammonium 4-nitrobenzenesulfonate) was filtered off and the filtrate extracted with water. The aqueous phase was back extracted with EtOAc and the combined organic phases washed with NaCl (sat.aq.), dried ($Na_2SO_4$) and concentrated under reduced pressure. The raw product (19.32 g) was absorbed on Isolute™ and subjected to chromatography (SNAP KP-Sil 340 g, 100 ml/min, 252 nm, A = n-hexane, B = EtOAc, 0%B 3 CV, 0% to 10% B in 9.8 CV, 10%B 1.4 CV, 10% to 19% B in 9.2 CV). Interesting fractions were combined to give the title compound 11.86 g (57%).

$^1H$ NMR ($CDCl_3$, 400MHz): δ (ppm) 8.37-8.42 (m, 2H), 8.12-8.18 (m, 2H), 5.71 (ddt, J=17.2, 10.9, 6.6 Hz, 1H), 4.99-5.06 (m, 3H), 3.67-3.70 (m, 3H), 1.92-2.22 (m, 3H).

$^{13}C$ NMR ($CDCl_3$, 101MHz): δ (ppm) 168.7, 150.8, 142.1, 135.5, 129.4 (2C), 124.2 (2C), 116.8, 77.9, 52.8, 31.1, 28.5.

**Step 3**

methyl (2S)-2-(1,4,7,10-tetraazacyclododecan-1-yl)hex-5-enoate

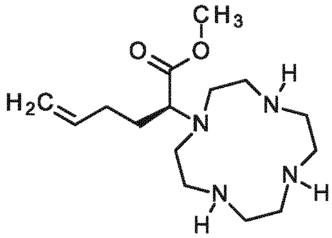

1,4,7,10-tetraazacyclododecane (2.62 g, 15.2 mmol) was dissolved in MeCN (25 ml), $K_2CO_3$ (1.05 g, 7.59 mmol) and a solution of methyl (2R)-2-[(4-nitrobenzene-1-sulfonyl)oxy]hex-5-enoate (2.50 g, 7.59 mmol) in MeCN (5 ml) added. After stirring at RT for 5h, the mixture was filtered, and the residue washed with MeCN. The combined filtrates were concentrated under reduced pressure to give 3.23 g of the title compound as raw product, which was used in the next step.

**Step 4**

methyl (2S)-2-[4,7,10-tris(2-tert-butoxy-2-oxoethyl)-1,4,7,10-tetraazacyclododecan-1-yl]hex-5-enoate

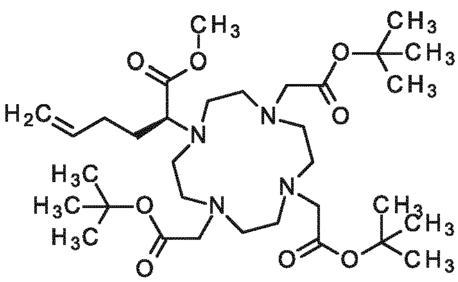

methyl (2S)-2-(1,4,7,10-tetraazacyclododecan-1-yl)hex-5-enoate (3.00 g, 10.1 mmol) was dissolved in MeCN

(150 ml), $K_2CO_3$ (4.86 g, 35.2 mmol) and tert-butyl bromoacetate (5.0 ml, 33 mmol) added dropwise and the mixture stirred at RT overnight. At this stage it was combined with the material from pilot experiment (0.23 g). The solids were filtered off and the filtrate concentrated under reduced pressure. The residue was partitioned between MTBE and water. The organic phase was washed with water, NaOH (1M, aq.) and water (2x) and then dried over $Na_2SO_4$. After removal of the solvent under reduced pressure, the residue (8.17 g) was absorbed on Isolute™ and subjected to chromatography (SNAP Ultra 100 g, 65 ml/min, 254 nm, A = Ethyl acetate, B = DCM, 0%B 2.7CV, 0% to 100%B in 15CV, 100%B 3CV). The interesting fractions were pooled and evaporated to give 2.25 g (3.34 mmol, 44% calc. from step 3) of the title compound.

Optical Rotation: $_{\alpha D}^{20}$ = -21.9° +/- 0.18° (c=1, $CHCl_3$).

LC-MS (Method 2): Rt = 1.02min; MS (ESIpos): m/z = 642 $[M+H]^+$.

LC-MS (Method 3): Rt = 0.94min (100% DAD); MS (ESIpos): m/z = 641 $[M + H]^+$, 321 $[M + 2H]^{++}$.

$^1H$ NMR (400 MHz, $CDCl_3$) $\delta$ 5.79 (tdd, J=6.72, 10.33, 17.05 Hz, 1H), 5.02 (qd, J=1.62, 17.20 Hz, 1H), 4.94-4.99 (m, 1H), 3.66 (s, 3H), 3.20-3.32 (m, 7H), 2.67-2.92 (m, 14H), 2.53-2.64 (m, 2H), 2.04-2.25 (m, 2H), 1.76-1.83 (m, 1H), 1.61-1.71 (m, 1H), 1.44 (s, 27H).

**Step 5**

methyl (2S,5E)-6-(4-ethoxyphenyl)-2-[4,7,10-tris(2-tert-butoxy-2-oxoethyl)-1,4,7,10-tetraazacyclododecan-1-yl]hex-5-enoate

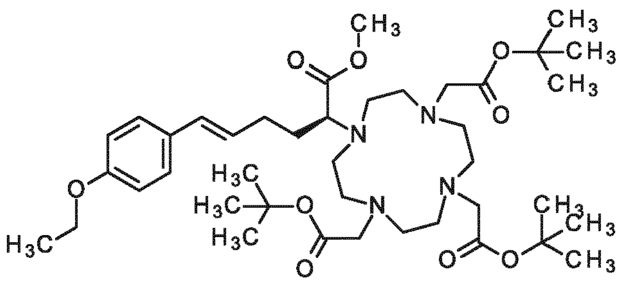

methyl (2S)-2-[4,7,10-tris(2-tert-butoxy-2-oxoethyl)-1,4,7,10-tetraazacyclododecan-1-yl]hex-5-enoate (2.06 g, 3.21 mmol) was dissolved in MeCN (170 ml), TEA (83 ml, 600 mmol) and the mixture deoxygenated by bubbling $N_2$ through the solution for 30min. 1-bromo-4-ethoxybenzene (450 µl, 3.1 mmol), tri-o-tolylphosphine (355 mg, 1.17 mmol; [6163-58-2]) and palladium(II) acetate (82 mg, 365µM) were added and the mixture stirred at 100°C overnight. The mixture was filtered through Celite® and evaporated to dryness under reduced pressure. The residue was partitioned between water and DCM, the organic phase separated and dried over $Na_2SO_4$. The title compound was obtained as raw material (3.13 g), which was used in the next step.

LC-MS (Method 2): Rt = 1.28min (79% ELSD); MS (ESIpos): m/z = 761 $[M+H]^+$.

**Step 6**

methyl (2S)-6-(4-ethoxyphenyl)-2-[4,7,10-tris(2-tert-butoxy-2-oxoethyl)-1,4,7,10-tetraazacyclododecan-1-yl]hexanoate

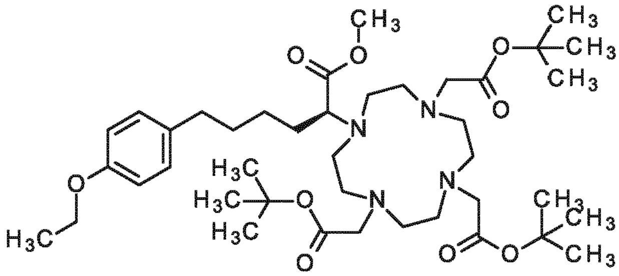

methyl (2S,5E)-6-(4-ethoxyphenyl)-2-[4,7,10-tris(2-tert-butoxy-2-oxoethyl)-1,4,7,10-tetraaza-cyclododecan-1-yl]hex-5-enoate (3.13 g, 4.11 mmol) was dissolved in EtOH (250 ml) and palladium (320 mg, 10% on activated carbon, 0.30 mmol; [7440-05-3]) added. The mixture was hydrogenated at RT overnight under ambient pressure. The catalyst was removed by filtration (PTFE-filter), washed with EtOH and the combined filtrates concentrated under reduced pressure. The raw product obtained (2.99 g) was used in the next step.

## Step 7

2,2',2''-{10-[(2S)-6-(4-ethoxyphenyl)-1-methoxy-1-oxohexan-2-yl]-1,4,7,10-tetraazacyclododecane-1,4,7-triyl}triacetic acid

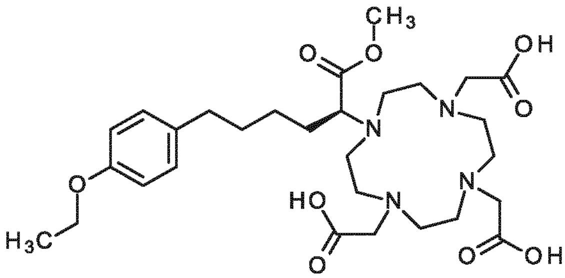

methyl (2S)-6-(4-ethoxyphenyl)-2-[4,7,10-tris(2-tert-butoxy-2-oxoethyl)-1,4,7,10-tetraazacyclododecan-1-yl] hexanoate (2.85 g, 3.74 mmol) was dissolved in formic acid (100 ml) and heated to 80°C for 8h. The solvent was removed under reduced pressure and the residue dried in vacuo (> 0.01 mbar) for 3h. The residue was dissolved in water, extracted with DCM and the aqueous phase lyophilized (2.37 g). The residue was absorbed on Isolute™ and subjected to RP-chromatography (SNAP C18 120 g, 45 ml/min, 254 nm, A = water, B = MeCN, 5% B 5 CV, 5% to 65%B in 15 CV, 65%B 2.3 CV) interesting fractions were pooled and lyophilized to give 630 mg (23%, calc. from step 5) of the title compound.

Specific Rotation: $\alpha_D^{20}$ = -23.3° +/- 0.40° (c=1, $H_2O$).

LC-MS (Method 2): $R_t$ = 0.75min; MS (ESIpos): m/z = 595 $[M+H]^+$.

LC-MS (Method 3): Rt = 0.67min (100% ESI TIC); MS (ESIpos): m/z = 298 $[2M + 2H]^{++}$, 595 $[M + H]^+$.

H-NMR indicated the hydrogenation (step 6) to be incomplete and revealed the presence of ca. 30% of the dehydrogenated compound.

$^{13}C$ NMR (101 MHz, $D_2O$) $\delta$ 174.4 (2C), 170.1, 169.1, 155.9, 135.8, 129.8 (2C), 114.7 (2C), 64.4, 59.9 (br), 56.6 (br, 2C), 56.2 (br), 53.0 (br, 2C), 52.3 (br, 2C), 52.0, 51.0 (br, 2C), 45.5 (br, 2C), 43.9 (br), 33.5, 30.6, 25.1 (br), 13.9.

## Step 7

gadolinium 2,2',2''-{10-[(1S)-1-carboxy-5-(4-ethoxyphenyl)pentyl]-1,4,7,10-tetraazacyclododecane-1,4,7-triyl}triacetate

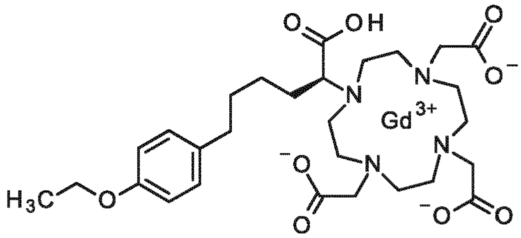

2,2',2''-{10-[(2S)-6-(4-ethoxyphenyl)-1-methoxy-1-oxohexan-2-yl]-1,4,7,10-tetraazacyclododecane-1,4,7-triyl} triacetic acid (630 mg, 1.06 mmol) was dissolved in water (40 ml) and $Gd_2O_3$ (192 mg, 530 μmol) added. The mixture was heated to 100°C for 22h. Chelex100™ (sodium form, ca. 5 g) were added and the mixture stirred at RT overnight. Xylenol orange test indicated the absence of free gadolinium, the mixture was filtrated, and the filtrate lyophilized. One obtained 800 mg of the title compound. (*The non-hydrogenated by-product can be seen in the MS, but a chromatographic separation was not seen feasible).*

The material was dissolved in water (20 ml) and EtOH (80 ml) and palladium (80 mg, 10% on activated carbon, 75 μM) were added. The mixture was hydrogenated at RT for 72h and filtrated to remove the catalyst. The filtrate was concentrated under reduced pressure and purified by RP-chromatography (SNAP C18 30 g, 20 ml/min, 254 nm, A = water, B = MeCN, 0%B 9.8 CV, 0% to 14%B in 6.4 CV, 14%B 3.2 CV, 14% to 46%B in 14.4 CV). One obtained 287.7 mg (0.39 mmol, 12% calc. from step 6) of the title compound, which still contained the dehydrogenated impurity.

From a different experiment, a small amount (5.7 mg) was obtained pure.

LC-MS (Method 2): Rt = 0.80min; MS (ESIpos): m/z = 726 $[M+H]^+$.

## Example 28

### Step 1

methyl (2S)-5-(4-ethoxyphenyl)-2-[(trifluoromethanesulfonyl)oxy]pentanoate

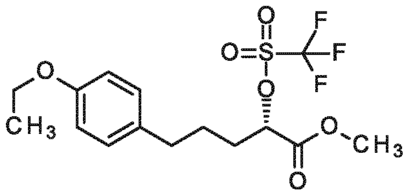

**Intermediate 20,** methyl (2S)-5-(4-ethoxyphenyl)-2-hydroxypentanoate, (500 mg, 1.98 mmol) was dissolved in DCM (5.0 ml), placed under $N_2$ and cooled to -60°C. 2,6-dimethylpyridine (280 μl, 2.4 mmol; [108-48-5]) was added, followed by dropwise addition of trifluoromethanesulfonic anhydride (2.1 ml, 1.0 M in DCM, 2.1 mmol; [358-23-6]). The reaction mixture was stirred at -60°C for 2h and was then allowed to warm to 8°C. Diethyl ether was added, and the precipitated triflate-salt removed by filtration. The filtrate and washings ($Et_2O$) were combined and extracted with water and 1 M HCl. After drying ($Na_2SO_4$) the solvent was removed under reduced pressure to give 746 mg (98%) methyl (2S)-5-(4-ethoxyphenyl)-2-[(trifluoromethanesulfonyl)oxy]pentanoate as raw product, which was directly used in the next step.

$^{19}F$ NMR (377 MHz, $CDCl_3$) δ -76.04 (s).

$^1H$ NMR (400 MHz, $CDCl_3$) δ 7.02-7.09 (m, 2H), 6.80-6.85 (m, 2H), 5.13 (t, J=6.21 Hz, 1H), 4.01 (q, *J*=7.10 Hz, 2H), 3.83 (s, 3H), 2.54-2.67 (m, 2H), 1.95-2.05 (m, 2H), 1.70-1.79 (m, 2H), 1.41 (t, *J*=7.10 Hz, 3H).

$^{13}C$ NMR (101 MHz, $CDCl_3$) δ 167.5, 157.4, 132.5, 129.2, 114.5, 118.4 (q, $^1J_{CF}$=319.6 Hz), 83.4, 63.4, 53.2, 33.9, 31.3, 26.2, 14.9.

**Step 2**

methyl (2R)-5-(4-ethoxyphenyl)-2-(1,4,7,10-tetraazacyclododecan-1-yl)pentanoate

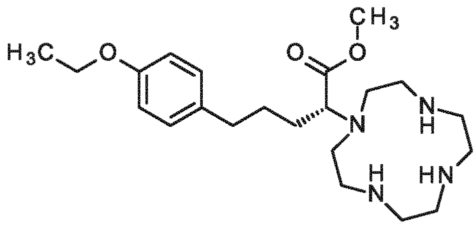

1,4,7,10-tetraazacyclododecane (382 mg, 2.22 mmol) was dissolved in MeCN (7.0 ml), $K_2CO_3$ (255 mg, 1.85 mmol) and a solution of methyl (2S)-5-(4-ethoxyphenyl)-2-[(trifluoromethanesulfonyl)oxy]pentanoate (710 mg, 1.85 mmol) in MeCN (3,5 ml) was added. The mixture was stirred at 60°C for 3h. The solids were removed by filtration. The filtrate and washings (MeCN) were combined and concentrated under reduced pressure to give 1.08 g raw product, which was used in the next step.

LC-MS (Method 2): Rt = 0.66min; MS (ESIpos): m/z = 407.5 $[M+H]^+$.

**Step 3**

5-(4-ethoxyphenyl)-2-[4,7,10-tris(carboxymethyl)-1,4,7,10-tetraazacyclododecan-1-yl] pentanoic acid

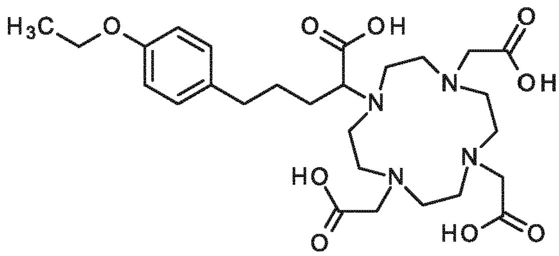

methyl (2R)-5-(4-ethoxyphenyl)-2-(1,4,7,10-tetraazacyclododecan-1-yl)pentanoate (1.08 g, 2.66 mmol) was dissolved in water (100 ml), chloroacetic acid (1.26 g, 13.3 mmol) added and the pH adjusted to 9 - 10 by addition of potassium hydroxide (50% in water). The mixture was stirred at 80°C for 8h, after which the pH was readjusted to 9 - 10. Chloroacetic acid (1.26 g, 13.3 mmol) was added, the pH brought to 9 - 10 and the mixture stirred at 100°C overnight. The mixture was neutralized by addition of HCl (1M, aq.) and concentrated under reduced pressure. The residue was extracted with EtOH and the extract concentrated in *vacuo.* The procedure was repeated with this residue to give 2.40 g raw product, which was purified by RP-chromatography (SNAP C18 60g, 40 ml/min, 254 nm. A = water/0.1% formic acid, B = MeCN, 2% B 5CV, 2% to 18%B in 4CV, 18%B 1.6CV, 18% to 21%B in 0.7CV, 21%B 3. CV, 21% to 60%B 10.2CV, 60%B 2CV). The interesting fractions were pooled and lyophilized to give 400 mg (35% from intermediate 20) of the title compound.

Specific Rotation: $\alpha_D^{20}$ = -1.6° +/- 0.42° (c=1, $H_2O$).

LC-MS (Method 3): Rt = 0.54min (100% DAD); MS (ESIpos): m/z = 284 $[M+2H]^{++}$, 567 $[M+H]^+$.

$^1H$ NMR (400 MHz, $D_2O$) $\delta$ 7.06-7.15 (m, 2H), 6.78-6.86 (m, 2H), 3.98 (q, *J*=7.01 Hz, 2H), 2.31-3.86 (m, 25H), 1.40-1.82 (m, 4H), 1.24 (t, *J*=6.97 Hz, 3H).

The specific rotation indicates that racemization had occurred. The product is regarded as a racemate.

**Step 4**

gadolinium 2,2',2''-{10-[1-carboxy-4-(4-ethoxyphenyl)butyl]-1,4,7,10-tetraazacyclododecane-1,4,7-triyl}triacetate

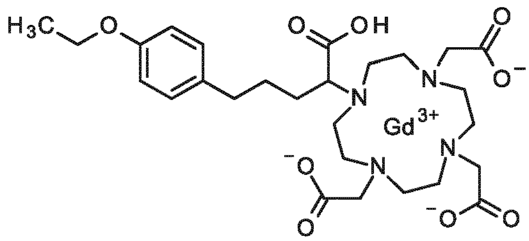

5-(4-ethoxyphenyl)-2-[4,7,10-tris(carboxymethyl)-1,4,7,10-tetraazacyclododecan-1-yl] pentanoic acid (400 mg, 706 μmol) was dissolved in water (14 ml), the pH adjusted, if necessary, to 3-4 (formic acid) and $Gd_2O_3$ (115 mg, 318 μmol, 0.9 eq) added. The mixture was stirred at 100°C overnight. The pH was readjusted, and stirring was continued overnight. $Gd_2O_3$ (38 mg, 0.10 mmol) was added and the mixture stirred overnight, after which the reaction was complete. Chelex100™ (sodium form, ca. 5 - 6 g) was added and left overnight at RT. Xylenol-orange test indicated the absence of free gadolinium, the mixture was filtered, and the filtrate concentrated under reduced pressure. RP-chromatography (SNAP C18 30g, 25 ml/min, 254 nm, A = water, B = MeCN, 5%B 5CV, 5% to 60%B in 15CV, 60%B 3.1CV). The interesting fractions were pooled and lyophilized and dried (50°C, 1 mbar) to give 246 mg (48%) of the title compound as white powder.

LC-MS (Method 3): Rt = 0.67min (100% DAD); MS (ESIpos): m/z = 361 $[M + 2H]^{++}$, 722 $[M + H]^{+}$ and some [2M + $2H]^{++}$, 731 $[2M + H_2O + 2H]^{++}$. The observed isotope pattern conforms to structure.

## Example 29

### Step 1

ethyl 3-(4-ethoxyphenyl)-2-[(methanesulfonyl)oxy]propanoate

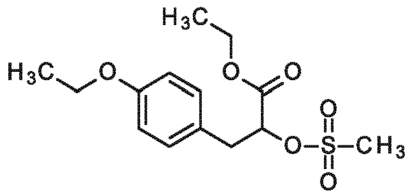

**Intermediate 21,** ethyl 3-(4-ethoxyphenyl)-2-hydroxypropanoate (1.19 g, 4.99 mmol) and MsCl (460 μl, 6.0 mmol; [124-63-0]) were dissolved in THF (15 ml) at 0°C and placed under $N_2$. TEA (1.7 ml, 12 mmol; [121-44-8]) was added and the mixture stirred at RT for 3h. The reaction mixture was poured into $NaHCO_3$ (0.6 M, aq.) and extracted with MTBE (3 x). The combined organic phases were washed with NaCl (sat. aq. 3 x), dried over $Na_2SO_4$ and concentrated under reduced pressure. 1.34 g (92% purity by NMR, 78%) of ethyl 3-(4-ethoxyphenyl)-2-[(methanesulfonyl)oxy]propanoate was obtained as an amorphous solid.

LC-MS (Method 3): Rt = 1.11min (100% DAD); MS (ESIpos): m/z = 339 $[M + Na]^{+}$.

$^1H$ NMR (DMSO-$d_6$, 400 MHz): δ (ppm) 7.13-7.20 (m, 2H), 6.82-6.88 (m, 2H), 5.26 (dd, J = 7.4, 5.3 Hz, 1H), 4.13 (q, *J* = 7.1 Hz, 2H), 3.98 (q, *J* = 7.1 Hz, 2H), 3.11 (dd, *J* = 14.4, 5.3 Hz, 1H), 3.04 (dd, *J* = 14.2, 7.1 Hz, 1H), 3.01 (s, 3H), 1.30 (t, *J* = 7.0 Hz, 3H), 1.15 (t, *J* = 7.1 Hz, 3H).

### Step 2

ethyl 2-[4,10-bis(2-tert-butoxy-2-oxoethyl)-1,4,7,10-tetraazacyclododecan-1-yl]-3-(4-ethoxyphenyl)propanoate

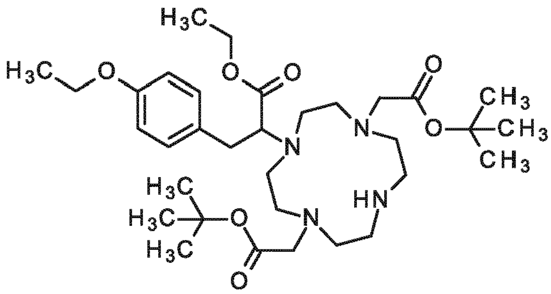

di-tert-butyl 2,2'-(1,4,7,10-tetraazacyclododecane-1,7-diyl)diacetate (978 mg, 2.44 mmol, [162148-48-3]) was dissolved in MeCN (6.7 ml) $K_2CO_3$ (743 mg, 5.73 mmol) and ethyl 3-(4-ethoxyphenyl)-2-[(methanesulfonyl)oxy]propanoate (850 mg, 2.69 mmol) were added. The mixture was heated to 80°C for 3d. The solids were filtered off and washed with EtOH. The combined filtrates were concentrated under reduced pressure to give raw product 1.75 g (>100%), which contains starting material (26%), monoalkylated product (40%) and dialkylated product (34%). The mixture was used in step 4.

LC-MS (Method 2): Rt = 1.16min; MS (ESIpos): m/z = 622.3 $[M+H]^+$.

__Step 3__

ethyl 2-[(trifluoromethanesulfonyl)oxy]pentanoate

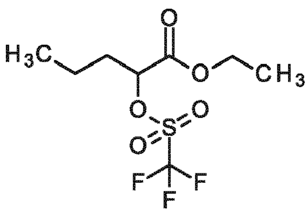

ethyl 2-hydroxypentanoate (600 mg, 4.10 mmol, [52089-55-1]) was dissolved in DCM (34 ml), placed under $N_2$ and cooled to -10°C. Lutidine (570 μl, 4.92 mmol) was added, followed by dropwise addition of trifluormethanesulfonic anhydride (730 μl, 4.3 mmol; [358-23-6]). The reaction was stirred at -10°C for 2h after which MTBE was added. The mixture was extracted with water, HCl (0.5 N. aq.) and NaCl (sat. aq.). The organic phase was dried over $Na_2SO_4$ and concentrated under reduced pressure to give the title compound 1.45 g (>100%, [168053-74-5]), which was used in the next reaction.

$^{19}F$ NMR (DMSO-$d_6$, 377 MHz): δ (ppm) -78.12 (s).

$^1H$ NMR (DMSO-$d_6$, 400 MHz): δ (ppm) 5.23 (dd, *J* = 7.6, 4.3 Hz, 1H), 4.19-4.33 (m, 2H), 1.73-1.92 (m, 2H), 1.28-1.45 (m, 2H), 1.25 (t, *J* = 7.1 Hz, 3H), 0.90 (t, *J* = 7.4 Hz, 3H).

__Step__ **4**

ethyl 2-{4,10-bis(2-tert-butoxy-2-oxoethyl)-7-[1-ethoxy-3-(4-ethoxyphenyl)-1-oxopropan-2-yl]-1,4,7,10-tetraazacyclododecan-1-yl}pentanoate

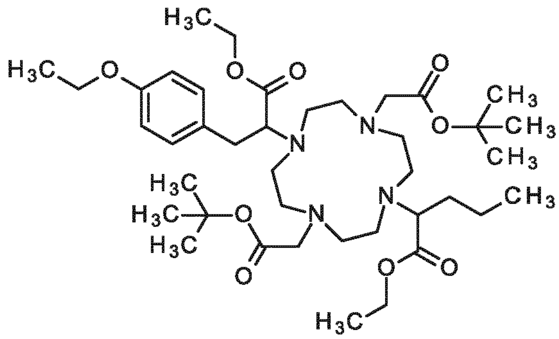

ethyl-2-[4,10-bis(2-tert-butoxy-2-oxoethyl)-1,4,7,10-tetraazacyclododecan-1-yl]-3-(4-ethoxyphenyl)propanoate (1.45 g, 2.34 mmol) was dissolved in MeCN (6.4 ml), DIPEA (890 µl) and ethyl 2-[(trifluoromethanesulfonyl)oxy] pentanoate (715 mg, 2.57 mmol) added and the mixture stirred at RT overnight. Ethyl acetate was added to the reaction mixture which was then extracted with water and NaCl (sat. aq.). The organic phase was dried over $Na_2SO_4$ and concentrated under reduced pressure. The raw product was combined with the raw product from an earlier experiment (0.15 g) to give the title compound (2.42 g, >100%), which was used as such in the next step.

LC-MS (Method 2): Rt = 1.35 - 1.41min; MS (ESIpos): m/z = 750.1 $[M+H]^+$.

## Step 5

2,2'-{4-[1-ethoxy-3-(4-ethoxyphenyl)-1-oxopropan-2-yl]-10-[1-ethoxy-1-oxopentan-2-yl]-1,4,7,10-tetraazacyclododecane-1,7-diyl}diacetic acid

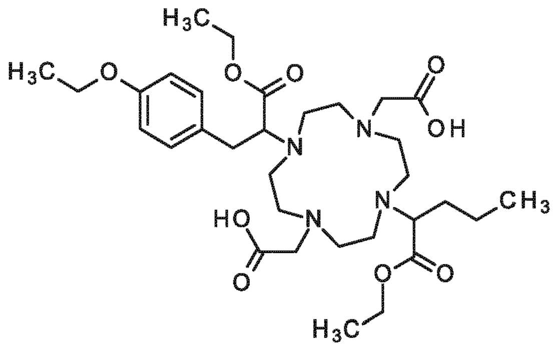

ethyl 2-{4,10-bis(2-tert-butoxy-2-oxoethyl)-7-[1-ethoxy-3-(4-ethoxyphenyl)-1-oxopropan-2-yl]-1,4,7,10-tetraazacyclododecan-1-yl}pentanoate (2.42 g, 3.23 mmol) dissolved in formic acid (41 ml) was and stirred at 70°C overnight. The formic acid was removed under reduced pressure and the residue subjected to RP-chromatography (Biotage SNAP Ultra C18 120g, 50 ml/min, A = water, B = MeCN, 0%B to 50%B in 25min, 100%B 10min). Interesting fractions were pooled and lyophilized to give 453 mg (29%, calc. over the 5 steps) of the title compound.

LC-MS (Method 2): Rt = 0.77min (100% ELSD); MS (ESIneg): m/z = 635 [M - H].

$^1H$ NMR (DMSO-$d_6$, 400 MHz): δ (ppm) 7.19-7.25 (m, 2H), 6.79 (d, *J* = 8.6 Hz, 2H), 4.09 (q, *J* = 7.1 Hz, 2H), 3.92-4.02 (m, 4H), 3.73 (br t, *J* = 7.6 Hz, 1H), 3.29-3.46 (m, 4H), 2.77-3.20 (m, 15H), 2.58-2.76 (m, 4H), 1.45-1.70 (m, 2H), 1.30 (t, *J* = 7.0 Hz, 3H), 1.15-1.26 (m, 5H), 1.05 (t, *J* = 7.2 Hz, 3H), 0.80-0.89 (m, 3H).

## Step 6

gadolinium 2-{7-[1-carboxy-2-(4-ethoxyphenyl)ethyl]-4,10-bis(carboxylatomethyl)-1,4,7,10-tetraazacyclododecan-1-yl} pentanoate

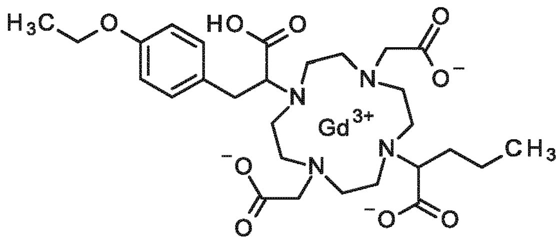

2,2'-{4-[1-ethoxy-3-(4-ethoxyphenyl)-1-oxopropan-2-yl]-10-[1-ethoxy-1-oxopentan-2-yl]-1,4,7,10-tetraazacyclododecane-1,7-diyl}diacetic acid (450 mg, 707 µmol) was dissolved in water (7.2 ml), $Gd_2O_3$ (115 mg, 318 µmol, 0.9 eq) added and the mixture heated to reflux for 24h. Chelex100™ (sodium form, ca. 5 g) was added, the pH adjusted to 5 and the mixture stirred at RT for 1h. The resin was filtered off, the filtrate concentrated under reduced pressure and purified by RP-chromatography (SNAP C18 60 g, 50ml/min, 254 nm, A = water, B = MeCN, 0% to 60%B in 15CV, 100%B 3.1CV). Interesting fractions were pooled and lyophilized to give the two diastereomers.

Diastereomer 1

228 mg (44 % yield)

LC-MS (Method 2): Rt = 0.71min; MS (pos): m/z = 736 $[M + H]^+$.

LC-MS (Method 3): Rt = 0.65min (92% DAD); MS (ESIpos): m/z = 368 $[M + 2H]^{++}$, 736 $[M + H]^+$ and some $[2M + 2H]^{++}$, 745 $[2M + H_2O + 2H]^{++}$. The observed isotope pattern conforms to a gadolinium complex.

**Example 30**

gadolinium 2-{7-[1-carboxy-2-(4-ethoxyphenyl)ethyl]-4,10-bis(carboxylatomethyl)-1,4,7,10-tetraazacyclododecan-1-yl} pentanoate

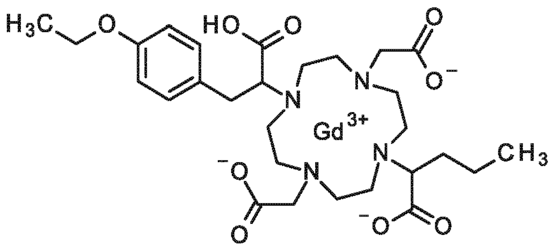

Diastereomer 2 from **Example 29.**

25 mg (5%)

LC-MS (Method 2): Rt = 0.68min; MS (pos): m/z = 736 $[M + H]^+$.

LC-MS (Method 3): Rt = 0.59min (100% DAD); MS (ESIpos): m/z = 368 $[M + 2H]^{++}$, 736 $[M + H]^+$ and some $[2M + 2H]^{++}$. The observed isotope pattern conforms to a gadolinium complex.

**Example 31**

**Step 1**

methyl (2R)-5-(4-butoxyphenyl)-2-[(trifluoromethanesulfonyl)oxy]pentanoate

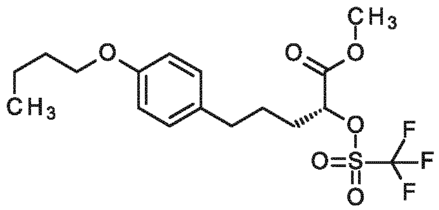

**Intermediate 22,** methyl (2R)-5-(4-butoxyphenyl)-2-hydroxypentanoate, (500 mg, 1.78 mmol) was dissolved in 5.0 ml DCM, placed under $N_2$ and cooled to -60°C. Lutidine (205 μl, 2.1 mmol) was added and then trifluormethanesulfonic anhydride (1.9 ml, 1.0 M in DCM, 1.9 mmol) added dropwise. The reaction was stirred for 3h after the end of the addition at -60°C and warming to ca. 8°C was allowed. Then diethyl ether was added to precipitate the lutidine triflate. The mixture was filtered and washed with diethyl ether. The combined filtrates were extracted with water and 1 M HCl, dried over $Na_2SO_4$ and concentrated under reduced pressure. 753 mg (>100%) raw product were obtained.

$^{19}$F NMR (377 MHz, $CDCl_3$) δ -76.03 (s).

$^{1}$H NMR (400 MHz, $CDCl_3$) δ 7.02-7.09 (m, 2H), 6.80-6.85 (m, 2H), 5.13 (t, J=6.08 Hz, 1H), 3.94 (t, J=6.59 Hz, 2H), 3.83 (s, 3H), 2.55-2.66 (m, 2H), 1.96-2.04 (m, 2H), 1.70-1.80 (m, 4H), 1.44-1.54 (m, 2H), 0.97 (t, J=7.35 Hz, 3H).

$^{13}$C NMR (101 MHz, $CDCl_3$) δ 167.5, 157.6, 132.4, 129.2 (2C), 114.5 (2C), 118.4 (q, $^{1}J_{CF}$=319.6 Hz), 83.4, 67.7, 53.3, 33.9, 31.4, 31.3, 26.2, 19.3, 13.9.

**Step 2**

methyl (2S)-5-(4-butoxyphenyl)-2-(1,4,7,10-tetraazacyclododecan-1-yl)pentanoate

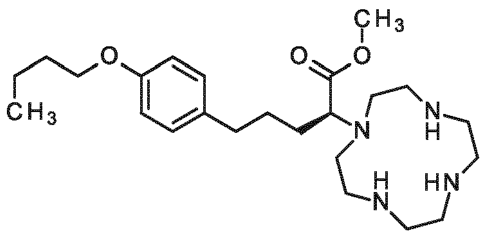

1,4,7,10-tetraazacyclododecane (377 mg, 2.19 mmol) was dissolved in MeCN (7 ml) and $K_2CO_3$ (252 mg, 1.83 mmol) added. A solution of (2R)-5-(4-butoxyphenyl)-2-[(trifluoromethanesulfonyl)oxy]pentanoate (753 mg, 1.83 mmol) in 3.5 ml MeCN was added and the mixture stirred for 3h at 60°C. The solids were filtered off and the filtrate concentrated under reduced pressure. The raw product (1.15 g) obtained, was used directly in the next step.

LC-MS (Method 2): Rt = 0.69min; MS (ESIpos): m/z = 435.4 $[M+H]^+$.

**Step 3**

methyl (2S)-5-(4-butoxyphenyl)-2-[4,7,10-tris(2-tert-butoxy-2-oxoethyl)-1,4,7,10-tetraazacyclododecan-1-yl]pentanoate

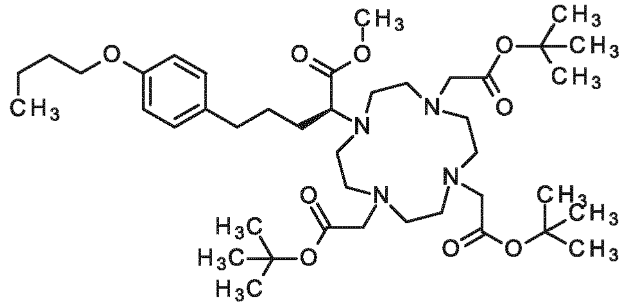

Methyl (2S)-5-(4-butoxyphenyl)-2-(1,4,7,10-tetraazacyclododecan-1-yl)pentanoate (1.15 g, 2.65 mmol) was dissolved in MeCN (35 ml) and $K_2CO_3$ (1.28 g, 9.26 mmol) added. Tert-butyl bromoacetate (1.3 ml, 8.7 mmol) was added dropwise and the suspension stirred overnight at RT. The reaction was filtered, and the filtrate concentrated under reduced pressure. The residue was dissolved in MTBE, washed with water, NaOH (1M, aq.) and water (twice) and dried over $Na_2SO_4$. The solvent was removed under reduced pressure to give 2.13 g raw material, which was used as such in the next step.

LC-MS (Method 2): Rt = 1.37min; MS (ESIpos): m/z = 777.9 $[M+H]^+$.

## Step 4

2,2',2"-{10-[(2S)-5-(4-butoxyphenyl)-1-methoxy-1-oxopentan-2-yl]-1,4,7,10-tetraazacyclododecane-1,4,7-triyl}triacetic acid

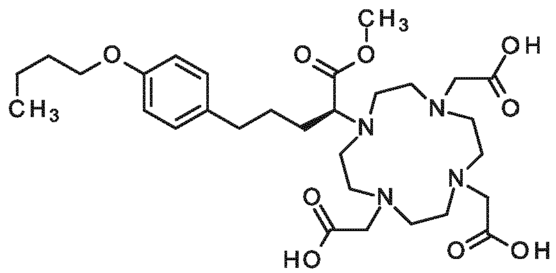

Methyl (2S)-5-(4-butoxyphenyl)-2-[4,7,10-tris(2-tert-butoxy-2-oxoethyl)-1,4,7,10-tetraazacyclododecan-1-yl] pentanoate (2.13 g, 2.74 mmol) was dissolved in formic acid (100 ml) and heated to 80°C for 8h. The solvent was removed under reduced pressure and the residue dried *in vacuo* to give 2.13 g raw material. RP-chromatography (SNAP C18 60 g, 50 ml/min, 254 nm, A = water, B = MeCN, 5%B 5CV, 5%B to 65%B in 15CV, 65%B 5.5CV). The interesting fractions were pooled and lyophilized to give 576 mg of the title compound.

Specific Rotation: $\alpha_D^{20}$ = -25.1° +/- 0.42° (c=1, $H_2O$).

LC-MS (Method 3): Rt = 0.76min (99% DAD); MS (ESIpos): m/z = 305 $[M+2H]^{++}$, 609 $[M+H]^+$.

$^1H$ NMR (400 MHz, $D_2O$) $\delta$ 7.09 (br d, J=8.62 Hz, 2H), 6.84 (br d, J=8.62 Hz, 2H), 3.92-4.00 (m, 3H), 3.60-3.87 (m, 6H), 3.58 (s, 3H), 2.95-3.44 (m, 12H), 2.79-2.94 (m, 3H), 2.54-2.66 (m, 2H), 2.30-2.41 (m, 1H), 1.40-1.70 (m, 6H), 1.28-1.39 (m, 2H), 0.82 (t, J=7.35 Hz, 3H).

$^{13}C$ NMR (101 MHz, $D_2O$) $\delta$ 174.3, 174.2, 170.1, 169.1, 156.2, 135.1, 129.8 (2C), 114.9 (2C), 68.6, 59.8, 56.6, 56.3, 52.9, 52.2 (br), 52.0, 51.0 (2C), 49.8 (br), 48.9, (br) 46.7 (br), 45.4, 43.8, 33.8, 30.5, 28.3 (br), 27.6, 18.6, 13.0.

## Step 5

gadolinium 2,2',2"-{10-[(1S)-4-(4-butoxyphenyl)-1-carboxybutyl]-1,4,7,10-tetraazacyclododecane-1,4,7-triyl}triacetate

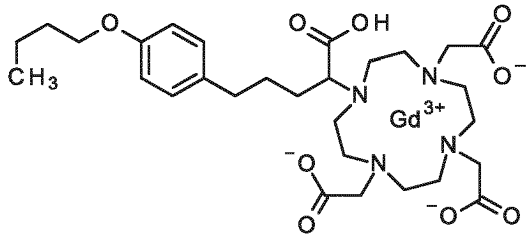

2,2',2"-{10-[(2S)-5-(4-butoxyphenyl)-1-methoxy-1-oxopentan-2-yl]-1,4,7,10-tetraazacyclododecane-1,4,7-triyl}triacetic acid (576 mg, 946 μmol) was dissolved in water (43 ml) at a pH of 3 - 4. $Gd_2O_3$ (172 mg, 473 μmol, 0.5 eq) was added and the reaction heated to 100°C overnight. After cooling to RT, Chelex100™ (sodium form, ca. 5 g, [11139-85-8]) was added and the mixture was stirred at RT overnight. The solution was tested negative for free gadolinium with the Xylenol-orange test and the pH adjusted to 6 - 7. After lyophilization, the raw material was purified by RP-chromatography (SNAP C18 30g, 25 ml/min, 254 nm, A = water, B = MeCN, 0%B 5 CV, 0 to 65%B in 15CV, 65%B 2CV). The interesting fractions were pooled and lyophilized to give 478 mg (35% over all steps) of the title compound as white lyophilizate.

Specific Rotation: $\alpha_D^{20}$ = +0.64° +/- 0.30° (c=1, $H_2O$, 20°C, 589 nm).

LC-MS (Method 3): $R_t$ = 0.90min (98% DAD); MS (ESIpos): m/z = 375 $[M+2H]^{++}$, 750 $[M+H]^+$ and $[2M+2H]^{2+}$, 759 $[2M + H_2O + 2H]^{2+}$. The observed isotope patterns conform to structure. The specific rotation indicates that racemization had occurred, and the compound was regarded as racemate.

**Example 32**

**Step 1**

ethyl 3-[4-(2-ethoxyethoxy)phenyl]-2-(1,4,7,10-tetraazacyclododecan-1-yl)propanoate

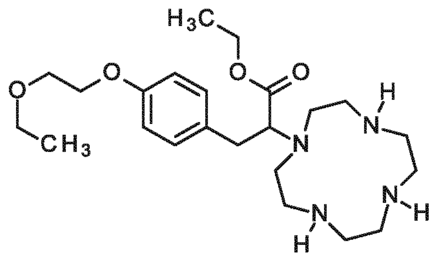

1,4,7,10-tetraazacyclododecane (1.19 g, 6.94 mmol) was dissolved in MeCN (19 ml), $K_2CO_3$ (959 mg, 6.94 mmol) and ethyl 3-[4-(2-ethoxyethoxy)phenyl]-2-[(methanesulfonyl)oxy]propanoate, (Example 7 - Step 1, 2.50 g, 6.94 mmol) added and the mixture heated to 55°C for 24h. The solids were filtered off and washed with EtOH. The combined filtrates were concentrated under reduced pressure to give 3.63 g (>100%) of the title compound, which was used as such in the next step.

LC-MS (Method 2): $R_t$ = 0.60min; MS (ESIpos): m/z = 438.1 $[M+H]^+$.

**Step 2**

ethyl 3-[4-(2-ethoxyethoxy)phenyl]-2-[4,7,10-tris(2-tert-butoxy-2-oxoethyl)-1,4,7,10-tetraazacyclododecan-1-yl]propanoate

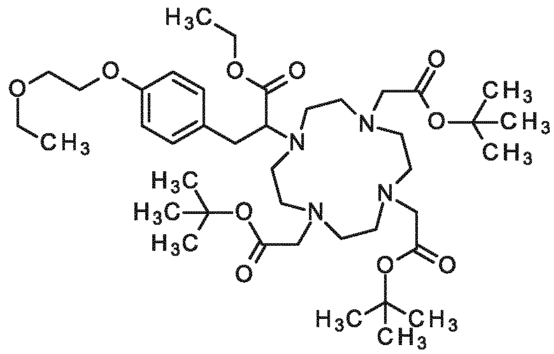

ethyl 3-[4-(2-ethoxyethoxy)phenyl]-2-(1,4,7,10-tetraazacyclododecan-1-yl)propanoate (3.63 g, 8.31 mmol) was dissolved in MeCN (32 ml) under $N_2$ and $K_2CO_3$ (9.77 g, 70.7 mmol) added. A solution of tert-butyl bromoacetate (4.3 ml, 29 mmol) in MeCN (11 ml) was added dropwise and the mixture stirred at RT overnight. The solids were filtered off, washed with EtOH and the combined filtrates concentrated under reduced pressure. The raw product 8.46 g (>100%) was used as such in the next step.

LC-MS (Method 2): Rt = 1.13min; MS (ESIpos): m/z = 780.8 $[M+H]^+$.

**Step 3**

2,2',2"-(10-{1-ethoxy-3-[4-(2-ethoxyethoxy)phenyl]-1-oxopropan-2-yl}-1,4,7,10-tetraazacyclododecane-1,4,7-triyl)triacetic acid

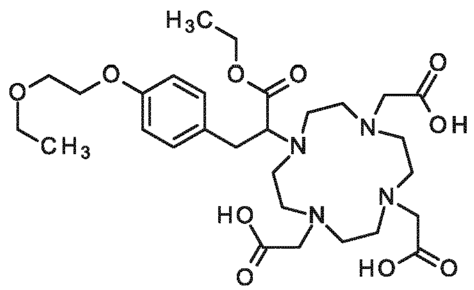

ethyl 3-[4-(2-ethoxyethoxy)phenyl]-2-[4,7,10-tris(2-tert-butoxy-2-oxoethyl)-1,4,7,10-tetraaza-cyclododecan-1-yl]propanoate (8.46 g, 10.9 mmol) was dissolved in formic acid (140 ml) and heated to 70°C overnight. The mixture was evaporated to dryness and purified by RP-chromatography (SNAP C18 120 g, 50 ml/min, A = water, B = MeCN, 0%B to 50%B in 30min, 100%B 10min). Interesting fractions were combined and lyophilized to give 1.75 g (2.87 mmol, 41% calc. from step 1) of the title compound.

LC-MS (Method 2): $R_t$ = 0.68min; MS (ESIpos): m/z = 611.7 $[M+H]^+$.

$^1H$ NMR (DMSO-$d_6$, 400 MHz): δ (ppm) 7.20 (d, *J* = 8.6 Hz, 2H), 6.81 (d, *J* = 8.9 Hz, 2H), 4.01-4.04 (m, 2H), 3.91-3.98 (m, 2H), 3.62 (t, *J* = 7.9 Hz, 1H), 3.66 (br dd, *J* = 5.2, 3.9 Hz, 2H), 3.48 (q, *J* = 7.1 Hz, 2H), 3.42-3.53 (m, 6H), 2.80-3.09 (m, 16H), 2.58 (br d, *J* = 9.9 Hz, 2H), 1.12 (t, *J* = 7.0 Hz, 3H), 1.04 (t, *J* = 7.1 Hz, 3H).

$^{13}C$ NMR (DMSO-$d_6$, 101 MHz): δ (ppm) 171.1, 170.8 (br), 169.6 (br, 2C), 157.0, 130.6 (2C), 129.8, 114.1 (2C), 68.4, 67.0, 65.7, 64.6 (br), 59.9, 55.3 (br), 55.1 (br, 2C), 51.5 (br, 2C), 51.2 (br, 2C), 49.1 (br, 2C), 46.7 (br, 2C), 33.8, 15.2, 14.2.

**Step 4**

gadolinium 2,2' 2"-(10-{1-carboxy-2-[4-(2-ethoxyethoxy)phenyl]ethyl}-1,4,7,10-tetraazacyclododecane-1,4,7-triyl)triacetate

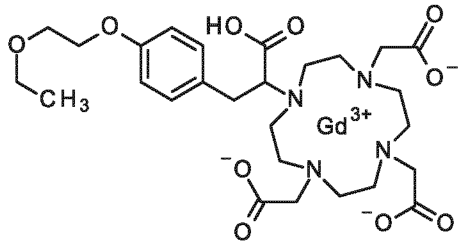

2,2',2''-(10-{1-ethoxy-3-[4-(2-ethoxyethoxy)phenyl]-1-oxopropan-2-yl}-1,4,7,10-tetraazacyclododecane-1,4,7-triyl)triacetic acid (1.75 g, 2.87 mmol) was dissolved in water (29 ml) and $Gd_2O_3$ (467 mg, 1.29 mmol, 0.9 eq) added. The mixture was heated at reflux for 48h. Chelex100™ (sodium form, ca. 5 g) was added, the pH adjusted to 5 and the mixture stirred at RT for 1h. RP-chromatography (SNAP C18 30 g, 25 ml/min, A = water, B = MeCN, 0%B to 50%B in 30min, 100%B 10min). Interesting fractions were combined and lyophilized to give 1.16 g (55 %) of the title compound as fluffy lyophilizate.

LC-MS (Method 2): Rt = 0.60min; MS (ESIpos): m/z = 738 $[M + H]^+$.

LC-MS (Method 3): Rt = 0.57min (100% DAD); MS (ESIpos): m/z = 369 $[M + 2H]^{++}$, 738 $[M + H]^+$ and some $[2M + 2H]^{++}$, 747 $[2M + H_2O + 2H]^{++}$. The observed isotope pattern conforms to a gadolinium complex.

## Example 33

### Step 1

ethyl 2-[(methanesulfonyl)oxy]-3-[4-(2,2,3,3-tetrafluoropropoxy)phenyl]propanoate

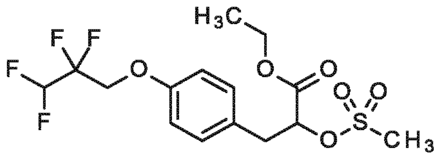

ethyl 2-hydroxy-3-[4-(2,2,3,3-tetrafluoropropoxy)phenyl]propanoate (4.42 g, 13.6 mmol) and TEA (4.2 ml, 30 mmol; [121-44-8]) were dissolved in THF (40 ml) at 0°C and placed under $N_2$. MsCl (1.2 ml, 15 mmol; [124-63-0]) was added dropwise and the mixture stirred at RT for 3h. The reaction mixture was poured into $NaHCO_3$ (0.6 M, aq.) and extracted with MTBE (3 x). The combined organic phases were washed with sat. NaCl (2 x), dried over $Na_2SO_4$ and concentrated under reduced pressure. 5.28 g (82%) of ethyl 2-[(methanesulfonyl)oxy]-3-[4-(2,2,3,3-tetrafluoropropoxy)phenyl]propanoate was obtained.

LC-MS (Method 4): $R_t$ = 1.24min; MS (ESIpos): m/z = 420.2 $[M + NH_4]^+$.

$^1H$ NMR (DMSO-$d_6$, 400 MHz): δ (ppm) 7.19-7.26 (m, 2H), 6.97-7.02 (m, 2H), 6.67 (tt, $^2J_{HF}$ = 51.7, $^3J_{HF}$ =5.6 Hz, 1H), 5.29 (dd, $J$ = 7.4, 5.3 Hz, 1H), 4.56 (br t, $J$ = 13.4 Hz, 2H), 4.13 (q, $J$ = 7.1 Hz, 2H), 3.14 (dd, $J$ = 14.2, 5.3 Hz, 1H), 3.07 (dd, $J$ = 14.2, 7.4 Hz, 1H), 3.03 (s, 3H), 1.16 (t, $J$ = 7.1 Hz, 3H).

### Step 2

ethyl 2-[4,10-bis(2-tert-butoxy-2-oxoethyl)-1,4,7,10-tetraazacyclododecan-1-yl]-3-[4-(2,2,3,3-tetrafluoropropoxy)phenyl]propanoate

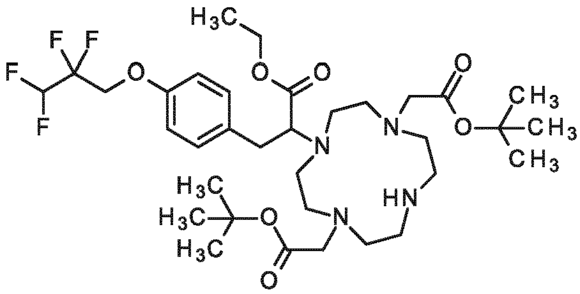

di-tert-butyl 2,2'-(1,4,7,10-tetraazacyclododecane-1,7-diyl)diacetate (2.06 g, 5.15 mmol, [162148-48-3]) was dissolved in MeCN (14 ml), $K_2CO_3$ (1.57 g, 11.3 mmol) and ethyl 2-[(methanesulfonyl)oxy]-3-[4-(2,2,3,3-tetrafluoropropoxy)phenyl]propanoate (2.28 g, 5.67 mmol) were added. The mixture was heated to 80°C for 24h. The solids were filtered off and washed with EtOH. The combined filtrates were concentrated under reduced pressure to give raw product 4.12 g (>100%), which contains starting material (25%), monoalkylated product (59%) and dialkylated product (16%). The mixture was used in step 4.

LC-MS (Method 2): Rt = 1.19min; MS (ESIpos): m/z = 707.9 $[M+H]^+$.

## Step 3

methyl (2R)-3-(benzyloxy)-2-[(methanesulfonyl)oxy]propanoate

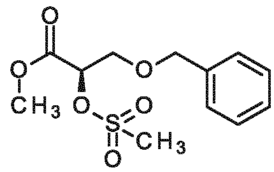

methyl (2R)-3-(benzyloxy)-2-hydroxypropanoate (1.00 g, 4.76 mmol, [209907-54-0]) was dissolved in THF (14 ml), placed under $N_2$ and cooled to 0°C. Triethylamine (1.5 ml, 10 mmol) and MsCl (400 µl, 5.2 mmol; [124-63-0]) were added and the mixture stirred at RT for 3h. The reaction mixture was poured into $NaHCO_3$ (0.6 M, aq.) and extracted with MTBE (3 x). The combined organic phases were washed with NaCl (sat. aq. 2 x), dried over $Na_2SO_4$ and concentrated under reduced pressure yielding 1.34 g (98%) of the title compound.

LC-MS (Method 4): $R_t$ = 1.03min; MS (ESIpos): m/z = 306.1 $[M+NH_4]^+$.

$^1H$ NMR (DMSO-$d_6$, 400 MHz): δ (ppm) 7.27-7.39 (m, 5H), 5.39 (dd, *J* = 4.8, 2.8 Hz, 1H), 4.58 (d, *J* = 12.2 Hz, 1H), 4.50 (d, *J* = 12.2 Hz, 1H), 3.87 (dd, *J* = 11.7, 4.8 Hz, 1H), 3.81 (dd, *J* = 11.4, 2.8 Hz, 1H), 3.72 (s, 3H), 3.26 (s, 3H).

## Step 4

methyl (2S)-3-(benzyloxy)-2-[4,10-bis(2-tert-butoxy-2-oxoethyl)-7-{1-ethoxy-1-oxo-3-[4-(2,2,3,3-tetrafluoropropoxy) phenyl]propan-2-yl}-1,4,7,10-tetraazacyclododecan-1-yl] propanoate

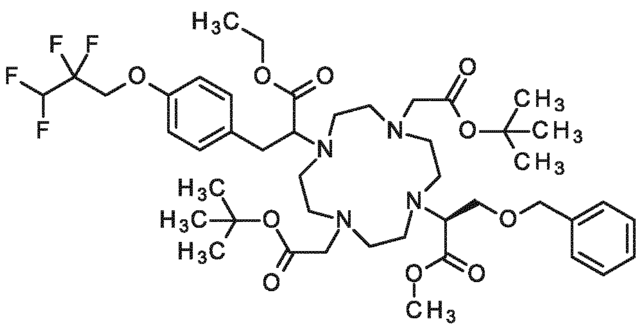

ethyl 2-[4,10-bis(2-tert-butoxy-2-oxoethyl)-1,4,7,10-tetraazacyclododecan-1-yl]-3-[4-(2,2,3,3-tetrafluoropropoxy)phenyl]propanoate (2.05 g, 2.90 mmol) was dissolved in MeCN, (9.5 ml), $K_2CO_3$ (802 mg, 5.80 mmol) and methyl (2R)-3-(benzyloxy)-2-[(methanesulfonyl)oxy]propanoate (1.34 g, 4.64 mmol) added and the mixture stirred at 80°C overnight. The solids were filtered off, washed with EtOH and the combined filtrated concentrated under reduced pressure. The residue obtained 3.19 g (>100%) was used without further purification.

LC-MS (Method 2): $R_t$ = 1.30min; MS (ESIpos): m/z = 899.9 $[M+H]^+$.

**Step 5**

2,2'-(4-[(2S)-3-(benzyloxy)-1-methoxy-1-oxopropan-2-yl]-10-{1-ethoxy-1-oxo-3-[4-(2,2,3,3-tetrafluoropropoxy)phenyl]propan-2-yl}-1,4,7,10-tetraazacyclododecane-1,7-diyl)diacetic acid

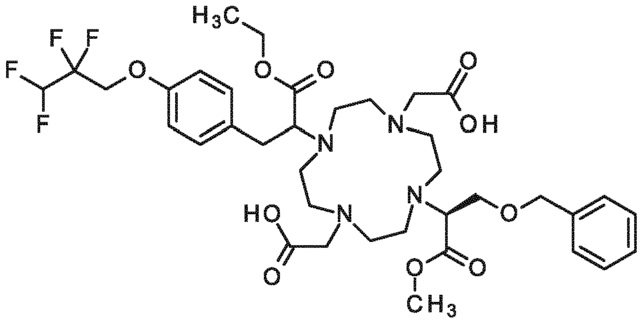

methyl (2S)-3-(benzyloxy)-2-[4,10-bis(2-tert-butoxy-2-oxoethyl)-7-{1-ethoxy-1-oxo-3-[4-(2,2,3,3-tetrafluoropropoxy)phenyl]propan-2-yl}-1,4,7,10-tetraazacyclododecan-1-yl] propanoate (3.19 g, 3.55 mmol) was dissolved in formic acid (46 ml) and heated to 70°C overnight. The mixture was concentrated under reduced pressure and purified by RP-chromatography (SNAP C18 60g, 50 ml/min, A = water, B = MeCN, 0%B to 50%B in 30min, 100%B 10min). Interesting fractions were combined and lyophilized to give the title compound 180 mg (6%).

LC-MS (Method 2): $R_t$ = 0.82 - 0.88min; MS (ESIpos): m/z = 787.8 $[M+H]^+$.

$^1$H NMR (DMSO-$d_6$, 400 MHz): $\delta$ (ppm) 7.18-7.42 (m, 7H), 6.93 (br d, $J$ = 8.4 Hz, 2H), 6.67 (br t, $^2J_{HF}$ = 51.7 Hz, 1H), 4.39-4.61 (m, 4H), 3.93-4.04 (m, 2H), 3.76-3.88 (m, 2H), 3.60-3.65 (m, 3H), 3.15-3.43 (m, 8H), 2.65-3.07 (m, 16H), 0.96-1.09 (m, 3H). The sample is a mixture of the diastereomers.

$^{19}$F NMR (DMSO-$d_6$, 377 MHz): $\delta$ (ppm) -125.33 (br s, 2F), -139.80 (br d, $J$ = 52.6 Hz, 2F).

**Step 6**

2,2'-(4-{1-ethoxy-1-oxo-3-[4-(2,2,3,3-tetrafluoropropoxy)phenyl]propan-2-yl}-10-[(2S)-3-hydroxy-1-methoxy-1-oxopropan-2-yl]-1,4,7,10-tetraazacyclododecane-1,7-diyl)diacetic acid

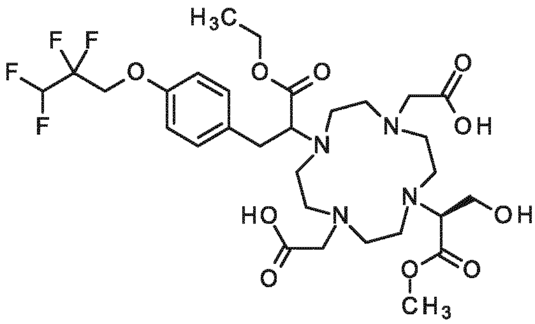

2,2'-(4-[(2S)-3-(benzyloxy)-1-methoxy-1-oxopropan-2-yl]-1 0-{1-ethoxy-1-oxo-3-[4-(2,2,3,3-tetrafluoropropoxy)phenyl]propan-2-yl}-1,4,7,10-tetraazacyclododecane-1,7-diyl)diacetic acid (180 mg, 229 µmol) was dissolved in EtOH (1.0 ml) and palladium (5.90 mg, 10 % on C, 5.55 µmol) was added and the mixture hydrogenated under 1 atm of

hydrogen. After 5d, acetic acid (1.3 µl, 23 µmol) was added. The mixture was further hydrogenated at RT and ambient pressure for 5d. The catalyst was filtered off and the filtrate concentrated under reduced pressure. The residue 79.0 mg (49%) was used as such in the next step.

LC-MS (Method 2): Rt = 0.80min (97% ELD); MS (ESIpos): m/z = 697 $[M + H]^+$.

$^{19}$F NMR (DMSO-$d_6$, 377 MHz): δ (ppm) -125.35 (br d, *J* = 5.7 Hz, 2F), -139.79 (br dd, *J* = 51.5, 5.7 Hz, 2F).

**Step 7**

gadolinium (2S)-2-[4,10-bis(carboxylatomethyl)-7-{1-carboxy-2-[4-(2,2,3,3-tetrafluoropropoxy)phenyl]ethyl}-1,4, 7,1 0-tetraazacyclododecan-1-yl]-3-hydroxypropanoate

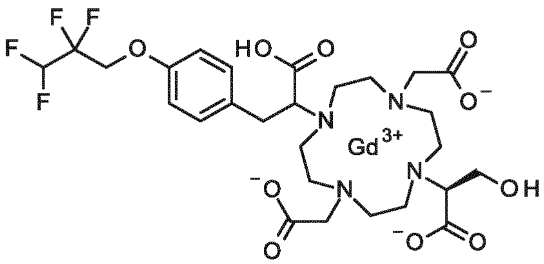

2,2'-(4-{1-ethoxy-1-oxo-3-[4-(2,2,3,3-tetrafluoropropoxy)phenyl]propan-2-yl}-10-[(2S)-3-hydroxy-1-methoxy-1-oxopropan-2-yl]-1,4,7,10-tetraazacyclododecane-1,7-diyl)diacetic acid (71.0 mg, 102 µmol) was dissolved in water (1.0 ml) and $Gd_2O_3$ (16.6 mg, 45.9 µmol, 0.9 eq) added. The mixture was stirred at 105°C for 24h and another 24h at 120°C, cooled to RT and Chelex100™ (sodium form, ca. 1 g) added. The mixture was stirred at pH 5 for 1h, the resin was filtered off and the filtrate concentrated under reduced pressure. The residue was purified by RP-chromatography (SNAP C18 12g, 12 ml/min, A = water, B = MeCN, 0%B to 50%B in 30min, 100%B 10min). Interesting fractions were pooled and lyophilized to give 14.2 mg (17%) of gadolinium 2-{7-[1-carboxy-2-hydroxyethyl]-4,10-bis(carboxylatemethyl)-1,4,7,10-tetraazacyclododecan-1-yl}-3-[4-(2,2,3,3-tetrafluoropropoxy)phenyl]propanoate

LC-MS (Method 2): Rt = 0.72min; MS (ESIneg): m/z = 808 [M - H].

LC-MS (Method 3): $R_t$ = 0.69min (8% DAD); MS (ESIpos): m/z = 405 $[M + 2H]^{++}$, 809 $[M + H]^+$; 0.72min (92% DAD); MS (ESIpos): m/z = 396 $[M + 2H - F]^{++}$, 405 $[M + 2H]^{++}$, 791 $[2M + 2H - HF - H_2O]^{++}$, 800 $[2M + 2H - HF]^+$, 809 $[M + H]^+$ and some $[2M + 2H]^{++}$. The observed isotope patterns conform to gadolinium complexes. One of the two diastereomers is predominating.

**Example 34**

**Step 1**

methyl (2R)-5-(3-butoxyphenyl)-2-[(trifluoromethanesulfonyl)oxy]pentanoate

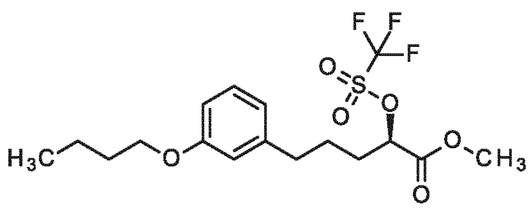

**Intermediate 24,** methyl (2R)-5-(3-butoxyphenyl)-2-hydroxypentanoate, (2.00 g, 7.13 mmol) in DCM (20 ml) was placed under $N_2$ and cooled to -60°C. Lutidine (1000 µl, 8.6 mmol; [108-48-5]) was added, followed by dropwise addition of trifluoromethanesulfonic anhydride (7.5 ml, 1.0 M in DCM, 7.5 mmol; [358-23-6]). The mixture was kept at this temperature for 2h and was then allowed to warm to ca. 8°C. Diethyl ether was added, and the precipitate removed by filtration. The filtrate and the washing (diethyl ether) were combined and extracted with water and HCl (1 M, aq.). The organic phase was dried ($Na_2SO_4$) and concentrated under reduced pressure to give 2,99 g (>100%) methyl (2R)-5-(3-butoxyphenyl)-2-[(trifluoromethanesulfonyl)oxy]pentanoate, which was used directly in the next step. The sample of an earlier experiment was characterized:

Specific rotation: $\alpha_D^{20}$ = +17.47° +/- 0.25° (c=1, $CHCl_3$) .

$^1$H NMR ($CDCl_3$, 400MHz): δ (ppm) 7.20 (t, J=7.8 Hz, 1H), 6.69-6.77 (m, 3H), 5.13 (t, J=6.1 Hz, 1H), 3.95 (t, J=6.6 Hz, 2H), 3.83 (s, 3H), 2.58-2.70 (m, 2H), 1.97-2.05 (m, 2H), 1.71-1.84 (m, 4H), 1.44-1.54 (m, 2H), 0.98 (t, J=7.4 Hz, 3H).

$^{13}$C NMR ($CDCl_3$, 101MHz): δ (ppm) 167.5, 159.3, 142.2, 129.5, 120.5, 118.4, 114.8 (q, J=319.59 Hz), 112.0, 83.4, 67.6, 53.3, 34.9, 31.4 (2C), 25.9, 19.3, 13.9.

**Step 2**

methyl (2S)-5-(3-butoxyphenyl)-2-(1,4,7,10-tetraazacyclododecan-1-yl)pentanoate

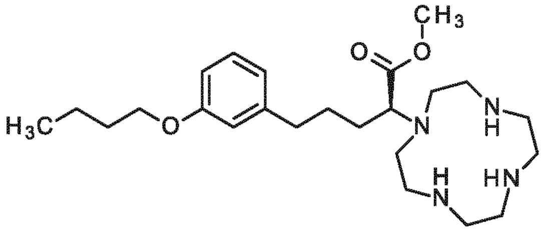

1,4,7,10-tetraazacyclododecane (1.20 g, 6.96 mmol) was dissolved in 23 ml MeCN and $K_2CO_3$ (802 mg, 5.80 mmol) added. A solution of (2R)-5-(4-butoxyphenyl)-2-[(trifluoromethanesulfonyl)oxy]pentanoate (2.99 g) obtained above in MeCN (1.0 ml) was added and the mixture stirred for 3h at 60°C and overnight at RT. The solids were filtered off and the filtrate concentrated under reduced pressure. The raw product (4.56 g) obtained, was used directly in the next step.

LC-MS (Method 2): Rt = 0.75min; MS (ESIpos): m/z = 436.0 $[M+H]^+$.

**Step 3**

methyl (2S)-5-(3-butoxyphenyl)-2-{4, 7,1 0-tris[(2S)-3-tert-butoxy-1-methoxy-1-oxopropan-2-yl]-1,4,7,10-tetraazacyclododecan-1-yl}pentanoate

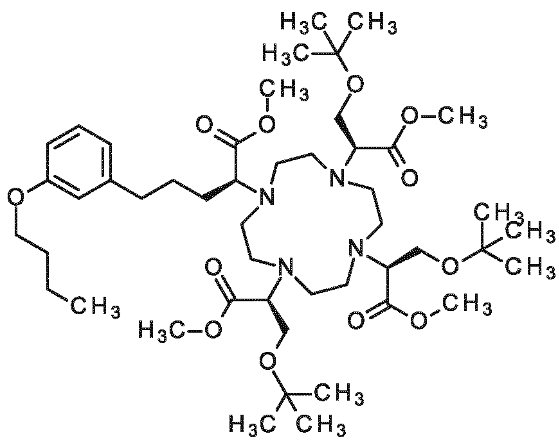

methyl (2S)-5-(3-butoxyphenyl)-2-(1,4,7,10-tetraazacyclododecan-1-yl)pentanoate (1.82 g, 70 % purity, 2.93 mmol) was dissolved in MeCN (35 ml) and $K_2CO_3$ (1.42 g, 10.2 mmol) added. Subsequently, a solution of **Intermediate 26,** methyl (2R)-3-tert-butoxy-2-[(trifluoromethanesulfonyl)oxy]propanoate (3.72 g, 90 % purity, 10.9 mmol) in MeCN (15 ml)

was added dropwise and the mixture stirred overnight at RT. The mixture was combined with the pilot reaction (0.2g), filtered and washed with MeCN. The combined filtrates were concentrated under reduced pressure to give 6.32 g (>100%) methyl (2S)-5-(3-butoxyphenyl)-2-{4,7,10-tris[(2S)-3-tert-butoxy-1-methoxy-1-oxopropan-2-yl]-1,4,7,10-tetraazacyclododecan-1-yl} pentanoate, which was used directly in the next step.

LC-MS (Method 2): Rt = 1.56min; MS (ESIpos): m/z = 910.0 $[M+H]^+$.

**Step 4**

methyl (2S)-5-(3-butoxyphenyl)-2-{4,7,10-tris[(2S)-3-hydroxy-1-methoxy-1-oxopropan-2-yl]-1,4,7,10-tetraazacyclododecan-1-yl}pentanoate

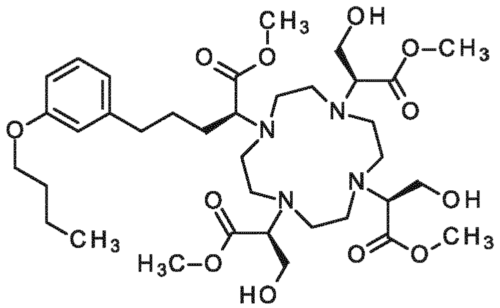

methyl (2S)-5-(3-butoxyphenyl)-2-{4,7,10-tris[(2S)-3-tert-butoxy-1-methoxy-1-oxopropan-2-yl]-1,4,7,10-tetraazacyclododecan-1-yl}pentanoate (5.22 g) was dissolved in 1,4-dioxane (65 ml) and three drops of water and HCl (11.4 ml 4 M in dioxane) was added. The mixture was stirred 4h at 80°C and then at RT overnight. The solvent was removed under reduced pressure and the raw material obtained (5.38 g) were used in the next step.

The raw material from a previous experiment with 1.00 g tetra ester was purified by RP-chromatography (SNAP C18 30g, 20 ml/min, 254 nm, A = water, B = MeCN, 20%B 5CV, 20%B to 100%B in 15CV, 100%B 2.6CV). The interesting fractions were pooled and lyophilized to give 143 mg of the title compound.

LC-MS (Method 3): Rt = 0.81min (100% DAD); MS (ESIpos): m/z = 371 $[M+2H]^{++}$, 741 $[M + H]^+$.

$^1$H NMR (DMSO-$d_6$, 400 MHz): δ (ppm) 7.15 (t, *J* = 7.9 Hz, 1H), 6.68-6.75 (m, 3H), 4.79 (br s, 3H), 3.92 (t, *J* = 6.5 Hz, 2H), 3.42-3.68 (m, 22H), 2.75-2.99 (m, 8H), 2.52-2.71 (m, 2H), 2.25-2.46 (m, 6H), 1.98-2.22 (m, 2H), 1.34-1.76 (m, 8H), 0.89-0.96 (m, 3H).

$^{13}$C NMR (DMSO-$d_6$, 101 MHz): δ (ppm) 172.6, 171.5 (2C), 171.4, 158.8, 143.6, 129.3, 120.4, 114.4, 111.8, 66.9, 62.9 (2C), 62.0, 61.8, 59.4 (2C), 58.8, 51.0, 51.0 (3C), 47.9 (2C), 47.8 (2C), 47.7 (2C), 47.5 (2C), 35.2, 30.9, 28.7 (br), 28.3 (br), 18.9, 13.8.

**Step 5**

(2S)-5-(3-butoxyphenyl)-2-{4,7,10-tris[(1S)-1-carboxy-2-hydroxyethyl]-1,4,7,10-tetraazacyclododecan-1-yl}pentanoic acid

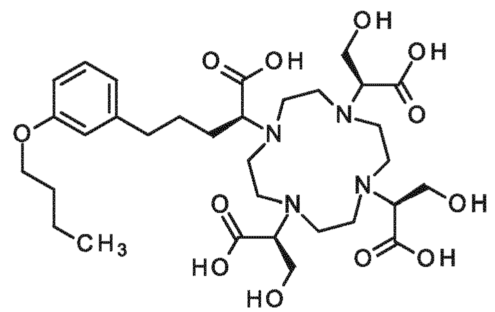

methyl (2S)-5-(3-butoxyphenyl)-2-{4,7,10-tris[(2S)-3-hydroxy-1-methoxy-1-oxopropan-2-yl]-1,4,7,10-tetraaza-

cyclododecan-1-yl}pentanoate (5.38 g) obtained above were dissolved in THF (30 ml). A solution of lithium hydroxide (696 mg, 29.0 mmol; [1310-65-2]) in water (30 ml) was added and the mixture stirred overnight at RT. The reaction was concentrated under reduced pressure to give 7.56 g raw material. It was purified by RP-chromatography (SNAP C18 400g, 65 ml/min, 254 nm, A = water, B = MeCN, 0% B 5CV, 0%B to 31%B in 10CV). The interesting fractions were pooled and lyophilized to give fraction 1 (772 mg) and fraction 2 (490 mg) of the title compound, which were both used separately in the final step.

LC-MS (Method 6): Rt = 0.24 / 0.39min; MS (ESIpos): m/z = 685.6 [M+H]$^+$.

**Step 6**

gadolinium (2S,2'S,2"S)-2,2',2"-{10-[(1S)-4-(3-butoxyphenyl)-1-carboxybutyl]-1,4,7,10-tetraazacyclododecane-1,4,7-triyl}tris(3-hydroxypropanoate)

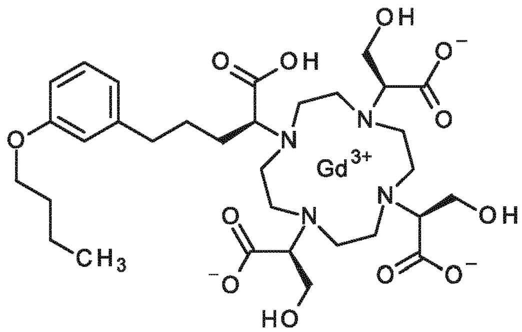

(2S)-5-(3-butoxyphenyl)-2-{4,7,10-tris[(1S)-1-carboxy-2-hydroxyethyl]-1,4,7,10-tetraazacyclo-dodecan-1-yl} pentanoic acid (490 mg, 716 μmol) was dissolved in water (40 ml) and brought to pH 4 by addition of formic acid. $Gd_2O_3$ (117 mg, 322 μmol, 0.9 eq) was added and the mixture stirred at 100°C for 4h and at RT overnight. Chelex100™ (sodium form, ca. 7 g) were added and the mixture stirred overnight at RT, after which the Xylenol-orange test for free gadolinium was negative. The pH was adjusted to 7 by addition of 25% ammonium hydroxide.

Fraction 1 from Step 5 was treated in the same way and both reactions were combined at this point.

The whole mixture (including the Chelex-resin) transferred into an empty Biotage cartridge connected to the RP-column. Chromatography (SNAP C18 120g, 45 ml/min, 254 nm, A = water, B = MeCN, 0%B 5CV, 0%B to 21%B in 4.8CV, 21% 0.9CV, 21%B to 65%B in 10 CV, 65%B 2.6CV) gave two fractions.

Fraction 1 52.5 mg

Specific rotation: $\alpha_D^{20}$ = -7.06° +/- 3.78° (c=1, MeOH).

LC-MS (Method 3): Rt = 0.85min (100% DAD); MS (ESIpos): m/z = 420 [M + 2H]$^{++}$, 840 [M + H]$^+$; 849 [M + $H_2O$ + 2H]$^{++}$. The observed isotope pattern conforms to a gadolinium complex. (stereoisomer of the target product).

Fraction 2: 645.2 mg (13%, calc. from intermediate 24) Example compound 34.

Specific rotation: $\alpha_D^{20}$ = +16.87° +/- 0.28° (c=1, $H_2O$).

LC-MS (Method 3): Rt = 0.90-0.92min (100% DAD); MS (ESIpos): m/z = 420 [M + 2H]$^{++}$, 840 [M + H]$^+$; 849 [M + $H_2O$ + 2H]$^{++}$. The observed isotope pattern conforms to a gadolinium complex.

**Example 35**

**Step 1**

dibenzyl 4-[(2R)-3-tert-butoxy-1-methoxy-1-oxopropan-2-yl]-1,4,7,10-tetraazacyclododecane-1,7-dicarboxylate

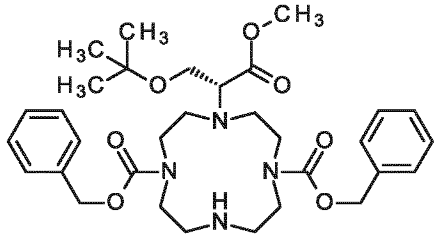

dibenzyl 1,4,7,10-tetraazacyclododecane-1,7-dicarboxylate, (10.0 g, 22.7 mmol, [162148-45-0]) was dissolved in chloroform (240 ml), DIPEA (5.9 ml, 34 mmol) and a solution of methyl (2S)-3-tert.-butoxy-2-[(trifluoromethanesulfonyl) oxy]propanoate, **Intermediate 28,** (4.90 g, 15.9 mmol) in chloroform (1.0 ml) added. The mixture was stirred at 40°C for 3h. Water and DCM were added, and the phases separated. The organic phase was washed with sat. NaCl, dried over $Na_2SO_4$ and concentrated under reduced pressure. The raw product obtained (14.17 g) was combined with the raw product from a previous experiment (3.92 g from 2.39 g, 5.43 mmol). The combined material (19.56 g) was absorbed on Isolute™ and purified by RP-chromatography (SNAP C18 60g, 50 ml/min, A = water/0.1% formic acid, B = MeCN, 210 nm, 10%B 2CV, 10%B to 25%B in 4,9CV, 25%B 1.3CV, 25%B to 70%B in 15CV). Interesting fractions were combined and lyophilized to give:

Fraction 1: 5.45 g (12.5 mmol, 46%) starting material.

Fraction 2: 4.97 g (8.30 mmol, 31 %) of the title compound.

Specific rotation: $\alpha_D^{20}$ = +9.21° +/- 0.25° (c=1, $CHCl_3$).

LC-MS (Method 3): Rt = 1.04min (100% DAD); MS (ESIpos): m/z = 599 $[M + H]^+$.

**Step 2**

dibenzyl 4-[(2R)-3-tert-butoxy-1-methoxy-1-oxopropan-2-yl]-10-[(2S)-5-{4-[2-(2-ethoxyethoxy)ethoxy]phenyl}-1-methoxy-1-oxopentan-2-yl]-1,4,7,10-tetraazacyclododecane-1,7-dicarboxylate

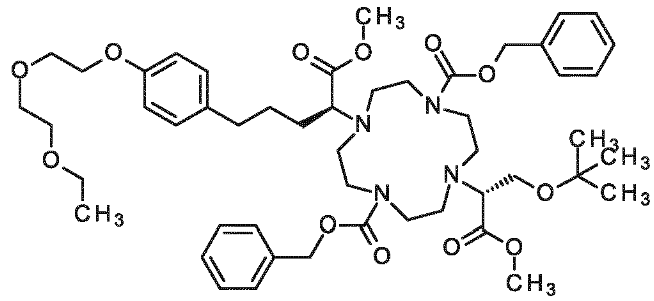

dibenzyl 4-[(2R)-3-tert-butoxy-1-methoxy-1-oxopropan-2-yl]-1,4,7,10-tetraazacyclododecane-1,7-dicarboxylate (4.68 g, 7.82 mmol) was dissolved in MeCN (300 ml) and $K_2CO_3$ (2.16 g, 15.6 mmol) added. A solution of methyl (2R)-5-{4-[2-(2-ethoxyethoxy)ethoxy]phenyl}-2-[(trifluoromethanesulfonyl)oxy]pentanoate (6.00 g, 10.2 mmol) in MeCN (50 ml) **(Step 1 of example 15)** was added dropwise and the mixture stirred at RT overnight. The mixture was combined with the pilot experiment (107 mg) and the solids removed by filtration. The filtrate and washings (MeCN) were concentrated under reduced pressure to give a residue (13.76 g), which was dissolved in DCM and extracted with water. The aqueous phase was extracted with DCM, the combined organic phases were washed with NaCl (sat. aq.) and dried over $Na_2SO_4$. Concentration under reduced pressure gave 9,6 g red-brown raw product, which was absorbed on to Isolute™ and purified by RP-chromatography (SNAP C18 120g, 50 ml/min, A = water/0.1% formic acid, B = MeCN, 254 nm, 10%B 2CV, 10%B to 38%B in 11.6CV, 38%B 2.8CV, 38%B to 70%B in 13.3CV, 70%B to 100%B in 6CV, 100%B 9CV). Interesting fractions were combined and lyophilized to give:

Fraction 1: 2.15 g (mixture of intermediate 12 and title compound)

Fraction 2: 3.30 g (38%)

Specific Rotation: $\alpha_D^{20}$ = -17.01° +/- 0.26° (c=1, $CHCl_3$).

LC-MS (Method 2): Rt = 1.72min; MS (ESIpos): m/z = 922 $[M+H]^+$.

$^{13}C$ NMR ($CDCl_3$, 101 MHz): δ (ppm) 173.2, 171.8, 156.8, 156.5 (2C), 136.8 (2C), 134.5 (br), 129.2 (2C), 128.4 (4C), 127.9 (2C), 127.9 (4C), 114.4 (2C), 73.3, 70.8, 69.8, 69.8, 67.4, 66.9 (br, 2C), 66.7, 65.2 (br), 62.6 (v br), 60.3 (br), 53.6 (br, 2C), 51.2, 51.1 (br, 2C), 50.9, 48.2 (br, 2C), 46.5 (br, 2C), 34.9, 29.1, 28.3, 27.4 (3C), 15.2.

**Step 3**

methyl (2S)-2-{7-[(2R)-3-tert-butoxy-1-methoxy-1-oxopropan-2-yl]-1,4,7,10-tetraazacyclododecan-1-yl}-5-{4-[2-(2-ethoxyethoxy)ethoxy]phenyl}pentanoate

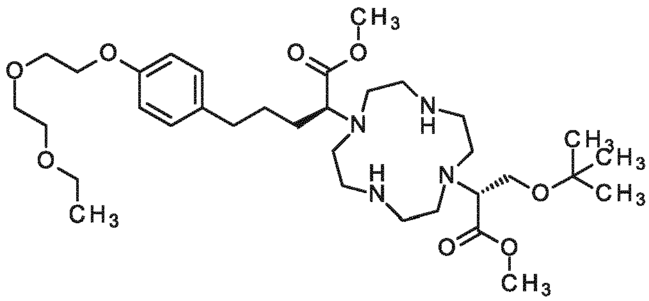

dibenzyl 4-[(2R)-3-tert-butoxy-1-methoxy-1-oxopropan-2-yl]-10-[(2S)-5-{4-[2-(2-ethoxyethoxy)ethoxy]phenyl}-1-methoxy-1-oxopentan-2-yl]-1,4,7,10-tetraazacyclododecane-1,7-dicarboxylate (2.99 g, 3.25 mmol) was dissolved in EtOH (70 ml), palladium (1.20 g, 10% on carbon, 1.3 mmol) added and the mixture hydrogenated at RT for 6h under ambient pressure. The reaction was combined with the pilot experiment (180 mg) and the catalyst removed by filtration. The filtrate was concentrated under reduced pressure to give the title compound (2.0 g, 89%).

LC-MS (Method 3): Rt = 0.64min (80% DAD); MS (ESIpos): m/z = 327 $[M + 2H]^{++}$, 653 $[M + H]^+$.

$^1H$ NMR ($CDCl_3$, 400 MHz): δ (ppm) 7.02-7.08 (m, 2H), 6.79-6.85 (m, 2H), 4.10 (dd, *J* = 5.3, 4.6 Hz, 2H), 3.84 (dd, *J* = 5.4, 4.4 Hz, 2H), 3.49-3.77 (m, 17H), 3.32 (br dd, *J* = 8.1, 5.3 Hz, 1H), 2.45-3.04 (m, 16H), 1.53-1.71 (m, 4H), 1.23 (t, *J* = 7.1 Hz, 3H), 1.14 (s, 7H).

$^{13}C$ NMR ($CDCl_3$, 101 MHz): δ (ppm) 173.4, 172.2, 157.1, 134.2, 129.3 (2C), 114.6 (2C), 73.5, 71.0, 70.0, 69.9, 67.5, 66.8, 64.5 (br), 64.1 (br), 61.2 (br), 51.5, 51.4, 49.9 (2C), 49.7 (br, 2C), 46.7 (br, 4C), 34.9, 29.3, 28.8, 27.5 (3C), 15.3.

**Step 4**

methyl (2S)-2-{7-[(2R)-3-tert-butoxy-1-methoxy-1-oxopropan-2-yl]-4,10-bis[(2S)-3-tert-butoxy-1-methoxy-1-oxopropan-2-yl]-1,4,7,10-tetraazacyclododecan-1-yl}-5-{4-[2-(2-ethoxyethoxy)ethoxy]phenyl}pentanoate

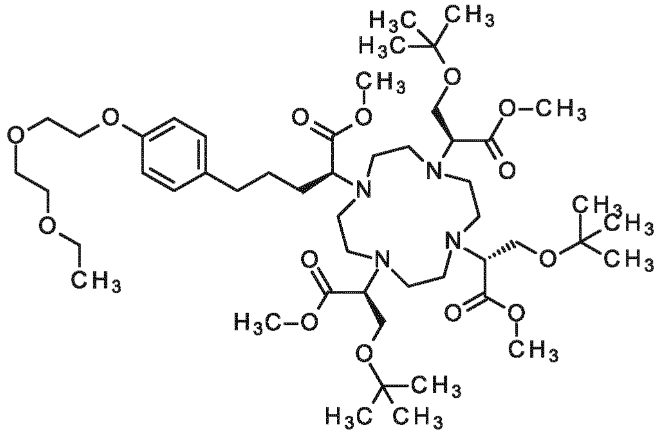

methyl (2S)-2-{7-[(2R)-3-tert-butoxy-1-methoxy-1-oxopropan-2-yl]-1,4,7,10-tetraazacyclo-dodecan-1-yl}-5-{4-[2-(2-ethoxyethoxy)ethoxy]phenyl}pentanoate (800 mg, 1.23 mmol) was dissolved in MeCN (30 ml), $K_2CO_3$ (423 mg, 3.06 mmol) and a solution of methyl (2R)-3-tert-butoxy-2-[(trifluoromethanesulfonyl)oxy]propanoate, **Intermediate 26,** (1.09 g, 2.82 mmol),in MeCN (1.9 ml) added. The mixture was stirred overnight at RTand was combined with the pilot experiment (100 mg). The mixture was filtrated, washed with MeCN and the combined filtrates concentrated under reduced pressure. The residue was dissolved in DCM and extracted with water and sat. NaCl. The organic phase was concentrated under reduced pressure to give the title compound as brown residue (1.83 g, >100%), which was used as such in subsequent experiments.

LC-MS (Method 2): Rt = 1.45min; MS (ESIpos): m/z = 970 $[M+H]^+$.

**Step 5**

methyl (2S)-5-{4-[2-(2-ethoxyethoxy)ethoxy]phenyl}-2-{7-[(2R)-3-hydroxy-1-methoxy-1-oxopropan-2-yl]-4,10-bis [(2S)-3-hydroxy-1-methoxy-1-oxopropan-2-yl]-1,4,7,10-tetraazacyclododecan-1-yl}pentanoate

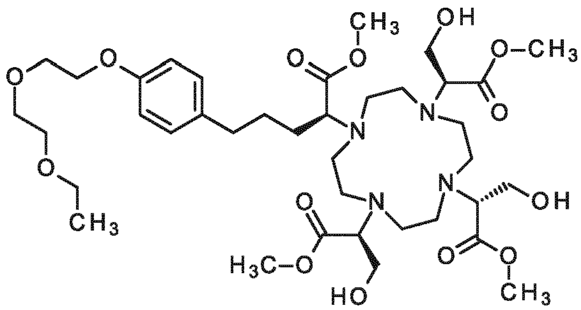

methyl (2S)-2-{7 -[(2R)-3-tert-butoxy-1-methoxy-1-oxopropan-2-yl]-4, 10-bis[(2S)-3-tert-butoxy-1-methoxy-1-oxopropan-2-yl]-1,4,7,10-tetraazacyclododecan-1-yl}-5-{4-[2-(2-ethoxyethoxy)ethoxy]phenyl}pentanoate (1.10 g, 1.14 mmol) was dissolved in 1,4-dioxane (20 ml), some drops of water and HCl (4.54 ml, 4.0 M in dioxane, 18.2 mmol) added and heated to 80°C for 4h. The mixture was combined with the pilot experiment (100 mg) and evaporated to dryness under reduced pressure. A brown residue (1.68 g, >100%) was obtained, that was used as such in the next step.

LC-MS (Method 2): $R_t$ = 0.97min; MS (ESIpos): m/z = 802.2 $[M+H]^+$.

**Step 6**

(2S)-2-{7-[(1R)-1-carboxy-2-hydroxyethyl]-4,10-bis[(1S)-1-carboxy-2-hydroxyethyl]-1,4,7,10-tetraazacyclododecan-1-yl}-5-{4-[2-(2-ethoxyethoxy)ethoxy]phenyl}pentanoic acid

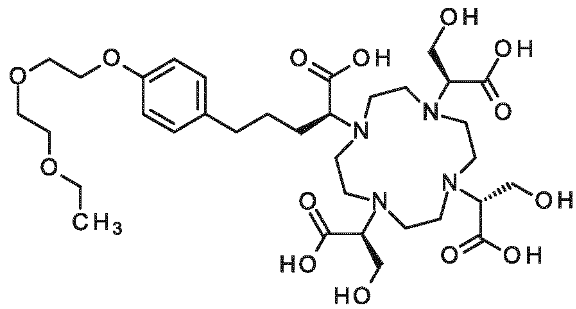

methyl (2S)-5-{4-[2-(2-ethoxyethoxy)ethoxy]phenyl}-2-{7 -[(2R)-3-hydroxy-1-methoxy-1-oxo-propan-2-yl]-4,10-bis[(2S)-3-hydroxy-1-methoxy-1-oxopropan-2-yl]-1,4,7,10-tetraazacyclo-dodecan-1-yl}pentanoate (900 mg, 1.12 mmol) was dissolved in THF (50 ml) and a solution of lithium hydroxide (161 mg, 6.74 mmol; [1310-65-2]) in water (50 ml) was added. The mixture was stirred at RT overnight. Another portion of lithium hydroxide (160 mg, 6.68 mmol) in water was added and the mixture heated to 80°C for 6h and overnight at RT. It was combined with an earlier reaction (90 mg) and evaporated to dryness under reduced pressure. The residue (2.0 g) was absorbed onto Isolute™ and purified by RP-chromatography (SNAP C18 120g, 50 ml/min, A = water/0.1% formic acid, B = MeCN, 250 nm, 0%B 6CV, 0%B to 50% B in 15CV). Interesting fractions were combined and lyophilized. The title compound was obtained in two fractions:

Fraction 1: 524.3 mg (0.704 mmol, 51% calc. from step 4)

Specific rotation: $\alpha_D^{20}$ = +12.5° +/- 0.9° (c=1, MeOH).

LC-MS (Method 2): $R_t$ = 0.79min; MS (ESIpos): m/z = 746 $[M+H]^+$

LC-MS (Method 3): Rt = 0.63min (100% DAD); MS (ESIpos): m/z = 373 $[M + 2H]^{++}$, 745 $[M + H]^+$.

$^1$H NMR ($D_2O$, 600 MHz): δ (ppm) 8.21 (s, 0.3H, formiate), 7.13-7.26 (m, 2H), 6.87-6.99 (m, 2H), 3.05-4.36 (m, 36H), 2.61 (br s, 2H), 1.60-1.96 (m, 4H), 1.14 (t, *J* = 7.0 Hz, 3H).

Fraction 2: 77.3 mg (0.104 mmol, 7.5% calc. from step 4).

Specific Rotation: $\alpha_D^{20}$ = +12.7° +/- 0.9° (c=1, MeOH).

LC-MS (Method 2): $R_t$ = 0.80min; MS (ESIpos): m/z = 745 $[M+H]^+$.

**Step 7**

gadolinium (2S,2'S)-2,2'-{4-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecane-1,7-diyl}bis(3-hydroxypropanoate)

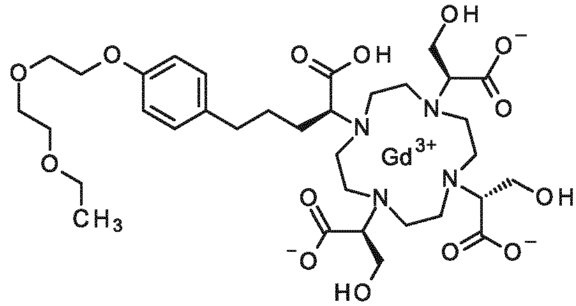

(2S)-2-{7-[(1R)-1-carboxy-2-hydroxyethyl]-4,10-bis[(1S)-1-carboxy-2-hydroxyethyl]-1,4,7,10-tetraazacyclododecan-1-yl}-5-{4-[2-(2-ethoxyethoxy)ethoxy]phenyl}pentanoic acid (482 mg, 647 μmol) was dissolved in water (15 ml) and $Gd_2O_3$ (106 mg, 291 μmol, 0.9 eq) were added. After stirring at 100°C for 8h, Chelex100™ (sodium form, ca. 5 g) was

added and the mixture stirred at RT overnight. After the xylenol-orange test indicated the absence of free gadolinium, the pH was adjusted to 8 (25% ammonium hydroxide) and the mixture (including resin) was loaded into an empty Biotage cartridge and directly subjected to RP-chromatography (SNAP C18 60g, 50 ml/min, A = water, B = MeCN, 275 nm, 0%B 6CV, 0%B to 13%B in 3.3CV, 13%B 5.8CV, 13%B to 60%B in 11.6CV). Interesting fractions were combined and lyophilized to give 294 mg (51 % yield) of the title compound.

Specific rotation: $\alpha_D^{20}$ = +2.9° +/- 0.2° (c=1, $H_2O$).

LC-MS (Method 2): Rt = 0.80min; MS (ESIpos): m/z = 900 $[M+H]^+$.

LC-MS (Method 3): Rt = 0.71min (98.5% DAD); MS (ESIpos): m/z = 450 $[M + 2H]^{++}$, 900 $[M + H]^+$ and some $[2M + 2H]^{++}$. The observed isotope pattern conforms to a gadolinium complex.

**Example 36**

**Step 1**

methyl (2S)-5-{4-[2-(2-ethoxyethoxy)ethoxy]phenyl}-2-{7-[2-oxooxolan-3-yl]-4,10-bis (2-oxooxolan-3-yl)-1,4,7,10-tetra-azacyclododecan-1-yl}pentanoate

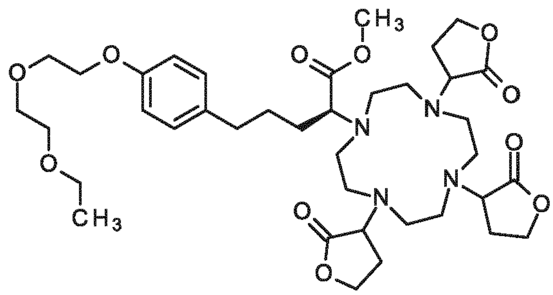

Under an atmosphere of $N_2$, racemic 3-hydroxyoxolan-2-one (350 mg, 3.43 mmol, [19444-84-9]) in DCM (11ml) was stirred at -70°C. 2,6-dimethylpyridine (460 μl, 3.9 mmol; [108-48-5]) was added, followed by the dropwise addition of trifluoromethanesulfonic anhydride (640 μl, 3.8 mmol). The mixture was then warmed to -40°C and stirred for 2h. In a separate reaction flask, methyl (2S)-5-{4-[2-(2-ethoxyethoxy)ethoxy]phenyl}-2-(1,4,7,10-tetraazacyclo-dodecan-1-yl) pentanoate, **(Example 15 Step 2,** 500 mg, 1.0 mmol), DIPEA (457 mg, 3.5 mmol) and MeCN (7.1 ml) were stirred under $N_2$ at -40°C. The two mixtures were combined at -40°C and allowed to warm to RT and stirred for 2d. The mixture was then added to water, extracted with DCM (2x), the organic phase washed with NaOH solution (0.1 M, aq. 2x), NaCl solution (sat. aq. 2x) and passed over a water repellent filter, and the mixture concentrated under reduced pressure. Due to incomplete alkylation, the crude mixture was then re-dissolved in MeCN (7.1 ml), $K_2CO_3$ was added (540 mg, 3.91 mmol; [584-08-7]). In a second flask, racemic 3-hydroxyoxolan-2-one (250 mg, 4.5 mmol, [19444-84-9]), THF (8.2 ml), and TEA (0.75 ml, 5.38 mmol) were stirred at 0°C, MsCl (0.21 ml, 2.7 mmol) was added dropwise, and the mixture stirred for 5min. The two mixtures were combined and stirred overnight at 60°C for 2d. The mixture was filtered and concentrated under reduced pressure yielding the crude product as a mixture of diastereomers and trialkylated by-products (1.02g).

LC-MS (Method 2): $R_t$ = 1.01min; MS (ESIpos): m/z = 747.7 $[M+H]^+$.

**Step 2**

(2S)-2-{7-[1-carboxy-3-hydroxypropyl]-4,10-bis[1-carboxy-3-hydroxypropyl]-1,4,7,10-tetraazacyclododecan-1-yl}-5-{4-[2-(2-ethoxyethoxy)ethoxy]phenyl}pentanoic acid

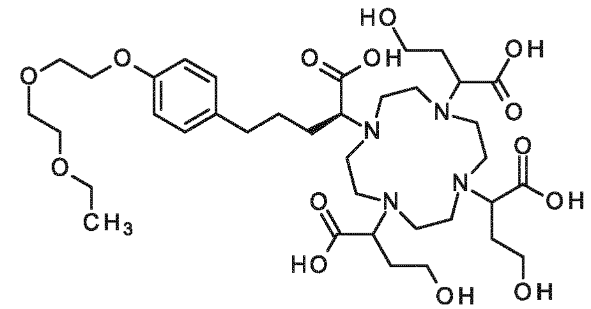

Crude product methyl (2S)-5-{4-[2-(2-ethoxyethoxy)ethoxy]phenyl}-2-{7-[2-oxooxolan-3-yl]-4,10-bis (2-oxooxolan-3-yl)-1,4,7,10-tetraazacyclododecan-1-yl}pentanoate (1.00 g, 1.34 mmol) was stirred in THF (21 ml), and a solution of NaOH (aq. 2.9 ml, 2.0 M, 5.9 mmol; [1310-73-2]) was added and the mixture stirred overnight at RT. Additional NaOH (5.4 ml, 2.0 M, 11 mmol; [1310-73-2]) was added and the mixture stirred for a further 1d. The mixture was neutralized with a solution of HCl (2N, aq.) and concentrated under reduced pressure yielding the crude product (1.0 g) also containing a trialkylated impurity.

LC-MS (Method 2): $R_t$ = 0.73min; MS (ESIpos): m/z = 787.7 $[M+H]^+$.

**Step 3**

gadolinium-2,2'-{4-[(1S)-1-carboxy-4-{4-[2-(2-ethoxyethoxy)ethoxy]phenyl}butyl]-10-[1-carboxylato-3-hydroxypropyl]-1,4,7,10-tetraazacyclododecane-1,7-diyl}bis(4-hydroxybutanoate)

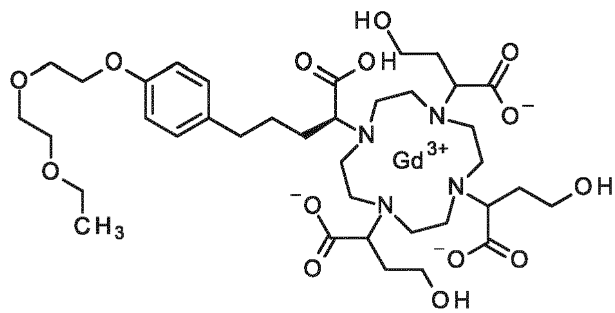

Crude, (2S)-2-{7-[1-carboxy-3-hydroxypropyl]-4,10-bis[1-carboxy-3-hydroxypropyl]-1,4,7,10-tetraazacyclododecan-1-yl}-5-{4-[2-(2-ethoxyethoxy)ethoxy]phenyl}pentanoic acid (1.0 g, 1.27 theoretical mmol), $Gd_2O_3$ (226 mg, 623 µmol; [12064-62-9]) and water (13 ml) were stirred at 105°C for 2d. Chelex100™(Sodium form, washed, approximately 5 g) was added, the mixture stirred for 1h, filtered and the solution purified by direct injection of the aqueous mixture by RP C18 chromatography (Biotage C18, 120 g, 50 ml/min, A = water, B = MeCN, 0%B to 50%B) yielding the title compound as a mixture of diastereomers (52.5 mg, 4 % yield over 3 steps).

LC-MS (Method 2): $R_t$ = 0.59min; MS (ESIpos): m/z = 942.6 $[M+H]^+$. Isotope pattern conforms to a gadolinium complex.

**Example 37**

gadolinium-2,2'-{4-[1-carboxy-2-{4-[2-(2-ethoxyethoxy)ethoxy]phenyl}ethyl]-10-[1-carboxylato-3-hydroxypropyl]-1,4,7,10-tetraazacyclododecane-1,7-diyl}bis(4-hydroxybutanoate)

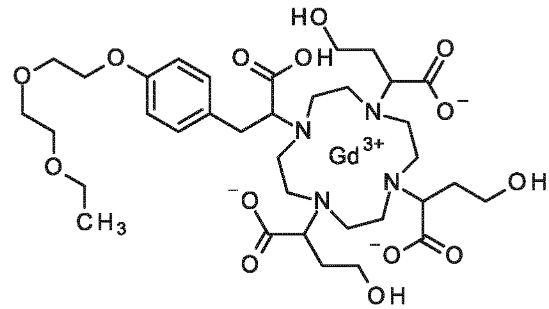

The title compound, present as a mixture of diastereomers, was produced by the methods described herein from **Intermediate 2.**

LC-MS (Method 3): Rt = 0.60 - 0.66min; MS (ESIpos): m/z = 457.6 $[M+2H]^{++}$, 914.3 $[M+H]^{+}$. Isotope pattern corresponds to anticipated gadolinium complex.

## Example 38

### Step 1

ethyl-2-{4, 1 0-bis(2- tert-butoxy-2-oxoethyl)-7 -[1-ethoxy-3-methoxy-1-oxopropan-2-yl]-1,4,7,10-tetraazacyclododecan-1-yl}-3-[4-(2-ethoxyethoxy)phenyl]propanoate

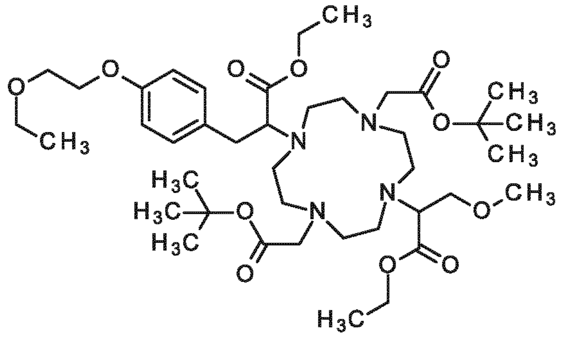

Ethyl-2-[4,10-bis(2-tert-butoxy-2-oxoethyl)-1,4,7,10-tetraazacyclododecan-1-yl]-3-[4-(2-ethoxyethoxy)phenyl] propanoate **(Example 7 - Step 2,** 1.95 g, 2.93 mmol) and ethyl-2-[(methanesulfonyl)oxy]-3-methoxypropanoate **(Intermediate 25,** 663 mg, 2.93 mmol), $K_2CO_3$ (810 mg, 5.86 mmol; [584-08-7]) and MeCN (9.6 ml) were heated at 70°C for 24h. The mixture was filtered, and filter cake washed with EtOH, the organic filtrates combined and concentrated under reduced pressure yielding the crude title compound as a mixture of diastereomers (2.5 g).

LC-MS (Method 2): $R_t$ = 1.20 - 1.40min; MS (ESIpos): m/z = 795.9 $[M+H]^{+}$.

### Step 2

2,2'-(4-{1-ethoxy-3-[4-(2-ethoxyethoxy)phenyl]-1-oxopropan-2-yl}-10-[1-ethoxy-3-methoxy-1-oxopropan-2-yl]-1,4,7,10-tetraazacyclododecane-1,7-diyl)diacetic acid

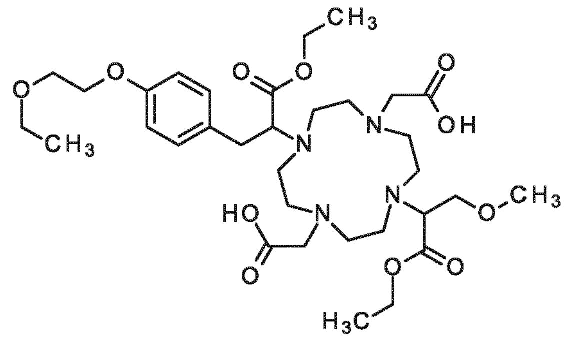

Crude ethyl-2-{4,10-bis(2-tert-butoxy-2-oxoethyl)-7-[(2SR)-1-ethoxy-3-methoxy-1-oxopropan-2-yl]-1,4,7,10-tetraazacyclododecan-1-yl}-3-[4-(2-ethoxyethoxy)phenyl]propanoate 2.50 g, 3.14 mmol) was stirred in formic acid (25 ml) at 60°C overnight. The reaction was concentrated under reduced pressure and purified by RP-chromatography (Biotage ULTRA C18, 60 g, 50 ml/min, A = water, B = MeCN, 0%B to 100%B) affording the title compound as a mixture of diastereomers (319 mg, 14 % yield).

LC-MS (Method 2): $R_t$ = 0.73min; MS (ESIpos): m/z = 683.6 $[M+H]^+$.

$^1$H NMR (DMSO-$d_6$, 400 MHz): $\delta$ (ppm) 7.09-7.35 (m, 2H), 6.75-6.91 (m, 2H), 3.74-4.23 (m, 8H), 3.60-3.71 (m, 4H), 3.42-3.56 (m, 4H), 3.14-3.34 (m, 7H), 2.55-3.13 (m, 16H), 0.98-1.24 (m, 9H).

**Step 3**

gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]-3-methoxypropanoate

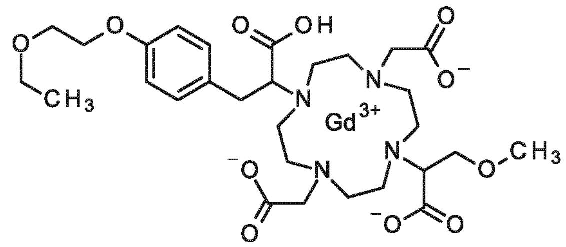

2,2'-(4-{1-ethoxy-3-[4-(2-ethoxyethoxy)phenyl]-1-oxopropan-2-yl}-10-[(2SR)-1-ethoxy-3-methoxy-1-oxopropan-2-yl]-1,4,7,10-tetraazacyclododecane-1,7-diyl)diacetic acid (319 mg, 467 μmol), $Gd_2O_3$ (76.1 mg, 210 μmol) and water were heated and stirred at 105°C in a crimp sealed reaction vessel, followed by heating for 5h at 120°C. The mixture was treated with Chelex100™(sodium form, washed, approximately 5 g), stirred for 1h, following which it was filtered, concentrated to dryness and purified by RP-chromatography (Biotage ULTRA C18 30g, 25 ml/min, A = water, B = MeCN, 0%B to 50%B in 25min,100%B 10min) yielding the title compound as a mixture of diastereomers (54.8 mg, 90 % purity, 15 % yield).

LC-MS (Method 3): $R_t$ = 0.57min (34%% DAD) and 0.63min (48% DAD); MS (ESIpos): m/z = 391.7 $[M+2H]^{++}$, 781.4 $[M+H]^+$. Isotope patterns match theoretical values for corresponding gadolinium complex.

**Example 39**

gadolinium 2,2',2''-{10-[2-{3,5-bis[2-(2-ethoxyethoxy)ethoxy]phenyl}-1-carboxyethyl]-1,4,7,10-tetraazacyclododecane-1,4,7-triyl}triacetate

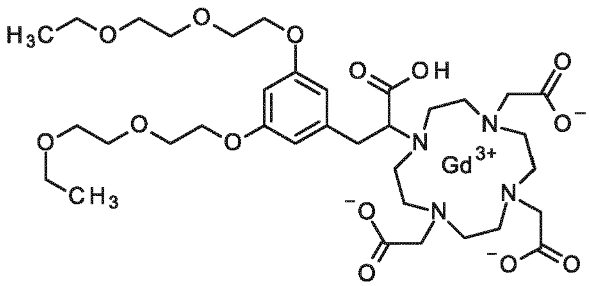

The title compound was produced by the methods described herein from 2-(2-ethoxyethoxy)EtOH ([111-90-0]) and 3,5-dihydroxybenzaldehyde ([26153-38-8]).

LC-MS (Method 3): Rt = 0.73min; MS (ESIpos): m/z = 457.8 $[M+2H]^{++}$, 914.6 $[M+H]^{+}$. Isotope patterns match theoretical values for corresponding gadolinium complex.

**Example 40**

gadolinium 2,2',2"-{10-[(1S)-2-(2,4-bis{2-[2-(2-ethoxyethoxy)ethoxy]ethoxy}phenyl)-1-carboxyethyl]-1,4,7,10-tetraazacyclododecane-1,4,7-triyl}triacetate

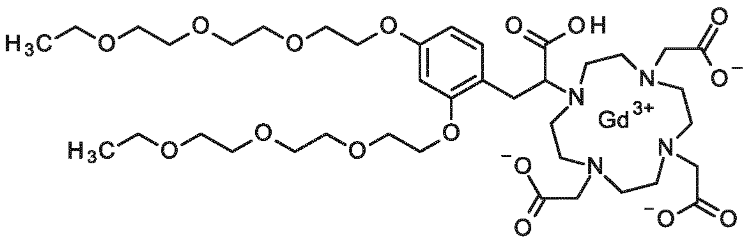

The title compound was produced by the methods described herein from 2-[2-(2-ethoxyethoxy)ethoxy]EtOH ([112-50-5]) and 2,4-dihydroxybenzaldehyde ([95-01-2]).

LC-MS (Method 3): $R_t$ = 0.82min; MS (ESIpos): m/z = 501.8 $[M+2H]^{++}$, 1002.6 $[M+H]^{+}$. Isotope patterns match theoretical values for corresponding gadolinium complex.

**Example 41**

gadolinium-2-{7-[1-carboxy-2-{4-[2-(2-ethoxyethoxy)ethoxy]phenyl}ethyl]-4,10-bis(carboxylatomethyl)-1,4,7,10-tetraazacyclododecan-1-yl}-3-hydroxypropanoate

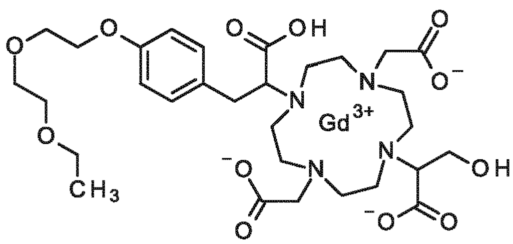

The title compound, present as a mixture of diastereomers, was produced by the methods described herein from **Intermediate 2, Intermediate 27,** and di-tert-butyl 2,2'-(1,4,7,10-tetraazacyclododecane-1,7-diyl)diacetate [162148-48-3].

LC-MS (Method 3): Rt = 0.64min; MS (ESIpos): m/z = 406.7 $[M+2H]^{++}$, 811.4 $[M+H]^{+}$. Isotope patterns match theoretical values for corresponding gadolinium complex.

**Example 42**

gadolinium-2-{7-[1-carboxy-2-(4-{2-[2-(2-ethoxyethoxy)ethoxy]ethoxy}phenyl)ethyl]-4,10-bis(carboxylatomethyl)-1,4,7,10-tetraazacyclododecan-1-yl}-3-hydroxypropanoate

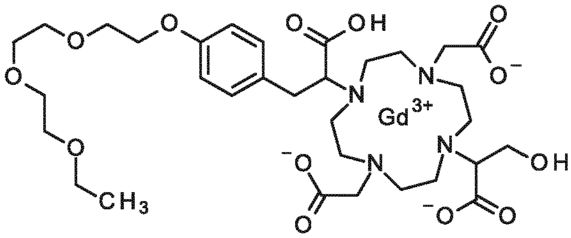

The title compound, present as a mixture of diastereomers, was produced by the methods described herein from **Intermediate 4,** and **Intermediate 27.**

LC-MS (Method 3): $R_t$ = 0.62 - 0.66min; MS (ESIpos): m/z = 428.7 $[M+2H]^{++}$, 856.4 $[M+H]^{+}$. Isotope patterns match theoretical values for corresponding gadolinium complex.

**Example 43**

gadolinium-2-{4,10-bis(carboxylatomethyl)-7-[1-carboxypropyl]-1,4,7,10-tetraazacyclododecan-1-yl}-3-{4-[2-(2-ethoxyethoxy)ethoxy]phenyl}propanoate

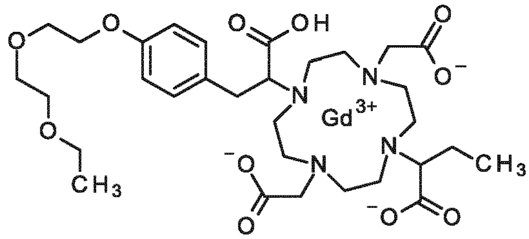

The title compound, present as a mixture of diastereomers, was produced by the methods described herein from **Intermediate 5,** di-tert-butyl 2,2'-(1,4,7,10-tetraazacyclododecane-1,7-diyl)diacetate [162148-48-3] and racemic ethyl-2-hydroxybutanoate ([ 52089-54-0]).

LC-MS (Method 3): $R_t$ = 0.60 - 0.64min; MS (ESIpos): m/z = 405.7 $[M+2H]^{++}$, 810.5 $[M+H]^{+}$. Isotope patterns match theoretical values for corresponding gadolinium complex.

**Example 44**

gadolinium 2,2',2''-{10-[2-(5-butoxypyridin-2-yl)-1-carboxyethyl]-1,4,7,10-tetraazacyclododecane-1,4,7-triyl}triacetate

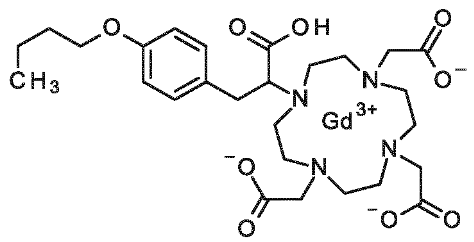

The title compound was produced by the methods described herein from 5-butoxypyridine-2-carbaldehyde

([ 66933-06-0]).

LC-MS (Method 3): $R_t$ = 0.44min; MS (ESIpos): m/z = 362.2 $[M+2H]^{++}$, 723.3 $[M+H]^+$. Isotope patterns match theoretical values for corresponding gadolinium complex.

## Example 45

gadolinium-2-{4,10-bis(carboxylatomethyl)-7-[1-carboxy-2-methoxyethyl]-1,4,7,10-tetraazacyclododecan-1-yl}-3-[4-(2,2,3,3-tetrafluoropropoxy)phenyl]propanoate

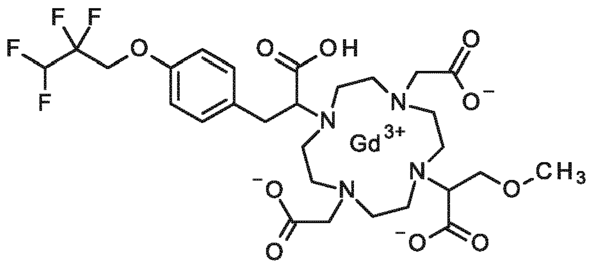

The title compound, present as a mixture of diastereomers, was produced by the methods described herein from **Intermediate 23, Intermediate 25** and di-tert-butyl 2,2'-(1,4,7,10-tetraazacyclododecane-1,7-diyl)diacetate [162148-48-3].

LC-MS (Method 3): $R_t$ = 0.72 - 0.77min; MS (ESIpos): m/z = 412.6 $[M+2H]^{++}$, 824.2 $[M+H]^+$. Isotope patterns match theoretical values for corresponding gadolinium complex.

## Example 46

gadolinium 2,2',2"-(10-{1-carboxy-2-[4-(2,2,3,3-tetrafluoropropoxy)phenyl]ethyl}-1,4,7,10-tetraazacyclododecane-1,4,7-triyl)triacetate

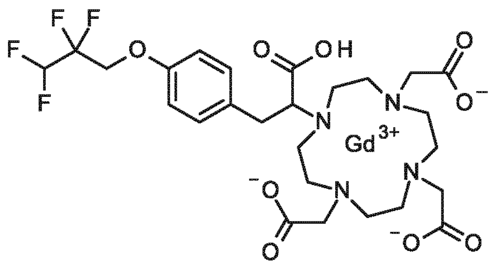

The title compound was produced by the methods described herein from Intermediate 23.

LC-MS (Method 3): $R_t$ = 0.71min; MS (ESIpos): m/z = 390.5 $[M+2H]^{++}$, 780.0 $[M+H]^+$. Isotope patterns match theoretical values for corresponding gadolinium complex.

## Example 47

gadolinium 2,2',2"-(10-{(1R)-1-carboxy-2-[4-(2,2,2-trifluoroethoxy)phenyl]ethyl}-1,4,7,10-tetraazacyclododecane-1,4,7-triyl)triacetate

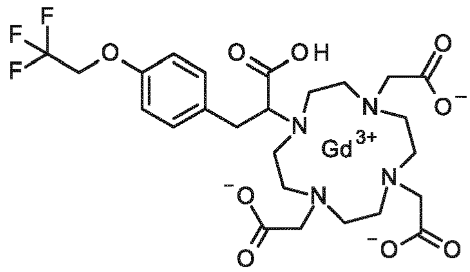

The title compound was produced by the methods described herein from 4-(2,2,2-trifluoroethoxy)-benzaldehyde ([76579-46-9]).

LC-MS (Method 3): Rt = 0.66min; MS (ESIpos): m/z = 374.6 $[M+2H]^{++}$, 748.2 $[M+H]^{+}$. Isotope patterns match theoretical values for corresponding gadolinium complex.

**Example 48**

gadolinium 2,2',2"-{10-[1-carboxy-2-(4-propoxyphenyl)ethyl]-1,4,7,10-tetraazacyclododecane-1,4,7-triyl}triacetate

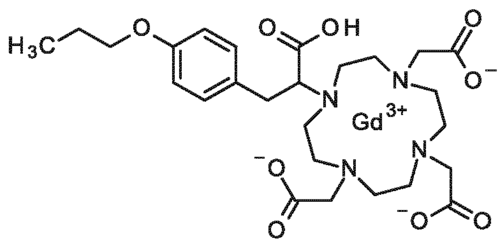

The title compound was produced by the methods described herein from 4-propoxy-benzaldehyde ([5736-85-6]).

LC-MS (Method 3): Rt = 0.64min; MS (ESIpos): m/z = 354.7 $[M+2H]^{++}$, 708.3 $[M+H]^{+}$. Isotope patterns match theoretical values for corresponding gadolinium complex.

**Example 49**

**Step 1**

methyl (2S)-2-{7-[(2R)-3-tert-butoxy-1-methoxy-1-oxopropan-2-yl]-4-[(2S)-3-tert-butoxy-1-methoxy-1-oxopropan-2-yl]-1,4,7,10-tetraazacyclododecan-1-yl}-5-{4-[2-(2-ethoxyethoxy) ethoxy]phenyl}pentanoate

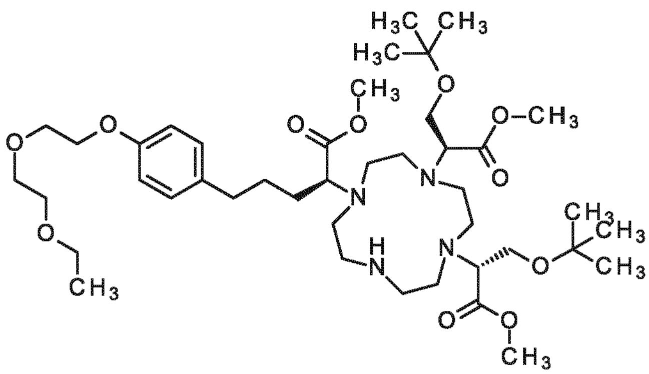

methyl (2S)-2-{7-[(2R)-3-tert-butoxy-1-methoxy-1-oxopropan-2-yl]-1,4,7,10-tetraazacyclodo-decan-1-yl}-5-{4-[2-(2-ethoxyethoxy)ethoxy]phenyl}pentanoate, **(Example 35** - **Step 3),** (960 mg, 1.47 mmol) was dissolved in

$CHCl_3$ (40 ml) and DIPEA( 260 µl, 1.5 mmol) added. Methyl (2R)-3-tert-butoxy-2-[(trifluoromethanesulfonyl)oxy]propanoate, **Intermediate 27,** (363 mg, 1.18 mmol) in $CHCl_3$ (1 ml) was added and the mixture stirred at RT overnight. The reaction was combined with the pilot experiment (100 mg), washed with water and sat NaCl, filtered through a water repellent filter and concentrated under reduced pressure. The residue (1.66g) was absorbed onto Isolute and subjected to RP-chromatography (SNAP C18 120 g, 50 ml/min, A = water/0.1% formic acid, B = MeCN, 10%B 2CV, 10%B to 26%B in2.6CV, 25%B 3.8CV, 26%B to 45%B in 5.2CV, 45%B 4.4CV, 45%B to 80%B in 10CV, 80%B 2CV, 225/276 nm). Interesting fractions were pooled, the MeCN removed under reduced pressure and the product extracted with DCM. Three fractions were obtained:

Fraction 1: 330 mg (31%, Unreacted starting material).

Fraction 3: 60 mg (4%, double alkylation product).

Fraction 2: 320 mg (24%, title compound).

Specific rotation: $a_D^{20}$ = 13.32° +/- 0.40° (c=1, $CHCl_3$).

LC-MS (Method 2): $R_t$ = 1.40 min; MS (ESIpos): m/z = 812 $[M+H]^+$.

LC-MS (Method 3): $R_t$ = 1.20 min (96% DAD); MS (ESIpos): m/z = 406 $[M + 2H]^{++}$, 811 $[M + H]^+$.

$^1H$ NMR ($CDCl_3$, 400 MHz): δ (ppm) 7.05 (d, *J* = 7.3 Hz, 2H), 6.79-6.84 (m, 2H), 4.10 (dd, *J* = 5.6, 4.3 Hz, 2H), 3.84 (dd, *J* = 5.6, 4.3 Hz, 2H), 3.49-3.76 (m, 21H), 2.53-3.35 (m, 19H), 1.81-2.17 (m, 4H), 1.21 (t, *J* = 7.0 Hz, 3H), 1.17 (s, 9H), 1.13-1.16 (m, 9H).

$^{13}C$ NMR ($CDCl_3$, 101 MHz): δ (ppm) 173.3, 172.0, 171.5, 157.4, 134.4, 129.6 (2C), 114.9 (2C), 74.5, 74.0, 71.2, 70.2, 70.1, 67.8, 67.1, 65.3 (br, 2C), 62.0 (br), 61.6 (br), 60.6 (br), 53.8 (br), 52.2 (br), 52.1, 52.0, 51.9, 50.1 (br), 49.0 (br), 48.2 (br), 47.7 (br), 46.7 (br), 35.1, 30.2 (br), 29.3, 27.8, 27.8, 15.5.

**Step** 2

methyl (2S)-2-{4,7-bis[(2R)-3-tert-butoxy-1-methoxy-1-oxopropan-2-yl]-10-[(2S)-3-tert-butoxy-1-methoxy-1-oxopropan-2-yl]-1,4,7,10-tetraazacyclododecan-1-yl}-5-{4-[2-(2-ethoxyethoxy) ethoxy]phenyl}pentanoate

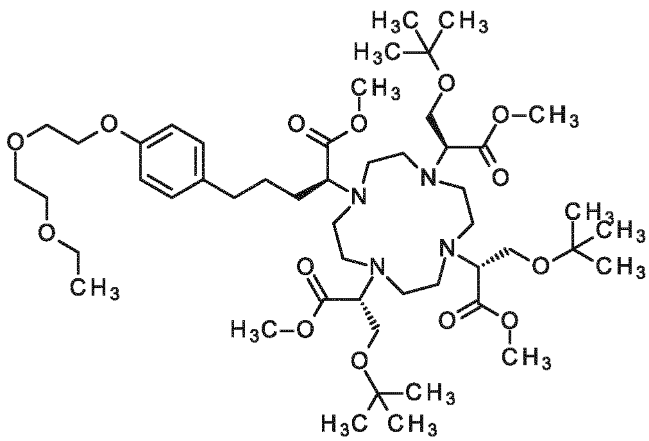

Methyl (2S)-2-{7-[(2R)-3-tert-butoxy-1-methoxy-1-oxopropan-2-yl]-4-[(2S)-3-tert-butoxy-1-methoxy-1-oxopropan-2-yl]-1,4,7,10-tetraazacyclododecan-1-yl}-5-{4-[2-(2-ethoxyethoxy) ethoxy]phenyl}pentanoate (259 mg, 320 µmol) was dissolved in MeCN, $K_2CO_3$ (66.3 mg, 480 µmol) and methyl (2S)-3-tert-butoxy-2-[(trifluoromethanesulfonyl)oxy]propanoate, **Intermediate 28,** (160 mg, 416 µmol) added. The mixture was stirred at RT over night and was combined with the pilot experiment (13 mg). The solids were removed by filtration, the filtrate concentrated under reduced pressure and the residue partitioned between DCM and water. The organic phase was washed with sat. NaCl, dried ($Na_2SO_4$) and concentrated to give 556.1 mg of the title compound as brown residue. The raw material was used as such in

the next step.

**Step 3**

methyl (2S)-2-{4,7-bis[(2R)-3-hydroxy-1-methoxy-1-oxopropan-2-yl]-10-[(2S)-3-hydroxy-1-methoxy-1-oxopropan-2-yl]-1,4,7,10-tetraazacyclododecan-1-yl}-5-{4-[2-(2-ethoxyethoxy) ethoxy]phenyl}pentanoate

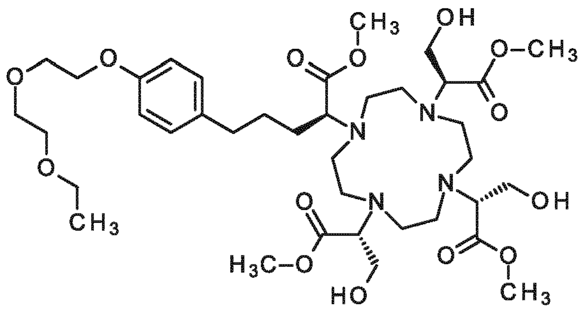

methyl (2S)-2-{4,7-bis[(2R)-3-tert-butoxy-1-methoxy-1-oxopropan-2-yl]-10-[(2S)-3-tert-butoxy-1-methoxy-1-oxopropan-2-yl]-1,4,7,10-tetraazacyclododecan-1-yl}-5-{4-[2-(2-ethoxyethoxy) ethoxy]phenyl}pentanoate (545 mg) and HCl (1.3 ml, 4.0 M, in dioxane, 5.1 mmol) and some drops of water were stirred at 80°C for 4h. The mixture was concentrated under reduced pressure and combined with the raw product of an earlier experiment (11 mg) to give 324 mg of the title compound as red-brown solid, which was used as such in the next step.

**Step 4**

(2S)-2-{4,7-bis[(1R)-1-carboxy-2-hydroxyethyl]-10-[(1S)-1-carboxy-2-hydroxyethyl]-1,4,7,10-tetraazacyclododecan-1-yl}-5-{4-[2-(2-ethoxyethoxy)ethoxy]phenyl}pentanoic acid

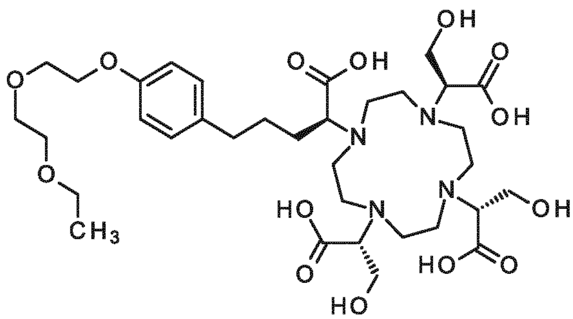

methyl (2S)-2-{4,7-bis[(2R)-3-hydroxy-1-methoxy-1-oxopropan-2-yl]-10-[(2S)-3-hydroxy-1-methoxy-1-oxopropan-2-yl]-1,4, 7, 1 0-tetraazacyclododecan-1-yl}-5-{4-[2-(2-ethoxyethoxy) ethoxy]phenyl}pentanoate (324 mg, 330 μmol) was dissolved in THF (2.0 ml) and a solution of lithium hydroxide (47.4 mg, 1.98 mmol) in water (2.0 ml) was added. The reaction was complete after stirring at RT overnight and the mixture evaporated to dryness under reduced pressure. The residue was absorbed on to Isolute and subjected to RP-chromatography. (SNAP C18 120 g, 50 ml/min, A = water/0.1% formic acid, B = MeCN, 0%B 5CV, 0%B to 20%B in 6CV, 20%B 1CV, 20 to 50%B in 9CV, 228/270 nm). Interesting fractions were combined and lyophilized to 118 mg (47%, calc. from step 2) of the title compound.

LC-MS (Method 2): Rt = 0.75 min; MS (ESIpos): m/z = 745 $[M+H]^+$.

**Step 5**

gadolinium (2R,2'R)-2,2'-{7-[(1S)-1-carboxy-4-{4-[2-(2-ethoxyethoxy)ethoxy]phenyl}butyl]-10-[(1S)-1-carboxylato-2-hydroxyethyl]-1,4,7,10-tetraazacyclododecane-1,4-diyl}bis(3-hydroxypropanoate)

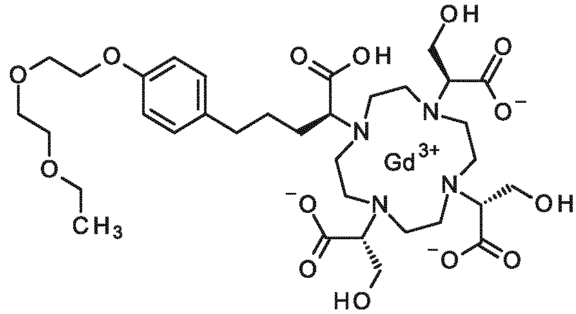

(2S)-2-{4,7-bis[(1R)-1-carboxy-2-hydroxyethyl]-10-[(1S)-1-carboxy-2-hydroxyethyl]-1,4,7,10-tetraazacyclododecan-1-yl}-5-{4-[2-(2-ethoxyethoxy)ethoxy]phenyl}pentanoic acid (116 mg, 156 μmol) and $Gd_2O_3$ (25.4 mg, 70.1 μmol) were placed in water (2.0 ml) and heated at 100°C for 8h. Chelex100 (sodium form, ca. 3 g) was added and the mixture stirred at RT for 4h. The pH was adjusted to 6 (ammonium hydroxide, 25% in water) and filtered. The filtrate was concentrated under reduced pressure and purified by RP-chromatography (SNAP C18 30 g, 25 ml/min, A = water, B = MeCN, 0%B 6CV, 0%B to 50%B 15CV, 216/275 nm). Interesting fraction were combined to give the title compound (95.2 mg, 67 %).

LC-MS (Method 2): $R_t$ = 0.76 min; MS (ESIpos): m/z = 899 $[M+H]^+$.

LC-MS (Method 3): Rt = 0.69 min (100% DAD); MS (ESIpos): m/z = 450 $[M + 2H]^{++}$, 600 $[2M + 3H]^{3+}$, 891 $[2M +2H - H_2O]^{2+}$, 900 $[M + H]^+$, 1349 $[3M + 2H]^{2+}$. The observed isotope patterns conform to a gadolinium complex.

**Example 50**

**Step 1**

methyl (2R)-3-tert-butoxy-2-(4,7-dibenzyl-1,4,7,10-tetraazacyclododecan-1-yl)propanoate

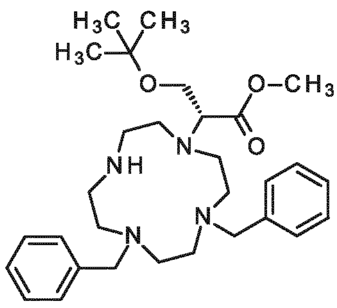

1,4-dibenzyl-1,4,7,10-tetraazacyclododecane (1.20 g, 3.40 mmol; [216101-03-0]) was dissolved in MeCN (20 ml), DIPEA (890 μl, 5.1 mmol), sodium trifluormethanesulfonate (586 mg, 3.40 mmol) and a solution of methyl (2S)-3-tert-butoxy-2-[(trifluoromethanesulfonyl)oxy]propanoate, **Intermediate 28,** (735 mg, 2.38 mmol) in MeCN (1 ml) added. The mixture was stirred at RT for 5h. The mixture was combined with the previous experiment (127 mg) and concentrated under reduced pressure. The residue (3.5 g) was partitioned between DCM and water. The aqueous phase was extracted with DCM, the combined organic phases washed with sat. NaCl, filtered through a water repellent and concentrated under reduced pressure to give 2.10 g of the title compound as red-brown raw product. It was absorbed onto Isolute™ and subjected to RP-chromatography (SNAP C18 120 g, 50 ml/min, A=water/0.1% formic acid, B = MeCN, 5%B 3CV, 5%B to 8%B in 0.9CV, 8%B 2.1V, 8%B to 16%B 2.7CV, 16%B 2.1CV, 16%B to 22%B in 2.1CV, 22%B 1.5CV, 22%B to 25%B in 0.8CV, 25%B 1.2CV, 25%B to 50%B in 8.3CV, 50%B to 60%B in 5CV, 210 nm). Interesting fractions were combined and

lyophilized to give 1.126 g (65%) of the title compound in three fractions:

Fraction 1: 227 mg (13%).

Specific Rotation: 1.7° (CHCL3, 20°C, 589 nm).

LC-MS (Method 3): Rt = 0.69 min (94% DAD); MS (ESIpos): m/z = 256 [M + 2H]$^{++}$, 455 [M + H - $C_4H_8$]$^+$, 511 [M + H]$^+$.

$^1$H NMR ($CDCl_3$, 400 MHz): δ (ppm) 7.23-7.36 (m, 8H), 7.07 (d, br, J = 7 Hz, 2H), 3.59-3.80 (m, 9H), 2.51-3.51 (m, 17H), 1.17 (s, 9H).

$^{13}$C NMR ($CDCl_3$, 101 MHz): δ (ppm) 171.7, 138.4, 135.4 (br), 129.8 (br, 2C), 129.6 (2C), 128.5 (2C), 128.4 (2C), 127.8, 127.4 (br), 73.6, 67.0 (br), 62.7 (br), 61.4 (br), 51.6 (br, 2C), 51.1 (br), 50.6 (br, 2C), 50.2 (br), 50.0 (br), 49.2 (br), 48.8 (br), 48.0 (br), 27.3 (3C).

Fraction 2: 516 mg (30%)

Specific Rotation: -7.8° (CHCL3, 20°C, 589 nm).

LC-MS (Method 2): Rt = 0.86 min; MS (ESIpos): m/z = 511 [M+H]$^+$.

LC-MS (Method 3): Rt = 0.70 min (100% DAD); MS (ESIpos): m/z = 256 [M + 2H]$^{++}$, 455 [M + H - $C_4H_8$]$^+$, 511 [M + H]$^+$.

$^1$H NMR ($CDCl_3$, 400 MHz): δ (ppm) 6.94-7.47 (m, 10H), 2.55-4.29 (m, 26H), 1.16 (br s, 9H).

Fraction 3: 383 mg (22%)

Specific Rotation: -7.7° (CHCL3, 20°C, 589 nm).

LC-MS (Method 2): $R_t$ = 0.85 min; MS (ESIpos): m/z = 511 [M+H]$^+$.

LC-MS (Method 3): Rt = 0.69 min (95% DAD); MS (ESIpos): m/z = 256 [M + 2H]$^{++}$, 455 [M + H - $C_4H_8$]$^+$, 511 [M + H]$^+$.

Despite their different retention times, all fractions behaved in an identical way in the subsequent transformations.

**Step 2**

methyl (2S)-2-{4,7-dibenzyl-10-[(2R)-3-tert-butoxy-1-methoxy-1-oxopropan-2-yl]-1,4,7,10-tetraazacyclododecan-1-yl}-5-{4-[2-(2-ethoxyethoxy)ethoxy]phenyl}pentanoate

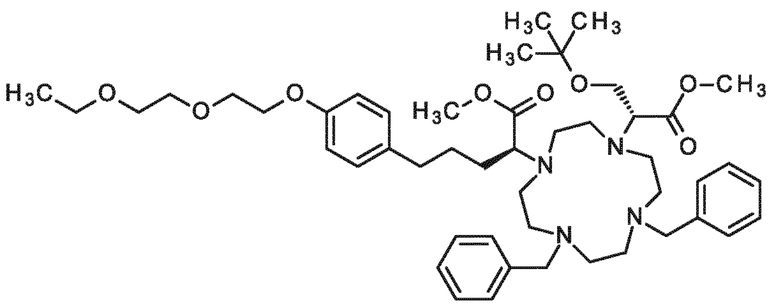

methyl (2R)-3-tert-butoxy-2-(4,7-dibenzyl-1,4,7,10-tetraazacyclododecan-1-yl)propanoate (450 mg, 881 μmol, Fraction 2, of step 1) was dissolved in MeCN in (15 ml) and potassium carbonate (207 mg, 1.50 mmol) added. A solution of methyl (2R)-5-{4-[2-(2-ethoxyethoxy)ethoxy]phenyl}-2-[(trifluoromethanesulfonyl)oxy]pentanoate (Example 15 -Step 1), (781 mg, 1.32 mmol) in MeCN (1 ml) was added and the mixture stirred at 60°C for 3h. The mixture was combined with the

two previous experiments (10.5 mg each), the solids removed by filtration, the filtrate and washings (MeCN) combined and concentrated under reduced pressure. The residue (1.2 g) was partitioned between DCM and water. The aqueous phase was extracted with DCM, the combined organic phases washed with sat. NaCl, filtered through a water repellent and concentrated under reduced pressure to give 727 mg of the title compound as red-brown raw product. 541 mg (other fractions of step 1) were treated in the same way to give further 1.51 g raw product, which were used as such in the next step.

**Step** 3

methyl (2S)-2-{4-[(2R)-3-tert-butoxy-1-methoxy-1-oxopropan-2-yl]-7,10-bis[(2S)-3-tert-butoxy-1-methoxy-1-oxopropan-2-yl]-1,4,7,10-tetraazacyclododecan-1-yl}-5-{4-[2-(2-ethoxyethoxy) ethoxy]phenyl}pentanoate

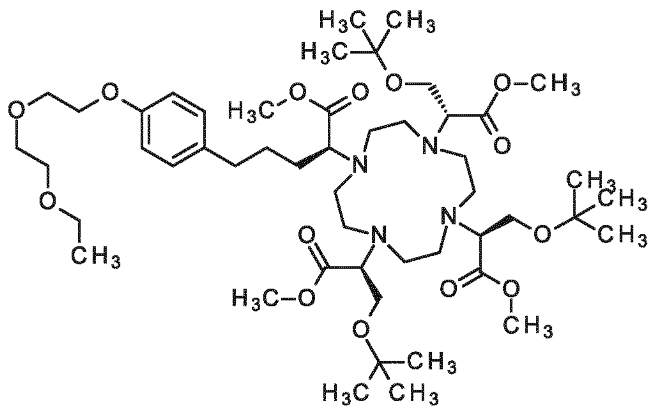

methyl (2S)-2-{4-[(2R)-3-tert-butoxy-1-methoxy-1-oxopropan-2-yl]-1,4,7,10-tetraazacyclodo-decan-1-yl}-5-{4-[2-(2-ethoxyethoxy)ethoxy]phenyl}pentanoate (1.65 g, 2.40 mmol) was dissolved in MeCN (30 ml) and potassium carbonate (1.16 g, 8.40 mmol) added, followed by a solution of methyl (2R)-3-tert-butoxy-2-[(trifluoromethanesulfonyl) oxy]propanoate, **Intermediate 26,** (2.78 g, 7.20 mmol) in MeCN (50 ml). The mixture was stirred at RT overnight, the solids removed by filtration and the filtrate and washings (MeCN) combined and concentrated under reduced pressure. The residue (6.3 g) was partitioned between DCM and water. The aqueous phase was extracted with DCM, the combined organic phases washed with sat. NaCl, filtered through a water repellent and concentrated under reduced pressure to give 3.4 g red-brown raw product. It was combined with the material from a previous experiment (400 mg from 223 mg), dissolved in DCM, absorbed onto Isolute™ and subjected to RP-chromatography (SNAP C18 120 g, 50 ml/min, A=water/0.1% ammonia, B = MeCN, C = MeOH, 10%B 3CV, 10%B to 100%B in 20CV, 100%B 5.7CV, 100%C 5CV, 220 nm). Interesting fractions were combined and lyophilized to give 332 mg (17%) of the title product.

Specific rotation: $\alpha_D^{20}$ = -17.28° +/- 0.62° (c=1, chloroform).

LC-MS (Standard Agilent): Rt = 1.27 min (67% DAD); MS (ESIpos): m/z = 485 $[M + 2H]^{++}$, 970 $[M + H]^+$; 1.32 min (22% DAD); 485 $[M + 2H]^{++}$, 970 $[M + H]^+$.

$^1$H NMR ($CDCl_3$, 400 MHz): δ (ppm) 7.06 (d, J = 8.6 Hz, 2H), 6.78-6.86 (m, 2H), 4.08-4.12 (m, 2H), 3.82-3.87 (m, 2H), 3.59-3.77 (m, 20H), 3.47-3.57 (m, 7H), 3.25-3.34 (m, 1H), 2.42-3.01 (m, 16H), 1.89-2.02 (m, 2H), 1.53-1.78 (m, 4H), 1.21 (t, J = 7.0 Hz, 3H), 1.11-1.18 (m, 27H).

$^{13}$C NMR ($CDCl_3$, 101 MHz): δ (ppm) 174.8 (br), 173.3 (br, 3C), 157.8, 135.4, 130.2 (2C), 115.4 (2C), 74.0 (br, 2C), 74.0 (br), 71.8, 70.8, 70.8, 68.4, 67.7, 64.7 (br), 64.7 (br), 64.2 (br), 64.1 (br), 62.8 (br), 62.7 (br), 62.2 (br), 52.7 (br, 2C), 52.2 (br, 2C), 52.0 (br), 51.9, 51.9, 51.8 (br, 2C), 51.7 (br), 51.4 (br), 50.8 (br), 35.9 (br), 30.7 (br), 29.5 (br), 28.3 (3C), 28.3 (6C), 16.1.

**Step 4**

methyl (2S)-5-{4-[2-(2-ethoxyethoxy)ethoxy]phenyl}-2-{4-[(2R)-3-hydroxy-1-methoxy-1-oxopropan-2-yl]-7,10-bis [(2S)-3-hydroxy-1-methoxy-1-oxopropan-2-yl]-1,4,7,10-tetraazacyclododecan-1-yl}pentanoate

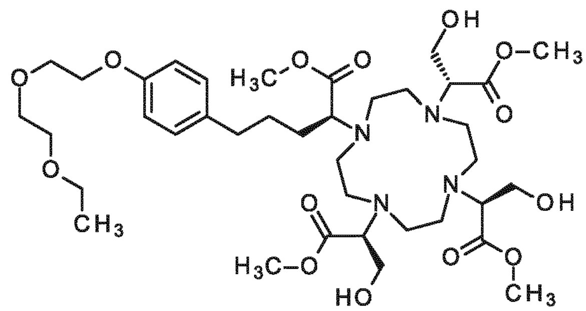

methyl (2S)-2-{4-[(2R)-3-tert-butoxy-1-methoxy-1-oxopropan-2-yl]-7,10-bis[(2S)-3-tert-butoxy-1-methoxy-1-oxopropan-2-yl]-1,4,7,10-tetraazacyclododecan-1-yl}-5-{4-[2-(2-ethoxyethoxy) ethoxy]phenyl}pentanoate (332 mg, 343 μmol) was dissolved in 1,4-dioxane, a few drops of water and HCl (1.4 ml, 4.0 M in dioxane, 5.5 mmol) were added and the mixture stirred at 80°C for 4h. The mixture was concentrated under reduced pressure to give the title compound (285 mg) as raw product, which was used in the next step.

**Step 5**

(2S)-2-{4-[(1R)-1-carboxy-2-hydroxyethyl]-7,10-bis[(1S)-1-carboxy-2-hydroxyethyl]-1,4,7,10-tetraazacyclododecan-1-yl}-5-{4-[2-(2-ethoxyethoxy)ethoxy]phenyl}pentanoic acid

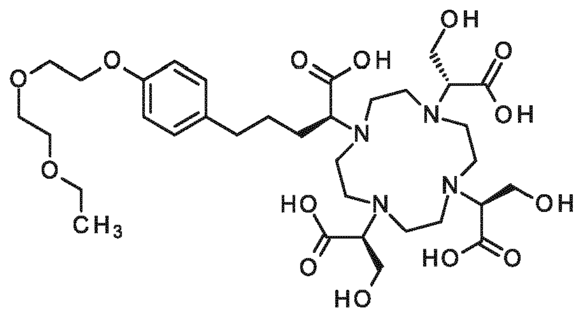

methyl (2S)-5-{ 4-[2-(2-ethoxyethoxy)ethoxy]phenyl}-2-{4-[(2R)-3-hydroxy-1-methoxy-1-oxopropan-2-yl]-7,10-bis[(2S)-3-hydroxy-1-methoxy-1-oxopropan-2-yl]-1,4,7,10-tetraazacyclododecan-1-yl}pentanoate (285 mg, 342 μmol) was dissolved in THF (2.0 ml) and a solution of lithium hydroxide (49.2 mg, 2.05 mmol) in water (2.0 ml) was added. The mixture was stirred at RT for 4h and then heated to 80°C for 3h. The mixture was concentrated under reduced pressure to give the title compound as red-brown raw product (302.9 mg), which was used as such in the next step.

**Step 6**

gadolinium (2S,2'S)-2,2'-{7-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecane-1,4-diyl}bis(3-hydroxypropanoate)

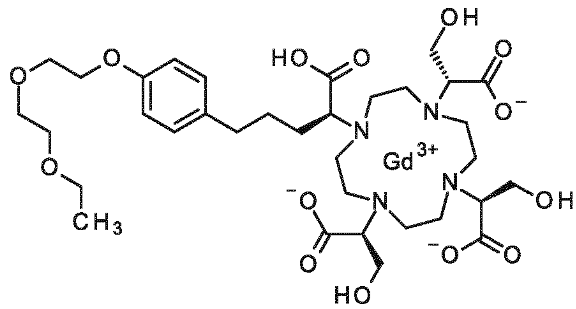

(2S)-2-{4-[(1R)-1-carboxy-2-hydroxyethyl]-7,10-bis[(1S)-1-carboxy-2-hydroxyethyl]-1,4,7,10-tetraazacyclododecan-1-yl}-5-{4-[2-(2-ethoxyethoxy)ethoxy]phenyl}pentanoic acid (303 mg, 346 μmol) was dissolved in water (4.0 ml), $Gd_2O_3$ (56.4 mg, 156 μmol) added and the mixture stirred at 100°C for 5h. Chelex 100™ (ca. 5 g, sodium form) was added and the mixture stirred at RT for 3d, after which the xylenol-orange test indicated the absence of free gadolinium. The mixture (including the resin) was adjusted to pH 7 (25% ammonium hydroxide), transferred to an empty Biotage cartridge and directly subjected to RP-chromatography (SNAP C18 60g, 50 ml/min, A = water, B = MeCN, 0%B 6CV, 0% to 15% in 4.4CV, 15%B 3.5CV, 15% to 40%B in 7.7CV, 227nm). Interesting fractions were pooled and lyophilized to give 104 mg of a slightly yellow material. It was dissolved in water, charcoal added, and the mixture heated to 100°C for 3h. After removal of the charcoal and lyophilization, 49 mg (16%) of the title compound were obtained as white lyophilizate.

Specific Rotation: 1.1° ($H_2O$, 20°C, 589 nm).

LC-MS (Method 2): $R_t$ = 0.77 min; MS (ESIpos): m/z = 900 $[M+H]^+$.

LC-MS (Method 3): $R_t$ = 0.71 min (100% DAD); MS (ESIpos): m/z = 450.8 $[M + 2H]^{++}$, 900.5 $[M + H]^+$; 1349 $[3M + 2H]^{2+}$. The observed isotope pattern conforms to a gadolinium complex.

**Reference Compounds**

**Reference compound 1**
Primovist® (Gd-EOB-DTPA, gadoxetate disodium, Bayer AG, Germany)

**Reference compound 2**
MultiHance® (Gd-BOPTA, gadobenic acid, Bracco, Italy)

**Reference compound 3**
Gadovist®/ Gadavist® (Gd-DO3A-butrol, gadobutrol, Bayer AG, Germany)

**In vitro and in vivo characterization of example compounds**

Examples were tested in selected assays one or more times. When tested more than once, data are reported as either average values or as median values, wherein

- the average value, also referred to as the arithmetic mean value, represents the sum of the values obtained divided by the number of times tested, and

- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from assays represent average values or median values calculated utilizing data sets obtained from testing of one or more synthetic batch.

**Example A - Water solubility**

The exploratory water solubility of the compounds is determined at room temperature (20°C) in 0.5 mL buffer solution (10 mM Tris-HCl, pH 7.4) in the microcentrifuge tubes (Eppendorf, 2.0 mL safe-lock caps). The solid compounds were added stepwise to the buffer solution. More substance is added once all the suspended material is dissolved until an

equilibrium between undissolved and dissolved substance is reached. The suspension was mixed using a shaker (Heidolph Reax 2000) and treated 5 min in an ultrasound bath (Bandelin, Sonorex Super RK255H). The Gadolinium concentration of the test substance in the clear solution is measured by ICP-MS. The results are summarized in Table 1.

**Table 1:** Solubilities of compounds in aqueous buffer at 20°C (pH 7.4).

| **Example No** | **Solubility** [mol/L] | **Example No** | **Solubility** [mol/L] |
|---|---|---|---|
| 1 | > 0.258 | 25 | 0.306 |
| 2 | >0.757 | 26 | > 0.062 |
| 3 | >0.331 | 27 | > 0.009 |
| 4 | > 0.075 | 28 | > 0.194 |
| 5 | > 0.059 | 29 | > 0.305 |
| 6 | > 0.217 | 30 | > 0.035 |
| 7 | > 0.243 | 31 | > 0.255 |
| 8 | 0.117 | 32 | > 0.198 |
| 9 | 0.002 | 33 | > 0.029 |
| 10 | > 0.272 | 34 | > 0.236 |
| 11 | n.d. | 35 | > 0.164 |
| 12 | n.d. | 36 | 0.085 |
| 13 | 0.067 | 37 | 0.018 |
| 14 | 0.005 | 38 | > 0.103 |
| 15 | > 0.500 | 39 | > 0.255 |
| 16 | > 0.416 | 40 | n.d. |
| 17 | > 0.217 | 41 | > 0.013 |
| 18 | > 0.354 | 42 | > 0.081 |
| 19 | > 0.410 | 43 | > 0.209 |
| 20 | > 0.381 | 44 | > 0.126 |
| 21 | > 0.368 | 45 | > 0.063 |
| 22 | > 0.308 | 46 | > 0.263 |
| 23 | > 0.241 | 47 | > 0.229 |
| 24 | > 0.261 | 48 | 0.016 |
| RC1 | > 0.250 | 49 | > 0.018 |
| | | 50 | > 0.019 |
| n.d. not determined | | | |

**Example B - Chemical stability**

Examples were separately dissolved in 10 mM Tris-HCl buffer, pH 7.4 at a final concentration of 1 mmol Gd/L. The solution was autoclaved three times at 1 bar, 121°C for 20 min. Before and after each autoclaving step an aliquot was removed, frozen at -20°C for later analytical analyses by HPLC-ICP-MS (Gd157) to determine the integrity of the compound.

Examples of used HPLC methods: Agilent 1290 Infinity II LC, Agilent ICP-MS 7900, Column: Waters BEH Acquity C18 UPLC, 1.7 μm, 2.1 x 50 mm. Solvent A: 20 mmol/L formic acid in water. Solvent B: MeOH. Gradient from 2% B to 98% B within 2.5 min, flow 0.8 mL/min or Solvent B: MeCN, isocratic 14% B, flow: 0.6 mL/min. Detection by ICP-MS, tuned to $^{157}$Gd (m/z) and $^{175}$Lu (internal standard). Before measuring via ICP the samples were mixed with 50% MeCN (+ 0.1% HNO3 + 0.5mM DTPA + 25nM Lu as IS). The chromatograms, displaying the intensity of the detected Gd, were visually compared. The compounds were valuated stable if changes in the chromatograms before and after autoclaving are < 1%

(Figure 1).

**Example C - Complex Stability (Transmetallation)**

The used method is described elsewhere (Laurent S et al., Stability of MRI paramagnetic contrast media: a proton relaxometric protocol for transmetallation assessment. Invest Radiol. 2001 Feb;36(2):115-22). In brief: A solution containing 2.5 mmol/L of test compound and 2.5 mmol/L $ZnCl_2$ in 26 mM phosphate buffer (KH2PO4 Merck; 1.09439.1000, #HC748513, pH 7,00), was incubated at 37°C over a period of at least 3 days. If test compounds are instable under these conditions, transmetallation during this test would lead to the formation of the $Zn^{2+}$ complex of the test item, while $Gd^{3+}$ is released and precipitated as highly insoluble $GdPO_4$. Since $Zn^{2+}$ does not influence the T1 relaxation time and $Gd^{3+}$ is withdrawn from the solution by precipitation, this ongoing exchange reaction would lead to increasing T1 relaxation times of the solution over time (measured at 1.41 T).

No change in T1 relaxation time was observed for investigated compounds after 3d of incubation, indicating that the compounds are highly stable against the dissociation of the Gd complex under these stress conditions. For comparison the results of some examples, the highly stable macrocyclic reference compound 3 (RC3), the less stable RC1 and least stable RC2 are included in Table 2. Values in % after 72h incubation are summarized in Table 2.

**Table 2:** Transmetallation stability [%] of paramagnetic contrast agents over 72 hours (2.5 mM $Zn^{2+}$, 26 mM phosphate buffer, 37°C, pH 7.4).

| Example No | Stability [%] | Example No | Stability [%] |
|---|---|---|---|
| 1 | 99 | 28 | 101 |
| 2 | 100 | 31 | 102 |
| 4 | 100 | 34 | 101 |
| 6 | 102 | 38 | 97 |
| 7 | 101 | 43 | 102 |
| 8 | 102 | 44 | 98 |
| 15 | 101 | 45 | 100 |
| 16 | 101 | 46 | 101 |
| 17 | 98 | 48 | 99 |
| 22 | 96 | RC1 | 44 |
| 25 | 103 | RC2 | 14 |
| 26 | 99 | RC3 | stable |

**Example D - Relaxivity measurements at 1.4 T**

Relaxivity measurements at 1.41 T were performed using a MiniSpec mq60 spectrometer (Bruker Analytik, Karlsruhe, Germany) operating at a resonance frequency of 60 MHz and a temperature of 37°C. The $T_1$ relaxation times were determined using the standard inversion recovery (IR) method with a fixed relaxation delay of at least 5 x $T_1$. The variable inversion time (TI) was calculated automatically by the standard software of the MiniSpec mq60 (8 steps). The $T_2$ measurements were performed by using a Carr-Purcell-Meiboom-Gill (CPMG) pulse sequence, applying a relaxation delay of at least 5 x $T_1$.

Each relaxivity measurement was performed using three different Gd concentrations (3 concentrations between 0.05 and 2 mM). The $T_1$ and $T_2$ relaxation times of the example compounds 1 to 10 were measured in different media for example in water and human plasma. Human plasma preparation: For each experiment fresh blood was taken from a volunteer using 10 mL citrate-tubes (Sarstedt S-Monovette 02.1067.001, 10 mL, Citrate). The 10 mL citrate- tubes were carefully inverted 10 times to mix blood and anticoagulant and centrifuged for 15min at 1811 g at RT (Eppendorf, Centrifuge 5810R).

The relaxivities $r_i$ (where i=1, 2) were calculated based on the measured relaxation rates $R_i$ in water and plasma:

$$r_i = (R_i - R_{i(0)}) / C_{Gd}$$

where $R_{i(0)}$ represent the relaxation rate of the respective solvent and $C_{Gd}$ the concentration of the compound normalized

to the Gadolinium. The Gadolinium concentrations of the investigated solutions were verified by Inductively Coupled Plasma Mass Spectrometry (ICP-MS Agilent 7500a, Waldbronn, Germany). The determined relaxivity values are summarized in Table 3.

**Table 3:** Relaxivities of investigated compounds in water and human plasma at 1.41 T and relaxivities of Reference compounds 1-3 (RC1-RC3) at 1.5 T in water and bovine plasma. All values are measured at 37°C and normalized to Gd and given in L $mmol^{-1}$ $s^{-1}$.

| Example No | $r_1$ water* | $r_2$ water* | $r_1$ human plasma* | $r_2$ human plasma* |
|---|---|---|---|---|
| 1 | 4.1 | 4.8 | 6.5 | 10.0 |
| 2 | 4.4 | 5.2 | 6.2 | 8.9 |
| 3 | 4.3 | 5.0 | 6.5 | 9.3 |
| 4 | 4.2 | 5.0 | 6.5 | 9.3 |
| 5 | 4.7 | 5.6 | 6.9 | 10.1 |
| 6 | 4.3 | 5.1 | 6.6 | 9.5 |
| 7 | 4.5 | 5.3 | 7.4 | 11.7 |
| 8 | 4.5 | 5.3 | 7.2 | 11.7 |
| 9 | n.d. | n.d. | n.d. | n.d. |
| 10 | 4.5 | 5.3 | 6.6 | 9.4 |
| 11 | 4.3 | 5.1 | 6.3 | 9.0 |
| 12 | 4.3 | 5.2 | 6.6 | 9.3 |
| 13 | 5.2 | 6.0 | 7.0 | 10.3 |
| 14 | 4.3 | 5.1 | 13.3 | 26.0 |
| 15 | 5.1 | 6.2 | 8.7 | 13.7 |
| 16 | 4.4 | 5.2 | 6.2 | 8.9 |
| 17 | 5.3 | 6.4 | 10.1 | 17.0 |
| 18 | 5.5 | 6.6 | 7.7 | 12.8 |
| 19 | 5.3 | 6.2 | 9.0 | 16.6 |
| 20 | 5.6 | 6.8 | 9.8 | 19.0 |
| 21 | 5.4 | 6.4 | 8.5 | 14.8 |
| 22 | 5.4 | 6.5 | 7.7 | 11.4 |
| 23 | 4.5 | 5.4 | 6.0 | 8.1 |
| 24 | 4.1 | 4.9 | 8.8 | 15.2 |
| 25 | 4.6 | 5.6 | 7.5 | 12.5 |
| 26 | 4.5 | 5.4 | 7.5 | 11.8 |
| 27 | n.d. | n.d. | n.d. | n.d. |
| 28 | 4.4 | 5.1 | 10.6 | 20.6 |
| 29 | 4.4 | 5.2 | 7.5 | 12.2 |
| 30 | 4.7 | 5.6 | 7.6 | 12.4 |
| 31 | 4.4 | 5.3 | 15.8 | 34.7 |
| 32 | 4.1 | 4.8 | 6.6 | 10.1 |
| 33 | 4.2 | 4.9 | 11.7 | 21.6 |
| 34 | 4.9 | 5.9 | 18.0 | 39.6 |
| 35 | 5.6 | 6.6 | 8.6 | 13.9 |

(continued)

| Example No | $r_1$ water* | $r_2$ water* | $r_1$ human plasma* | $r_2$ human plasma* |
|---|---|---|---|---|
| 36 | 6.1 | 7.5 | 8.5 | 13.1 |
| 37 | 6.4 | 7.4 | 8.4 | 12.9 |
| 38 | 5.6 | 6.6 | 7.5 | 11.0 |
| 39 | 4.8 | 5.8 | 7.8 | 11.4 |
| 40 | 5.4 | 6.4 | 7.3 | 9.9 |
| 41 | 4.5 | 5.4 | 5.7 | 8.2 |
| 42 | 5.0 | 5.8 | 7.2 | 10.4 |
| 43 | 4.8 | 5.7 | 6.9 | 10.1 |
| 44 | 4.0 | 4.7 | 7.9 | 14.0 |
| 45 | 4.5 | 5.4 | 11.4 | 21.5 |
| 46 | 3.5 | 4.2 | 10.8 | 20.7 |
| 47 | 3.7 | 4.4 | 7.8 | 13.0 |
| 48 | 4.0 | 5.1 | 9.3 | 17.2 |
| 49 | n.d. | n.d. | n.d. | n.d. |
| 50 | n.d. | n.d. | n.d. | n.d. |
| RC1^ | 3.3 | 3.9 | 5.2 | 6.1 |
| RC2^ | 3.3 | 3.9 | 4.1 | 4.6 |
| RC3^ | 3.3 | 3.9 | 5.2 | 6.1 |

* values are depicted in L $mmol^{-1}$ $s^{-1}$, n.d. not determined
^ Relaxivities from reference compounds from Rohrer et. al. at 1.5 T (Invest. Radiol. 2005; 40, 11: 715-724) and in bovine plasma (Kreaber GmbH, Pharmaceutical Raw Material, Ellerbek, Germany) instead of human plasma

**Example E - Uptake into freshly isolated rat hepatocytes**

Freshly isolated rat hepatocytes were received as described before (Papeleu P. et al. (2006) Isolation of Rat Hepatocytes. In: Phillips I.R., Shephard E.A. (eds) Cytochrome P450 Protocols. Methods in Molecular Biology, vol 320. Humana Press, Totowa, NJ. https://doi.org/10.1385/1-59259-998-2:229). The rat hepatocytes were incubated ($2.5 \cdot 10^5$ cells/well) for 2.5 to 4h in collagen coated 24-well plates (Fa. Beckton Dickinson Biocoat, cell environments, Collagen I Cellware) in Williams E-medium (Fa. Sigma W1878) in an incubator (37°C, 5% $CO_2$ and 95% oxygen). After incubation the adherent hepatocytes were washed once with 37°C Hepes carbonate transport buffer (500 µL/well). The test items and RC1 were prepared from stock solutions (180 to 500 mM concentration) in Hepes carbonate transport buffer at pH 7.4 to reach a final concentration of 100 µmol Gd/L. The specific uptake of the test item or RC1 into rat hepatocytes via OATP1B1 and OATP1B3 was challenged using a known OATP1B1/1B3 inhibitor (Rifampicine, Fa. Sigma R3501, test item and inhibitor 1:1 each 100 µM final concentration). The prepared rat hepatocyte plates were incubated with test solutions (500 µL/well) at 37°C for 10 min using a thermoshaker (Fa. Grant Bio, PHMP). Triplicates were performed for each test item. After incubating the cells were washed once with cold PBS buffer (Fa. Gibco, Dulbeccos PBS (+)). After washing the intact hepatocytes were lysed by addition of 500 µL cold 0.01 % Triton-X 100 in Millipore water for 1h. The strongly hypo-osmolar water led to cell membrane rupture and the release of the intracellular Gd-compound. After 1h 500 µL of 10 nM Terbium ICP-MS standard was added in 2% nitric acid and 0.01% Triton 100 before the Gadolinium concentrations in the lysate were measured using ICP-MS (Agilent 8900, singleQuad, integr. Time 0.3 s, no gas, 5 replicates, Gd m/z 157, Tb m/z 159). As a reference standard RC1 is used in the same well plate. The hepatocyte uptake of the test compound is calculated relative to RC1 (resulting in the unit % of RC1). The results different are summarized in the Table 4.

**Table 4:** Uptake of compounds into freshly isolated rat hepatocytes in % compared to reference compound 1 (RC1, Gd-EOB-DTPA).

| Example No | Uptake rat hepatocytes [% of RC1] | Inhibition Rifampicine [% inhibition] | Example No | Uptake rat hepatocytes [% of RC1] | Inhibition Rifampicine [% inhibition] |
|---|---|---|---|---|---|
| 1 | 29 | 93 | 25 | 82 | 96 |
| 2 | 52 | 85 | 26 | 77 | 97 |
| 3 | 47 | 96 | 27 | 69 | 98 |
| 4 | 72 | 98 | 28 | 72 | 98 |
| 5 | 28 | 92 | 29 | 87 | 99 |
| 6 | 62 | 96 | 30 | 75 | 99 |
| 7 | 89 | 99 | 31 | 87 | 94 |
| 8 | 83 | 98 | 32 | 54 | 93 |
| 9 | 54 | 46 | 33 | 82 | 94 |
| 10 | 49 | 90 | 34 | 98 | 90 |
| 11 | 39 | 93 | 35 | n.d. | n.d. |
| 12 | 53 | 97 | 36 | n.d. | n.d. |
| 13 | 49 | 86 | 37 | 26 | 37 |
| 14 | 96 | 98 | 38 | 62 | 94 |
| 15 | 109 | 94 | 39 | 17 | 64 |
| 16 | 16 | 92 | 40 | 20 | 88 |
| 17 | 71 | 91 | 41 | 52 | 56 |
| 18 | 75 | 91 | 42 | 44 | 95 |
| 19 | 92 | 92 | 43 | 90 | 97 |
| 20 | 71 | 91 | 44 | 70 | 96 |
| 21 | 81 | 91 | 45 | 68 | 82 |
| 22 | 41 | 83 | 46 | 106 | 90 |
| 23 | 17 | 73 | 47 | 55 | 82 |
| 24 | 40 | 96 | 48 | 77 | 98 |
| RC1^ | 100 | 97 | 49 | n.d. | n.d. |
| RC2^ | 9 | 79 | 50 | n.d. | n.d. |
| RC3^ | 0.7 | 0 | | | |
| n.d. not determined | | | | | |

**Example F - Uptake into stable transfected OATP1B1 HEK 293 cells**

Human stable transfected OATP1B1 human embryonic kidney 293 cells (OATP1B1 HEK 293, SLCO1B1, Entrez Gene ID: 10599) were used (Leonhardt M et al. Drug Metab Dispos. 2010 Jul;38(7):1024-8). The following culture medium was used: 500 mL DMEM (Dulbecco's Modified Eagle Medium - high Glucose, D6429), 50 mL FCS (fetal bovine serum, Fa. Sigma, F7524), 5.8 mL G418 (Geneticin, Fa. Gibco 10131-027) and 5 mL Pen-Strep. (Penicillin-Streptomycin, Fa. Gibco, Ref 15140-122). Cryopreserved cells were shortly thawed in water bath and diluted in 10 mL warm culture medium (37°C). After medium change the cells were grown for 3d in a 25 $cm^2$ cell culture flask. After 3 to 5d the cells were transferred into 75 $cm^2$ flasks. For the splitting the cells were incubated with 1.5 mL trypsin (Trypsin-EDTA (1x) 0,05%, Ref 25300-054 100ml, Fa. Gibco) for 3-5min in the incubator (37°C, 5% $CO_2$ and 95% oxygen). After centrifugation and washing with medium the cells were counted (50 µL cell suspension and 50 µL trypan blue (Trypan Blue Solution,

93595-50ml, Fa. Fluka). Cells were cultured (1x$10^5$ cells/well) in poly-D-lysine coated 24-well plates (Fa. Corning, Ref: 354414) in 1.5 mL medium per well. After 48h in the incubator the medium was changed and 1.5 mL/well sodium butyrate containing medium were added (40 mL culture medium plus 400 μL Na-Butyrate, Fa. Sigma Aldrich B5887-5G, 250 mg in 2.27 mL MilliQ water). After 24h in the incubator the medium was vacuumed off and 1.5 mL transport buffer was added and incubated for 10min. The transport buffer contains the following ingredients: 500 mL HBSS (Hank's balanced salt solution, Hyclone, SH30268.01), 5 mL HEPES buffer (4-(2-hydroxyethyl)-1-piperazine-ethanesulfonic acid, Gibco 15630-056) and 3.9 mL glucose (Sigma, G8270-100, 90 g/200mL MilliQ water). All test items were prepared in transport buffer at a final concentration of 100 μM from stock solutions (180 to 500 mM concentration). The OATP1B1 HEK 293 cells were incubated with test solutions (test item in transport buffer 500 μL) for 40min on a thermoshaker without shaking (PHMP, Fa. Grant Bio). Triplicates were performed for each test item. After incubation the cells were washed with 2 mL of cold PBS (+) buffer (Dulbeccos PBS +, Fa. Gibco). The OATP1B1 HEK 293 cells were lysed by addition of 500 μL cold 0.01 % Triton-X 100 in Millipore water for 1h. The strongly hypo-osmolar water led to cell membrane rupture and the release of the intracellular Gd-compound. After 1h 500 μL of 1 nM Terbium ICP-MS standard was added in 2% nitric acid and 0.01% Triton 100. Before the Gadolinium concentrations in the lysate were measured using ICP-MS (Agilent 8900, singleQuad, integr. Time 0.3 s, no gas, 5 replicates, Gd m/z 157, Tb m/z 159) the samples were centrifuged at 4000 rpm for 2min. As a reference standard Gd-EOB-DTPA (gadoxetate, BAY 864873) is used in the same well plate. The OATP1B1 HEK 293 uptake of the test compound is calculated relative to gadoxetate (resulting in the unit % of RC1). The results are summarized in Table 5.

**Table** 5: Uptake of compounds into stably transfected OATP1B1-HEK 293 cells in % compared to reference compound 1 (RC1, Gd-EOB-DTPA).

| Example No | Uptake into OATP1B1-HEK 293 cells [% of RC1] | Inhibition Rifampicine [% inhibition] | Example No | Uptake into OATP1B1-HEK 293 cells [% of RC1] | Inhibition Rifampicine [% inhibition] |
|---|---|---|---|---|---|
| 1 | 187 | 92 | 25 | 301 | 75 |
| 2 | 324 | 45 | 26 | 386 | 73 |
| 3 | 63 | 29 | 27 | 284 | 93 |
| 4 | 126 | 40 | 28 | 294 | 94 |
| 5 | 79 | 50 | 29 | 493 | 96 |
| 6 | 121 | 66 | 30 | 169 | 90 |
| 7 | 217 | 91 | 31 | 1919 | 98 |
| 8 | 271 | 90 | 32 | 147 | 72 |
| 9 | 498 | 73 | 33 | 367 | 88 |
| 10 | 87 | 43 | 34 | 947 | 96 |
| 11 | 154 | 40 | 35 | 184 | 87 |
| 12 | 86 | 0 | 36 | n.d. | n.d. |
| 13 | 109 | 30 | 37 | 1558 | 13 |
| 14 | 856 | 98 | 38 | 137 | 65 |
| 15 | 280 | 91 | 39 | 59 | 12 |
| 16 | 66 | 35 | 40 | 107 | 22 |
| 17 | 178 | 88 | 41 | 218 | 30 |
| 18 | 169 | 76 | 42 | 89 | 62 |
| 19 | 249 | 92 | 43 | 122 | 62 |
| 20 | 99 | 87 | 44 | 37 | 78 |
| 21 | 249 | 75 | 45 | 492 | 95 |
| 22 | 77 | 51 | 46 | 356 | 95 |
| 23 | 46 | 0 | 47 | 13 | 93 |

(continued)

| Example No | Uptake into OATP1B1-HEK 293 cells [% of RC1] | Inhibition Rifampicine [% inhibition] | Example No | Uptake into OATP1B1-HEK 293 cells [% of RC1] | Inhibition Rifampicine [% inhibition] |
|---|---|---|---|---|---|
| 24 | 229 | 87 | 48 | 240 | 92 |
| RC1^ | 100 | 81 | 49 | 86 | 71 |
| RC2^ | 31 | 61 | 50 | 240 | 84 |
| n.d. not determined | | | | | |

**Example G - Uptake into stable transfected OATP1B3 HEK 293 cells**

Human transfected OATP1B3 human embryonic kidney 293 cells (OATP1B3 HEK 293, SLCO1B3, Entrez Gene ID: 28234) were used (Leonhardt M et al. Drug Metab Dispos. 2010 Jul;38(7):1024-8). The following culture medium was used: 500 mL DMEM (Dulbecco's Modified Eagle Medium - high Glucose, D6429), 50 mL FCS (fetal bovine serum, Fa. Sigma, F7524), 5.8 mL G418 (Geneticin, Fa. Gibco 10131-027) and 5 mL Pen-Strep. (Penicillin-Streptomycin, Fa. Gibco, Ref 15140-122). Cryopreserved cells were shortly thawed in water bath and diluted in 10 mL warm culture medium (37°C). After medium change the cells were grown for 3d in a 25 $cm^2$ cell culture flask. After 3 to 5d the cells were transferred into 75 $cm^2$ flasks. For the splitting of cells these were incubated with 1.5 mL trypsin (Trypsin-EDTA (1x) 0,05%, Ref 25300-054 100ml, Fa. Gibco) for 3-5 min in the incubator (37°C, 5% $CO_2$ and 95% oxygen). After centrifugation and washing with medium the cells were counted (50 $\mu$L cell suspension and 50 $\mu$L trypan blue (Trypan Blue Solution, 93595-50ml, Fa. Fluka). Cells were cultured (1x$10^5$ cells/well) in poly-D-lysine coated 24-well plates (Fa. Corning, Ref: 354414) in 1.5 mL medium per well. After 48 hours in the incubator the medium was changed and 1.5 mL/well sodium butyrate containing medium were added (40 mL culture medium plus 400 $\mu$L Na-Butyrate, Fa. Sigma Aldrich B5887-5G, 250 mg in 2.27 mL MilliQ water). After 24 hours in the incubator the medium was vacuumed off and 1.5 mL transport buffer was added and incubated for 10 min. The transport buffer contains the following ingredients: 500 mL HBSS (Hank's balanced salt solution, Hyclone, SH30268.01), 5 mL HEPES buffer (4-(2-hydroxyethyl)-1-piperazine-ethanesulfonic acid, Gibco 15630-056) and 3.9 mL glucose (Sigma, G8270-100, 90 g/200mL MilliQ water). All test items were prepared in transport buffer at a final concentration of 100 $\mu$M from stock solutions (180 to 500 mM concentration). The OATP1B3 HEK 293 cells were incubated with test solutions (test item in transport buffer 500 $\mu$L) for 40min on a thermoshaker without shaking (PHMP, Fa. Grant Bio). Triplicates were performed for each test item. After incubation the cells were washed with 2 mL of cold PBS (+) buffer (Dulbeccos PBS +, Fa. Gibco). The OATP1B3 HEK 293 cells were lysed by addition of 500 $\mu$L cold 0.01 % Triton-X 100 in Millipore water for 1h. The strongly hypo-osmolar water led to cell membrane rupture and the release of the intracellular Gd-compound. After 1 hour 500 $\mu$L of 1 nM Terbium ICP-MS standard was added in 2% nitric acid and 0.01% Triton 100. Before the Gadolinium concentrations in the lysate were measured using ICP-MS (Agilent 8900, singleQuad, integr. Time 0.3 s, no gas, 5 replicates, Gd m/z 157, Tb m/z 159) the samples were centrifuged at 4000 rpm for 2 min. As a reference standard Gd-EOB-DTPA (gadoxetate) is used in the same well plate. The OATP1B3 HEK 293 uptake of the test compound is calculated relative to gadoxetate (resulting in the unit % of RC1). The results are summarized in Table 6.

**Table 6:** Uptake of compounds into stably transfected OATP1B3-HEK 293 cells in % compared to reference compound 1 (RC1, Gd-EOB-DTPA).

| Example No | Uptake into OATP1B3-HEK 293 cells [% of RC1] | Inhibition Rifampicine [% inhibition] | Example No | Uptake into OATP1 B3-HEK 293 cells [% of RC1] | Inhibition Rifampicine [% inhibition] |
|---|---|---|---|---|---|
| 1 | 24 | 97 | 25 | 58 | 93 |
| 2 | 56 | 80 | 26 | 64 | 93 |
| 3 | 18 | 89 | 27 | 24 | 96 |
| 4 | 58 | 98 | 28 | 36 | 97 |
| 5 | 41 | 97 | 29 | 75 | 99 |
| 6 | 46 | 97 | 30 | 62 | 98 |
| 7 | 76 | 99 | 31 | 194 | 99 |

(continued)

| Example No | Uptake into OATP1B3-HEK 293 cells [% of RC1] | Inhibition Rifampicine [% inhibition] | Example No | Uptake into OATP1 B3-HEK 293 cells [% of RC1] | Inhibition Rifampicine [% inhibition] |
|---|---|---|---|---|---|
| 8 | 90 | 99 | 32 | 22 | 94 |
| 9 | 124 | 95 | 33 | 71 | 98 |
| 10 | 13 | 95 | 34 | 140 | 98 |
| 11 | 16 | 73 | 35 | 55 | 98 |
| 12 | 24 | 87 | 36 | n.d. | n.d. |
| 13 | 31 | 93 | 37 | 123 | 44 |
| 14 | 69 | 97 | 38 | 24 | 94 |
| 15 | 78 | 98 | 39 | 26 | 88 |
| 16 | 25 | 94 | 40 | 27 | 92 |
| 17 | 61 | 98 | 41 | 47 | 74 |
| 18 | 52 | 96 | 42 | 20 | 91 |
| 19 | 62 | 96 | 43 | 111 | 99 |
| 20 | 75 | 99 | 44 | 41 | 97 |
| 21 | 72 | 94 | 45 | 106 | 98 |
| 22 | 46 | 96 | 46 | 43 | 97 |
| 23 | 14 | 71 | 47 | 19 | 90 |
| 24 | 17 | 94 | 48 | 11 | 81 |
| RC1^ | 100 | 99 | 49 | 26 | 94 |
| RC2^ | 4 | 87 | 50 | 51 | 93 |
| n.d. not determined | | | | | |

**Example H** - **Plasma protein binding**

The binding of Example 1 and 15 to plasma proteins of different species (human, dog, monkey, rabbit, mouse and rat) was investigated *in vitro* by using equilibrium dialysis in reusable 96-well Micro-Equilibrium Dialysis Devices (ht dialysis) (Banker MJ et al. J Pharm Sci . 2003 May;92(5):967-74). Data indicate that the compounds show low binding to plasma proteins of all species tested (Table 7).

**Table 7:** Fraction unbound (fu %) of different species of examples 1,2 and 15.

| Species | Example 1 fu (%) | Example 2 fu (%) | Example 15 fu (%) |
|---|---|---|---|
| Rat | 92 | > 95 | 71 |
| Dog | 96 | > 95 | 91 |
| Monkey | 94 | > 95 | 88 |
| Human | 99 | > 95 | 86 |

**Example I** - **In vivo Pharmacokinetics Following i.v. Administration in Rat**

Pharmacokinetic parameters of Example 1 and 15 and were determined in male and female rats (Han-Wistar, n=4). The compounds were administered as a sterile aqueous solution into the tail vein as bolus of 25 μmol Gd/kg bodyweight. Plasma was sampled before and 1, 3, 5, 10, 15, 30, 60, 90, 120, 240, 360 and 1440 min post administration and the Gd concentration was determined by Inductively Coupled Plasma Mass Spectrometry (ICP-MS Agilent 7500a,

Waldbronn, Germany). The samples were digested using strong acidic and oxidizing conditions at elevated temperature following dilution in 1% nitric acid to bring the samples in the quantification range of the method. The lower limit of quantitation amounts to 1 nmol/L for all elements and the upper limit to 1000 nmol/L in the diluted sample.

The fit of the obtained data to a three-compartment model (Phoenix, WinNonlin 6.4, 3- compartment mode) yielded the pharmacokinetic parameters which are shown in Table 8.

**Table 8:** Pharmacokinetic parameters of blood plasma levels.

| Parameter | | unit | Example 1 | Example 1 | Example 15 | RC1 |
|---|---|---|---|---|---|---|
| Dose | | μmol/kg bw | 25 | 250 | 25 | 100 |
| t½ α | Half-life, compartme nt V1 | [min] | 1,8 | 3 | 3 | 2 |
| t½ β | Half-life, compartme nt V2 | [min] | 13 | 11 | 12 | 8 |
| t½ γ | Half-life, compartme nt V3 | [h] | 1.9 | 6.6 | 9.8 | 6.5 |
| AUC | Area under the curve | $AUC_\infty$ [μmol · h/L]<br>$AUC_{norm}$ [h · kg/L] | $AUC_\infty$ 16 | $AUC_\infty$ 315 | $AUC_{norm}$ 0.38 | $AUC_{norm}$ 0.40 |
| $V_{ss}$ | Volume of distribution at steady state | [L/kg] | 0.4 | 0.2 | 0.4 | 0.9 |
| $Cl_{plasma}$ | Plasma clearance | [L/h/kg] | 1.6 | 0.8 | 2.7 | 2.4 |

**Example J - Biliary elimination in bile duct cannulated (BDC) rats**

The biliary excretions of compounds were investigated in bile duct cannulated (BDC) rats (Burden N et al. Lab Anim. 2017 Oct; 51(5): 457-464). All animals are initially anesthetized using a body weight-adjusted intramuscular injection of a mixture (1+2) of xylazine hydrochloride (20 mg/mL, Rompun 2%, Bayer Vital GmbH, Leverkusen) and ketamine hydrochloride (100 mg/mL, Ketavet, Pfizer, Pharmacia GmbH, Berlin) using 1 mL/kg body weight. The continuous anesthesia of the animals is achieved by the intravenous injection of 1.5 mL per hour (Braun perfusor infusion of 1+2, 1:20 saline, xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin) via tail vein (Introcan, 24G, yellow). The animals received 0.1 mmol Gd/kg bw as intravenous bolus injection via tail vein (50 mmol Gd/L formulation). Bile was sampled 0-0.5h, 0.5-1h, 1-2h, 2-3h und 3-4h post injection. The Gadolinium concentrations in the bile fractions were determined by Inductively Coupled Plasma Mass Spectrometry (ICP-MS Agilent 7500a, Waldbronn, Germany). The bile fractions (n=3 times 10 μL) were dried for 2h at 90°C and 50 μL 65% nitric acid ($HNO_3$, Fa. Fisher, Optima Grade #1219010) and 30 μL hydrogen peroxide 30% (H2O2 Fa VWR Chemicals, AnalaR NORMAPUR, #18J024022) were added and processed in microwave oven (5 cycles: 5min 40W, 5min 120W, 10min 200W, 10min 320W and 25min 400W, Fa. CEM, Mars5 Xpress). After the samples have cooled down to room temperature 920 μL of ICP diluent were added (1% HNO3 + 0.01% Triton Tx100 and internal standard 50 μM Terbium), samples were adequately diluted, and the Gadolinium concentrations were measured via ICP-MS. The results of cumulative dose are summarized in Table 9.

**Table 9:** Biliary excretion in bile duct cannulated rats.

| | % of injected dose Mean ± SD (cumulative) | | |
|---|---|---|---|
| **Bile fractions** | **Example 1** | **Example 15** | **RC1** |
| 0-0.5 h | 55.5 ± 1.7% | 73.8 ± 2.3% | 64.1 ± 2.4% |
| 0.5-1 h | 62.6 ± 0.7% | 77.4 ± 2.8% | 71.8 ± 3.7% |
| 1-2 h | 65.1 ± 1.3% | 78.6 ± 2.6% | 75.1 ± 3.1% |
| 2-3 h | 65.6 ± 1.6% | 79.6 ± 2.2% | 76.0 ± 3.2% |
| 3-4 h | 65.8 ± 1.7% | 80.5 ± 2.7% | 76.4 ± 3.3% |

**Example K - Excretion study in rats (5 days) and residual Gadolinium organ distribution (7 days)**

The excretion and organ distribution of Example 1 and 15 were determined in male rats (Han-Wistar, n=3). The compound was administered as a sterile aqueous solution (50 mmol Gd/L) as a bolus in the tail vein of the animals. The dose was 0.1 mmol Gd/kg bw. Urine was collected in the following time periods 0-1h, 1-3h, 3-6h, 6-24h, 1-2d and 2-5d post injection and feces 0-1h, 1-3h, 3-6h, 6-24h, 1-2d and 2-5d post injection and feces 0-1d, 1-2d and 2-5d post injection. As a control, 3 animals were treated in the same way with RC1. On day 7 the animals were sacrificed, and the following organs were excised: blood, liver, kidney, spleen, heart, lung, brain, muscle, skin, stomach, gut, bone and bone marrow. The remaining carcass was freeze dried and ground to a fine powder. The Gd concentration in the organs and the carcass was determined by ICP-MS (ICP-MS Agilent 7500a, Waldbronn, Germany). The results of the organ distribution of investigated examples and Reference compound 1 are summarized in Table 10. The compounds are excreted via dual pathway (liver and kidney). Less than 1% of the administered dose was present in the body 7 days after the injection.

**Table 10:** Excretion and organ distribution of Example 1, 15 and RC1 in rats.

| | **Example 1** [% Dose] | **Example 15** [% Dose] | **RC1** [% Dose] |
|---|---|---|---|
| **Time period post injection** | **Urine** | **Urine** | **Urine** |
| 0-1 h | 39.8 ± 6.8% | 12.6 ± 11.1% | 23.2 ± 1.6% |
| 1-3 h | 40.3 ± 6.8% | 17.3 ± 3.4% | 23.5 ± 1.5% |
| 3-6 h | 40.3 ± 6.8% | 17.4 ± 3.3% | 23.6 ± 1.5% |
| 6-24 h | 40.8 ± 7.4% | 17.6 ± 3.3% | 23.7 ± 1.5% |
| 1-2 d | 40.9 ± 7.4% | 17.7 ± 3.3% | 23.7 ± 1.5% |
| 2-5 d | 40.9 ± 7.4% | 17.7 ± 3.3% | 23.7 ± 1.5% |
| **Time period post injection** | **Feces** | **Feces** | **Feces** |
| 0-1 d | 55.6 ± 5.6% | 78.6 ± 6.2% | 77.5 ± 2.7% |
| 1-2 d | 56.9 ± 6.6% | 80.0 ± 5.3% | 80.6 ± 2.9% |
| 2-5 d | 57.0 ± 6.6% | 80.0 ± 5.3% | 80.8 ± 2.9% |
| **Time point post injection** | **Σ organs and carcass** | **Σ organs and carcass** | **Σ organs and carcass** |
| 7d | 0.65 ± 0.07% | 0.11 ± 0.03 % | 0.14 ± 0.08 % |
| **Total recovery** | **98.6 ± 2.1 %** | **97.9 ± 5.4%** | **104.6 ± 2.4 %** |

**Example L - In vitro metabolism**

The in vitro metabolism was investigated in cryopreserved human and rat hepatocytes (Li A P et al. Biomark Med. 2014;8(2):173-83). Cryopreserved human (WSS, male) and rat hepatocytes (Wistar rat, RWH137, male) were incubated with the test compound (incubation concentration: 5 μM). The amount of remaining compound was related to the initial amount (0 mins). The cells were removed from the liquid nitrogen. The vial was immersed in the 37°C water bath and shaken gently (by hand) until most of the ice was melted (approx. 90 sec.). The cell suspensions were transferred e.g. into a 50 ml-centrifuge tube and 12 ml of 4°C-cold buffer (e.g. Krebs-Henseleit or Williams Medium E) were added. The cell suspension was centrifuged at 50xg for 5 mins. The buffer was aspirated, and the cells resuspended in 5 ml of fresh warm buffer. An aliquot of 50 μl was mixed with 200 μl trypan blue to determine the cell density and cell viability. The cell suspension was adjusted to a final concentration of 0.8 Mio hepatocytes per mL. The incubations were performed in 96 well plates (1.2 ml volume, negative control without cells). The 0 min samples were stopped immediately by adding 250 μl of ice-cold methanol. All other samples are shaken on an Heidolph®-rotor during incubation in a 37°C incubator for the indicated period of 4 hours. Aliquots of the samples were used for analysis. All samples were analyzed using LC-MS at 0h, 1h, 2h and 4h (exemplary LC-MS: Accucore C-18 column, 30°C, 0.35 mL/min flow rate, solvent A: 10 mM Ammonium formate + 5% MeCN, pH4 and solvent B: MeCN + 0.1% formic acid; 10 mL injection volume, gradient: 90% A, 10% B, 5 min 70% A and 30% B, 8 min 0% A and 100% B).

No metabolites were detected for Example 1, 2 and 15 in hepatocyte incubations of rat and human origin during an incubation time of 4h (1x$10^6$ cells/mL).

**Example M - Partition coefficient (logP butanol/buffer)**

The partition coefficient P (log P= log($C_{butanol}$/$C_{buffer}$)) was determined in buffered aqueous solution (10 μM of compound in 0.5 mL 50 mM Tris-HCl saturated with Butanol, pH 7.4) and 1-butanol 1+1 and the mixture was shaken for 2h at room temperature (n=3). The Gd-concentrations in each phase. $C_{butanol}$ and $C_{buffer}$ were measured by ICP-MS (The Chemistry of Contrast Agents in Medical Magnetic Resonance Imaging" 2nd Ed. Merbach AS, Helm L, Toth E, eds. Hoboken, NJ: Wiley 2013).

**Table 11:** Partition coefficient P (log P= log(butanol/buffer, pH 7.4))

| Example No | Log P | Example No | Log P) |
|---|---|---|---|
| 1 | -0.98 | 25 | -1.00 |
| 2 | -1.63 | 26 | -1.01 |
| 3 | -1.61 | 27 | -0.41 |
| 4 | -1.61 | 28 | -0.71 |
| 5 | -1.52 | 29 | -0.53 |
| 6 | -1.44 | 30 | -0.60 |
| 7 | -1.09 | 31 | 0.20 |
| 8 | -1.04 | 32 | -1.53 |
| 9 | 0.26 | 33 | -0.55 |
| 10 | -1.70 | 34 | 0.12 |
| 11 | -1.68 | 35 | -1.02 |
| 12 | -1.68 | 36 | -1.08 |
| 13 | -1.53 | 37 | -1.16 |
| 14 | -0.38 | 38 | -1.41 |
| 15 | -1.00 | 39 | -1.32 |
| 16 | -1.81 | 40 | -1.43 |
| 17 | n.d. | 41 | -1.61 |
| 18 | -1.11 | 42 | -1.60 |
| 19 | -0.95 | 43 | -1.19 |
| 20 | -1.11 | 44 | -0.83 |
| 21 | -1.03 | 45 | -0.36 |
| 22 | n.d. | 46 | -0.57 |
| 23 | -1.94 | 47 | -0.77 |
| 24 | -0.71 | 48 | -0.83 |
| RC1^ | -2.12 | 49 | n.d. |
| RC2^ | -2.07 | 50 | n.d. |
| | | RC3^ | -2.22 |
| n.d. not determined | | | |

**Example N - NOAEL**

The no-observed-adverse-effect level (NOAEL) was tested in mice using a modified 4-level up procedure with increasing doses of the test items. The observed NOAEL denotes the level of exposure, at which no severe adverse effects were observed (Dorato et al. Regul Toxicol Pharmacol. 2005 Aug;42(3):265-74). The test items were applicated as intravenous bolus injection via the tail vein. The starting dose was 0.2 mmol/kg bw (8-fold of the clinical dose of RC1), and

the dose for the next level was increased to 0.5 mmol Gd/kg bw if the animals showed no adverse effects and decreased if the mice showed any adverse effects. The next level was 1.0 or 1.5 mmol Gd/kg bw. At the highest dose level of 2.5 mmol Gd/kg bw 3 animals were injected. Symptoms were observed continuously during the whole experiment (2h after contrast agent administration). Animals that survived were euthanized in deep anesthesia 48h post injection (xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin). Macroscopic analysis was performed in all mice immediately after death. Blood was collected by puncture of vena cava and ALT, AST, GGT, GLDH, creatinine and BUN were analyzed (300 μL serum). The NOAEL levels are depicted in relation to their lipophilicity (logP values) in Figure 2.

**Example O - Contrast-Enhanced Liver MRI in healthy mice**

Dynamic liver contrast-enhanced magnetic resonance imaging (CE-MRI) was performed in healthy mice. The liver MRI signal intensity change over time was compared to RC1 (Primovist). The studies were performed at a 4.7T preclinical MRI scanner equipped with a dedicated transmit-receive mouse body volume coil (Bruker Biospec 70/20, Bruker BioSpin, Ettlingen, Germany). Studies were done using female NMRI mice (~25 g, n=3, Charles River Kisslegg). The animals were anesthetized using a mixture of isoflurane (initial 3%, then 2 %), oxygen gas (ca. 0.5 L/min) and nitrous oxide (flow ca. 1 L/min). CE-MRI was done using a T1-weighted FLASH sequence with retrospective respiratory gating. Different imaging slices (kidney, liver and muscle) were investigated. The acquisition time per image was 32 seconds and 110 repetitions were performed in each study. After 4 repetitions the example compound (0.025 mmol/kg bw), reference compound 1 (RC1, Primovist, 0.025 mmol/kg bw) or reference compound 2 (RC2, Multihance, 0.05 mmol/kg bw) was injected. Three different regions-of interest within the liver were evaluated. The relative signal enhancement over time was compared to that of RC1 and RC2, as very well known liver specific MRI market products. Representative images of the liver before and ~10min after administration are depicted in Figure 3.

**Example P - Contrast-Enhanced Liver MRI of tumor bearing rabbits at 1.5T**

Dynamic liver CE-MRI was performed in VX2 tumor rabbit model, as an established model for HCC (Keller S et al. J Magn Reson Imaging. 2020 Sep;52(3):668-685). Therefore, a small piece of frozen VX2 tumor (stored in 10% DMSO in fetal bovine serum, WNS 924084) was washed in medium (RPMI 1640) and injected using a trocar into the left femoral muscle of one donor rabbit. After 21d the tumor tissue was dissected and small pieces of the VX2 carcinoma were prepared. A total number of 6 male White New Zealand rabbits (Charles River) were investigated in 3-way crossover study. The animals were inoculated with 1 × 1 mm pieces of VX2 carcinoma tissue into the left medial liver lobe by laparotomy. In 5 / 6 animal the tumor was successfully developed at the start of MRI examinations (day 12 /13 after inoculation) at the end of the study (day 16/17 post inoculation) a tumor was detected in the liver of all 6 animals. Each rabbit received 3 contrast enhanced multi-phase liver MRI examinations using example compound and the comparators RC1 and RC2. Examinations were performed ever second day to allow complete contrast media excretion and recovery from anesthesia. The order of contrast media application was randomized between example compound and RC2 for the first and second examination. RC2 was always applied at the third scan day. For MRI the rabbits were investigated under general anesthesia induced with intramuscular injection of 5 mg/kg of xylazine and 60 mg/kg of ketamine. Anesthesia was maintained by intravenous infusion of 0.9 ml/kg/h Propofol. They were orotracheally intubated, ventilated with 55% oxygen, and placed in the scanner in a supine position. Ventilation was stopped during image acquisitions to enable breath-hold imaging. Example compounds were formulated to a concentration of 50 mmol/L with 10 mM Tris-HCL (pH 7.4) and 0.1 mol% CaNa-Butrol. RC1 was diluted with 0.9% saline to an identical concentration, RC2 was diluted to 100 mmol /L. RC1 was administered at the human standard dose (0.025 mmol/kg). The identical dose was used for the example compounds. RC2 was used at the human standard dose for liver MRI (0.05 mmol/kg). Contrast injection was performed with the MRXperion injection system with a flow rate of 0.9 ml/s for all contrast agents. MRI was performed on 1.5T MR system (Magnetom Avanto fit) and a 32-channel knee coil using a T1 weighted fat-saturated 3D gradient echo sequence (Volumetric Interpolated Breath-bold Examination, VIBE) sequence covering the entire liver with 36 slices a 2 mm. After axial pre-contrast image acquisition injection of contrast agent and coronal bolus tracking scans (thoracic region) were started simultaneously. Immediately after detection of contrast enhancement on the pulmonary artery, 5 continuous axial dynamic scans were obtained (6s each) in which the 1st and 2nd phases corresponded to early and late arterial phases. After this dynamic phase image acquisition additional scans were performed at 3, 5, 10, 15, 20, 40, 60min after contrast injection. For quantitative image evaluation the signal intensities (SIs) of the liver tissue, the aorta and the portal vein were measured by manually placed regions of interest (ROIs). For liver and aorta, the SIs of 3 ROIs were averaged, for the portal vein the mean of 2 ROIs was used. The image analysis was done for all time points. Exemplarily liver MRI of the tumor sliced as an intraindividual comparison in rabbits of the different liver imaging phases are depicted in Figure 4.

**Example Q - Contrast-Enhanced Liver and Biliary MRI of pig at 1.5T**

Contrast-enhanced magnetic resonance imaging (CE-MRI) of liver and bile was performed in healthy Goettingen minipig. The study was performed at a 1.5T preclinical MRI scanner equipped with a spine and body flex coil (Siemens Avanto Fit, Erlangen, Germany). For MRI the pig was investigated under general anesthesia induced with intramuscular injection of 2 mg/kg of azaperone (Stresnil, Elanco GmbH, Cuxhaven, Germany) and 15 mg/kg of ketamine (Ketaset, 100 mg/mL, Fa. Zoetis, Berlin, Germany). Anesthesia was maintained by intravenous infusion of 12 mg/kg/h Propofol after initial Propofol bolus of 3 mg/kg (Propofol-Liiuro, 10mg/ml, Braun, Melsungen, Germany). Animals received 8 mL/kg/h 0.9% saline/h as intravenous infusion. They were orotracheally intubated, ventilated with 50% oxygen, and placed in the scanner in a supine position. Ventilation was stopped in end-expiratory breath hold during image acquisitions. Example compounds were formulated to a concentration of 250 mmol Gd/L in 10 mM Tris-HCL (pH 7.4) and 0.1 mol% CaNa-Butrol. T1-weighted 3D DIXON VIBE sequence (TR=6.9 ms, TE1/2=2.39/4.77 ms, flip angle 10°, IPAT=CAIPIRINIA PAT 3; PE=1/3D=3) covering the entire liver with 88 slices a 1.5 mm were used (FOV 350x284, matrix 256x156, phase resolution 75%, in-plane resolution 1.4x1.4 mm, slide resolution 50%) was used. The acquisition time per image was 23 seconds and different time point post after contrast agent application were investigated. Representative images of the liver before and after administration of contrast agents (A) and exemplarily Maximum Intensity Projections (MIPS 2 cm) of Example 15 and RC1 are depicted to visualize the elimination of the contrast agent into the bile Figure 5 (B).

## Claims

**1.** A compound of general formula (I),

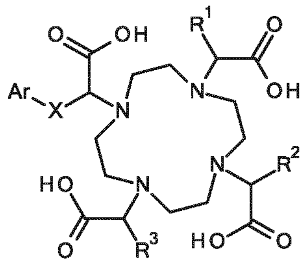

(I) ,

in which

Ar represents a group selected from

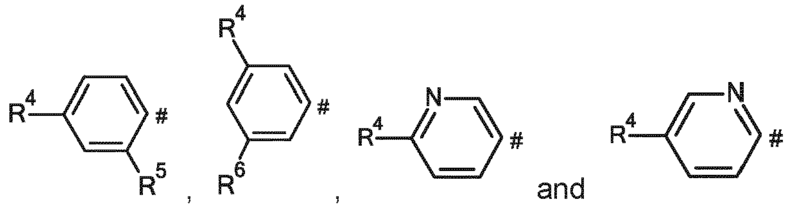

, , and ,

wherein # indicates the point of attachment to X,
X represents a group selected from $CH_2$, $(CH_2)_2$, $(CH_2)_3$, $(CH_2)_4$ and $*-(CH_2)_2-O-CH2-^{\#}$,
wherein * indicates the point of attachment to Ar and $^{\#}$ indicates the point of attachment to the acetic acid moiety,
$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,
$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,
$R^5$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-, and

$R^6$ represents a hydrogen atom or a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,

or a stereoisomer, a tautomer, an N-oxide, a hydrate, a solvate, or a salt thereof, or a mixture of same.

**2.** The compound according to claim 1, wherein

Ar represents a group selected from

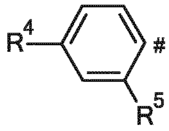 , 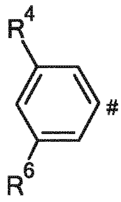 , 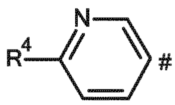 and 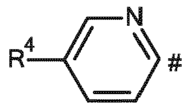 ,

wherein # indicates the point of attachment to X,
X represents a group selected from $CH_2$, $(CH_2)_2$, $(CH_2)_3$, $(CH_2)_4$ and *-$(CH_2)_2$-O-$CH_2$-#,
wherein * indicates the point of attachment to Ar and # indicates the point of attachment to the acetic acid moiety,
$R^1$, $R^2$ and $R^3$ represent, independently for each occurrence, a hydrogen atom or a group selected from $C_1$-$C_3$-alkyl, -$CH_2OH$, -$(CH_2)_2OH$ and -$CH_2OCH_3$,
$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,
$R^3$ represents a hydrogen atom, and
$R^6$ represents a hydrogen atom,

or a stereoisomer, a tautomer, an N-oxide, a hydrate, a solvate, or a salt thereof, or a mixture of same.

**3.** The compound according to any of claims 1 or 2, wherein

Ar represents a group selected from

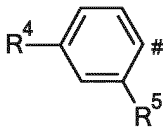 and 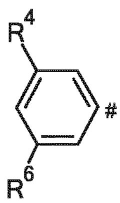 ,

wherein # indicates the point of attachment to X,
X represents a group selected from $CH_2$ and $(CH_2)_3$,
$R^1$ and $R^3$ represent, independently for each occurrence, a hydrogen atom or a -$CH_2OH$ group,
$R^2$ represents a hydrogen atom or a group selected from $C_1$-$C_3$-alkyl, -$CH_2OH$, - $(CH_2)_2OH$ and -$CH_2OCH_3$,
$R^4$ represents a group selected from $C_2$-$C_5$-alkoxy, ($C_1$-$C_3$-alkoxy)-$(CH_2)_2$-O-, ($C_1$-$C_3$-alkoxy)-$(CH_2)_2$-O-$(CH_2)_2$-O- and ($C_1$-$C_3$-alkoxy)-$(CH_2)_2$-O-$(CH_2)_2$-O-$(CH_2)_2$-O-,
wherein said $C_1$-$C_3$-alkoxy groups and $C_2$-$C_5$-alkoxy groups are optionally substituted, one, two, three or four times, with a fluorine atom,
$R^5$ represents a hydrogen atom, and
$R^6$ represents a hydrogen atom,

or a stereoisomer, a tautomer, a hydrate, a solvate, or a salt thereof, or a mixture of same.

**4.** The compound according to any of claims 1 or 2, wherein

Ar represents a group selected from

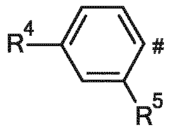 and 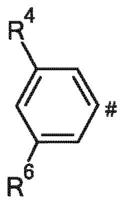 ,

wherein # indicates the point of attachment to X,
X represents a group selected from $CH_2$, $(CH_2)_2$, $(CH_2)_3$, $(CH_2)_4$ and $*-(CH_2)_2-O-CH_2-^{\#}$,
wherein * indicates the point of attachment to Ar and # indicates the point of attachment to the acetic acid moiety,
$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,
$R^4$ represents a group selected from $C_2$-$C_4$-alkoxy, $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom, and
$R^6$ represents a hydrogen atom,

or a stereoisomer, a tautomer, a hydrate, a solvate, or a salt thereof, or a mixture of same.

**5.** The compound according to any of claims 1, 2 or 4, wherein

Ar represents a group selected from

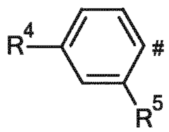 and 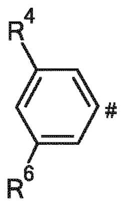 ,

wherein # indicates the point of attachment to X,
X represents a group selected from $CH_2$ and $(CH_2)_3$,
$R^1$, $R^2$ and $R^3$ represent, independently for each occurrence, a hydrogen atom or a group selected from $C_1$-$C_3$-alkyl, $-CH_2OH$, $-(CH_2)_2OH$ and $-CH_2OCH_3$,
$R^4$ represents a group selected from $(H_3C-CH_2)-O-(CH_2)_2-O-$, $(H_3C-CH_2)-O-(CH_2)_2-O-(CH_2)_2-O-$ and $(H_3C-CH_2)-O-(CH_2)_2-O-(CH_2)_2-O-(CH_2)_2-O-$,
$R^5$ represents a hydrogen atom, and
$R^6$ represents a hydrogen atom,

or a stereoisomer, a tautomer, a hydrate, a solvate, or a salt thereof, or a mixture of same.

**6.** The compound of general formula (I) according to any one of claims 1 to 5 in the form of a complex with $Gd^{3+}$, or a stereoisomer, a tautomer, an N-oxide, a hydrate, a solvate, or a salt thereof, or a mixture of same.

**7.** A compound according to claim 6 selected from the group consisting of

gadolinium 2,2',2''-(10-{1-carboxy-2-[2-(4-ethoxyphenyl)ethoxy]ethyl}-1,4,7,10-tetraazacyclododecane-1,4,7-triyl)triacetate,
gadolinium 2,2',2''-{10-[1-carboxy-2-{4-[2-(2-ethoxyethoxy)ethoxy] phenyl}ethyl]-1,4,7,10-tetraazacyclododecane-1,4,7-triyl}triacetate,
gadolinium 2,2',2''-{10-[(1S)-1-carboxy-2-{4-[2-(2-ethoxyethoxy)ethoxy] phenyl}ethyl]-1,4,7,10-tetraazacyclododecane-1,4,7-triyl}triacetate,
gadolinium 2,2',2''-{10-[(1R)-1-carboxy-2-{4-[2-(2-ethoxyethoxy)ethoxy] phenyl}ethyl]-1,4,7,10-tetraazacyclododecane-1,4,7-triyl}triacetate,

gadolinium 2-[7-(1-carboxy-2-{4-[2-(2-ethoxyethoxy)ethoxy]phenyl} ethyl)-4,10-bis(carboxylatomethyl)-1,4,7,10-tetraazacyclododecan-1-yl]propanoate,

gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]butanoate,

gadolinium (2S,2'S,2"S)-2,2',2"-{10-[(1S)-5-(4-butoxyphenyl)-1-carboxypentyl]-1,4,7,10-tetraazacyclododecane-1,4,7-triyl}tris(3-hydroxypropanoate),

gadolinium 2,2',2"-{10-[1-carboxy-2-(4-{2-[2-(2-ethoxyethoxy)ethoxy] ethoxy}phenyl)ethyl]-1,4,7,10-tetraazacyclododecane-1,4,7-triyl} triacetate,

gadolinium 2,2',2"-{10-[(1S)-1-carboxy-2-(4-{2-[2-(2-ethoxyethoxy)ethoxy]ethoxy}phenyl)ethyl]-1,4,7,10-tetraazacyclododecane-1,4,7-triyl}triacetate,

gadolinium 2,2',2"-{10-[(1R)-1-carboxy-2-(4-{2-[2-(2-ethoxyethoxy)ethoxy]ethoxy}phenyl)ethyl]-1,4,7,10-tetraazacyclododecane-1,4,7-triyl}triacetate,

gadolinium 2-{7-[1-carboxy-2-(4-{2-[2-(2-ethoxyethoxy)ethoxy]ethoxy} phenyl)ethyl]-4,10-bis(carboxylatomethyl)-1,4,7,10-tetraazacyclodo decan-1-yl}-3-methoxypropanoate,

gadolinium 2,2',2"-{10-[2-(4-butoxyphenyl)-1-carboxyethyl]-1,4,7,10-tetraazacyclododecane-1,4,7-triyl}triacetate,

gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),

gadolinium 2,2',2"-{10-[1-carboxy-2-{6-[2-(2-ethoxyethoxy)ethoxy] pyridin-3-yl}ethyl]-1,4,7,10-tetraazacyclododecane-1,4,7-triyl}triacetate,

gadolinium-2,2',2"-{10-[1-carboxy-4-{4-[2-(2-ethoxyethoxy)ethoxy] phenyl}butyl]-1,4,7,10-tetraazacyclododecane-1,4,7-triyl}tris(3-hydroxypropanoate),

gadolinium (2S,2'S,2"S)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),

gadolinium (2R,2'R,2"R)-2,2',2"-{10-[(1R)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),

gadolinium (2R,2'R,2"R)-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),

gadolinium-2,2',2"-{10-[(1S)-1-carboxy-4-{4-[2-(2-ethoxyethoxy)ethoxy] phenyl}butyl]-1,4,7,10-tetraazacyclododecane-1,4,7-triyl}tris(3-hydroxypropanoate),

gadolinium 2,2',2"-{10-[1-carboxy-2-{4-[2-(2-ethoxyethoxy)ethoxy] phenyl}ethyl]-1,4,7,10-tetraazacyclododecane-1,4,7-triyl}tris(3-hydroxypropanoate),

gadolinium 2,2',2"-{10-[1-carboxy-2-{5-[2-(2-ethoxyethoxy)ethoxy] pyridin-2-yl}ethyl]-1,4,7,10-tetraazacyclododecane-1,4,7-triyl}triacetate,

gadolinium 2,2',2"-{10-[1-carboxy-3-(4-propoxyphenyl)propyl]-1,4,7,10-tetraazacyclododecane-1,4,7-triyl}triacetate,

gadolinium 2,2',2"-{10-[1-carboxy-4-{4-[2-(2-ethoxyethoxy)ethoxy] phenyl}butyl]-1,4,7,10-tetraazacyclododecane-1,4,7-triyl}triacetate,

gadolinium 2,2',2"-{10-[(1S)-1-carboxy-4-{4-[2-(2-ethoxyethoxy)ethoxy] phenyl}butyl]-1,4,7,10-tetraazacyclododecane-1,4,7-triyl}triacetate,

gadolinium 2,2',2"-{10-[(1S)-1-carboxy-5-(4-ethoxyphenyl)pentyl]-1,4,7,10-tetraazacyclo-dodecane-1,4,7-triyl} triacetate,

gadolinium 2,2',2"-{10-[1-carboxy-4-(4-ethoxyphenyl)butyl]-1,4,7,10-tetraazacyclododecane-1,4,7-triyl}triacetate,

gadolinium 2-{7-[1-carboxy-2-(4-ethoxyphenyl)ethyl]-4,10-bis(carboxylatomethyl)-1,4,7,10-tetraazacyclododecan-1-yl}pentanoate,

gadolinium 2,2',2"-{10-[(1S)-4-(4-butoxyphenyl)-1-carboxybutyl]-1,4,7,10-tetraazacyclododecane-1,4,7-triyl} triacetate,

gadolinium 2,2',2"-(10-{1-carboxy-2-[4-(2-ethoxyethoxy)phenyl]ethyl}-1,4,7,10-tetraazacyclododecane-1,4,7-triyl)triacetate,

gadolinium (2S)-2-[4,10-bis(carboxylatomethyl)-7-{1-carboxy-2-[4-(2,2,3,3-tetrafluoropropoxy)phenyl] ethyl}-1,4,7,10-tetraazacyclododecan-1-yl]-3-hydroxypropanoate,

gadolinium (25,2'S,2"S)-2,2',2"-{10-[(1S)-4-(3-butoxyphenyl)-1-carboxybutyl]-1,4,7,10-tetraazacyclododecane-1,4,7-triyl}tris(3-hydroxypropanoate),

gadolinium (2S,2'S)-2,2'-{4-[(1S)-1-carboxy-4-{4-[2-(2-ethoxyethoxy) ethoxy]phenyl}butyl]-10-[(1R)-1-carboxylato-2-hydroxyethyl]-1,4,7,10-tetraazacyclododecane-1,7-diyl}bis(3-hydroxypropanoate),

gadolinium-2,2'-{4-[(1S)-1-carboxy-4-{4-[2-(2-ethoxyethoxy)ethoxy] phenyl}butyl]-10-[1-carboxylato-3-hydroxypropyl]-1,4,7,10-tetraazacyclododecane-1,7-diyl}bis(4-hydroxybutanoate),

gadolinium-2,2'-{4-[1-carboxy-2-{4-[2-(2-ethoxyethoxy)ethoxy] phenyl}ethyl]-10-[1-carboxylato-3-hydroxypropyl]-1,4,7,10-tetraazacyclododecane-1,7-diyl}bis(4-hydroxybutanoate),
gadolinium 2-[7-{1-carboxy-2-[4-(2-ethoxyethoxy)phenyl]ethyl}-4,10-bis(carboxylatomethyl)-1,4,7,10-tetraazacyclododecan-1-yl]-3-methoxypropanoate,
gadolinium 2,2',2"-{10-[2-{3,5-bis[2-(2-ethoxyethoxy)ethoxy]phenyl}-1-carboxyethyl]-1,4,7,10-tetraazacyclododecane-1,4,7-triyl}triacetate,
gadolinium 2,2',2"-{10-[(1S)-2-(2,4-bis{2-[2-(2-ethoxyethoxy)ethoxy]ethoxy}phenyl)-1-carboxyethyl]-1,4,7,10-tetraazacyclododecane-1,4,7-triyl}triacetate,
gadolinium-2-{7-[1-carboxy-2-{4-[2-(2-ethoxyethoxy)ethoxy]phenyl} ethyl]-4,10-bis(carboxylatomethyl)-1,4,7,10-tetraazacyclododecan-1-yl}-3-hydroxypropanoate,
gadolinium-2-{7-[1-carboxy-2-(4-{2-[2-(2-ethoxyethoxy)ethoxy]ethoxy} phenyl)ethyl]-4,10-bis(carboxylatomethyl)-1,4,7,10-tetraazacyclododecan-1-yl}-3-hydroxypropanoate,
gadolinium-2-{4,10-bis(carboxylatomethyl)-7-[1-carboxypropyl]-1,4,7,10-tetraazacyclododecan-1-yl}-3-{4-[2-(2-ethoxyethoxy)ethoxy]phenyl}propanoate,
gadolinium 2,2',2"-{10-[2-(5-butoxypyridin-2-yl)-1-carboxyethyl]-1,4,7,10-tetraazacyclododecane-1,4,7-triyl} triacetate,
gadolinium-2-{4,10-bis(carboxylatomethyl)-7-[1-carboxy-2-methoxyethyl]-1,4,7,10-tetraazacyclododecan-1-yl}-3-[4-(2,2,3,3-tetrafluoropropoxy)phenyl]propanoate,
gadolinium 2,2',2"-(10-{1-carboxy-2-[4-(2,2,3,3-tetrafluoropropoxy) phenyl]ethyl}-1,4,7,10-tetraazacyclododecane-1,4,7-triyl)triacetate,
gadolinium 2,2'2"-(10-{(1R)-1-carboxy-2-[4-(2,2,2-trifluoroethoxy) phenyl]ethyl}-1,4,7,10-tetraazacyclododecane-1,4,7-triyl)triacetate,
gadolinium 2,2',2"-{10-[1-carboxy-2-(4-propoxyphenyl)ethyl]-1,4,7,10-tetraazacyclododecane-1,4,7-triyl}triacetate,
gadolinium (2R,2'R)-2,2'-{7-[(1S)-1-carboxy-4-{4-[2-(2-ethoxyethoxy)ethoxy]phenyl}butyl]-10-[(1S)-1-carboxylato-2-hydroxyethyl]-1,4,7,10-tetraazacyclododecane-1,4-diyl}bis(3-hydroxypropanoate) and
gadolinium (2S,2'S)-2,2'-{7-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecane-1,4-diyl}bis(3-hydroxypropanoate)
or a stereoisomer, a tautomer, an N-oxide, a hydrate, a solvate, or a salt thereof, or a mixture of same.

**8.** The compound of general formula (I) according to any one of claims 6 or 7 in the form of a sodium ($Na^+$) salt of a complex with $Gd^{3+}$, or a stereoisomer, a tautomer, an N-oxide, a hydrate or a solvate thereof, or a mixture of same.

**9.** A method of preparing a compound of general formula (I) in the form of a complex with $Gd^{3+}$ according to any of claims 6 or 7, said method comprising the step of allowing a compound of general formula (I)

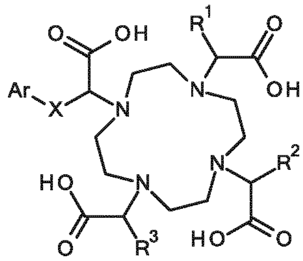

(I) ,

or a stereoisomer, tautomer, N-oxide, hydrate, solvate, or salt thereof, or mixtures of same in which Ar, X, $R^1$, $R^2$ and $R^3$ are as defined for the compounds of general formula (I), according to any one of claims 1 to 5, to react with a Gadolinium (III) salt,
thereby giving a compound of general formula (I), in which Ar, X, $R^1$, $R^2$ and $R^3$ are as defined for the compounds of general formula (I), according to any one of claims 1 to 5, in the form of a complex with $Gd^{3+}$.

**10.** Use of a compound of any one of claims 1 to 8 for diagnostic imaging.

**11.** Use of a compound of any one of claims 1 to 8 for magnetic resonance imaging, preferably of the vascular, renal or of the hepatobiliary system or of the gastrointestinal tract, more preferably for magnetic resonance imaging of the liver.

**12.** The compounds according to any one of claims 1 to 8 for use in diagnostic imaging.

**13.** The compounds according to any one of claims 1 to 8 for use in magnetic resonance imaging, preferably of the vascular, renal or of the hepatobiliary system or of the gastrointestinal tract, more preferably for magnetic resonance imaging of the liver.

**14.** Use of the compounds according to any one of claims 1 to 8 or mixtures thereof for the manufacture of diagnostic agents.

**15.** Use of the compounds according to any one of claims 1 to 8 or mixtures thereof for the manufacture of contrast agents for magnetic resonance imaging.

**16.** A method of imaging body tissue in a patient, comprising the steps of administering to the patient an effective amount of one or more compounds according to any one of claims 6 to 8 in a pharmaceutically acceptable carrier, and subjecting the patient to magnetic resonance imaging.

**17.** Use of a compound of general formula (I)

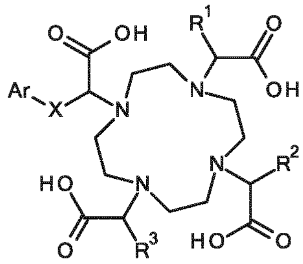

(I) ,

or a stereoisomer, tautomer, N-oxide, hydrate, solvate, or salt thereof, or mixtures of same in which Ar, X, $R^1$, $R^2$ and $R^3$ are as defined for the compounds of general formula (I), according to any one of claims 1 to 5, for the preparation of a compound of general formula (I) in the form of a complex with $Gd^{3+}$ according to any one of claims 6 or 7.

**Patentansprüche**

**1.** Verbindung der allgemeinen Formel (I),

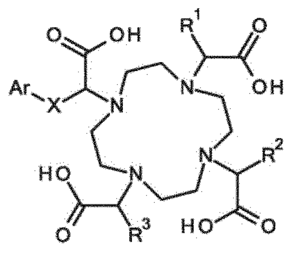

(I),

in der

Ar für eine Gruppe steht, die aus

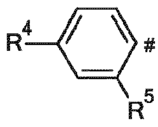, 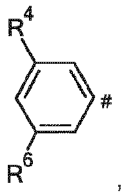, 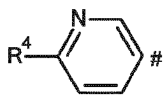 und 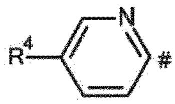,

ausgewählt ist,
wobei # den Punkt der Verknüpfung mit X anzeigt,
X für eine Gruppe steht, die aus $CH_2$, $(CH_2)_2$, $(CH_2)_3$, $(CH_2)_4$ und $*-(CH_2)_2-O-CH_2-^{\#}$ ausgewählt ist,
wobei * den Punkt der Verknüpfung mit Ar anzeigt und # den Punkt der Verknüpfung mit der Essigsäuregruppierung anzeigt,
$R^1$, $R^2$ und $R^3$ unabhängig für jedes Auftreten für ein Wasserstoffatom oder eine Gruppe, die aus $C_1$-$C_3$-Alkyl,-$CH_2OH$, -$(CH_2)_2OH$ und -$CH_2OCH_3$ ausgewählt ist, stehen,
$R^4$ für eine Gruppe steht, die aus $C_2$-$C_5$-Alkoxy, ($C_1$-$C_3$-Alkoxy)-$(CH_2)_2$-O-, ($C_1$-$C_3$-Alkoxy)-$(CH_2)_2$-O-$(CH_2)_2$-O- und ($C_1$-$C_3$-Alkoxy)-$(CH_2)_2$-O-$(CH_2)_2$-O-$(CH_2)_2$-O- ausgewählt ist,
wobei die $C_1$-$C_3$-Alkoxygruppen und $C_2$-$C_5$-Alkoxygruppen gegebenenfalls ein-, zwei-, drei- oder vierfach durch ein Fluoratom substituiert sind,
$R^5$ für ein Wasserstoffatom oder eine Gruppe, die aus $C_2$-$C_5$-Alkoxy, ($C_1$-$C_3$-Alkoxy)-$(CH_2)_2$-O-, ($C_1$-$C_3$-Alkoxy)-$(CH_2)_2$-O-$(CH_2)_2$-O- und ($C_1$-$C_3$-Alkoxy)-$(CH_2)_2$-O-$(CH_2)_2$-O-$(CH_2)_2$-O- ausgewählt ist, steht
und
$R^6$ für ein Wasserstoffatom oder eine Gruppe, die aus $C_2$-$C_5$-Alkoxy, ($C_1$-$C_3$-Alkoxy)-$(CH_2)_2$-O-, ($C_1$-$C_3$-Alkoxy)-$(CH_2)_2$-O-$(CH_2)_2$-O- und ($C_1$-$C_3$-Alkoxy)-$(CH_2)_2$-O-$(CH_2)_2$-O-$(CH_2)_2$-O- ausgewählt ist, steht,
oder ein Stereoisomer, ein Tautomer, ein N-Oxid, ein Hydrat, ein Solvat oder ein Salz davon oder ein Gemisch davon.

**2.** Verbindung nach Anspruch 1, wobei:

Ar für eine Gruppe steht, die aus

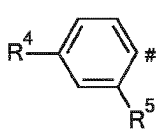, 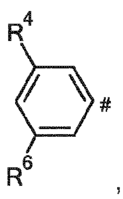, 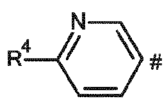 und 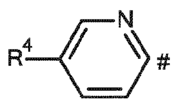,

ausgewählt ist,
wobei # den Punkt der Verknüpfung mit X anzeigt,
X für eine Gruppe steht, die aus $CH_2$, $(CH_2)_2$, $(CH_2)_3$, $(CH_2)_4$ und $*-(CH_2)_2-O-CH_2-^{\#}$ ausgewählt ist,
wobei * den Punkt der Verknüpfung mit Ar anzeigt und # den Punkt der Verknüpfung mit der Essigsäuregruppierung anzeigt,
$R^1$, $R^2$ und $R^3$ unabhängig für jedes Auftreten für ein Wasserstoffatom oder eine Gruppe, die aus $C_1$-$C_3$-Alkyl,-$CH_2OH$, -$(CH_2)_2OH$ und -$CH_2OCH_3$ ausgewählt ist, stehen,
$R^4$ für eine Gruppe steht, die aus $C_2$-$C_5$-Alkoxy, ($C_1$-$C_3$-Alkoxy)-$(CH_2)_2$-O-, ($C_1$-$C_3$-Alkoxy)-$(CH_2)_2$-O-$(CH_2)_2$-O- und ($C_1$-$C_3$-Alkoxy)-$(CH_2)_2$-O-$(CH_2)_2$-O-$(CH_2)_2$-O- ausgewählt ist,
wobei die $C_1$-$C_3$-Alkoxygruppen und $C_2$-$C_5$-Alkoxygruppen gegebenenfalls ein-, zwei-, drei- oder vierfach durch ein Fluoratom substituiert sind,
$R^5$ für ein Wasserstoffatom steht
und
$R^6$ für ein Wasserstoffatom steht
oder ein Stereoisomer, ein Tautomer, ein N-Oxid, ein Hydrat, ein Solvat oder ein Salz davon oder ein Gemisch davon.

**3.** Verbindung nach Anspruch 1 oder 2, wobei:

Ar für eine Gruppe steht, die aus

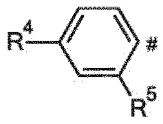 und 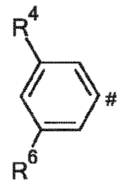 ,

ausgewählt ist,
wobei # den Punkt der Verknüpfung mit X anzeigt,
X für eine Gruppe steht, die aus $CH_2$ und $(CH_2)_3$ ausgewählt ist,
$R^1$ und $R^3$ unabhängig für jedes Auftreten für ein Wasserstoffatom oder eine $-CH_2OH$-Gruppe stehen,
$R^2$ für ein Wasserstoffatom oder eine Gruppe, die aus $C_1$-$C_3$-Alkyl, $-CH_2OH$, $-(CH_2)_2OH$ und $-CH_2OCH_3$ ausgewählt ist, steht,
$R^4$ für eine Gruppe steht, die aus $C_2$-$C_5$-Alkoxy, ($C_1$-$C_3$-Alkoxy)-$(CH_2)_2$-O-, ($C_1$-$C_3$-Alkoxy)-$(CH_2)_2$-O-$(CH_2)_2$-O- und ($C_1$-$C_3$-Alkoxy)-$(CH_2)_2$-O-$(CH_2)_2$-O-$(CH_2)_2$-O- ausgewählt ist,
wobei die $C_1$-$C_3$-Alkoxygruppen und $C_2$-$C_5$-Alkoxygruppen gegebenenfalls ein-, zwei-, drei- oder vierfach durch ein Fluoratom substituiert sind,
$R^5$ für ein Wasserstoffatom steht
und
$R^6$ für ein Wasserstoffatom steht
oder ein Stereoisomer, ein Tautomer, ein Hydrat, ein Solvat oder ein Salz davon oder ein Gemisch davon.

**4.** Verbindung nach Anspruch 1 oder 2, wobei:

Ar für eine Gruppe steht, die aus

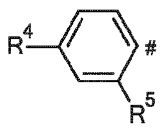 und 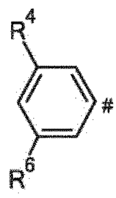 ,

ausgewählt ist,
wobei # den Punkt der Verknüpfung mit X anzeigt,
X für eine Gruppe steht, die aus $CH_2$, $(CH_2)_2$, $(CH_2)_3$, $(CH_2)_4$ und $*-(CH_2)_2-O-CH_2-^{\#}$ ausgewählt ist,
wobei * den Punkt der Verknüpfung mit Ar anzeigt und $^{\#}$ den Punkt der Verknüpfung mit der Essigsäuregruppierung anzeigt,
$R^1$, $R^2$ und $R^3$ unabhängig für jedes Auftreten für ein Wasserstoffatom oder eine Gruppe, die aus $C_1$-$C_3$-Alkyl,$-CH_2OH$, $-(CH_2)_2OH$ und $-CH_2OCH_3$ ausgewählt ist, stehen,
$R^4$ für eine Gruppe steht, die aus $C_2$-$C_4$-Alkoxy, $(H_3C-CH_2)-O-(CH_2)_2-O-$, $(H_3C-CH_2)-O-(CH_2)_2-O-(CH_2)_2-O-$ und $(H_3C-CH_2)-O-(CH_2)_2-O-(CH_2)_2-O-(CH_2)_2-O-$ausgewählt ist,
$R^5$ für ein Wasserstoffatom steht
und
$R^6$ für ein Wasserstoffatom steht
oder ein Stereoisomer, ein Tautomer, ein Hydrat, ein Solvat oder ein Salz davon oder ein Gemisch davon.

**5.** Verbindung nach Anspruch 1, 2 oder 4, wobei:

Ar für eine Gruppe steht, die aus

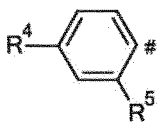 und 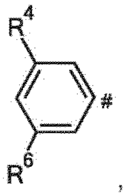 ,

ausgewählt ist,
wobei # den Punkt der Verknüpfung mit X anzeigt,
X für eine Gruppe steht, die aus $CH_2$ und $(CH_2)_3$ ausgewählt ist,
$R^1$, $R^2$ und $R^3$ unabhängig für jedes Auftreten für ein Wasserstoffatom oder eine Gruppe, die aus $C_1$-$C_3$-Alkyl,-$CH_2OH$, -$(CH_2)_2OH$ und -$CH_2OCH_3$ ausgewählt ist, stehen,
$R^4$ für eine Gruppe steht, die aus $(H_3C$-$CH_2)$-O-$(CH_2)_2$-O-, $(H_3C$-$CH_2)$-O-$(CH_2)_2$-O-$(CH_2)_2$-O- und $(H_3C$-$CH_2)$-O-$(CH_2)_2$-O-$(CH_2)_2$-O-$(CH_2)_2$-O- ausgewählt ist,
$R^5$ für ein Wasserstoffatom steht
und
$R^6$ für ein Wasserstoffatom steht
oder ein Stereoisomer, ein Tautomer, ein Hydrat, ein Solvat oder ein Salz davon oder ein Gemisch davon.

**6.** Verbindung der allgemeinen Formel (I) nach einem der Ansprüche 1 bis 5 in Form eines Komplexes mit $Gd^{3+}$ oder ein Stereoisomer, ein Tautomer, ein N-Oxid, ein Hydrat, ein Solvat oder ein Salz davon oder ein Gemisch davon.

**7.** Verbindung nach Anspruch 6, die ausgewählt ist aus der Gruppe bestehend aus:

Gadolinium-2,2',2"-(10-{1-carboxy-2-[2-(4-ethoxyphenyl)ethoxy]ethyl}-1,4,7,10-tetraazacyclododecan-1,4,7-triyl)triacetat,
Gadolinium-2,2',2"-{10-[1-carboxy-2-{4-[2-(2-ethoxyethoxy)ethoxy]phenyl}ethyl]-1,4,7,10-tetraazacyclododecan-1,4,7-triyl}triacetat,
Gadolinium-2,2',2"-{10-[(1S)-1-carboxy-2-{4-[2-(2-ethoxyethoxy)ethoxy]phenyl}ethyl]-1,4,7,10-tetraazacyclododecan-1,4,7-triyl}triacetat,
Gadolinium-2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat,
Gadolinium-2-[7-(1-carboxy-2-{4-[2-(2-ethoxyethoxy)ethoxy]phenyl}-ethyl)-4,10-bis(carboxylatomethyl)-1,4,7,10-tetraazacyclododecan-1-yl]propanoat,
Gadolinium-2-[7-{1-carboxy-2-[4-(2-ethoxyethoxy)phenyl]ethyl}-4,10-bis(carboxylatomethyl)-1,4,7,10-tetraazacyclododecan-1-yl]butanoat,
Gadolinium-(2S,2'S,2"S)-2,2',2"-{10-[(1S)-5-(4-butoxyphenyl)-1-carboxypentyl]-1,4,7,10-tetraazacyclododecan-1,4,7-triyl}tris(3-hydroxypropanoat),
Gadolinium-2,2',2"-{10-[1-carboxy-2-(4-{2-[2-(2-ethoxyethoxy)ethoxy]ethoxy}phenyl)ethyl]-1,4,7,10-tetraazacyclododecan-1,4,7-triyl}triacetat,
Gadolinium-2,2',2"-{10-[(1S)-1-carboxy-2-(4-{2-[2-(2-ethoxyethoxy)ethoxy]ethoxy}phenyl)ethyl]-1,4,7,10-tetraazacyclododecan-1,4,7-triyl}triacetat,
Gadolinium-2,2',2"-{10-[(1R)-1-carboxy-2-(4-{2-[2-(2-ethoxyethoxy)ethoxy]ethoxy}phenyl)ethyl]-1,4,7,10-tetraazacyclododecan-1,4,7-triyl}triacetat,
Gadolinium-2-{7-[1-carboxy-2-(4-{2-[2-(2-ethoxyethoxy)ethoxy]ethoxy}phenyl)ethyl]-4,10-bis(carboxylatomethyl)-1,4,7,10-tetraazacyclododecan-1-yl}-3-methoxypropanoat,
Gadolinium-2,2',2"-{10-[2-(4-butoxyphenyl)-1-carboxyethyl]-1,4,7,10-tetraazacyclododecan-1,4,7-triyl}triacetat,
Gadolinium-(25,2'S,2"S)-2,2',2"-{10-[(1S)-1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-2,2',2"-{10-[1-carboxy-2-{6-[2-(2-ethoxyethoxy)ethoxy]pyridin-3-yl}ethyl]-1,4,7,10-tetraazacyclododecan-1,4,7-triyl}triacetat,
Gadolinium-2,2',2"-{10-[1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-(25,2'S,2"S)-2,2',2"-{10-[(1R)-1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-(2R,2'R,2"R)-2,2',2"-{10-[(1R)-1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-(2R,2’R,2"R)-2,2’,2"-{10-[(1S)-1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-2,2’,2"-{10-[(1S)-1-carboxy-4-{4-[2-(2-ethoxyethoxy)ethoxy]phenyl}butyl]-1,4,7,10-tetraazacyclododecan-1,4,7-triyl}tris(3-hydroxypropanoat),
Gadolinium-2,2’,2"-{10-[1-carboxy-2-{4-[2-(2-ethoxyethoxy)ethoxy]phenyl}ethyl]-1,4,7,10-tetraazacyclododecan-1,4,7-triyl}tris(3-hydroxypropanoat),
Gadolinium-2,2’,2"-{10-[1-carboxy-2-{5-[2-(2-ethoxyethoxy)ethoxy]pyridin-2-yl}ethyl]-1,4,7,10-tetraazacyclododecan-1,4,7-triyl}triacetat,
Gadolinium-2,2’,2"-{10-[1-carboxy-3-(4-propoxyphenyl)propyl]-1,4,7,10-tetraazacyclododecan-1,4,7-triyl}triacetat,
Gadolinium-2,2’,2"-{10-[1-carboxy-4-{4-[2-(2-ethoxyethoxy)ethoxy]phenyl}butyl]-1,4,7,10-tetraazacyclododecan-1,4,7-triyl}triacetat,
Gadolinium-2,2’,2"-{10-[(1S)-1-carboxy-4-{4-[2-(2-ethoxyethoxy)ethoxy]phenyl}butyl]-1,4,7,10-tetraazacyclododecan-1,4,7-triyl}triacetat,
Gadolinium-2,2’,2"-{10-[(1S)-1-carboxy-5-(4-ethoxyphenyl)pentyl]-1,4,7,10-tetraazacyclo-dodecan-1,4,7-triyl} triacetat,
Gadolinium-2,2’,2"-{10-[1-carboxy-4-(4-ethoxyphenyl)butyl]-1,4,7,10-tetraazacyclododecan-1,4,7-triyl}triacetat,
Gadolinium-2-{7-[1-carboxy-2-(4-ethoxyphenyl)ethyl]-4,10-bis(carboxylatomethyl)-1,4,7,10-tetraazacyclododecan-1-yl}pentanoat,
Gadolinium-2,2’,2"-{10-[(1S)-4-(4-butoxyphenyl)-1-carboxybutyl]-1,4,7,10-tetraazacyclododecan-1,4,7-triyl} triacetat,
Gadolinium-2,2’,2"-(10-{1-carboxy-2-[4-(2-ethoxyethoxy)phenyl]ethyl}-1,4,7,10-tetraazacyclododecan-1,4,7-triyl)triacetat,
Gadolinium-(2S)-2-[4,10-bis(carboxylatomethyl)-7-{1-carboxy-2-[4-(2,2,3,3-tetrafluorpropoxy)phenyl] ethyl}-1,4,7,10-tetraazacyclododecan-1-yl]-3-hydroxypropanoat,
Gadolinium-(25,2’S,2"S)-2,2’,2"-{10-[(1S)-4-(3-butoxyphenyl)-1-carboxybutyl]-1,4,7,10-tetraazacyclododecan-1,4,7-triyl}tris(3-hydroxypropanoat),
Gadolinium-(2S,2’S)-2,2’-{4-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecan-1,7-diyl}bis(3-hydroxypropanoat),
Gadolinium-2,2’-{4-[(1S)-1-carboxy-4-{4-[2-(2-ethoxyethoxy)ethoxy]phenyl}butyl]-10-[1-carboxylato-3-hydroxypropyl]-1,4,7,10-tetraazacyclododecan-1,7-diyl}bis(4-hydroxybutanoat),
Gadolinium-2,2’-{4-[1-carboxy-2-{4-[2-(2-ethoxyethoxy)ethoxy]phenyl}ethyl]-10-[1-carboxylato-3-hydroxypropyl]-1,4,7,10-tetraazacyclododecan-1,7-diyl}bis(4-hydroxybutanoat),
Gadolinium-2-[7-{1-carboxy-2-[4-(2-ethoxyethoxy)phenyl]ethyl}-4,10-bis(carboxylatomethyl)-1,4,7,10-tetraazacyclododecan-1-yl]-3-methoxypropanoat,
Gadolinium-2,2’,2"-{10-[2-{3,5-bis[2-(2-ethoxyethoxy)ethoxy]phenyl}-1-carboxyethyl]-1,4,7,10-tetraazacyclododecan-1,4,7-triyl}triacetat,
Gadolinium-2,2’,2"-{10-[(1S)-2-(2,4-bis{2-[2-(2-ethoxyethoxy)ethoxy]ethoxy}phenyl)-1-carboxyethyl]-1,4,7,10-tetraazacyclododecan-1,4,7-triyl}triacetat,
Gadolinium-2-{7-[1-carboxy-2-{4-[2-(2-ethoxyethoxy)ethoxy]phenyl}ethyl]-4,10-bis(carboxylatomethyl)-1,4,7,10-tetraazacyclododecan-1-yl}-3-hydroxypropanoat,
Gadolinium-2-{7-[1-carboxy-2-(4-{2-[2-(2-ethoxyethoxy)ethoxy]ethoxy}phenyl)ethyl]-4,10-bis(carboxylatomethyl)-1,4,7,10-tetraazacyclododecan-1-yl}-3-hydroxypropanoat,
Gadolinium-2-{4,10-bis(carboxylatomethyl)-7-[1-carboxypropyl]-1,4,7,10-tetraazacyclododecan-1-yl}-3-{4-[2-(2-ethoxyethoxy)ethoxy]phenyl}propanoat,
Gadolinium-2,2’,2"-{10-[2-(5-butoxypyridin-2-yl)-1-carboxyethyl]-1,4,7,10-tetraazacyclododecan-1,4,7-triyl} triacetat,
Gadolinium-2-{4,10-bis(carboxylatomethyl)-7-[1-carboxy-2-methoxyethyl]-1,4,7,10-tetraazacyclododecan-1-yl}-3-[4-(2,2,3,3-tetrafluorpropoxy)phenyl]propanoat,
Gadolinium-2,2’,2"-(10-{1-carboxy-2-[4-(2,2,3,3-tetrafluorpropoxy)phenyl]ethyl}-1,4,7,10-tetraazacyclododecan-1,4,7-triyl)triacetat,
Gadolinium-2,2’,2"-(10-{(1R)-1-carboxy-2-[4-(2,2,2-trifluorethoxy)phenyl]ethyl}-1,4,7,10-tetraazacyclododecan-1,4,7-triyl)triacetat,
Gadolinium-2,2’,2"-{10-[1-carboxy-2-(4-propoxyphenyl)ethyl]-1,4,7,10-tetraazacyclododecan-1,4,7-triyl}triacetat,
Gadolinium-(2R,2’R)-2,2’-{7-[(1S)-1-carboxy-4-{4-[2-(2-ethoxyethoxy)ethoxy]phenyl}butyl]-10-[(1S)-1-carboxylato-2-hydroxyethyl]-1,4,7,10-tetraazacyclododecan-1,4-diyl}bis(3-hydroxypropanoat) und Gadolinium-(2S,2'S)-2,2'-{7-[(1S)-1-carboxy-4-{4-[2-(2-ethoxyethoxy)ethoxy]phenyl}butyl]-10-[(1R)-1-carboxylato-2-hydroxyethyl]-1,4,7,10-tetraazacyclododecan-1,4-diyl}bis(3-hydroxypropanoat),
oder ein Stereoisomer, ein Tautomer, ein N-Oxid, ein Hydrat, ein Solvat oder ein Salz davon oder ein Gemisch davon.

**8.** Verbindung der allgemeinen Formel (I) nach Anspruch 6 oder 7 in Form eines Natriumsalzes ($Na^+$-Salzes) eines Komplexes mit $Gd^{3+}$ oder ein Stereoisomer, ein Tautomer, ein N-Oxid, ein Hydrat oder ein Solvat davon oder ein Gemisch davon.

**9.** Verfahren zur Herstellung einer Verbindung der allgemeinen Formel (I) in Form eines Komplexes mit $Gd^{3+}$ nach Anspruch 6 oder 7, wobei das Verfahren den Schritt des Reagierenlassens einer Verbindung der allgemeinen Formel (I)

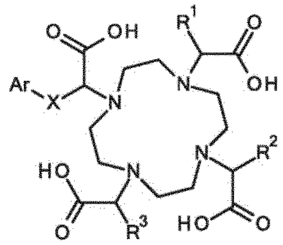

(I),

oder eines Stereoisomers, Tautomers, N-Oxids, Hydrats, Solvats oder Salzes davon oder von Gemischen davon, wobei Ar, X, $R^1$, $R^2$ und $R^3$ wie für die Verbindungen der allgemeinen Formel (I) nach einem der Ansprüche 1 bis 5 definiert sind, mit einem Gadolinium(III)-Salz,
wodurch eine Verbindung der allgemeinen Formel (I), in der Ar, X, $R^1$, $R^2$ und $R^3$ wie für die Verbindungen der allgemeinen Formel (I) nach einem der Ansprüche 1 bis 5 definiert sind, in Form eines Komplexes mit $Gd^{3+}$ erhalten wird.

**10.** Verwendung einer Verbindung nach einem der Ansprüche 1 bis 8 für die diagnostische Bildgebung.

**11.** Verwendung einer Verbindung nach einem der Ansprüche 1 bis 8 für die Magnetresonanztomographie, vorzugsweise des Gefäß-, Nieren- oder Leber-Galle-Systems oder des Magen-Darm-Trakts, besonders bevorzugt für die Magnetresonanztomographie der Leber.

**12.** Verbindungen nach einem der Ansprüche 1 bis 8 zur Verwendung bei der diagnostischen Bildgebung.

**13.** Verbindungen nach einem der Ansprüche 1 bis 8 zur Verwendung bei der Magnetresonanztomographie, vorzugsweise des Gefäß-, Nieren- oder Leber-Galle-Systems oder des Magen-Darm-Trakts, besonders bevorzugt für die Magnetresonanztomographie der Leber.

**14.** Verwendung der Verbindungen nach einem der Ansprüche 1 bis 8 oder von Mischungen davon zur Herstellung von Diagnostika.

**15.** Verwendung der Verbindungen nach einem der Ansprüche 1 bis 8 oder von Mischungen davon zur Herstellung von Kontrastmitteln für die Magnetresonanztomographie.

**16.** Verfahren zur Abbildung von Körpergewebe in einem Patienten, umfassend die Schritte des Verabreichens einer wirksamen Menge einer oder mehrerer Verbindungen nach einem der Ansprüche 6 bis 8 in einem pharmazeutisch unbedenklichen Träger an den Patienten und der Durchführung von Magnetresonanzbildgebung an dem Patienten.

**17.** Verwendung einer Verbindung der allgemeinen Formel (I)

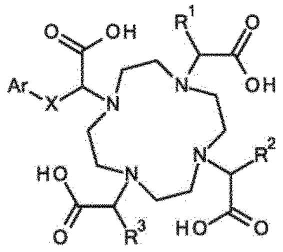

(I),

oder eines Stereoisomers, Tautomers, N-Oxids, Hydrats, Solvats oder Salzes davon oder von Gemischen davon, wobei Ar, X, $R^1$, $R^2$ und $R^3$ wie für die Verbindungen der allgemeinen Formel (I) nach einem der Ansprüche 1 bis 5 definiert sind, zur Herstellung einer Verbindung der allgemeinen Formel (I) in Form eines Komplexes mit $Gd^{3+}$ nach Anspruch 6 oder 7.

## Revendications

**1.** Composé de formule générale (I),

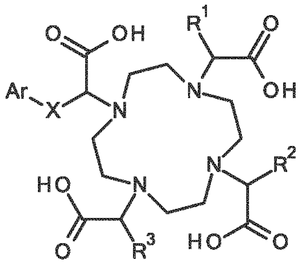

(I),

dans laquelle

Ar représente un groupe choisi parmi

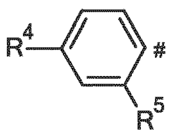 , 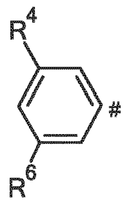 , 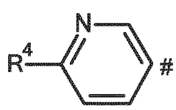 et 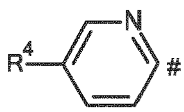 ,

dans laquelle # indique le point de fixation à X,
X représente un groupe choisi parmi $CH_2$, $(CH_2)_2$, $(CH_2)_3$, $(CH_2)_4$ et *- $(CH_2)_2$-O-$CH_2$-#,
dans laquelle * indique le point de fixation à Ar et # indique le point de fixation au groupement acide acétique,
$R^1$, $R^2$ et $R^3$ représentent, indépendamment pour chaque occurrence, un atome d'hydrogène ou un groupe choisi parmi $C_1$-$C_3$-alkyle, -$CH_2OH$, -$(CH_2)_2OH$ et -$CH_2OCH_3$,
$R^4$ représente un groupe choisi parmi $C_2$-$C_5$-alcoxy, ($C_1$-$C_3$-alcoxy)-$(CH_2)_2$-O-, ($C_1$-$C_3$-alcoxy)-$(CH_2)_2$-O-$(CH_2)_2$-O- et ($C_1$-$C_3$-alcoxy)-$(CH_2)_2$-O-$(CH_2)_2$-O-$(CH_2)_2$-O-,
dans laquelle lesdits groupes $C_1$-$C_3$-alcoxy et groupes $C_2$-$C_5$-alcoxy sont éventuellement substitués une, deux, trois ou quatre fois, par un atome de fluor,
$R^5$ représente un atome d'hydrogène ou un groupe choisi parmi
$C_2$-$C_5$-alcoxy, ($C_1$-$C_3$-alcoxy)-$(CH_2)_2$-O-, ($C_1$-$C_3$-alcoxy)-$(CH_2)_2$-O-$(CH_2)_2$-O- et ($C_1$-$C_3$-alcoxy)-$(CH_2)_2$-O-$(CH_2)_2$-O-$(CH_2)_2$-O-,
et
$R^6$ représente un atome d'hydrogène ou un groupe choisi parmi

$C_2$-$C_5$-alcoxy, ($C_1$-$C_3$-alcoxy)-$(CH_2)_2$-O-, ($C_1$-$C_3$-alcoxy)-$(CH_2)_2$-O-$(CH_2)_2$-O- et ($C_1$-$C_3$-alcoxy)-$(CH_2)_2$-O-$(CH_2)_2$-O-$(CH_2)_2$-O-,
ou stéréoisomère, forme tautomère, N-oxyde, hydrate, solvate, ou sel correspondant, ou mélange de ceux-ci.

**2.** Composé selon la revendication 1, dans lequel

Ar représente un groupe choisi parmi

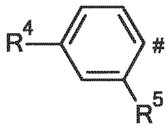 , 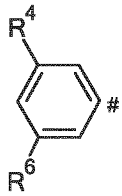 , 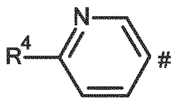 et 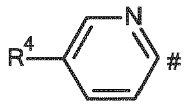 ,

dans laquelle # indique le point de fixation à X,
X représente un groupe choisi parmi $CH_2$, $(CH_2)_2$, $(CH_2)_3$, $(CH_2)_4$ et *-$(CH_2)_2$-O-$CH_2$-#,
dans laquelle * indique le point de fixation à Ar et # indique le point de fixation au groupement acide acétique,
$R^1$, $R^2$ et $R^3$ représentent, indépendamment pour chaque occurrence, un atome d'hydrogène ou un groupe choisi parmi $C_1$-$C_3$-alkyle, -$CH_2OH$, -$(CH_2)_2OH$ et -$CH_2OCH_3$,
$R^4$ représente un groupe choisi parmi $C_2$-$C_5$-alcoxy, ($C_1$-$C_3$-alcoxy)-$(CH_2)_2$-O-, ($C_1$-$C_3$-alcoxy)-$(CH_2)_2$-O-$(CH_2)_2$-O- et ($C_1$-$C_3$-alcoxy)-$(CH_2)_2$-O-$(CH_2)_2$-O-$(CH_2)_2$-O-,
dans laquelle lesdits groupes $C_1$-$C_3$-alcoxy et groupes $C_2$-$C_5$-alcoxy sont éventuellement substitués une, deux, trois ou quatre fois, par un atome de fluor,
$R^5$ représente un atome d'hydrogène,
et
$R^6$ représente un atome d'hydrogène,
ou stéréoisomère, forme tautomère, N-oxyde, hydrate, solvate, ou sel correspondant, ou mélange de ceux-ci.

**3.** Composé selon l'une quelconque des revendications 1 ou 2, dans lequel

Ar représente un groupe choisi parmi

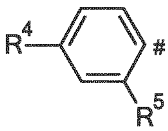 et 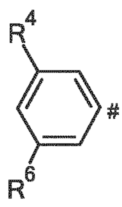 ,

dans laquelle # indique le point de fixation à X,
X représente un groupe choisi parmi $CH_2$ et $(CH_2)_3$,
$R^1$ et $R^3$ représentent, indépendamment pour chaque occurrence, un atome d'hydrogène ou un groupe -$CH_2OH$,
$R^2$ représente un atome d'hydrogène ou un groupe choisi parmi $C_1$-$C_3$-alkyle, -$CH_2OH$, -$(CH_2)_2OH$ et -$CH_2OCH_3$,
$R^4$ représente un groupe choisi parmi $C_2$-$C_5$-alcoxy, ($C_1$-$C_3$-alcoxy)-$(CH_2)_2$-O-, ($C_1$-$C_3$-alcoxy)-$(CH_2)_2$-O-$(CH_2)_2$-O- et ($C_1$-$C_3$-alcoxy)-$(CH_2)_2$-O-$(CH_2)_2$-O-$(CH_2)_2$-O-,
dans laquelle lesdits groupes $C_1$-$C_3$-alcoxy et groupes $C_2$-$C_5$-alcoxy sont éventuellement substitués une, deux, trois ou quatre fois, par un atome de fluor,
$R^5$ représente un atome d'hydrogène,
et
$R^6$ représente un atome d'hydrogène,
ou stéréoisomère, forme tautomère, hydrate, solvate, ou sel correspondant, ou mélange de ceux-ci.

**4.** Composé selon l'une quelconque des revendications 1 ou 2, dans lequel

Ar représente un groupe choisi parmi

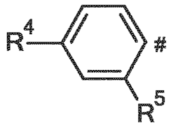 et 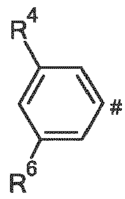 ,

dans laquelle # indique le point de fixation à X,
X représente un groupe choisi parmi $CH_2$, $(CH_2)_2$, $(CH_2)_3$, $(CH_2)_4$ et $*-(CH_2)_2-O-CH_2-^{\#}$,
dans laquelle * indique le point de fixation à Ar et # indique le point de fixation au groupement acide acétique,
$R^1$, $R^2$ et $R^3$ représentent, indépendamment pour chaque occurrence, un atome d'hydrogène ou un groupe choisi parmi $C_1$-$C_3$-alkyle, $-CH_2OH$, $-(CH_2)_2OH$ et $-CH_2OCH_3$,
$R^4$ représente un groupe choisi parmi $C_2$-$C_4$-alcoxy, $(H_3C-CH_2)-O-(CH_2)_2-O-$, $(H_3C-CH_2)-O-(CH_2)_2-O-(CH_2)_2-O-$ et $(H_3C-CH_2)-O-(CH_2)_2-O-(CH_2)_2-O-(CH_2)_2-O-$,
$R^5$ représente un atome d'hydrogène,
et
$R^6$ représente un atome d'hydrogène,
ou stéréoisomère, forme tautomère, hydrate, solvate, ou sel correspondant, ou mélange de ceux-ci.

**5.** Composé selon l'une quelconque des revendications 1, 2 ou 4, dans lequel

Ar représente un groupe choisi parmi

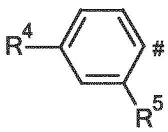 et 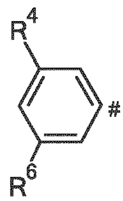 ,

dans laquelle # indique le point de fixation à X,
X représente un groupe choisi parmi $CH_2$ et $(CH_2)_3$,
$R^1$, $R^2$ et $R^3$ représentent, indépendamment pour chaque occurrence, un atome d'hydrogène ou un groupe choisi parmi $C_1$-$C_3$-alkyle, $-CH_2OH$, $-(CH_2)_2OH$ et $-CH_2OCH_3$,
$R^4$ représente un groupe choisi parmi $(H_3C-CH_2)-O-(CH_2)_2-O-$, $(H_3C-CH_2)-O-(CH_2)_2-O-(CH_2)_2-O-$ et $(H_3C-CH_2)-O-(CH_2)_2-O-(CH_2)_2-O-(CH_2)_2-O-$,
$R^5$ représente un atome d'hydrogène,
et
$R^6$ représente un atome d'hydrogène,
ou stéréoisomère, forme tautomère, hydrate, solvate, ou sel correspondant, ou mélange de ceux-ci.

**6.** Composé de formule générale (I) selon l'une quelconque des revendications 1 à 5 sous la forme d'un complexe avec $Gd^{3+}$, ou stéréoisomère, forme tautomère, N-oxyde, hydrate, solvate, ou sel correspondant, ou mélange de ceux-ci.

**7.** Composé selon la revendication 6, choisi dans le groupe constitué par

2,2',2"-(10-{1-carboxy-2-[2-(4-éthoxyphényl)éthoxy]éthyl}-1,4,7,10-tétraazacyclododécane-1,4,7-triyl)triacétate de gadolinium,
2,2',2"-{10-[1-carboxy-2-{4-[2-(2-éthoxyéthoxy)éthoxy]phényl}éthyl]-1,4,7,10-tétraazacyclododécane-1,4,7-triyl}triacétate de gadolinium,
2,2',2"-{10-[(1S)-1-carboxy-2-{4-[2-(2-éthoxyéthoxy)éthoxy]phényl}éthyl]-1,4,7,10-tétraazacyclododécane-1,4,7-triyl}triacétate de gadolinium,
2,2',2"-{10-[(1R)-1-carboxy-2-{4-[2-(2-éthoxyéthoxy)éthoxy]phényl}éthyl]-1,4,7,10-tétraazacyclododécane-1,4,7-triyl}triacétate de gadolinium,

2-[7-(1-carboxy-2-{4-[2-(2-éthoxyéthoxy)éthoxy]phényl}éthyl)-4,10-bis(carboxylatométhyl)-1,4,7,10-tétraazacyclododécane-1-yl]propanoate de gadolinium,
2-[7-{1-carboxy-2-[4-(2-éthoxyéthoxy)phényl]éthyl}-4,10-bis(carboxylatométhyl)-1,4,7,10-tétraazacyclododécane-1-yl]butanoate de gadolinium, (2S,2'S,2"S)-2,2',2"-{10-[(1S)-5-(4-butoxyphényl)-1-carboxypentyl]-1,4,7,10-tétraazacyclododécane-1,4,7-triyl}tris(3-hydroxypropanoate) de gadolinium,
2,2',2"-{10-[1-carboxy-2-(4-{2-[2-(2-éthoxyéthoxy)éthoxy]éthoxy}phényl)éthyl]-1,4,7,10-tétraazacyclododécane-1,4,7-triyl}triacétate de gadolinium,
2,2',2"-{10-[(1S)-1-carboxy-2-(4-{2-[2-(2-éthoxyéthoxy)éthoxy]éthoxy}phényl)éthyl]-1,4,7,10-tétraazacyclododécane-1,4,7-triyl}triacétate de gadolinium,
2,2',2"-{10-[(1R)-1-carboxy-2-(4-{2-[2-(2-éthoxyéthoxy)éthoxy]éthoxy}phényl)éthyl]-1,4,7,10-tétraazacyclododécane-1,4,7-triyl}triacétate de gadolinium,
2-{7-[1-carboxy-2-(4-{2-[2-(2-éthoxyéthoxy)éthoxy]éthoxy}phényl)éthyl]-4,10-bis(carboxylatométhyl)-1,4,7,10-tétraazacyclododécane-1-yl}-3-méthoxypropanoate de gadolinium,
2,2',2"-{10-[2-(4-butoxyphényl)-1-carboxyéthyl]-1,4,7,10-tétraazacyclododécane-1,4,7-triyl}triacétate de gadolinium,
(2S,2'S,2"S)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
2,2',2"-{10-[1-carboxy-2-{6-[2-(2-éthoxyéthoxy)éthoxy]pyridin-3-yl}éthyl]-1,4,7,10-tétraazacyclododécane-1,4,7-triyl}triacétate de gadolinium,
2,2',2"-{10-[1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
(2S,2'S,2"S)-2,2',2"-{10-[(1R)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
(2R,2'R,2"R)-2,2',2"-{10-[(1R)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
(2R,2'R,2"R)-2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
2,2',2"-{10-[(1S)-1-carboxy-4-{4-[2-(2-éthoxyéthoxy)éthoxy]phényl}butyl]-1,4,7,10-tétraazacyclododécane-1,4,7-triyl}tris(3-hydroxypropanoate) de gadolinium,
2,2',2"-{10-[1-carboxy-2-{4-[2-(2-éthoxyéthoxy)éthoxy]phényl}éthyl]-1,4,7,10-tétraazacyclododécane-1,4,7-triyl}tris(3-hydroxypropanoate) de gadolinium,
2,2',2"-{10-[1-carboxy-2-{5-[2-(2-éthoxyéthoxy)éthoxy]pyridin-2-yl}éthyl]-1,4,7,10-tétraazacyclododécane-1,4,7-triyl}triacétate de gadolinium,
2,2',2"-{10-[1-carboxy-3-(4-propoxyphényl)propyl]-1,4,7,10-tétraazacyclododécane-1,4,7-triyl}triacétate de gadolinium,
2,2',2"-{10-[1-carboxy-4-{4-[2-(2-éthoxyéthoxy)éthoxy]phényl}butyl]-1,4,7,10-tétraazacyclododécane-1,4,7-triyl}triacétate de gadolinium,
2,2',2"-{10-[(1S)-1-carboxy-4-{4-[2-(2-éthoxyéthoxy)éthoxy]phényl}butyl]-1,4,7,10-tétraazacyclododécane-1,4,7-triyl}triacétate de gadolinium,
2,2',2"-{10-[(1S)-1-carboxy-5-(4-éthoxyphényl)pentyl]-1,4,7,10-tétraazacyclo-dodécane-1,4,7-triyl}triacétate de gadolinium,
2,2',2"-{10-[1-carboxy-4-(4-éthoxyphényl)butyl]-1,4,7,10-tétraazacyclododécane-1,4,7-triyl}triacétate de gadolinium,
2-{7-[1-carboxy-2-(4-éthoxyphényl)éthyl]-4,10-bis(carboxylatométhyl)-1,4,7,10-tétraazacyclododécane-1-yl} pentanoate de gadolinium,
2,2',2"-{10-[(1S)-4-(4-butoxyphényl)-1-carboxybutyl]-1,4,7,10-tétraazacyclododécane-1,4,7-triyl}triacétate de gadolinium,
2,2',2"-(10-{1-carboxy-2-[4-(2-éthoxyéthoxy)phényl]éthyl}-1,4,7,10-tétraazacyclododécane-1,4,7-triyl)triacétate de gadolinium,
(2S)-2-[4,10-bis(carboxylatométhyl)-7-{1-carboxy-2-[4-(2,2,3,3-tétrafluoropropoxy)phényl]éthyl}-1,4,7,10-tétraazacyclododécane-1-yl]-3-hydroxypropanoate de gadolinium,
(2S,2'S,2"S)-2,2',2"-{10-[(1S)-4-(3-butoxyphényl)-1-carboxybutyl]-1,4,7,10-tétraazacyclododécane-1,4,7-triyl} tris(3-hydroxypropanoate) de gadolinium,
(2S,2'S)-2,2'-{4-[(1S)-1-carboxy-4-{4-[2-(2-éthoxyéthoxy) éthoxy]phényl}butyl]-10-[(1R)-1-carboxylato-2-hydroxyéthyl]-1,4,7,10-tétraazacyclododécane-1,7-diyl}bis(3-hydroxypropanoate) de gadolinium,
2,2'-{4-[(1S)-1-carboxy-4-{4-[2-(2-éthoxyéthoxy)éthoxy]phényl}butyl]-10-[1-carboxylato-3-hydroxypropyl]-1,4,7,10-tétraazacyclododécane-1,7-diyl}bis(4-hydroxybutanoate) de gadolinium,
2,2'-{4-[1-carboxy-2-{4-[2-(2-éthoxyéthoxy)éthoxy]phényl}éthyl]-10-[1-carboxylato-3-hydroxypropyl]-1,4,7,10-tétraazacyclododécane-1,7-diyl}bis(4-hydroxybutanoate) de gadolinium,
2-[7-{1-carboxy-2-[4-(2-éthoxyéthoxy)phényl]éthyl}-4,10-bis(carboxylatométhyl)-1,4,7,10-tétraazacyclododécane-1-yl]-3-méthoxypropanoate de gadolinium,
2,2',2"-{10-[2-{3,5-bis[2-(2-éthoxyéthoxy) éthoxy]phényl}-1-carboxyéthyl]-1,4,7,10-tétraazacyclododécane-1,4,7-triyl}triacétate de gadolinium,
2,2',2"-{10-[(1S)-2-(2,4-bis{2-[2-(2-éthoxyéthoxy) éthoxy]éthoxy}phényl)-1-carboxyéthyl]-1,4,7,10-tétraazacyclododécane-1,4,7-triyl}triacétate de gadolinium,
2-{7-[1-carboxy-2-{4-[2-(2-éthoxyéthoxy)éthoxy]phényl} éthyl]-4,10-bis(carboxylatométhyl)-1,4,7,10-tétraazacyclododécane-1-yl}-3-hydroxypropanoate de gadolinium,
2-{7-[1-carboxy-2-(4-{2-[2-(2-éthoxyéthoxy)éthoxy]éthoxy} phényl)éthyl]-4,10-bis(carboxylatométhyl)-1,4,7,10-tétraazacyclododécane-1-yl}-3-hydroxypropanoate de gadolinium,
2-{4,10-bis(carboxylatométhyl)-7-[1-carboxypropyl]-1,4,7,10-tétraazacyclododécane-1-yl}-3-{4-[2-(2-éthoxyéthoxy)éthoxy]phényl}propanoate de gadolinium,
2,2',2"-{10-[2-(5-butoxypyridin-2-yl)-1-carboxyéthyl]-1,4,7,10-tétraazacyclododécane-1,4,7-triyl}triacétate de gadolinium,
2-{4,10-bis(carboxylatométhyl)-7-[1-carboxy-2-méthoxyéthyl]-1,4,7,10-tétraazacyclododécane-1-yl}-3-[4-(2,2,3,3-tétrafluoropropoxy)phényl]propanoate de gadolinium,
2,2',2"-(10-{1-carboxy-2-[4-(2,2,3,3-tétrafluoropropoxy) phényl]éthyl}-1,4,7,10-tétraazacyclododécane-1,4,7-triyl)triacétate de gadolinium,
2,2',2"-(10-{(1R)-1-carboxy-2-[4-(2,2,2-trifluoroéthoxy) phényl]éthyl}-1,4,7,10-tétraazacyclododécane-1,4,7-triyl)triacétate de gadolinium,
2,2',2"-{10-[1-carboxy-2-(4-propoxyphényl)éthyl]-1,4,7,10-tétraazacyclododécane-1,4,7-triyl}triacétate de gadolinium,
(2R,2'R)-2,2'-{7-[(1S)-1-carboxy-4-{4-[2-(2-éthoxyéthoxy)éthoxy]phényl}butyl]-10-[(1S)-1-carboxylato-2-hydroxyéthyl]-1,4,7,10-tétraazacyclododécane-1,4-diyl}bis(3-hydroxypropanoate) de gadolinium et
(2S,2'S)-2,2'-{7-[(1S)-1-carboxy-4-{4-[2-(2-éthoxyéthoxy)éthoxy]phényl}butyl]-10-[(1R)-1-carboxylato-2-hydroxyéthyl]-1,4,7,10-tétraazacyclododécane-1,4-diyl}bis(3-hydroxypropanoate) de gadolinium
ou stéréoisomère, forme tautomère, N-oxyde, hydrate, solvate, ou sel correspondant, ou mélange de ceux-ci.

8. Composé de formule générale (I) selon l'une quelconque des revendications 6 ou 7 sous la forme d'un sel de sodium ($Na^+$) d'un complexe avec $Gd^{3+}$, ou stéréoisomère, forme tautomère, N-oxyde, hydrate ou solvate correspondant, ou mélange de ceux-ci.

9. Procédé de préparation d'un composé de formule générale(I) sous la forme d'un complexe avec $Gd^{3+}$ selon l'une quelconque des revendications 6 ou 7, ledit procédé comprenant l'étape du fait de laisser réagir un composé de formule générale (I)

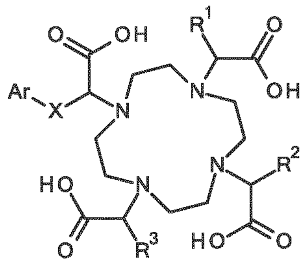

(I),

ou un stéréoisomère, une forme tautomère, N-oxyde, hydrate, solvate ou un sel correspondant, ou des mélanges de ceux-ci dans laquelle Ar, X, $R^1$, $R^2$ et $R^3$ sont tels que définis pour les composés de formule générale (I), selon l'une quelconque des revendications 1 à 5, avec un sel de gadolinium (III),
donnant ainsi un composé de formule générale (I), dans laquelle Ar, X, $R^1$, $R^2$ et $R^3$ sont tels que définis pour les composés de formule générale (I), selon l'une quelconque des revendications 1 à 5, sous la forme d'un complexe avec $Gd^{3+}$.

10. Utilisation d'un composé selon l'une quelconque des revendications 1 à 8 pour l'imagerie diagnostique.

11. Utilisation d'un composé selon l'une quelconque des revendications 1 à 8 pour l'imagerie par résonance magnétique,

de préférence du système vasculaire, rénal ou hépatobiliaire ou du tractus gastro-intestinal, plus préférablement pour l'imagerie par résonance magnétique du foie.

**12.** Composés selon l'une quelconque des revendications 1 à 8, destinés à être utilisés dans l'imagerie diagnostique.

**13.** Composés selon l'une quelconque des revendications 1 à 8, destinés à être utilisés dans l'imagerie par résonance magnétique, de préférence du système vasculaire, rénal ou hépatobiliaire ou du tractus gastro-intestinal, plus préférablement pour l'imagerie par résonance magnétique du foie.

**14.** Utilisation des composés selon l'une quelconque des revendications 1 à 8 ou de mélanges de ceux-ci pour la fabrication d'agents de diagnostic.

**15.** Utilisation des composés selon l'une quelconque des revendications 1 à 8 ou de mélanges de ceux-ci pour la fabrication d'agents de contraste pour l'imagerie par résonance magnétique.

**16.** Procédé d'imagerie d'un tissu corporel chez un patient, comprenant les étapes d'administration au patient d'une quantité efficace d'un ou plusieurs composés selon l'une quelconque des revendications 6 à 8 dans un support pharmaceutiquement acceptable, et de soumission du patient à une imagerie par résonance magnétique.

**17.** Utilisation d'un composé de formule générale (I)

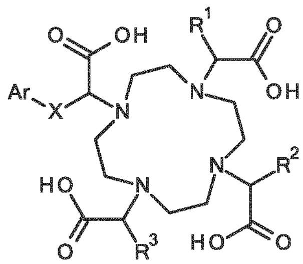

(I),

ou d'un stéréoisomère, d'une forme tautomère, d'un N-oxyde, d'un hydrate, d'un solvate ou d'un sel correspondant, ou de mélanges de ceux-ci dans laquelle Ar, X, $R^1$, $R^2$ et $R^3$ sont tels que définis pour les composés de formule générale (I), selon l'une quelconque des revendications 1 à 5, pour la préparation d'un composé de formule générale (I) sous la forme d'un complexe avec $Gd^{3+}$ selon l'une quelconque des revendications 6 ou 7.

## FIGURES – Figure 1

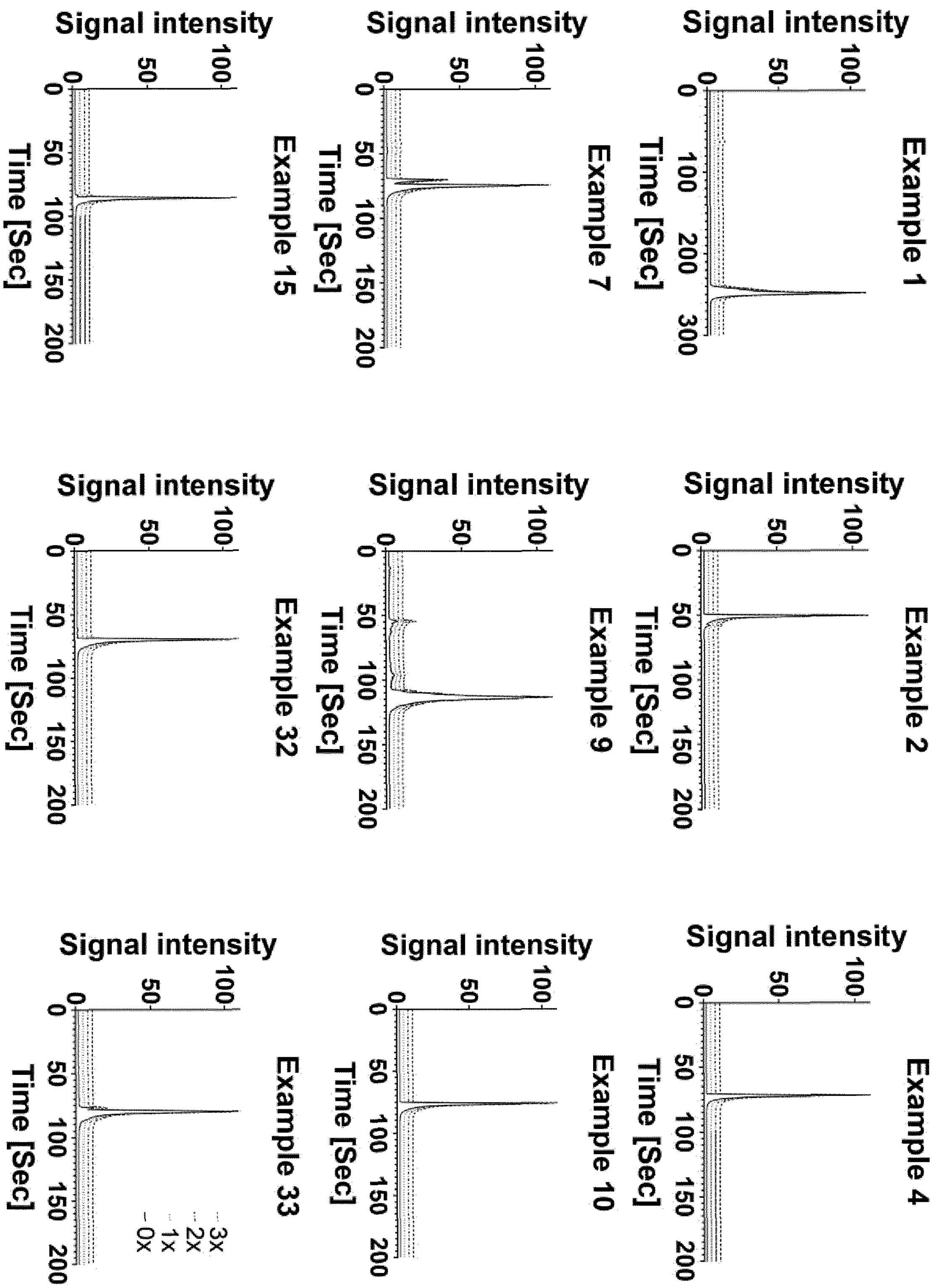

Figure 2

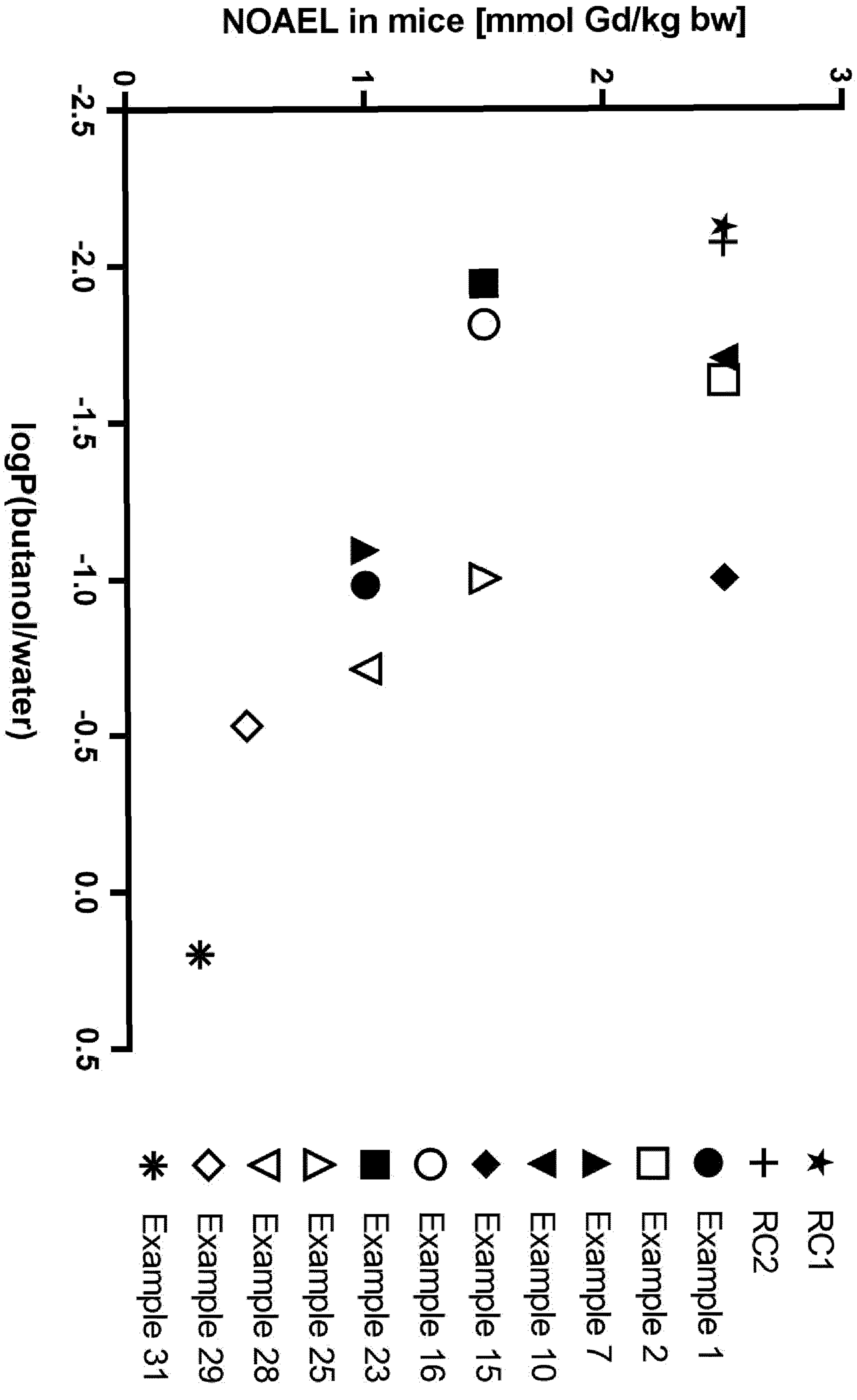
NOAEL in mice [mmol Gd/kg bw]
0
1
2
3
-2.5
-2.0
-1.5
-1.0
-0.5
0.0
0.5
logP(butanol/water)
RC1
RC2
Example 1
Example 2
Example 7
Example 10
Example 15
Example 16
Example 23
Example 25
Example 28
Example 29
Example 31

Figure 3

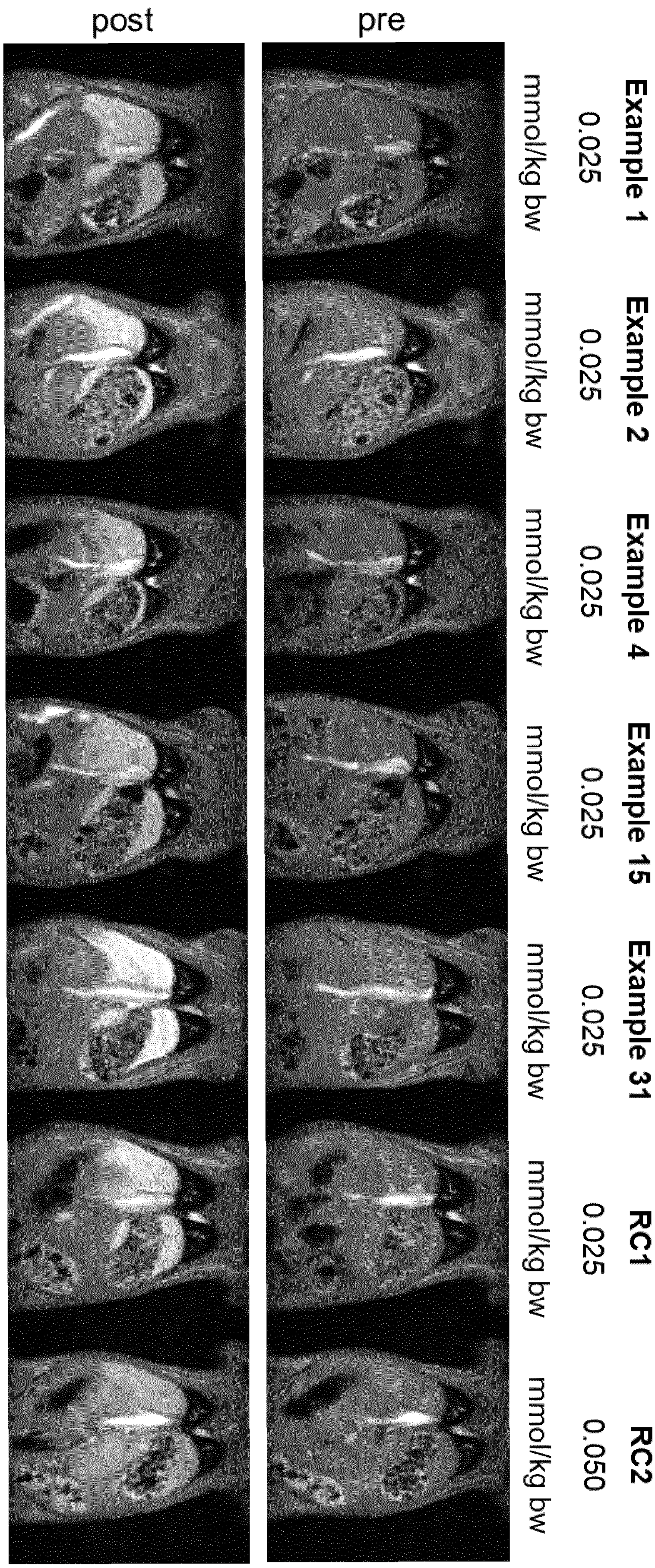
post
pre
Example 1
0.025
mmol/kg bw
Example 2
0.025
mmol/kg bw
Example 4
0.025
mmol/kg bw
Example 15
0.025
mmol/kg bw
Example 31
0.025
mmol/kg bw
RC1
0.025
mmol/kg bw
RC2
0.050
mmol/kg bw

Figure 4

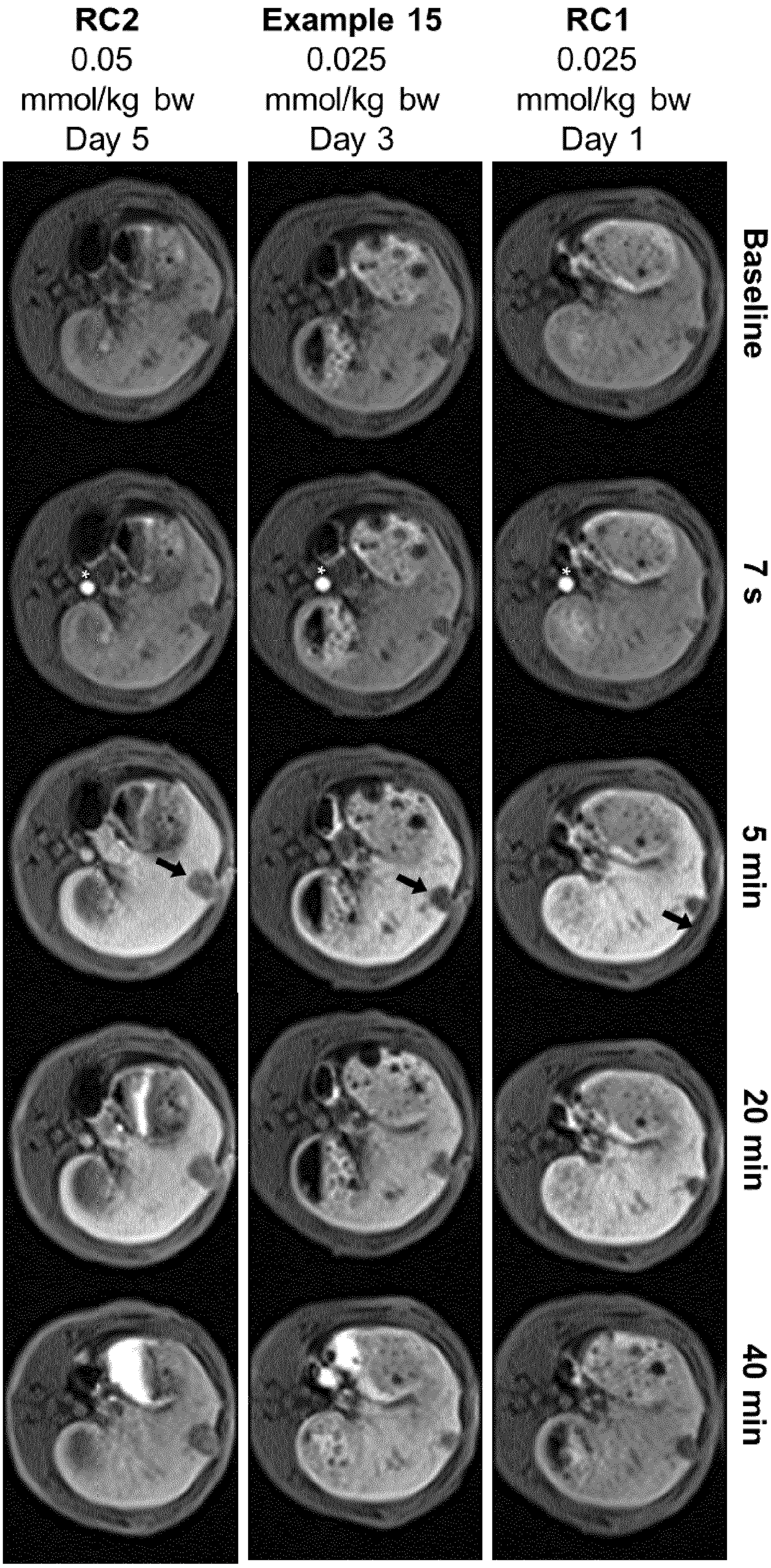
RC2
0.05
mmol/kg bw
Day 5
Example 15
0.025
mmol/kg bw
Day 3
RC1
0.025
mmol/kg bw
Day 1
Baseline
7 s
5 min
20 min
40 min
*

Figure 5 (A)

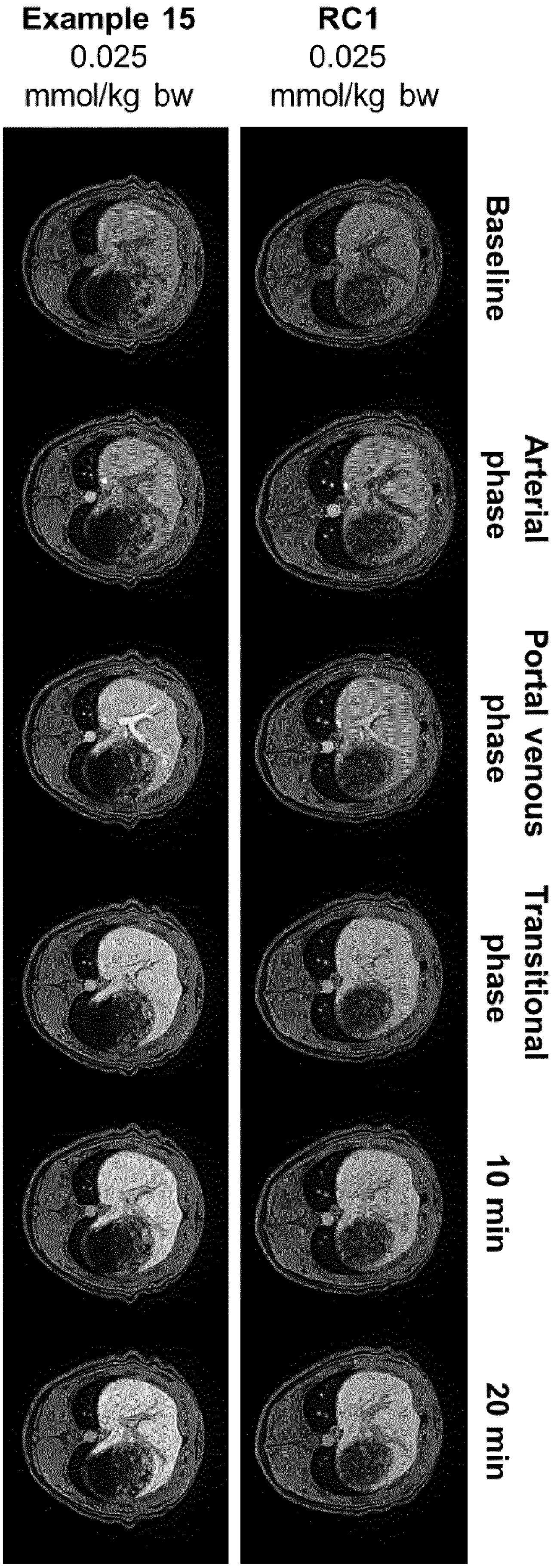
Example 15
0.025
mmol/kg bw
RC1
0.025
mmol/kg bw
Baseline
Arterial phase
Portal venous phase
Transitional phase
10 min
20 min

Figure 5 (B)

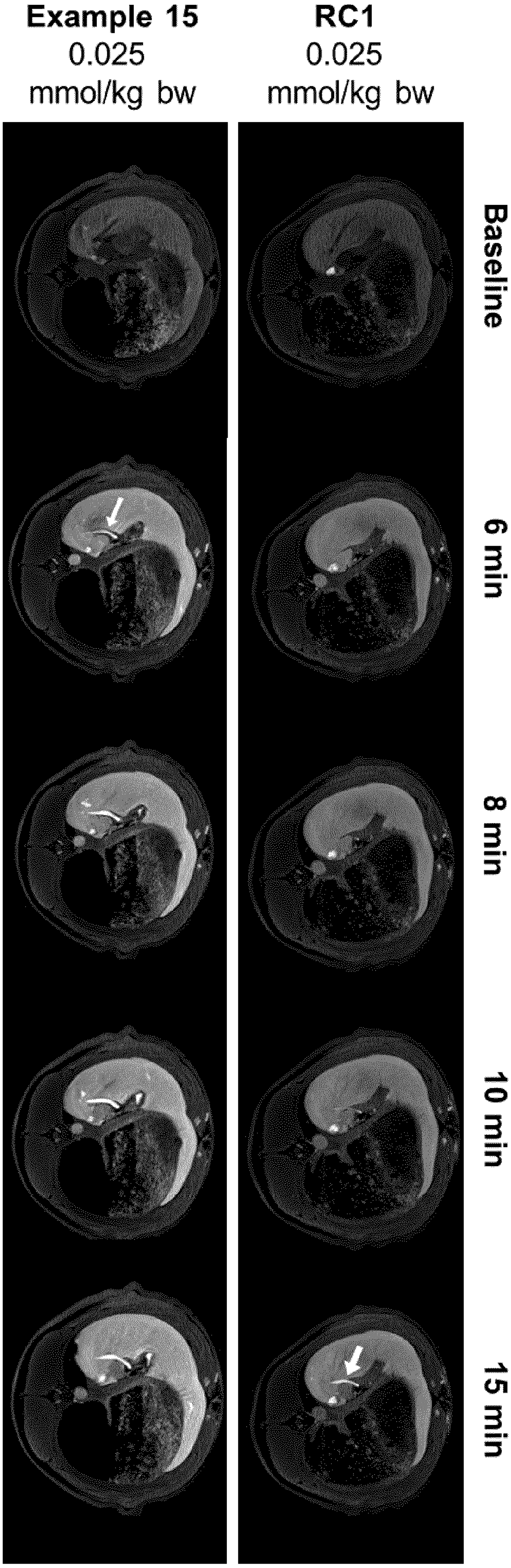
Example 15
0.025
mmol/kg bw
RC1
0.025
mmol/kg bw
Baseline
6 min
8 min
10 min
15 min

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199532741 A
- WO 2001082795 A
- WO 2007009638 A
- WO 2004006965 A
- WO 1997032862 A
- WO 1999005145 A
- WO 1996016677 A
- WO 1995028392 A
- WO 2013083535 A
- EP 405704 A
- US 5560903 A


### Non-patent literature cited in the description


- **LAUFFER RB et al.** Paramagnetic metal complexes as water proton relaxation agents for NMR imaging: theory and design. *Chem Rev.*, 1987, vol. 87 (5), 901-27
- **CARAVAN P et al.** Gadolinium(III) chelates as MRI contrast agents: structure, dynamics, and applications. *Chem Rev.*, 1999, vol. 99 (9), 2293-352
- **SEALE MK et al.** *Radiographics*, 2009, vol. 29, 1725-1748
- **FIDLER J et al.** *Hepatology*, 2011, vol. 53 (2), 678-82
- **PARK Y et al.** *Korean J Radiol*, 2010, vol. 11 (4), 433-40
- **BLUEMKE DA et al.** *Radiology*, 2005, vol. 237, 89-98
- **RINGE KI et al.** *American Journal of Roentgenology*, 2010, vol. 195, 13-28
- **LEONHARDT M et al.** *Drug Metab Dispos*, July 2010, vol. 38 (7), 1024-8
- **WEINMANN HJ et al.** *Magn Reson Med*, 1991, vol. 22, 233-237
- **ROHRER M et al.** *Invest Radiol*, November 2005, vol. 40 (11), 715-24
- **KAHN J et al.** *Radiology*, 2017, vol. 282 (3), 708-716
- **FRENZEL et al.** *Invest Radiol*, December 2008, vol. 43 (12), 817-28
- **S. M. BERGE et al.** Pharmaceutical Salts. *J. Pharm. Sci*, 1977, vol. 66, 1-19
- *CHEMICAL ABSTRACTS*, 162148-48-3
- *CHEMICAL ABSTRACTS*, 162148-45-0
- *CHEMICAL ABSTRACTS*, 216101-03-0
- **T.W. GREENE** ; **P.G.M. WUTS**. Protective Groups in Organic Synthesis. Wiley, 1999
- *Tetrahedron*, 2006, vol. 62, 10255-10270
- *Chemical & Pharmaceutical Bulletin*, 1995, vol. 43 (10), 1821-3
- *CHEMICAL ABSTRACTS*, 54678-23-8
- *CHEMICAL ABSTRACTS*, 2622-05-1
- *CHEMICAL ABSTRACTS*, 111058-32-3
- **LAURENT S et al.** Stability of MRI paramagnetic contrast media: a proton relaxometric protocol for transmetallation assessment. *Invest Radiol*, February 2001, vol. 36 (2), 115-22
- Isolation of Rat Hepatocytes. In: Phillips I.R., Shephard E.A. (eds) Cytochrome P450 Protocols. **PAPELEU P et al.** Methods in Molecular Biology. Humana Press, 2006, vol. 320
- **BANKER MJ et al.** *J Pharm Sci*, May 2003, vol. 92 (5), 967-74
- **BURDEN N et al.** *Lab Anim*, October 2017, vol. 51 (5), 457-464
- **LI A P et al.** *Biomark Med*, 2014, vol. 8 (2), 173-83
- **DORATO et al.** *Regul Toxicol Pharmacol*, August 2005, vol. 42 (3), 265-74
- **KELLER S et al.** *J Magn Reson Imaging*, September 2020, vol. 52 (3), 668-685